# EUROPEAN PATENT SPECIFICATION

(11) **EP 1 539 739 B1**
(45) Date of publication and mention of the grant of the patent: **24.11.2010**
(21) Application number: 03791686.3
(22) Date of filing: 15.08.2003
(51) Int. Cl.: C07D 401/12, C07D 401/14, C07D 417/04, C07D 405/14, A61K 31/4523, A61P 35/00, A61P 19/02, A61P 13/12, A61P 17/06, A61P 19/10

(54) **PIPERIDINYL COMPOUNDS THAT SELECTIVELY BIND INTEGRINS**
PIPERIDINYLVERBINDUNGEN, DIE SELEKTIV AN INTEGRINE BINDEN
COMPOSES DE PIPERIDINYLE LIANT SELECTIVEMENT LES INTEGRINES

(30) Priority: 16.08.2002 US 404239 P
(43) Date of publication of application: 15.06.2005
(73) Proprietor: Janssen Pharmaceutica NV, 2340 Beerse (BE)
(72) Inventor: DE CORTE, Bart, South Hampton, PA 18966 (US); KINNEY, William, A., Newtown, PA 18940 (US); MARYANOFF, Bruce, E., Forest Grove, PA 18922 (US); GHOSH, Shyamali, Norristown, PA 19403 (US); LIU, Li, Doylestown, PA 18901 (US)
(74) Representative: Fisher, Adrian John
(86) International application number: PCT/US2003/025782
(87) International publication number: WO 2004/020435

(56) References cited:
- WO-A-94/09029
- US-A- 4 289 772
- US-B1- 6 211 191
- KATANO, KIYOAKI ET AL: "Tetrahydrothienopyridine derivatives as novel GPIIb/IIIa antagonists" BIOORGANIC & MEDICINAL CHEMISTRY LETTERS (1996), 6(21), 2601-2606 , XP004135921
- KLEIN, SCOTT I. ET AL: "Design of a New Class of Orally Active Fibrinogen Receptor Antagonists" JOURNAL OF MEDICINAL CHEMISTRY (1998), 41(14), 2492-2502 , XP001040436
- GRUMEL, VALERIE ET AL: "Synthesis of substituted oxazolo[4,5-b]pyridine derivatives" HETEROCYCLES (2001), 55(7), 1329-1345 , XP001156308
- FISHER, MATTHEW J. ET AL: "Fused bicyclic Gly-Asp.beta.-turn mimics with potent affinity for GPIIb-IIIa. Exploration of the arginine isostere" BIOORGANIC & MEDICINAL CHEMISTRY LETTERS (2000), 10(4), 385-389 , XP004189939
- LAWSON, E. C. ET AL: "1,2,4-Triazolo[4,3-a]pyridine as a novel, constrained template for fibrinogen receptor (GPIIb/IIIa) antagonists" BIOORGANIC & MEDICINAL CHEMISTRY LETTERS (2001), 11(19), 2619-2622 , XP002261904
- SU T ET AL: "FIBRINOGEN RECEPTOR (GPIIB-IIIA) ANTAGONISTS DERIVED FROM 5,6-BICYCLIC TEMPLATES. AMIDINOINDOLES, AMIDINOINDAZOLES, AND AMIDINOBENZOFURANS CONTAINING THE N-ALPHA-SULFONAMIDE CARBOXYLIC ACID FUNCTION AS POTENT PLATELET AGGREGATION INHIBITORS" JOURNAL OF MEDICINAL CHEMISTRY, AMERICAN CHEMICAL SOCIETY. WASHINGTON, US, vol. 40, no. 26, 1997, pages 4308-4318, XP000915289 ISSN: 0022-2623
- VARON D ET AL: "INHIBITION OF INTEGRIN-MEDIATED PLATELET AGGREGATION, FIBRINOGEN-BINDING, AND INTERACTIONS WITH EXTRACELLULAR MATRIX BY NONPEPTIDIC MIMETICS OF ARG-GLY-ASP" THROMBOSIS AND HAEMOSTASIS, STUTTGART, DE, vol. 70, no. 6, 1993, pages 1030-1036, XP000979521 ISSN: 0340-6245
- SAMANEN J ET AL: "VASCULAR INDICATIONS FOR INTEGRIN ALPHAV ANTAGONISTS" CURRENT PHARMACEUTICAL DESIGN, BENTHAM SCIENCE PUBLISHERS, SCHIPHOL, NL, vol. 3, 1997, pages 545-584, XP000872090 ISSN: 1381-6128 cited in the application
- VALERIE A ALABASTER ET AL: "2,4-Diamino-6,7-dimethoxyquinazolines. 2. 2-(4-Carbamoylpiperidino) Derivatives as alpha1-Adrenoceptor Antagonists and Antihypertensive Agents" JOURNAL OF MEDICINAL CHEMISTRY, AMERICAN CHEMICAL SOCIETY. WASHINGTON, US, vol. 30, 1987, pages 999-1003, XP002159406 ISSN: 0022-2623
- ISHIHARA Y ET AL: "REGIOSELECTIVE FRIEDEL-CRAFTS ACYLATION OF 2,3,4,5-TETRAHYDRO-1H-2-BE NZAZEPINE AND RELATED NITROGEN HETEROCYCLES" JOURNAL OF THE CHEMICAL SOCIETY, PERKIN TRANSACTIONS 1, CHEMICAL SOCIETY. LETCHWORTH, GB, vol. 20, 1994, pages 2993-2999, XP001084788 ISSN: 0300-922X

## Description

### FIELD OF THE INVENTION

This invention relates to novel compounds and methods for use in treating an integrin mediated disorder. More particularly, this invention relates to piperidinyl compounds that selective bind integrin receptors and methods for treating an integrin mediated disorder.

### BACKGROUND OF THE INVENTION

Integrins are a family of transmembrane receptors, each of which is composed of a pair of heterodimeric, noncovalently associated glycoproteins, designated as a and P chains. The a subunit contains heavy and light chains as part of its extracellular domain, with 3-4 divalent-cation binding sites; the light chain also contains transmembrane and intracellular domains. The β-subunit contains a large extracellular domain, as well as transmembrane and intracellular domains. Integrins are cell surface receptors, which bind to extracellular matrix adhesive proteins such as fibrinogen, fibronectin, vitronectin and osteopontin. These transmembrane glycoproteins are classified by the β subunits. The β3 class of integrin family has received the most attention in recent drug discovery efforts (W.J. Hoekstra, Current Medicinal Chemistry, 1998, 5, 195), however, the β5 class has also become a focus of attention. Some of the disease states that have been associated with a strong β3 and β5 integrin component in their etiologies are thrombosis (integrin α2bβ3 also called GPIIb/IIIa); unstable angina (GPIIb/IIIa); restenosis (GPIIb/IIIa and integrin αvβ3); arthritis, vascular disorders or osteoporosis (αvβ3); tumor angiogenesis, multiple sclerosis, neurological disorders, asthma, vascular injury or diabetic retinopathy (αvβ3 or αvβ5) and tumor metastasis (αvβ3). See S.A. Mousa, et al., Emerging Therapeutic Targets, 2000, 4(2) 148-149; and W.H. Miller, et al., Drug Discovery Today, 2000, 5(9), 397-40. Antibodies and/or low-molecular weight compound antagonists of αvβ3 have shown efficacy against these respective disease states in animal models (J. Samanen, Current Pharmaceutical Design, 1997, 3 545-584) and thereby offer promise as therapeutic agents. Several patents have described compounds that could interact with these integrins. For example, United States Patents 5,919,792 B1, 6,211,191 B1, and WO 01/96334 and WO 01/23376 describe αvβ3 and αvβ5 integrin receptor antagonists.

The present invention provides a new class of piperidinyl compounds, which selective bind to β3, β5 or dual integrin receptors (e.g. αvβ3 and αvβ5) for the treatment of a wide variety of integrin mediated disease states.

### SUMMARY OF THE INVENTION

The present invention provides compounds, as set out in the appended claims. The present invention also provides compositions and medical uses as set out in the appended claims.

The present disclosure is more generally directed to piperidinyl compounds of Formula (I): 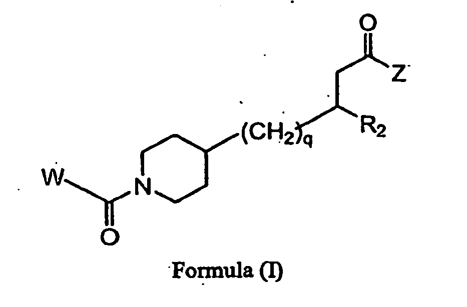 and Formula (II) 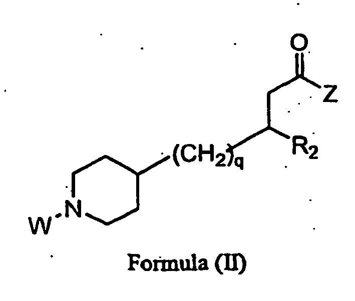 wherein
- W: is selected from the group consisting of-C₀₋₆alkyl (R₁), -C₁₋₆alkyl(R₁ₐ), -C₀₋₆alkyl-aryl(R₁,R₈), -C₀₋₆alkyl-heterocyclyl(R₁,R₈), -C₀₋₆alkoxy(R₁), -C₀₋₆alkoxy-aryl(R₁,R₈), and -C₀₋₆alkoxy-heterocyclyl(R₁,R₈),
- R₁ is: selected from the group consisting of hydrogen, -N(R₄)₂, -N(R₄)(R₅), -N(R₄)(R₆), -heterocyclyl(R₈) and -heteroaryl(R₈);
- R₁ₐ: is selected from the group consisting of -C(R₄)(=N-R₄), -C(=N-R₄)-N(R₄)₂, -C(=N-R₄)-N(R₄)(R₆), -C(=N-R₄)-N(R₄)-C(=O)-R₄, -C(=N-R₄)-N(R₄)-C(=O)-N(R₄)₂, -C(=N-R₄)-N(R₄)-CO₂-R₄, -C(=N-R₄)-N(R₄)-SO₂-C₁₋₈alkyl(R₇) and -C(=N-R₄)-N(R₄)-SO₂-N(R₄)₂;
- R₄: is selected from the group consisting of hydrogen and -C₁₋₈alkyl(R₇);
- R₅: is selected from the group consisting of -C(=O)-R₄, -C(=O)-N(R₄)₂, -C(=O)-cycloalkyl(R₈), -C(=O)-heterocyclyl(R₈), -C(=O)-aryl(R₈), -C(=O)-heteroaryl(R₈), -C(=O)-N(R₄)-cycloalkyl(R₈), -C(=O)-N(R₄)-aryl(R₈), -CO₂-R₄, -CO₂-cycloalkyl(R₈), -CO₂-aryl(R₈), -C(R₄)(=N-R₄), -C(=N-R₄)-N(R₄)₂, -C(=N-R₄)-N(R₄)(R₆), -C(=N-R₄)-N(R₄)-C(=O)-R₄, -C(=N-R₄)-N(R₄)-C(=O)-N(R₄)₂, -C(=N-R₄)-N(R₄)-CO₂-R₄, -C(=N-R₄)-N(R₄)-SO₂-C₁₋₈alkyl(R₇), -C(=N-R₄)-N(R₄)-SO₂-N(R₄)₂, -N(R₄)-C(R₄)(=N-R₄), -N(R₄)-C(=N-R₄)-N(R₄)₂, -N(R₄)-C(=N-R₄)-N(R₄)(R₆), -N(R₄)-C(=N-R₄)-N(R₄)-C(=O)-R₄, -N(R₄)-C(=N-R₄)-N(R₄)-C(=O)-N(R₄)₂, -N(R₄)-C(=N-R₄)-N(R₄)-CO₂-R₄, -N(R₄)-C(=N-R₄)-N(R₄)-SO₂-C₁₋₈alkyl(R₇), -N(R₄)-C(=N-R₄)-N(R₄)-SO₂-N(R₄)₂, -SO₂-C₁₋₈alkyl(R₇), -SO₂-N(R₄)₂, -SO₂-cycloalkyl(R₈) and -SO₂-aryl(R₈);
- R₆: is selected from the group consisting of -cycloalkyl(R₈), -heterocyclyl(R₈), -aryl(R₈) and -heteroaryl(R₈);
- R₇: is one to two substituents independently selected from the group consisting of hydrogen, -C₁₋₈alkoxy(R₉), -NH₂, -NH-C₁₋₈alkyl(R₉), -N(C₁₋₈alkyl(R₉))₂, -C(=O)H, -C(=O)-C₁₋₈alkyl(R₉), -C(=O)-NH₂, -C(=O)-NH-C₁₋₈alkyl(R₉), -C(=O)-N(C₁₋₈alkyl(R₉))₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₈alkyl(R₉), -CO₂-aryl(R₁₀), -C(=NH)-NH₂, -SH, -S-C₁₋₈alkyl(R₉), -S-C₁₋₈alkyl-S-C₁₋₈alkyl(R₉), -S-C₁₋₈alkyl-C₁₋₈alkoxy(R₉), -S-C₁₋₈alkyl-NH-C₁₋₈alkyl(R₉), -SO₂-C₁₋₈alkyl(R₉), -SO₂-NH₂, -SO₂-NH-C₁₋₈alkyl(R₉), -SO₂-N(C₁₋₈alkyl(R₉))₂, -SO₂-aryl(R₁₀), cyano, (halo)₁₋₃, hydroxy, nitro, oxo, -cycloalkyl(R₁₀), -heterocyclyl(R₁₀), -aryl(R₁₀) and -heteroaryl(R₁₀);
- R₈: is one to four substituents independently selected from the group consisting of hydrogen, -C₁₋₈alkyl(R₉), -C(=O)H, -C(=O)-C₁₋₈alkyl(R₉), -C(=O)-NH₂, -C(=O)-NH-C₁₋₈alkyl(R₉), -C(=O)-N(C₁₋₈alkyl(R₉))₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₈alkyl(R₉), -CO₂-aryl(R₁₀), -C(=NH)-NH₂, -SO₂-C₁₋₈alkyl(R₉), -SO₂-NH₂, -SO₂-NH-C₁₋₈alkyl(R₉), -SO₂-N(C₁₋₈alkyl(R₉))₂, -SO₂-aryl(R₁₀), -cycloalkyl(R₁₀) and -aryl(R₁₀) when attached to a nitrogen atom; and, wherein R₈ is one to four substituents independently selected from the group consisting of hydrogen, -C₁₋₈alkyl(R₉), -C₁₋₈alkoxy(R₉), -O-cycloalkyl(R₁₀), -O-aryl(R₁₀), -C(=O)H, -C(=O)-C₁₋₈alkyl(R₉), -C(=O)-NH₂, -C(=O)-NH-C₁₋₈alkyl(R₉), -C(=O)-N(C₁₋₈alkyl(R₉))₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₈alkyl(R₉), -CO₂-aryl(R₁₀), -C(=NH)-NH₂, -SO₂-C₁₋₈alkyl(R₉), -SO₂-NH₂, -SO₂-NH-C₁₋₈alkyl(R₉), -SO₂-N(C₁₋₈alkyl(R₉))₂, -SO₂-aryl(R₁₀), -SH, -S-C₁₋₈alkyl(R₉), -S-C₁₋₈alkyl-S-C₁₋₈alkyl(R₉), -S-C₁₋₈alkyl-C₁₋₈alkoxy(R₉), -S-C₁₋₈alkyl-NH-C₁₋₈alkyl(R₉), -NH₂, -NH-C₁₋₈alkyl(R₉), -N(C₁₋₈alkyl(R₉))₂, cyano, halo, hydroxy, nitro, oxo, -cycloalkyl(R₁₀), -heterocyclyl(R₁₀); -aryl(R₁₀) and -heteroaryl(R₁₀) when attached to a carbon atom;
- R₉: is selected from the group consisting of hydrogen, -C₁₋₈alkoxy, -NH₂, -NH-C₁₋₈alkyl, -N(C₁₋₈alkyl)₂, -C(=O)H, -C(=O)-NH₂, -C(=O)-NH-C₁₋₈alkyl, -C(=O)-N(C₁₋₈alkyl)₂, -CO₂H, -CO₂-C₁₋₈alkyl, -SO₂-C₁₋₈alkyl, -SO₂-NH₂, -SO₂-NH-C₁₋₈alkyl, -SO₂-N(C₁₋₈alkyl)₂, cyano, (halo)₁₋₃, hydroxy, nitro and oxo;
- R₁₀: is one to four substituents independently selected from from the group consisting of hydrogen, -C₁₋₈alkyl, -C(=O)H, -C(=O)-C₁₋₈alkyl, -C(=O)-NH₂, -C(=O)-NH-C₁₋₈alkyl, -C(=O)-N(C₁₋₈alkyl)₂, -CO₂H, -CO₂- C₁₋₄alkyl, -SO₂-C₁₋₈alkyl, -SO₂-NH₂, -SO₂-NH-C₁₋₈alkyl and -SO₂-N(C₁₋₈alkyl)₂ when attached to a nitrogen atom; and, wherein R₁₀ is one to four substituents independently selected from the group consisting of hydrogen, -C₁₋₈alkyl, -C₁₋₈alkoxy, -C(=O)H, -C(=O)-C₁₋₈alkyl, -C(=O)-NH₂, -C(=O)-NH-C₁₋₈alkyl, -C(=O)-N(C₁₋₈alkyl)₂, -CO₂H, -CO₂- C₁₋₄alkyl, -SO₂-C₁₋₈alkyl, -SO₂-NH₂, -SO₂-NH-C₁₋₈alkyl, -SO₂-N(C₁₋₈alkyl)₂,-NH₂, -NH-C₁₋₈alkyl, -N(C₁₋₈alkyl)₂, cyano, halo, hydroxy, nitro and oxo when attached to a carbon atom;
- R₂: is selected from the group consisting of hydrogen, -C₁₋₈alkyl(R₇), -C₂₋₈alkenyl(R₇), -C₂₋₈alkynyl(R₇), -cycloalkyl(R₈), -heterocyclyl(R₈), -aryl(R₈) and -heteroaryl(R₈);
- q: is 0, 1, 2 or 3;
- Z: is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈alkoxy, -O- C₁₋₈alkylcarbonylC₁₋₈alkyl, -O-C₁₋₈alkyl-CO₂H, -O-C₁₋₈alkyl-C(O)O-C₁₋₈alkyl, -O- C₁₋₈alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(C₁₋₈alkyl)₂, -O-C₁₋₈alkylamide, -O-C₁₋₈alkyl-C(O)-NH-C₁₋₈alkyl, -O-C₁₋₈alkyl-C(O)-N(C₁₋₈alkyl)₂ and -NHC(O)C₁₋₈alkyl.
and pharmaceutically acceptable salts, racemic mixtures and enantiomers thereof.

The present disclosure is also directed to methods for producing the instant piperidinyl compounds and pharmaceutical compositions and medicaments thereof.

Since this disclosure is broader than the invention as claimed, references herein to this disclosure encompass references to this invention, to the extent that those references relate to subject-matter within the claims.

The present invention is further directed to a compound, for the medical uses set out in claims 25 to 28.

### DETAILED DESCRIPTION OF THE INVENTION

Another aspect of the present disclosure includes compounds of Formula (I) and Formula (II) wherein W is preferably is selected from the group consisting of -C₀₋₄alkyl(R₁), -C₁₋₄alkyl(R₁ₐ), -C₀₋₄alkyl-aryl(R₁,R₈), -C₀₋₄alkyl-heterocyclyl(R₁,R₈), -C₀₋₄alkoxy(R₁),-C₀₋₄alkoxy-aryl(R₁,R₈), and -C₀₋₄alkoxy-heterocyclyl(R₁,R₈).

Aspects of the present disclosure include compounds of Formula (I) and Formula (II) wherein W is preferably -C₀₋₄alkyl(R₁) or -C₀₋₄alkyl-aryl(R₁,R₈).

Another aspect of the present disclosure includes compounds of Formula (I) and Formula (II) wherein W is preferably -C₀₋₄alkyl(R₁) or -C₀₋₄alkyl-phenyl(R₁,R₈).

Aspects of the present disclosure include compounds of Formula (I) and Formula (II) wherein R₁ is-N(R₄)(R₆),-heterocyclyl(R₈) or -heteroaryl(R₈).

Another aspect of the present disclosure includes compounds of Formula (I) and Formula (II) wherein R₁ is -N(R₄)(R₆), -dihydro-1H-pyrrolo[2,3-b]pyridinyl(R₈), -tetrahydropyrimidinyl(R₈), -tetrahydro-1,8-naphthyridinyl(R₈), -tetrahydro-1H-azepino[2,3-b]pyridinyl(R₈)or-pyridinyl(R₈).

Another aspect of the present disclosure includes compounds of Formula (I) and Formula (II) wherein R₁ is N(R₄)(R₆), -tetrahydropyrimidinyl(R₈) or -tetrahydro-1,8-naphthyridinyl(R₈).

Aspects of the present disclosure include compounds of Formula (I) and Formula (II) wherein R₁ₐ is -C(R₄)(=N-R₄), -C(=N-R₄)-N(R₄)₂, -C(=N-R₄)-N(R₄)(R₆), -C(=N-R₄)-N(R₄)-C(=O)R₄, -C(=N-R₄)-N(R₄)-C(=O)N(R₄)₂, -C(=N-R₄)-N(R₄)-CO₂-R₄,-C(=N-R₄)-N(R₄)-SO₂-C₁₋₄alkyl(R₇) or -C(=N-R₄)-N(R₄)-SO₂-N(R₄)₂.

Aspects of the present disclosure include compounds of Formula (I) and Formula (II) wherein R₄ is hydrogen or -C₁₋₄alkyl(R₇).

Another aspect of the present disclosure includes compounds of Formula (I) and Formula (II) wherein R₄ is hydrogen.

Aspects of the present disclosure include compounds of Formula (I) and Formula (II) wherein R₅ is -C(=O)-R₄, -C(=O)-N(R₄)₂, -C(=O)-cycloalkyl(R₈), -C(=O)-heterocyclyl(R₈), -C(=O)-aryl(R₈), -C(=O)-heteroaryl(R₈), -C(=O)-N(R₄)-cycloalkyl(R₈), -C(=O)-N(R₄)-aryl(R₈), -CO₂-R₄, -CO₂-cycloalkyl(R₈), -CO₂-aryl(R₈), -C(R₄)(=N-R₄), -C(=N-R₄)-N(R₄)₂, -C(=N-R₄)-N(R₄)(R₆), -C(=N-R₄)-N(R₄)-C(=O)-R₄, -C(=N-R₄)-N(R₄)-C(=O)-N(R₄)₂, -C(=N-R₄)-N(R₄)CO₂-R₄, -C(=N-R₄)-N(R₄)-SO₂-C₁₋₄alkyl(R₇), -C(=N-R₄)-N(R₄)-SO₂-N(R₄)₂, -N(R₄)-C(R₄)(=N-R₄), -N(R₄)-C(=N-R₄)-N(R₄)₂, . -N(R₄)-C(=N-R₄)-N(R₄)(R₆), -N(R₄)-C(=N-R₄)-N(R₄)-C(=O)-R₄, -N(R₄)-C(=N-R₄)-N(R₄)-C(=O)-N(R₄)₂,-N(R₄)-C(=N-R₄)-N(R₄)-CO₂-R₄, -N(R₄)-C(=N-R₄)-N(R₄)-SO₂-C₁₋₄alkyl(R₇),-N(R₄)-C(=N-R₄)-N(R₄)-SO₂-N(R₄)₂, -SO₂-C₁₋₄alkyl(R₇), -SO₂-N(R₄)₂, -SO₂-cycloalkyl(R₈) or -SO₂-aryl(R₈).

Another aspect of the present disclosure includes compounds of Formula (I) and Formula (II) wherein R₅ is -C(-O)-R₄, -C(=O)-N(R₄)₂, -CO₂-R₄, -C(R₄)(=N-R₄), -C(=N-R₄)-N(R₄)₂,-C(=N-R₄)-N(R₄)(R₆),-N(R₄)-C(R₄)(=N-R₄), -N(R₄)-C(=N-R₄)-N(R₄)₂, -N(R₄)-C(=N-R₄)-N(R₄)(R₆), -SO₂-C₁₋₄alkyl(R₇) or -SO₂-N(R₄)₂.

Aspects of the present disclosure include compounds of Formula (I) and Formula (II) wherein R₆ is -heterocyclyl(R₈) or -heteroaryl(R₈).

Another aspect of the present disclosure includes compounds of Formula (I) and Formula (II) wherein R₆ is -dihydroimidazolyl(R₈), -tetrahydropyridinyl(R₈), -tetrahydropyrimidinyl(R₈) or -pyridinyl(R₈).

Aspects of the present disclosure include compounds of Formula (I) and Formula (II) wherein R₇ is one to two substituents independently selected from hydrogen, -C₁₋₄alkoxy(R₉), -NH₂, -NH-C₁₋₄alkyl(R₉), -N(C₁₋₄alkyl(R₉))₂, -C(=O)H, -C(=O)-C₁₋₄alkyl(R₉), -C(=O)-NH₂, -C(=O)-NH-C₁₋₄alkyl(R₉), -C(=O)-N(C₁₋₄alkyl(R₉))₂, -C(=O)-NH-aryl(R₁₀), -C(=O)-cycloalkyl (R₁₀), -C(=O)-heterocyclyl(R₁₀), -C(=O)-aryl(R₁₀), -C(=O)-heteroaryl(R₁₀), -CO₂H, -CO₂-C₁₋₄alkyl(R₉), -CO₂-aryl(R₁₀), -C(=NH)-NH₂, -SH, -S-C₁₋₄alkyl(R₉), -S-C₁₋₄alkyl-S-C₁₋₄alkyl(R₉), -S-C₁₋₄alkyl-C₁₋₄alkoxy(R₉), -S-C₁₋₄alkyl-NH-C₁₋₄alkyl(R₉), -SO₂-C₁₋₄alkyl(R₉), -SO₂-NH₂, -SO₂-NH-C₁₋₄alkyl(R₉), -SO₂-N(C₁₋₄alkyl(R₉))₂, -SO₂-aryl(R₁₀), cyano, (halo)₁₋₃, hydroxy, nitro, oxo, -cycloalkyl(R₁₀), -heterocyclyl(R₁₀), -aryl(R₁₀) or-heteroaryl(R₁₀).

Another aspect of the present disclosure includes compounds of Formula (I) and Formula (II) wherein R₇ is one to two substituents independently selected from hydrogen, -C₁₋₄alkoxy(R₉), -NH₂, -NH-C₁₋₄alkyl(R₉), -N(C₁₋₄alkyl(R₉))₂, (halo)₁₋₃, hydroxy or oxo.

A further aspect of the present disclosure includes compounds of Formula (I) and Formula (II) wherein R₇ is hydrogen.

Aspects of the present disclosure include compounds of Formula (I) and Formula (II) wherein R₈ is one to four substituents independently selected from hydrogen, -C₁₋₄alkyl(R₉), -C(=O)H, -C(=O)-C₁₋₄alkyl(R₉), -C(=O)-NH₂, -C(=O)-NH-C₁₋₄alkyl(R₉), -C(=O)-N(C₁₋₄alkyl(R₉))₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₄alkyl(R₉), -CO₂-aryl(R₁₀), -C(=NH)-NH₂, -SO₂-C₁₋₄alkyl(R₉), -SO₂₋NH₂, -SO₂-NH-C₁₋₄alkyl(R₉), -SO₂-N(C₁₋₄alkyl(R₉))₂, -SO₂-aryl(R₁₀), -cycloalkyl(R₁₀) or -aryl(R₁₀) when attached to a nitrogen atom; and, wherein R₈ is one to four substituents independently selected from hydrogen, -C₁₋₄alkyl(R₉), -C₁₋₄alkoxy(R₉), -O-cycloalkyl(R₁₀), -O-aryl(R₁₀), -C(=O)H, -C(=O)-C₁₋₄alkyl(R₉), -C(=O)-NH₂, -C(=O)-NH-C₁₋₄alkyl(R₉), -C(=O)-N(C₁₋₄alkyl-R₁₁)₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₄alkyl(R₉), -CO₂-aryl(R₁₀), -C(=NH)-NH₂, -SO₂-C₁₋₄alkyl(R₉), -SO₂-NH₂, -SO₂-NH-C₁₋₄alkyl(R₉), -SO₂-N(C₁₋₄alkyl(R₉))₂, -SO₂-aryl(R₁₀), -SH, -S-C₁₋₄alkyl(R₉), -S-C₁₋₄alkyl-S-C₁₋₄alkyl(R₉), -S-C₁₋₄alkyl-C₁₋₄alkoxy(R₉), -S-C₁₋₄alkyl-NH-C₁₋₄alkyl(R₉), -NH₂, -NH-C₁₋₄alkyl(R₉), -N(C₁₋₄alkyl(R₉))₂, cyano, halo, hydroxy, nitro, oxo, -cycloalkyl(R₁₀), -heterocycl(R₁₀), -aryl(R₁₀) or -heteroaryl(R₁₀) when attached to a carbon atom.

Another aspect of the present disclosure includes compounds of Formula (I) and Formula (II) wherein R₈ is one to four substituents independently selected from hydrogen, -C₁₋₄alkyl(R₉), -C(=O)H, -C(=O)-NH₂, -C(=O)-NH-C₁₋₄alkyl(R₉), -C(=O)-N(C₁₋₄alkyl(R₉))₂, -CO₂H, -CO₂C₁₋₄alkyl(R₉) or -SO₂-NH₂ when attached to a nitrogen atom; and, wherein R₈ is one to four substituents independently selected from hydrogen, -C₁₋₄alkyl(R₉), -C₁₋₄alkoxy(R₉), -O-aryl(R₁₀), -C(=O)H, -C(=O)-NH₂, -C(=O)-NH-C₁₋₄alkyl(R₉), -C(=O)-N(C₁₋₄alkyl(R₉))₂, -CO₂H, -CO₂-C₁₋₄alkyl(R₉), -SO₂-NH₂, -NH₂, -NH-C₁₋₄alkyl(R₉), -N(C₁₋₄alkyl(R₉))₂, cyano, halo, hydroxy, nitro or oxo when attached to a carbon atom.

Another aspect of the present disclosure includes compounds of Formula (I) and Formula (II) wherein R₈ is one to four substituents independently selected from hydrogen or -C₁₋₄alkyl(R₉) when attached to a nitrogen atom; and, wherein R₈ is one to four substituents independently selected from hydrogen, -C₁₋₄alkyl(R₉), -C₁₋₄alkoxy(R₉), -O-aryl(R₁₀), -NH₂, -NH-C₁₋₄alkyl(R₉), -N(C₁₋₄alkyl(R₉))₂, halo, hydroxy or oxo when attached to a carbon atom.

A further aspect of the present disclosure includes compounds of Formula (I) and Formula (II) wherein R₈ is one to four substituents independently selected from hydrogen or -C₁₋₄alkyl(R₉) when attached to a nitrogen atom; and, wherein R₈ is one to four substituents independently selected from hydrogen, -C₁₋₄alkyl(R₉), -C₁₋₄alkoxy(R₉) -O-aryl(R₁₀) or hydroxy when attached to a carbon atom.

Aspects of the present disclosure include compounds of Formula (I) and Formula (II) wherein R₉ is hydrogen, -C₁₋₄alkoxy, -NH₂, -NH-C₁₋₄alkyl, -N(C₁₋₄alkyl)₂, -C(=O)H; -C(=O)-NH₂, -C(=O)-NH-C₁₋₄alkyl, -C(=O)-N(C₁₋₄alkyl)₂, -CO₂H, -CO₂-C₁₋₄alkyl, -SO₂-C₁₋₄alkyl, -SO₂-NH₂, -SO₂-NH-C₁₋₄alkyl, -SO₂-N(C₁₋₄alkyl)₂, cyano, (halo)₁₋₃, hydroxy, nitro or oxo.

Another aspect of the present disclosure includes compounds of Formula (I) and Formula (II) wherein R₉ is hydrogen, -C₁₋₄alkoxy, -NN₂, -NH-C₁₋₄alkyl, -N(C₁₋₄alkyl)₂, -C(=O)H, -CO₂H, -C(=O)-C₁₋₄alcoxy, (Halo)₁₋₃, hydroxy or oxo.

A further aspect of the present disclosure includes compounds of Formula (I) wherein R₉ is hydrogen, -C₁₋₄alkoxy, NH₂, -NH-C₁₋₄alkyl, -N(C₁₋₄alkyl)₂, (halo)₁₋₃ or hydroxy.

Aspects of the present disclosure include compounds of Formula (I) and Formula (II) wherein R₁₀ is one to four substituents independently selected from hydrogen, -C₁₋₄alkyl, -C(=O)H, -C(=O)-C₁₋₄alkyl, -C(=O)-NH₂, -C(=O)-NH-C₁₋₄-alkyl, -C(=O)-N(C₁₋₄alkyl)₂, -CO₂H, -CO₂-C₁₋₄-alkyl, -SO₂-C₁₋₄alkyl, -SO₂-NH₂, -SO₂-NH-C₁₋₄alkyl or -SO₂-N(C₁₋₄alkyl)₂ when attached to a nitrogen atom; and, wherein R₁₀ is one to four substituents independently selected from hydrogen, -C₁₋₄alkyl, -C₁₋₄alkoxy, -C(=O)H, -C(=O)-C₁₋₄alkyl, -C(=O)-NH₂, -C(=O)-NH-C₁₋₄alkyl, -C(=O)-N(C₁₋₄alkyl)₂, -CO₂H, -CO₂-C₁₋₄alkyl, -SO₂-C₁₋₄alkyl, -SO₂NH₂, -SO₂-NH-C₁₋₄alkyl, -SO₂-N(C₁₋₄alkyl)₂, -NH₂, -NH-C₁₋₄alkyl, -N(C₁₋₄alkyl)₂, cyano, halo, hydroxy, nitro or oxo when attached to a carbon atom.

Another aspect of the present disclosure includes compounds of Formula (I) and Formula (II) wherein (R₁₀)₁₋₄ is hydrogen, -C₁₋₄alkyl, -C₁₋₄alkoxy, -C(=O)H, -C(=O)-C₁₋₄alkyl, -CO₂H, -CO₂-C₁₋₄alkyl, -NN₂, -NH-C₁₋₄alkyl, -N(C₁₋₄alkyl)₂, halo, hydroxy, nitro or oxo when attached to a carbon atom.

A further aspect of the present disclosure includes compounds of Formula (I) and Formula (II) wherein R₁₀ is hydrogen.

Aspects of the present disclosure include compounds of Formula (I) and Formula (II) wherein R₂ is hydrogen, -C₁₋₄alkyl(R₇), -C₂₋₄alkenyl(R₇), -C₂₋₄alkynyl(R₇), -cycloalkyl(R₈), -heterocyclyl(R₈), -aryl(R₈) or -heteroaryl(R₈).

Another aspect of the present disclosure includes compounds of Formula (I) and Formula (II) wherein R₂ is hydrogen, -cycloalkyl(R₈), -heterocyclyl(R₈), -aryl(R₈) or -heteroaryl(R₈).

Another aspect of the present disclosure includes compounds of Formula (I) and Formula (II) wherein R₂ is hydrogen, -cycloalkyl(R₈), -heterocyclyl(R₈), -phenyl(R₈), -naphthatenyl(R₈) or -heteroaryl(R₈).

Another aspect of the present disclosure includes compounds of Formula (I) and Formula (II) wherein R₂ is hydrogen, -tetrahydropyrimidinyl(R₈), -1,3-benzodioxolyl(R₈), -dihydrobenzofuranyl(R₈), -tetrahydroquinolinyl(R₈), -phenyl(R₈), -naphthalenyl(R₈), -pyridinyl(R₈), -pyrimidinyl(R₈) or -quinolinyl(R₈).

Aspects of the present disclosure include a composition comprising a compound of Formula (I) and Formula (II) wherein q is 1, 2 or 3.

Aspects of the present disclosure include a composition comprising a compound of Formula (I) and Formula (II) wherein Z is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₄alkoxy, -O-C₁₋₈alkylcarbonylC₁₋₄alkyl, -O-C₁₋₈alkyl-CO₂H, -O-C₁₋₈alkyl-C(O)O-C₁₋₆alkyl, -O-C₁₋₈alkyl-O-C(O)C₁₋₈alkyl, -O-C₁₋₈alkyl-NH₂, -O-C₁₋₈alkyl-NH-C₁₋₈alkyl, -O-C₁₋₈alkyl-N(C₁₋₈alkyl)₂, -O-C₁₋₈alkylamide -O-C₁₋₈alkyl-C(O)-NH-C₁₋₈alkyl, -O-C₁₋₈alkyl-C(O)-N(C₁₋₈alkyl)₂ and -NHC(O)C₁₋₈alkyl. NHC(O)C₁₋₈alkyl..

Aspects of the present disclosure include a composition comprising compound of Formula (I) 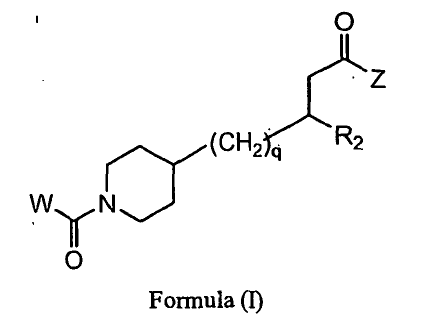 wherein the compound is selected from the group consisting of:

| | | | | | **Stereo** | |
|---|---|---|---|---|---|---|
| **Cpd** | **W** | **R₁** | **R₂** | **q** | **chem** | **Z** |
| | | -NH-1,4,5,6-tetrahydro-pyrimidin- | | | | |
| **1** | -CH₂-Ph(3-R₁) | 2-yl -NH-1,4,5,6-tetrahydro-pyrimidin- | H | 0 | | OH |
| **2** | -(CH₂)₂-Ph(3-R₁) | 2-yl -NH-1,4,5,6-tetrahydro-5-OH- | H | 0 | | OH |
| **3** | -CH₂-Ph(3-R₁) | pyrimidin-2-yl 5,6,7,8-tetrahydro- | quinolin-3-yl | 0 | | OH |
| **4** | -(CH₂)₃-R₁ | [1,8]naphthyridin-2-yl 5,6,7,8-tetrahydro- | quinolin-3-yl | 0 | | OH |
| **5** | -(CH₂)₃-R₁ | [1,8]naphthyridin-2-yl 5,6,7,8-tetrahydro- | 1,2,3,4-tetrahydro-quinolin-3-yl | 0 | | OH |
| **5-1** | -(CH₂)₃-R₁ | [1,8]naphthyridin-2-yl 5,6,7,8-tetrahydro- | 1,2,3,4-tetrahydro-quinolin-3-yl | 0 | Isomer 1 | OH |
| **5-2** | -(CH₂)₃-R₁ | [1,8]naphthyridin-2-yl 5,6,7,8-tetrahydro- | 1,2,3,4-tetrahydro-quinolin-3-yl | 0 | Isomer 2 | OH |
| **5-3** | -(CH₂)₃-R₁ | [1,8]naphthyridin-2-yl | 1,2,3,4-tetrahydro-quinolin-3-yl | 0 | Isomer 3 | OH |
| | | 5,6,7,8-tetrahydro- | | | | |
| **5-4** | -(CH₂)₃-R₁ | [1,8]naphthyridin-2-yl -NH-1,4,5,6-tetrahydro-pyrimidin- | 1,2,3,4-tetrahydro-quinolin-3-yl | 0 | Isomer 4 | OH |
| **6** | Ph(3-R₁) | 2-yl -NH-1,4,5,6-tetrahydro-5-OH- | pyridin-3-yl | 2 | | OH |
| **7** | Ph(3-R₁) | pyrimidin-2-yl 5,6,7,8-tetrahydro-[1,8]naphthyridin-2- | pyridin-3-yl | 2 | | OH |
| **8** | -(CH₂)₂-R₁ | yl | pyridin-3-yl | 2 | | OH |
| **9** | -(CH₂)₃-R₁ | -NH-pyridin-2-yl -NH-1,4,5,6- | pyridin-3-yl | 2 | | OH |
| **10** | Ph(3-R₁) | tetrahydro-5-OH-pyrimidin-2-yl | (6-OCH₃)-pyridin-3-yl | 2 | | OH |
| **11** | -(CH₂)₂-R₁ | 5,6,7,8-tetrahydro-[1,8]naphthyridin-2-yl | 1,3-benzodioxol-5-yl | 1 | | OH |
| | | -NH-1,4,5,6-tetrahydro-pyrimidin- | | | | |
| **12** | Ph(3-R₁) | 2-yl | quinolin-3-yl | 2 | | OH |
| | | 5,6,7,8-tetrahydro-[1,8]naphthyridin-2 | | | | |
| **13** | -(CH₂)₂-R₁ | -yl | phenyl | 1 | | OH |
| | | 5,6,7,8-tetrahydro-[1,8]naphthyridin-2- | 1,3-benzodioxol-5- | | | |
| **14** | -(CH₂)₂-R₁ | yl 5,6,7,8-tetrahydro-[1,8]naphthyridin-2- | yl 1,3-benzodioxol-5- | 0 | | OH |
| **15** | -(CH2)3-R₁ | yl 5,6,7,8-tetrahydro-[1,8]naphthyridin-2- | yl 1,3-benzodioxol-5- | 0 | | OH |
| **16** | -CH₂-R₁ | yl | yl | 0 | | OH |
| **17** | -(CH₂)₃-R₁ | 5,6,7,8-tetrahydro-[1,8]naphthyridin-2-yl | (6-OCH₃)-pyridin-3-yl | 0 | | OH |
| **18** | -(CH₂)₂-R₁ | 5,6,7,8-tetrahydro-[1,8]naphthyridin-2-yl | 1,4,5,6-tetrahydro-2-Me-pyrimidin-5-yl | | | OH |
| **19** | -(CH₂)₂-R₁ | 5,6,7,8-tetrahydro-[1,8]naphthyridin-2-yl | 1,2,3,4-tetrahydro-quinolin-3-yl | 1 | | OH |
| **19-1** | -(CH₂)₂-R₁ | 5,6,7,8-tetrahydro-[1,8]naphthyridin-2-yl | 1,2,3,4-tetrahydro-quinolin-3-yl | 1 | Isomer 1 | OH |
| **19-2** | -(CH₂)₂-R₁ | 5,6,7,8-tetrahydro-[1,8]naphthyridin-2-yl | 1,2,3,4-tetrahydro-quinolin-3-yl | 1 | Isomer 2 | OH |
| **19-3** | -(CH₂)₂-R₁ | 5,6,7,8-tetrahydro-[1,8]naphthyridin-2-yl | 1,2,3,4-tetrahydro-quinolin-3-yl | 1 | Isomer 3 | OH |
| **19-4** | -(CH₂)₂-R₁ | 5,6,7,8-tetrahydro-[1,8]naphthyridin-2-yl | 1,3,3,4-tetrahydro-quinolin-3-yl | 1 | Isomer 4 | OH |
| **20** | -(CH₂)₂-R₁ | 5,6,7,8-tetrahydro-[1,8]naphthyridin-2-yl | 1,3-benzodioxol-5-yl | 2 | | OH |
| **21** | -(CH₂)₂-R₁ | 5,6,7,8-tetrahydro-[1,8]naphthyridin-2-yl | (6-OCH₃)-pyridin-3-yl | 2 | | OH |
| **21a** | -(CH₂)₂-R₁ | 5,6,7,8-tetrahydro-[1,8]naphthyridin-2-yl | (6-OCH₃)-pyridin-3-yl | 2 | Isomer a | OH |
| **21b** | -(CH₂)₂-R₁ | 5,6,7,8-tetrahydro-[1,8]naphthyridin-2-yl | (6-OCH₃)-pyridin-3-yl | 2 | Isomer b | OH |
| **22** | -(CH₂)₃-R₁ | -NH-pyridin-2-yl | quinolin-3-yl | 2 | | OH |
| **23** | -(CH₂)₃-R₁ | -NH-Pyridin-2-yl | 1,3-benzodioxol-5-yl | 2 | | OH |
| **24** | -(CH₂)₃-R₁ | -NH-pyridin-3-yl | 1,3-benzodioxol-5-yl | 0 | | OH |
| **25** | -(CH₂)₃-R₁ | -NH-pyridin-2-yl | (6-OCH₃)-pyridin-3-yl | 2 | | OH |
| **26** | -(CH₂)₃-R₁ | 5,6,7,8-tetrahydro-[1,8]naphthyridin-2-yl | 1,3-benzodioxol-5-yl | 1 | | OH |
| **27** | Ph(3-R₁) | -NH-1,4,5,6-tetrahydro-5-OH-pyrimidin-2-yl | 1,3-benzodioxol-5-yl | 1 | | OH |
| **28** | -(CH₂)₂-R₁ | 5,6,7,8-tetrahydro-[1,8]naphthyridin-2-yl | (6-OCH₃)-pyridin-3-yl | 1 | | OH |
| **28a** | -(CH₂)₂-R₁ | 5,6,7,5-tetrahydro-[1,8]naphthyridin-2-yl | (6-OCH₃)-pyridin-3-yl | 1 | Isomer a | OH |
| **28b** | -(CH₂)₂-R₁ | 5,6,7,8-tetrahydro-[1,8]naphthyridin-2-yl | (6-OCH₃)-pyridin-3-yl | I | Isomer b | OH |
| **29** | -(CH₂)₃-R₁ | 5,6,7,8-tetrahydro-[1,8]naphthyridin-2-yl | quinolin-3-yl | 1 | | OH |
| **30** | -(CH₂)₂-R₁ | 5,6,7,8-tetrahydro-[1,8]naphthyridin-2-yl | (3-F)phenyl | 1 | | OH |
| **30a** | -(CH₂)₂-R₁ | 5,6,7,8-tetrahydro-[1,8]naphthyridin-2-yl | (3-F)phenyl | 1 | Isomer a | OH |
| **30b** | -(CH₂)₂-R₁ | 5,6,7,8-tetrahydro-[1,8]naphthyridin-2-yl | (3-F)phenyl | 1 | Isomer b | OH |
| **31** | -(CH₂)₃-R₁ | 5,6,7,8-tetrahydro-[1,8]naphthyridin-2-yl | (3-F)phenyl | 1 | | OH |
| **32** | -(CH₂)₂-R₁ | 5,6,7,8-tetrahydro-[1,8]naphthyridin-2-yl | quinolin-3-yl | 1 | | OH |
| **33** | -(CH₂)₂-R₁ | 5,6,7,8-tetrahydro-[1,8]naphthyridin-2- yl | (4-F)phenyl | 1 | | OH |
| **34** | -(CH₂)₃-R₁ | 5,6,7,8-tetrahydro-[1,8]naphthyridin-2-yl | (4-F)phenyl | 1 | | OH |
| **35** | -(CH₂)₂-R₁ | 5,6,7,8-tetrahydro-[1,8]naphthyridin-2-yl | (2-CH₃)pyrimidin-5-yl | 1 | | OH |
| **36** | -(CH₂)₂-R₁ | 5,6,7,8-tetrahydro-[1,8]naphthyridin-2-yl | 2,3-dihydro-benzofuran-6-yl | 1 | | OH |
| **36a** | -(CH₂)₂-R₁ | 5,6,7,8-tetrahydro-[1,8]naphthyridin-2-yl | 2,3-dihydro-benzofuran-6-yl | 1 | Isomer a | OH |
| **36b** | -(CH₂)₂-R₁ | 5,6,7,8-tetrahydro-[1,8]naphthyridin-2-yl | 2,3-dihydro-benzofuran-6-yl | 1 | Isomer b | OH |
| **37** | -(CH₂)₂-R₁ | 5,6,7,8-tetrahydro-[1,8]naphthyridin-2-yl | (3,5-difluoro)-phenyl | 1 | | OH |
| **38** | -(CH₂)₃-R₁ | 5,6,7,8-tetrahydro-[1,8]naphthyridin-2-yl | (3,5-difluoro)-phenyl | 1 | | OH |
| **39** | -(CH₂)₂-R₁ | 5,6,7,8-tetrahydro-[1,8]naphthyridin-2-yl | (3-CF₃)-phenyl | 1 | | OH |
| **40** | -(CH₂)₂-R₁ | 5,6,7,8-tetrahydro-[1,8]naphthyridin-2-yl | (4-OCF₃)-phenyl | 1 | | OH |
| **41** | -(CH₂)₂-R₁ | 5,6,7,8-tetrahydro-[1,8]naphthyridin-2-yl | (3-F-4-Ph)-phenyl | 1 | | OH |
| **42** | -(CH₂)₂-R₁ | 5,6,7,8-tetrahydro-[1,8]naphthyridin-2-yl | (3-F-4-OCH₃)-phenyl | 1 | | OH |
| **43** | -(CH₂)₂-R₁ | 5,6,7,8-tetrahydro-[1,8]naphthyridin-2-yl | (4-Oph)-phenyl | 1 | | OH |
| **44** | -(CH₂)₂-R₁ | 5,6,7,8-tetrahydro-[1,8]naphthyridin-2-yl | isoquinolin-4-yl | 1 | | OH |
| **45** | -(CH₂)₂-R₁ | 5,6,7,8-tetrahydro-[1,8]naphthyridin-2-yl | pyridin-3-yl | 1 | | OH |
| **46** | -(CH₂)₂-R₁ | 5,6,7,8-tetrahydro-[1,8]naphthyridin-2-yl | dihydrobenzofuran -5-yl | 1 | | OH |
| **47** | -(CH₂)₂-R₁ | 5,6,7,8-tetrahydro-[1,8]naphthyridin-2-yl | (2,4-OCH₃)-pyrimidin-5-yl | 1 | | OH |
| **48** | -(CH₂)₂-R₁ | 5,6,7,8-tetrahydro-[1,8]naphthyridin-2-yl | (2-OCH₃)-pyrimidin-5-yl | 1 | | OH |
| **49** | Ph(3-R₁) | -NH-1,4,5,6-tetrahydro-5-OH-pyrimidin-2-yl | quinolin-3-yl | 2 | | OH |
| **50** | Ph(3-R₁) | -NH-1,4,5,6-tetrahydro-pyridin-2-yl | quinolin-3-yl | 2 | | OH |
| **51** | -(CH₂)₂-R₁ | 5,6,7,8-tetrahydro-[1,8]naphthyridin-2-yl | quinolin-3-yl | 2 | | OH |
| **52** | Ph(3-R₁) | -NH-3,4,5,6-tetrahydro-pyrimidin-2-yl | 1,3-benzodioxol-5-yl | 2 | | OH |
| **53** | Ph(3-R₁) | -NH-3,4,5,6-tetrahydro-pyridin-2-yl | 1,3-benzodioxol-5-yl | 2 | | OH |
| **54** | Ph(3-R₁) | NH-1,4,5,6-tetrahydro-5-OH-pyrintidin-2-yl | 1,3-benzodioxol-5-yl | 2 | | OH |
| **55** | -CH₂-R₁ | 5,6,7,8-tetrahydro-[1,8]naphthyridin-2-yl | 1,3-benzodioxol-5-yl | 2 | | OH |
| **56** | -(CH₂)₂-R₁ | 5,6,7,8-tetrahydro-[1,8]naphthyridin-2-yl | naphthalene-2-yl | 1 | | OH |
| **56a** | -(CH₂)₂-R₁ | 5,6,7,8-tetrahydro-[1,8]naphthyridin-2-yl | naphthalen-2-yl | 1 | Isomer a | OH |
| **56b** | -(CH₂)₂-R₁ | 5,6,7,8-tetrahydro-[1,8]naphthyridin-2-yl | naphthalen-2-yl | 1 | Isomer b | OH |
| **57** | -(CH₂)₂-R₁ | 5,6,7,8-tetrahydro-[1,8]naphthyridin-2-yl | 5,6,7,8-tetrahydro-quinolin-3-yl | 1 | racemic | OH |
| **58a** | -(CH₂)₃-R₁ | 5,6,7,8-tetrahydro-[1,8]naphthyridin-2-yl | 5,6,7,8-tetrahydro-quinolin-3-yl | 0 | Isomer a | OH |
| **58b** | -(CH₂)₃-R₁ | 5,6,7,8-tetrahydro-[1,8]naphthyridin-2-yl | 5,6,7,8-tetrahydro-quinolin-3-yl | 0 | Isomer b | OH |
| **59** | -(CH₂)₂-R | 5,6,7,8-tetrahydro-[1,8]naphthyridin-2-yl | (3-OCH₃)phenyl | 1 | racemic | OH |
| **60** | -(CH₂)₂-R₁ | 5,6,7,8-tetrahydro-[1,8]naphthyridin-2-yl | (4-OCH₃)phenyl | 1 | racemic | OH |
| **61** | -(CH₂)₂-R₁ | 5,6,7,8-tetrahydro-[1,8]naphthyridin-2-yl | H | 1 | | OH |
| **62** | -(CH₂)₂-R₁ | 5,6,7,8-tetrahydro-[1,8]naphthyridin-2-yl | tetrahydrofuran-3-yl | 1 | racemic | OH |
| **63** | -(CH₂)₂-R₁ | 5,6,7,8-tetrahydro-[1,8]naphthyridin-2-yl | thiophen-2-yl | 1 | racemic | OH |
| **64** | -(CH₂)₂-R₁ | 5,6,7,8-tetrahydro-[1,8]naphthyridin-2-yl | (3-F)phenyl | 1 | racemic | NH₂ |
| **65** | -(CH₂)₂-R₁ | 5,6,7,8-tetrahydro-[1,8]naphthyridin-2-yl | 2,3-dihydro-benzo[1,4]-dioxin-6-yl | 1 | racemic | OH |
| **66** | -(CH₂)₂-R₁ | 5,6,7,8-tetrahydro-[1,8]naphthyridin-2-yl | (3-SCH₃)phenyl | 1 | racemic | OH |
| **67** | -(CH₂)₂-R₁ | 5,6,7,8-tetrahydro-[1,8]naphthyridin-2-yl | N-methyl-1,2,3,4-tetrahydro-quinolin-3-yl | 1 | racemic | OH |
| **68** | -(CH₂)₂-R₁ | 5,6,7,8-tetrahydro-[1,8]naphthyridin-2-yl | H | 1 | | -O-ethyl |
| **69** | -(CH₂)₂-R₁ | 5,6,7,8-tetrahydro-[1,8]naphthyridin-2-yl | H | 1 | | -O-2-propyl |
| **70** | -(CH₂)₂-R₁ | 5,6,7,8-tetrahydro-[1,8]naphthyridin-2-yl | H | 1 | | -O-t-butyl |
| **71** | -(CH₂)₂-R₁ | 5,6,7,8-tetrahydro-[1,8]naphthyridin-2-yl | H | 1 | | -O-n-octyl |
| **72** | -(CH₂)₂-R₁ | 5,6,7,8-tetrahydro-[1,8]naphthyridin-2-yl | H | 1 | | -*O*-*s*-butyl |
| **73** | -(CH₂)₂-R₁ | 5,6,7,8-tetrahydro-[1,8]naphthyridin-2-yl | H | 1 | | -O--methyl |
| **74** | -(CH₂)₂-R₁ | 5,6,7,8-tetrahydro-[1,8]naphthyridin-2-yl | H | 1 | racemic | -O-CH₂-OC(O)-t-butyl |
| **75** | -(CH₂)₂-R₁ | 5,6,7,8-tetrahydro-[1,8]naphthyridin-2-yl | (3-(NMe₂)phenyl | 1 | racemic | OH |
| **76** | -(CH₂)₂-R₁ | 5,6,7,8-tetrahydro-[1,8]naphthyridin-2-yl | (3-OMe-4-OH)phenyl | 1 | racemic | OH |
| **76a** | -(CH₂)₂-R₁ | 5,6,7,8-tetrahydro-[1,8]naphthyndin-2-yl | (3-OMe-4-OH)phenyl | 1 | Isomer a | OH |
| **77** | Ph(3-R₁) | -NH-4,5-dihydro-1*H*-imidazol-2-yl | (3-F)phenyl | 1 | racemic | OH |
| **78** | -(CH₂)₂-R₁ | 5,6,7,8-tetrahydro-[1,8]naphthyridin-2-yl | (3-NHEt)phenyl | 1 | racimic | OH |
| **79** | -(CH₂)₂-R₁ | 5,6,7,8-tetrahydro-[1,8]naphthyridin-2-yl | (3-NHMe)phenyl | 1 | racemic | OH |
| **80** | -(CH₂)₃-R₁ | 5,6,7,8-tetrahydro-[1,8]naphthyridin-2-yl | dihydrobenzofuran -6-yl | | 0 | OH |

Aspects of the present disclosure include a composition comprising a compound of Formula (II) 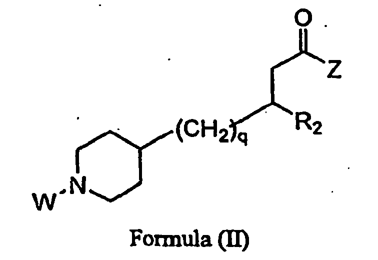 wherein W, R₁, R₂, q and Z are as previously defined and preferably are

| | | | | | **Stereo** | |
|---|---|---|---|---|---|---|
| **Cpd** | **W** | **R₁** | **R₂** | **q** | **chem** | **Z** |
| 81 | -(CH₂)₃-R₁ | 5,6,7,8-tetrahydro-[1,8]naphthyridin-2-yl | (3-F)phenyl | 1 | racemic | OH |

Aspects of the present disclosure include a composition comprising a compound of Formula (I) wherein the compound is selected from the group consisting of
a compound of Formula (I) wherein W is -CH₂-Ph(3-R₁); R₁ is -NH-1,4,5,6-tetrahydro-pyrimidin-2-yl; R₂ is H, q is 0 and Z is OH;
a compound of Formula (I) wherein W is -(CH₂)₂-Ph(3-R₁); R₁ is -NH-1,4,5,6-tetrahydro-pyrimidin-2-yl; R₂ is H, q is 0 and Z is OH;
a compound of Formula (1) wherein W is -CH₂-Ph(3-R₁); R₁ is -NH-1,4,5,6-tetrahydro-5-OH-pyrimidin-2-yl; R₂ is -3-quinolinyl, q is 0 and Z is OH;
a compound of Formula (I) wherein W is -(CH₂)₃-R₁; R₁ is -5,6,7,8-tetrahydro-1,8-naphthyridin-2-yl; R₂ is -3-quinolinyl, q is 0 and Z is OH;
a compound of Formula (I) wherein W is -(CH₂)₃-R₁; R₁ is -5,6,7,8-tetrahydro-1,8-naphthyridin-2-yl; R₂ is -1,2,3,4-tetrahydro-3-quinolinyl, q is 0 and Z is OH
a compound of Formula (I) wherein W is -Ph(3-R₁); R₁ is -NH-1,4,5,6-tetrahydro-pyrimidin-2-yl; R₂ is -3-pyridinyl, q is 2 and Z is OH;
a compound of Formula (I) wherein W is -Ph(3-R₁); R₁ is -NH-1,4,5,6-tetrahydro-5-OH-pyrimidin-2-yl; R₂ is -3-pyridinyl, q is 2 and Z is OH;
a compound of Formula (I) wherein W is -(CH₂)₂-R₁; R₁ is -5,6,7,8-tetrahydro-1,8-naphthyridin-2-yl; R₂ is -3-pyridinyl, q is 2 and Z is OH;
a compound of Formula (I) wherein W is -(CH₂)₃-R₁; R₁ is -NH-pyridin-2-yl; R₂ is -3-pyridinyl, q is 2, and Z is OH;
a compound of Formula (I) wherein W is -Ph(3-R₁); R₁ is -NH-1,4,5,6-tetrahydro-5-OH-pyrimidin-2-yl; R₂ is -(6-MeO)pyridin-3-yl, q is 2 and Z is OH;
a compound of Formula (I) wherein W is -(CH₂)₂-R₁; R₁ is -5,6,7,8-tetrahydro-1,8-naphthyridin-2-yl; R₂ is -1,3-benzodioxol-5-yl, q is 1 and Z is OH;
a compound of Formula (I) wherein W is -Ph(3-R₁); R₁ is -NH-1,4,5,6-tetrahydro-pyrimidin-2-yl; R₂ is -3-quinolinyl, q is 2 and Z is OH; a compound of Formula (I) wherein W is -(CH₂)₂-R₁; R₁ is -5,6,7,8-tetrahydro-1,8-naphthyridin-2-yl; R₂ is -Ph, q is 1 and Z is OH;
a compound of Formula (I) wherein W is -(CH₂)₂-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -(CH₂)₃-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -CH₂-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -(CH₂)₃-R₁; R₁ is -5,6,7,8-tetrahydro-1,8-naphthyridin-2-yl; R₂ is -(6-MeO)pyridin-3-yl, q is 0 and Z is OH;
a compound of Formula (I) wherein W is -(CH₂)₂-R₁; R₁ is -5,6,7,8-tetrahydro-1,8-naphthyridin-2-yl; R₂ is -1,4,5,6-tetrahydro-2-Me-pyrimidin-5-yl, q is 1 and Z is OH;
a compound of Formula (I) wherein W is -(CH₂)₂-R₁; R₁ is -5,6,7,8-tetrahydro-1,8-naphthyridin-2-yl; R₂ is -1,2,3,4-tetrahydro-3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -1,3-benzodioxol-5-yl, q is 2 and Z is OH;
a compound of Formula (1) wherein W is -(CH₂)₂-R₁; R₁ is -5,6,7,8-tetrahydro-1,8-naphthyridin-2-yl; R₂ is -(6-MeO)pyridin-3-yl, q is 2 and Z is OH;
a compound of Formula (I) wherein W is -(CH₂)₃-R₁; R₁ is -NH-pyridin-2-yl; R₂ is -3-quinolinyl, q is 2 and Z is OH;
a compound of Formula (I) wherein W is -(CH₂)₃-R₁; R₁ is -NH-pyridin-2-yl; R₂ is -1,3-benzodioxol-5-yl, q is 2 and Z is OH;
a compound of Formula (I) wherein W is -(CH₂)₃-R₁; R₁ is -NH-pyridin-2-yl; R₂ is . -1,3-benzodioxol-5-yl, q is 0 and Z is OH;
a compound of Formula (I) wherein W is -(CH₂)₃-R₁; R₁ is -NH-pyridin-2-yl; R₂ is -(6-MeO)pyridin-3-yl, q is 2 and Z is OH;
a compound of Formula (I) wherein W is -(CH₂)₃-R₁; R₁ is -5,6,7,8-tetrahydro-1,8-naphthyridin-2-yl; R₂ is -1,3-benzodioxol-5-yl, q is 1 and Z is OH;
a compound of Formula (I) wherein W is -Ph(3-R₁); R₁ is -NH-1,4,5,6-tetrahydro-5-OH-2-pyrimidinyl; R₂ is -1,3-benzodioxol-5-yl, q is 1 and Z is OH;
a compound of Formula (I) wherein W is -(CH₂)₂-R₁; R₁ is -5,6,7,8-tetrahydro-1,8-naphthyridin-2-yl; R₂ is -(6-MeO)pyridin-3-yl, q is 1 and Z is OH;
a compound of Formula (I) wherein W is -(CH₂)₃-R₁; R₁ is -5,6,7,8-tetrahydro-1,8-naphthyridin-2-yl; R₂ is -3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -(3-F)Ph, q is 1 and Z is OH;
a compound of Formula (I) wherein W is -(CH₂)₃-R₁; R₁ is -5,6,7,8-tetrahydro-1,8-naphthyridin-2-yl; R₂ is -(3-F)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -(4-F)Ph, q is 1 and Z is OH;
a compound of Formula (I) wherein W is -(CH₂)₃-R₁; R₁ is -5,6,7,8-tetrahydro-1,8-naphthyridin-2-yl; R₂ is -(4-F)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -(2-Me)pyrimidin-5-yl, q is 1 and Z isOH;
a compound of Formula (I) wherein W is -(CH₂)₂-R₁; R₁ is -5,6,7,8-tetrahydro-1,8-naphthyridin-2-yl; R₂ is -2,3-dihydro-benzofuran-6-yl, q is 1 and Z is OH;
a compound of Formula (I) wherein W is -(CH₂)₂-R₁; R₁ is -5,6,7,8-tetrahydro-1,8-naphthyridin-2-yl; R₂ is -(3,5-F₂)Ph, q is 1 and Z is-OH;
a compound of Formula (I) wherein W is -(CH₂)₃-R₁; R₁ is -5,6,7,8-tetrahydro-1,8-naphthyridin-2-yl; R₂ is -(3,5-F₂)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -(3-CF₃)Ph, q is I and Z is OH;
a compound of Formula (I) wherein W is -(CH₂)₂-R₁; R₁ is -5,6,7,8-tetrahydro-1,8-naphthyridin-2-yl; R₂ is -(4-OCF₃)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -(3-F-4-Ph)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -(3-F-4-OMe)Ph, q is 1, and Z is OH;
a compound of Formula (I) wherein W is -(CH₂)₂-R₁; R₁ is -5,6,7,8-tetrahydro-1,8-naphthyridin-2-yl; R₂ is -(4-OPh)Ph, q is 1 and Z is OH;
a compound of Formula (I) wherein W is -(CH₂)₂-R₁; R, is -5,6,7,8-tetrahydro-1,8-naphthyridin-2-yl; R₂ is -4-isoquinolinyl, q is 1, and Z is OH;
a compound of Formula (I) wherein W is -(CH₂)₂-R₁; R₁ is -5,6,7,8-tetrahydro-1,8-naphthyridin-2-yl; R₂ is -3-pyridinyl, q is 1 and Z is OH;
a compound of Formula (I) wherein W is -(CH₂)₂-R₁; R₁ is -5,6,7,8-tetrahydro-1,8-naphthyridin-2-yl; R₂ is -5-dihydrobenzofuranyl, q is 1 and Z is OH;
a compound of Formula (I) wherein W is -(CH₂)₂-R₁; R₁ is -5,6,7,8-tetrahydro-1,8-naphthyridin-2-yl; R₂ is -2,4-(OMe)₂-pyrimid-5-yl, q is 1 and Z is OH;
a compound of Formula (I) wherein W is -(CH₂)₂-R₁; R₁ is -5,6;7,8-tetrahydro-1,8-naphthyridin-2-yl; R₂ is -(2-OMe)pyrimidin-5-yl, q is 1 and Z is OH;
a compound of Formula (I) wherein W is -Ph(3-R₁); R₁ is -NH-1,4,5,6-tetrahydro-5-OH-pyrimidin-2-yl; R₂ is -3-quinolinyl, q is 2 and Z is OH;
a compound of Formula (I) wherein W is -Ph(3-R₁); R₁ is -NH-3,4,5,6-tetrahydro-pyridin-2-yl; R₂ is -3-quinolinyl, q is 2 and Z is OH;
a compound of Formula (I) wherein W is -(CH₂)₂-R₁; R₁ is -5,6,7,8-tetrahydro-1,8-naphthyridin-2-yl; R₂ is -3-quinolinyl, q is 2 and Z is OH;
a compound of Formula (I) wherein W is -Ph(3-R₁); R₁ is -NH-3,4,5,6-tetrahydro-pyrimidin-2-yl; R₂ is -1,3-benzodioxol-5-yl, q is 2 and Z is OH;
a compound of Formula (I) wherein W is -Ph(3-R₁); R₁ is -NH-3,4,5,6-tetrahydro-pyridin-2-yl; R₂ is -1,3-benzodioxol-5-yl, q is 2 and Z is OH;
a compound of Formula (I) wherein W is -Ph(3-R₁); R₁ is -NH-1,4,5,6-tetrahydro-5-OH-pyrimidin-2-yl; R₂ is -1,3-benzodiaxol-5-yl, q is 2 and Z is OH;
a compound of Formula (I) wherein W is -CH₂-R₁; R₁ is -5,6,7,8-tetrahydro-1,8-naphthyridin-2-yl; R₂ is -1,3-benzodioxol-5-yl, q is 2 and Z is OH; and,
a compound of Formula (I) wherein W is -(CH₂)₂-R₁; R₁ is -5,6,7,8-tetrahydro-1,8-naphthyridin-2-yl; R₂ is -2-naphthalenyl, q is 1 and Z is OH.

Another aspect of the present disclosure includes a composition comprising a compound of Formula (I) wherein the compound is selected from the group consisting of:
a compound of Formula (I) wherein W is -(CH₂)₃-R₁; R₁ is -5,6,7,8-tetrahydro-1,8-naphthyridin-2-yl; R₂ is -1,2,3,4-tetrahydro-3-quinolinyl, q is 0 and Z is OH;
a compound of Formula (1) wherein W is -(CH₂)₃-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -(CH₂)₂-R₁; R₁ is -5,6,7,8-tetrahydro-1,8-naphthyridin-2-yl; R₂ is -1,2,3,4-tetrahydro-3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -(6-MeO)pyridin-3-yl, q is 1 and Z is OH;
a compound of Formula (I) wherein W is -(CH₂)₂-R₁; R₁ is -5,6,7,8-tetrahydro-1,8-naphthyridin-2-yl; R₂ is -(3-F)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -(2-Me)pyrimidin-5-yl, q is 1 and Z is OH;
a compound of Formula (I) wherein W is -(CH₂)₂-R₁; R₁ is -5,6,7,8-tetrahydro-1,8-naphthyridin-2-yl; R₂ is -2,3-dihydro-benzofuran-6-yl, q is 1 and Z is OH;
a compound of Formula (I) wherein W is -(CH₂)₂-R₁; R₁ is -5,6,7,8-tetrahydro-1,8-naphthyridin-2-yl; R₂ is -4-isoquinolinyl, q is I, and Z is OH;
a compound of Formula (I) wherein W is -(CH₂)₂-R₁; R₁ is -5,6,7,8-tetrahydro-1,8-naphthyridin-2-yl; R₂ is -3-pyridinyl, q is 1 and Z is OH;
a compound of Formula (I) wherein W is -(CH₂)₂-R₁; R₁ is -5,6,7,8-tetrahydro-1,8-naphthyridin-2-yl; R₂ is -2,4-(OMe)₂-pyrimid-5-yl, q is 1 and Z is OH; and,
a compound of Formula (I) wherein W is -(CH₂)₂-R₁; R₁ is -5,6,7,8-tetrahydro-1,8-naphthyridin-2-yl; R₂ is -(2-OMe)pyrimidin-5-yl, q is 1 and Z is OH.

Another aspect of the present disclosure includes a compound of Formula (I) wherein W is -(CH₂)₃-R₁; R₁ is -5,6,7,8-tetrahydro-1,8-naphthyridin-2-yl; R₂ is -1,2,3,4-tetrahydro-3-quinolinyl, q is 0 and Z is OH.

Another aspect of the present disclosure includes a compound of Formula (I) wherein W is -(CH₂)₃-R₁; R₁ is -5,6,7,8-tetrahydro-1,8-naphthyridin-2-yl; R₂ is -1,3-benzodioxol-5-yl, q is 0 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -1,2,3,4-tetrahydro-3-quinolinyl, q is 1 and Z is OH;

Another aspect of the present disclosure includes a compound of Formula (I) wherein W is -(CH₂)₂-R₁; R₁ is -5,6,7,8-tetrahydro-1,8-naphthyridin-2-yl; R₂ is -(6-MeO)pyridin-3-yl, q is 1 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -(3-F)Ph, q is 1 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -3-quinolinyl, q is 1 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -(2-Me)pyrimidin-5-yl, q is 1 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -2,3-dihydro-benzofuran-6-yl, q is 1 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -4-isoquinolinyl, q is 1 and Z is OH.

Another aspect of the present disclosure includes a compound of Formula (I) wherein W is-(CH₂)₂-R₁; R₁ is -5,6,7,8-tetrahydro-1,8-naphthyridin-2-yl; R₂ is -3-pyridinyl, q is 1 and Z is OH.

Another aspect of the present disclosure includes a compound of Formula (I) wherein W is -(CH₂)₂-R₁; R₁ is -5,6,7,8-tetrahydro-1,8-naphthyridin-2-yl; R₂ is -2,4-(OMe)₂-pyrimid-5-yl, q is 1 and Z is OH.

Another aspect of the present disclosure includes a compound of Formula (I) wherein W is -(CH₂)₂-R₁; R₁ is -5,6,7,8-tetrahydro-1,8-naphtlryridin-2-yl; R₂ is -(2-OMe)pyrimidin-5-yl, q is 1 and Z is OH.

Aspects of the present disclosure include a compound of Formula (I): 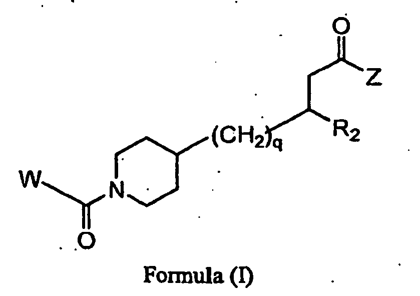 wherein W, R₁, R₂, R₆, R₈, R₉, q and Z are as previously defined; and, preferably,
wherein
- W: is -C₀₋₄alkyl(R₁) or -C₀₋₄alkyl-phenyl(R₁,R₈);
- R₁: is -NH(R₆);
- R₂: is hydrogen, -tetrahydropyrimidinyl(R₈), 1,3-benzodioxolyl(R₈), -dihydrobenzofuranyl(R₈), -tetrahydroquinolinyl(R₈), -phenyl(R₈), -naphthalenyl(R₈), -pyridinyl(R₈), -pyrimidinyl(R₈) or -quinolinyl(R₈);
- R₆: is -dihydroimidazolyl(R₈), -tetrahydropyridinyl(R₈), -tetrahydropyrimidinyl(R₈) or -pyridinyl(R₈);
- R₈: is one to four substituents independently selected from hydrogen or -C₁₋₄alkyl(R₉) when attached to a nitrogen atom; and, wherein R₈ is one to four substituents independently selected from hydrogen, -C₁₋₄alkyl(R₉), -C₁₋₄alkoxy(R₉),-O-aryl(R₁₀) or hydroxy when attached to a carbon atom;
- R₉: is hydrogen, -C₁₋₄alkoxy, -NH₂, -NH-C₁₋₄alkyl, -N(C₁₋₄alkyl)₂, (halo)₁₋₃ or hydroxy; and,
- q: is 1, 2 or 3;
Z is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈akoxy, -O- C₁₋₈alkylcarbonylC₁₋₈alkyl, -O-C₁₋₈alkyl-CO₂H, -O-C₁₋₈alkyl-C(O)O-C₁₋₈alkyl, -O- C₁₋₈-alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(C₁₋₈alkyl)₂, -O-C₁₋₈alkylamide, -O-C₁₋₈alkyl-C(O)-NH-C₁₋₈alkyl, -O-C₁₋₈alkyl-C(O)-N(C₁₋₈alkyl)₂ and -NHC(O)C₁₋₈alkyl;
and pharmaceutically acceptable salts, racemic mixtures and enantiomers thereof.

Aspects of the present disclosure include a compound of Formula (I) wherein the compound is a compound of Formula (I.2): 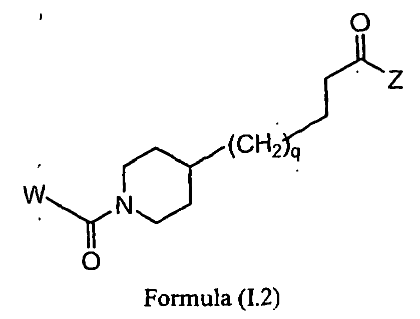 wherein W, R₁, R₆, R₈, R₉, q and Z are as previously defined; and, preferably, wherein
- W: is -C₀₋₄alkyl(R₁) or -C₀₋₄alkyl-phenyl(R₁,R₈);
- R₁: is NH(R₆), -dihydro-1*H*-pyrrolo[2,3-*b*]pyridinyl(R₈), -tetrahydropyrimidinyl(R₈), -tetrahydro-1,8-naphoyridinyl(R₈), -tetrahydro-1*H*-azepino[2,3-*b*]pyridinyl(R₈) or -pyridinyl(R₈);
- R₆: is -dihydroimidazolyl(R₈), -tetrahydropyridinyl(R₈), -tetrahydropyrimidinyl(R₈) or -pyridinyl(R₈);
- R₈: is one to four substituents independently selected from hydrogen or -C₁₋₄alkyl(R₉) when attached to a nitrogen atom; and, wherein R₈ is one to four substituents independently selected from hydrogen, -C₁₋₄alkyl(R₉), -C₁₋₄alkoxy(R₉), -O-aryl(R₁₀) or hydroxy when attached to a carbon atom;
- R₉: is hydrogen, -C₁₋₄alkoxy, -NH₂, -NH-C₁₋₄alk-yl, -N(C₁₋₄alkyl)₂, (halo)₁₋₃ or hydroxy; and,
- q is: 1, 2 or 3;
Z is selected from the group consisting hydroxy, NH₂, -NH-C₁₋₈alk-yl, -N(C₁₋₈alkyl)₂, -O-C₁₋₈alkyl, -O-C₁₋₈alkyl-OH, -O-C₁₋₈alkylC₁₋₈alkoxy, -O- C₁₋₈alkylcaronylC₁₋₈alkyl, -O-C₁₋₈alkyl-CO₂H, -O-C₁₋₈alkyl-C(O)O-C₁₋₈alkyl, -O- C₁₋₈alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(C₁₋₈alkyl)₂, -O-C₁₋₈alkylamide, -O-C₁₋₈alkyl-C(O)-NH-C₁₋₈alkyl, -O-C₁- ₈alkyl-C(O)-N(C₁₋₈alkyl)₂ and -NHC(O)C₁₋₈alkyl;
and pharmaceutically acceptable salts, racemic mixtures and enantiomers thereof.

Another aspect of the present disclosure includes compounds of Formula (I.2) wherein R₁ is -NH(R₆), -tetrahydropyrimidinyl(R₈) or -tetrahydro-1,8-naphthyridinyl(R₈); and, all other variables are as previously defined.

Aspects of the present disclosure include a compound of Formula (I) wherein the compound is a compound of Formula (I.3): 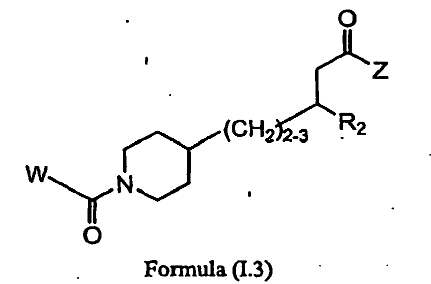 wherein W, R₁, R₂, R₆, R₈, R₉ and Z are as previously defined; and, preferably, wherein
- W: is -C₀₋₄alkyl(R₁) or -C₀₋₄alkyl-phenyl(R₁,R₈);
- R₁: is -NH(R₆), -dihydro-1*H*-pyrrolo[2,3-*b*]pyridinyl(R₈), -tetrahydropyrimidinyl(R₈), -tetrahydro-1,8-naphthyridinyl(R₈), -tetrahydro-1*H*-azepino[2,3-*b*]pyridinyl(R₈) or -pyridinyl(R₈);
- R₂: is hydrogen, -tetrahydropyrimidinyl(R₈), -1,3-benzodioxolyl(R₈), -dihydrobenzofuranyl(R₈), -tetrahydroquinolinyl(R₈), -phenyl(R₈), -naphthalenyl(R₈), -pyridinyl(R₈), -pyrimidinyl(R₈) or -quinolinyl(R₈);
- R₆: is -dihydroimidazolyl(R₈), -tetrahydropyridinyl(R₈), -tetrahydropyrimidinyl(R₈) or -pyridinyl(R₈);
- R₈: is one to four substituents independently selected from hydrogen or -C₁₋₄lkyl(R₉) when attached to a nitrogen atom; and, wherein R₈ is one to four substituents independently selected from hydrogen, -C₁₋₄alkyl(R₉), -C₁₋₄alkoxy(T₉), -O-aryl(R₁₀) or hydroxy when attached to a carbon atom; and,
- R₉: is hydrogen, -C₁₋₄alkoxy, -NH₂, -NH-C₁₋₄akyl, -N(C₁₋₄alkyl)₂, (halo)₁₋₃ or hydroxy;
Z is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈alkoxy, -O- C₁₋₈alkylcarbonylC₁₋₈alkyl, -O-C₁₋₈alkyl-CO₂H, -O-C₁₋₈alkyl-C(O)O-C₁₋₈alkyl, -O- C₁₋₈alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(C₁₋₈alkyl)₂, -O-C₁₋₈alkylamide, -O-C₁₋₈alkyl-C(O)-NH-C₁₋₈alkyl, -O-C₁₋₈alkyl-C(O)-N(C₁₋₈alkyl)₂ and -NHC(O)C₁₋₈alkyl;
and pharmaceutically acceptable salts, racemic mixtures and enantiomers thereof.

Another aspect of the present disclosure includes compounds of Formula (I.3) wherein R₁ is -NH(R₆), -tetrahydropyrimidinyl(R₈) or -tetrahydro-1,8-naphthyridinyl(R₈); and, all other variables are as previously defined.

Aspects of the present disclosure include a compound of Formula (I) wherein the compound is a compound of Formula (I.4): 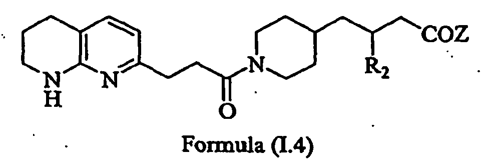 wherein R₂ and Z are as previously defined; and, further, R₂ is selected from the group consisting of -2-benzofuranyl, -3-benzofuranyl, -4-benzofuranyl; -5-benzofuranyl, -6-benzofuranyl, -7-benzofuranyl, -benzo[*b*]thien-2-yl, -benzo[b]thien-3-yl, -benzo[b]thien-4-yl,-benzo[*b*]thien-5-yl, -benzo[*b*]thien-6-yl, -benzo[*b*]thien-7-yl, -1*H-*indol-2-yl, -1*H-*indol-3-yl, -1*H-*indol-4-yl, -1*H-*indol-5-yl, -1*H-*indol-6-yl, -1*H-*indol-7-yl, -2-benzoxazolyl, -4-benzoxazolyl, -5-benzoxazolyl, -6-benzoxazolyl, -7-benzoxazolyl, -2-benzothiazolyl,-3-benzothiazolyl, -4-benzothiazolyl, -5-benzothiazolyl, -6-benzothiazolyl, -7-benzothiazolyl, -1*H-*benzimidazolyl-2-yl, -1*H-*benzimidazolyl-4-yl, -1*H-*benzimidazolyl-5-yl, -1*H-*benzimidazolyl-6-yl, -1*H-*benzimidazolyl-7-yl, -2-quinolinyl, -3-quinolinyl, -4-quinolinyl, -5-quinolinyl, -6-quinolinyl, -7-quinolinyl, -8-quinolinyl, -2*H-*1-benzopyran-2-yl, -2*H-*1-benzopyran-3-yl, -2*H-*1-benzopyran-4-yl, -2*H-*1-benzopyran-5-yl, -2*H-*1-benzopyran-6-yl, -2*H-*1-benzopyran-7-yl, -2*H-*1-benzopyran-8-yl, -4*H-*1-benzopyran-2-yl, -4*H-*1-benzopyran-3-yl, -4*H-*1-benzopyran-4-yl, -4*H-*1-benzopyran-5-yl, -4*H-*1-benzopyran-6-yl, -4*H-*1-benzopyran-7-yl, -4*H-*1-benzopyran-8-yl, -1*H-*2-benzopyran-1-yl, -1*H-*2'-benzopyran-3-yl, -1*H-*2-benzopyran-3-yl, -1*H-*2-benzopyran-5-yl, -1*H-*2-benzopyran-6-yl, -1*H-*2-benzopyran-7-yl, -1*H-*2-benzopyran-8-yl, -1,2,3,4-tetrahydro-1-naphthalenyl, -1,2,3,4-tetrahydro-2-naphthalenyl, -1,2,3,4-tetrahydro-5-naphthalenyl, -1,2,3,4-tetrahydra-6-naphthalenyl, -2,3-dihydro-2-benzofuranyl, -2,3-dihydro-3-benzofuranyl, -2,3-dihydro-4-benzofuranyl, -2,3-dihydro-5, benzofuranyl, -2,3-dihydro-6-benzofuranyl, -2,3-dihydro-7-benzofuranyl, -2,3-dihydrobenzo[*b*]thien-2-yl, -2,3-dihydrobenzo[*b*]thien-3-yl, -2,3-dihydrobenzo[*b*]thien-4-yl, -2,3-dihydrobenzo[*b*]thien-5-yl, -2,3-dihydrobenzo[*b*]thien-6-yl, -2,3-dihydrobenzo[*b*]thien-7-yl, -2,3-dihydro-1*H-*indol-2-yl, -2,3-dihydro-1*H-*indol-3-yl, -2,3-dihydro-1H indol-4-yl, -2,3-dihydro-1*H-*indol-5-yl, -2,3-dihydro-1*H-*indol-6-yl, -2,3-dihydro-1*H-*indol-7-yl, -2,3-dihydro-2-benzoxazolyl, -2,3-dihydro-4-benzoxazolyl, -2,3-dihydro-5-benzoxazolyl, -2,3-dihydro-6-benzoxazolyl, -2,3-dihydro-7-benzoxazolyl, -2,3-dihydro-1*H*-benzimidazol-2-yl, -2,3-dihydro-1*H-*benzimidazol-4-yl, -2,3-dihydro-1*H-*benzimidazol-5-yl, -2,3-dihydro-1*H-*benzimidazol-6-yl, -2,3-dihydro-1*H-*benzimidazol-7-yl, -3,4-dihydro-1(2*H*)-quinolinyl, -1,2,3,4-tetrahydro-2-quinolinyl, -1,2,3,4-tetrahydro-3-quinolinyl, -1,2,3,4-tetrahydro-4-quinolinyl, -1,2,3,4-tetrahydro-5-quinolinyl, -1,2,3,4-tetrahydro-6-quinolinyl, -1,2,3,4-tetrahydro-7-quinolinyl, -1,2,3,4-tetrahydro-8-quinolinyl, -3,4-dihydro-2*H-*1-benzopyran-2-yl, -3,4-dihydro-2*H-*1-benzopyran-3-yl, -3,4-dihydro-2*H-*1-benzopyran-4-yl, -3,4-dihydro-2*H-*1-benzopyrm-5-yl, -3,4-dihydro-2*H-*1-benzopyran-6-yl, -3,4-dihydro-2*H-*1-benzopyran-7-yl, -3,4-dihydro-2*H-*1-benzopyran-8-yl, -3,4-dihydro-4*H-*1-benzopyran-2-yl; -3,4-dihydro-4*H-*1-benzopyran-3-yl, -3,4-dihydro-4*H-*1-benzopyran-4-yl, -3,4-dihydro-4*H-*1-benzopyran-5-yl, -3,4-dihydro-4*H-*1-benzopyran-6-yl, -3,4-diydro-4*H-*1-benzopyran-7-yl, -3,4-dihydro-4*H-*1-benzopyran-8-yl, -3,4-dihydro-1*H-*2-benzopyran-2-yl, -3,4-dihydro-1*H-*2-benzopyran-3-yl, -3,4-dihydro-1*H-*2-benzopyran-4-yl, -3,4-dihydro-1*H-*2-benzopyran-5-yl, -3,4-dihydro-1*H-*2-benzopyran-6-yl, -3,4-dihydro-1*H-*2-benzopyran-7-yl and -3,4-dihydro-1*H-*2-benzopyran-8-yl optionally substituted when allowed by available valences with up to 7 substituents independently selected from methyl when attached to a nitrogen atom; and, independently selected from methyl, methoxy or fluoro when attached to a carbon atom;
Z is selected from the group, consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈alkoxy, -O-C₁₋₈alkylcarbonylC₁₋₈alkyl, -O-C₁₋₈alkyl-CO₂H, -O-C₁₋₈alkyl-C(O)O-C₁₋₈alkyl, -O-C₁₋₈alkyl-O-C(O)C₁₋₈alkyl, -O-C₁₋₈alkyl-NH₂, -O-C₁₋₈alkyl-NH-C₁₋₈alkyl, -O-C₁₋₈alkyl-N(C₁₋₈alkyl)₂, -O-C₁₋₈alkylamide, -O-C₁₋₈alkyl-C(O)-NH-C₁₋₈alkyl, -O-C₁-₈alkyl-C(O)-N(C₁₋₈alkyl)₂ and -NHC(O)C₁₋₈alkyl;
pharmaceutically acceptable salts, racemic mixtures and enantiomers thereof.

The compounds of the present disclosure may also be present in the form of pharmaceutically acceptable salts. For use in medicine, the salts of the compounds of this diclosure refer to non-toxic "pharmaceutically acceptable salts" (*Ref.* International J. Pharm., 1986, 33, 201-217; J. Pharm.Sci., 1977 (Jan), 66, 1, 1). Other salts may, however, be useful in the preparation of compounds according to this disclosure or of their pharmaceutically acceptable salts. Representative organic or inorganic acids include, but are not limited to, hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic acid. Representative organic or inorganic bases include, but are not limited to, basic or cationic salts such as benzathine, chloroprocaine, choline, diethanolamine, ethylenediamine, meglumine, procaine, aluminum, calcium, lithium, magnesium, potassium, sodium and zinc.

This document also discloses prodrugs of the compounds of this disclosure. In general, such prodrugs will be functional derivatives of the compounds which are readily convertible in vivo into the required compound. Thus, in the methods of treatment of the present disclosure, the term "administering" shall encompass the treatment of the various disorders described with the compound specifically disclosed or with a compound which may not be specifically disclosed, but which converts to the specified compound in vivo after administration to the subject. Conventional procedures for the selection and preparation of suitable prodrug derivatives are described, for example, in "Design of Prodrugs", ed. H. Bundgaard, Elsevier, 1985.

Where the compounds according to this disclosure have at least one chiral center, they may accordingly exist as enantiomers. Where the compounds possess two or more chiral centers, they may additional exist as diastereomers. Where the processes for the preparation of the compounds according to the disclosure give rise to mixtures of stereoisomers, these isomers may be separated by conventional techniques such as preparative chromatography. The compounds may be prepared in racemic form or as individual enantiomers or diasteromers by either stereospecific synthesis or by resolution. The compounds may be resolved into their component enantiomers or diasteromers by standard techniques. It is to be understood that all stereoisomers, racemic mixtures, diastereomers and enantiomers thereof are encompassed within the scope of the present disclosure.

During any of the processes for preparation of the compounds of the present disclosure, it may be necessary and/or desirable to protect sensitive or reactive groups on

any of the molecules concerned. This may be achieved by means of conventional protecting groups, such as those described in Protective Groups in Organic Chemistry ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known in the art.

Furthermore, some of the crystalline forms for the compounds may exist as polymorphs and as such are intended to be included in the present disclosure. In addition, some of the compounds may form solvates with water (i.e., hydrates) or common organic solvents and such solvates are also intended to be encompassed within the scope of this disclosure.

As used herein, the following underlined terms are intended to have the following meanings:
The term "C*_{a-b}*" (where a and b are integers referring to a designated number of carbon atoms) refers to an alkyl, alkenyl, alkynyl, alkoxy or cycloalkyl radical or to the alkyl portion of a radical in which alkyl appears as the prefix root containing from a to *b* carbon atoms inclusive. For example, C₁₋₃ denotes a radical containing 1, 2 or 3 carbon atoms.
The term "alkyl" refers to a saturated branched, straight-chain or cyclic monovalent hydrocarbon radicals derived by the removal of one hydrogen atom from a single carbon atom of an alkane molecule, thus forming the point of attachment. The term "alkenyl" refers to a partially unsaturated branched or straight-chain monovalent hydrocarbon radical having at least one carbon-carbon double bond and derived by the removal of one hydrogen atom from a single carbon atom of an alkene molecule, thus forming the point of attachment. The radical may be in either the *cis* or *trans* conformation about the double bond(s). The term "alkynyl" refers to a partially unsaturated branched or straight-chain monovalent hydrocarbon radical having at least one carbon-carbon triple bond and derived by the removal of one hydrogen atom from a single carbon atom of an alkyne molecule, thus forming the point of attachment. The term "alcoxy" refers to a saturated or partially unsaturated, branched, straight-chain monovalent hydrocarbon radical derived by the removal of the hydrogen atom from the single oxygen atom of an alkane, alkene or alkyne molecule, thus forming the point of attachment.
The term "-C₁₋₈alkyl(R*ₓ*)" (where x is an integer referring to a designated substitutent group) refers to an R*ₓ* substituent group which may be substituted within an alykl chain, on a terminal carbon atom and may be similarly substituted on an alkenyl, alkynyl or alkoxy radical with a designated amount of substituents where allowed by available chemical bond valences. The term "-C₀₋₈alkyl(R*ₓ*)" refers to an R*ₓ* substituent group which may also be directly substituted on a pointof attachment without an alkyl linking group (wherein C₀ is a placeholder for the R*ₓ* substituent with a direct bond to the point of attachment).
The term "cycloalkyl" refers to saturated cyclic monovalent hydrocarbon radical consistent with the definitions of alkyl, alkanyl, alkenyl and alkynyl. Specifically included within the definition of cycloalkyl are fused polycyclic ring systems in which one or more rings are aromatic and one or more rings are saturated or partially unsaturated (it being understood that the radical may also occur on the aromatic ring). For example, the cycloalkyl groups are saturated or partially unsaturated or monocyclic alkyl radicals of from 3-8 carbon atoms (derived from a molecule such as cyclopropane, cyclobutane, cyclopentane, cyclohexane or cycloheptane); saturated or partially unsaturated fused or benzofused cyclic alkyl radicals of from 9 to 12 carbon atoms; or, saturated or partially unsaturated fused or benzofused tricyclic or polycyclic alkyl radicals of from 13 to 20 carbon atoms.
The term "heterocyclyl" refers to a saturated or partially unsaturated cyclic alkyl radical in which one or more carbon atoms are independently replaced with the same or different heteroatom. Specifically included within the definition of heterocyclyl are fused polycyclic ring systems in which one or more rings are aromatic and one or more rings are saturated or partially unsaturated (it being understood that the radical may also occur on the aromatic ring). Typical heteroatoms to replace the carbon atom(s) include, but are not limited to, N, O, S and the like. For example, the heterocyclyl group is a saturated or partially unsaturated five membered monocyclic alkyl ring of which at least one member is replaced by a N, O or S atom and which optionally contains one additional O atom replacing an additional member of the alkyl ring or one additional N atom replacing a member of the alkyl ring; a saturated or partially unsaturated six membered monocyclic alkyl ring of which one, two or three members of the alkyl ring are replaced by a N atom and optionally one member of the alkyl ring is replaced by a O or S atom or two members of the alkyl ring are replaced,by O or S atoms; a saturated or partially unsaturated 5-6 membered heterocylic ring as previously defined fused to a heteroaryl as hereinafter defined; a saturated, partially unsaturated or benzofused nine or 10 membered bicyclic alkyl wherein at least one member of the ring is replaced by N, O, or S atom and which optionally one or two additional members of the bicyclic alkyl are replaced by N, O or S atoms; or, a saturated, partially unsaturated or benzofused 11 to 20 membered polycyclic alkyl of which at least one member is replaced by a N, O or S atom and which optionally one, two or three additional members of the polycyclic alkyl are replaced by N atoms. Examples of saturated or partially unsaturated heterocyclyl radicals include, but are not limited to, 2-pyrrolinyl, 3-pyrrolinyl, pyrrolidinyl, 1,3-dioxolanyl, 2-imidazolinyl, imidazolidinyl, dihydroimdazolyl, 2-pyrazolinyl, pyrazolidinyl, piperidinyl, morpholinyl, tetrahydropyrimidinyl, piperazinyl, dihydro-1H-pyrrolo[2,3-b]pyridinyl, tetrahydro-1, 8-naphthyridinyl, tetrahydro-1H-azepino[2,3-b]pyridinyl, 1,3-benzodioxol-5-yl, 1,2,3,4-tetrahydro-3-quinolinyl or dihydrobenzofuranyl.
The term "aryl" refers to a monovalent aromatic hydrocarbon radical derived by the removal of one hydrogen atom from a single carbon atom of an aromatic ring system, thus forming the point of attachment for the radical. For example, the aryl group is derived from an unsaturated aromatic monocyclic ring system containing 5 to 6 carbon atoms (such as phenyl, derived from benzene); an unsaturated aromatic bicyclic ring system containing 9 to 10 carbon atoms (such as naphthyl, derived from naphthalene); or, an unsaturated aromatic tricyclic ring system containing 13 to 14 hydrogen carbon atoms (such as anthracenyl, derived from anthracene). The term "aromatic ring system" refers to an unsaturated cyclic or polycyclic ring system having an "aromatic" conjugated π electron system. Specifically excluded from the definition of aryl are fused ring systems in which one or more rings are saturated or partially unsaturated. Typical aryl groups include, but are not limited to, anthracenyl, naphthalenyl, azulenyl, benzenyl and the like
The term "heteroaryl" refers to a monovalent heteroaromatic radical derived by the removal of one hydrogen atom from a single atom of a heteroaromatic ring system, thus forming the point of attachment for the radical. The term "heteroaromatic ring system" refers to an aromatic ring system in which one or more carbon atoms are each independently replaced with a heteroatom. Typical heteratoms to replace the carbon atoms include, but are not limited to, N, O, S, and the like. Specifically excluded from the definition of heteroaromatic ring system are fused ring systems in which one or more rings are saturated or partially unsaturated. For example, the heteroaryl group is derived from a heteroaromatic monocyclic ring system containing five members of which at least one member is a N, O or S atom and which optionally contains one, two or three additional N atoms; a heteroaromatic monocyclic ring system having six members of which one, two or three members are an N atom; a heteroaromatic fused bicyclic ring system having nine members of which at least one member is a N, O or S atom and which optionally contains one, two or three additional N atoms; a heteroaromatic fused bicyclic ring system having ten members of which one, two or three members are a N atom; a heteroaromatic fused tricyclic ring system containing 13 or 14 members of which at least one member is a N, O or S atom and which optionally contains one, two or three additional N atoms; or, a heteroaromatic fused polycyclic ring system containing 15 to 20 members of which at least one member is a N, O or S atom and which optionally contains one, two or three additional N atoms. Typical heteroaryls include, but are not limited to, cinnolinyl, furanyl, imidazolyl, indazolyl, indolyl, indolinyl, indolizinyl, isobenzofuranyl, isoquinolinyl, isothiazolyl, isoxazolyl, naphthyridinyl, oxazolyl, phenanthridinyl, phenanthrolinyl, purinyl, pyranyl, pyrazinyl, pyrazolyl, pyridazinyl, pyridinyl, pyrimidinyl, pyrrolyl, quinazolinyl, quinolinyl, quinoxalinyl, tetrazole, thiadiazole, thiazole, thiophene, triazole and the like.
The term "independently" means that when a group is substituted with more than one substituent that the substituents may be the same or different The term "dependently" means that the substituents are specified in an indicated combination of structure variables.

Under standard nomenclature rules used throughout this disclosure, the terminal portion of the designated side chain is described first followed by the adjacent functionality toward the point of attachment. Thus, for examples, a "phenylC₁-₆alkylamidoC₁₋₆alkyl" substituent refers to a group of the formula:

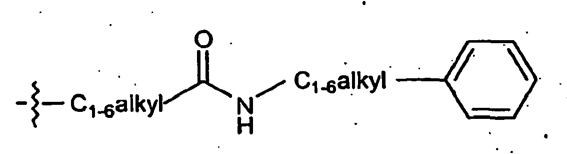

A substituent's point of attachment may also be indicated by a dashed line to indicate the point(s) of attachment, followed by the adjacent functionality and ending with the terminal functionality such as, for example, -(C₁₋₆)alkyl-carbonyl-NH-(C₁₋₆)alkyl-phenyl.

It is intended that the definition of any substituent or variable at a particular location in a molecule be independent of its definitions elsewhere in that molecule. It is understood that substituents and substitution patterns on the compounds of this disclosure can be selected by one of ordinary skill in the art to provide compounds that are chemically stable and that can be readily synthesized by techniques known in the art as well as those methods set forth herein.

Integrins are a widely expressed family of calcium or magnesium dependent a or β heterodimeric cell surface receptors, which bind to extracellular matrix adhesive proteins such as fibrinogen, fibronectin, vitronectin and osteopontin. The integrin receptors are transmembrane glycoproteins (GP's) known for their large extracellular 40 domains and are classified by at least 8 known β subunits and 14 α subunits (S. A. Mousa, et al., Emerging Theraupeutic Targets, 2000, 4, (2), 143-153).

For example, the β1 subfamily has the largest number of integrins wherein the various α subunits associate with various β subunits: β3, β5, β6 and β8 (S. A. Mousa, et al., Emerging Theraupeutic Targets, 2000, 4, (2), 144-147). Some of the disease states that have a strong αvβ3, αvβ5 and αIIbβ3 (also referred to as GPIIb/IIIa) integrin component in their etiologies are, unstable angina, thromboembolic disorders or atherosclerosis (GPIIb/IIIa); thrombosis or restenosis (GPIIb/IIIa or αvβ3); restenosis (dual αvβ3/GPIIbIIIIa); rheumatoid arthritis, vascular disorders or osteoporosis (αvβ3); tumor angiogenesis, tumor metastasis, tumor growth, multiple sclerosis, neurological disorders, asthma, vascular injury or diabetic retinopathy (αvβ3 or αvβ5); and, angiogenesis (dual αvβ3/αvβ5) (S. A. Mousa, et al., Emerging Theraupeutic Targets, 2000, 4, (2), 148-149; W. H. Miller, et al., Drug Discovery Today 2000, 5 (9), 397-407; and, S. A. Mousa, et al., Exp. Opin. Ther. Patents, 1999, 9 (9), 1237-1248). The β3 subunit has received significant attention in recent drug discovery efforts. (W. J. Hoekstra, Current Medicinal Chemistry 1998, 5, 195). Antibodies and/or low-molecular weight compound antagonists of αvβ3 have shown efficacy in animal models (J. Samanen, Current Pharmaceutical Design 1997, 3, 545) and, thereby, offer promise as medicinal agents.

Integrin antagonists have typically been designed after the bioactive arginine-glycine-aspartate (RGD) conformation of peptides derived from the primary ligand vitronectin. The RGD motif is the general cell attachment sequence of many extracellular matrix, blood and cell surface proteins, as half of the approximately 20 known integrins bind the RGD-containing adhesion ligands. To discover RGD peptides with integrin selectivity, peptides with both restricted conformations and alterations of flanking residues have been studied. In particular, the structural requirements for interaction of the RGD sequence with GPIIb/IIIa and the inhibitory potential of a series of nonpeptidic mimetics on platelet aggregation and interactions with the extracellular matrix have been described (D. Varon, et al., Thromb. Haemostasis, 1993, 70(6), 1030-1036). Iterative synthesis of cyclic and alicyclic peptides and computer modelling have provided potent, selective agents as a platform for nonpeptide αv (as in αvβ3) integrin antagonist design.

Integrin antagonists have been implicated as useful for inhibiting bone resorption (S.B. Rodan and G.A. Rodan, Integrin Function In Osteoclasts, Journal of Endocrinology, 1997, 154: S47-S56). In vertebrates, bone resorption is mediated by the action of cells known as osteoclasts, large multinucleated cells of up to about 400 mm in diameter that resorb mineralized tissue, chiefly calcium carbonate and calcium phosphate. Osteoclasts are actively motile cells that migrate along the surface of bone and can bind to bone, secrete necessary acids and proteases, thereby causing the actual resorption of mineralized tissue from the bone. More specifically, osteoclasts are believed to exist in at least two physiological states, namely, the secretory state and the migratory or motile state. In the secretory state, osteoclasts are flat, attach to the bone matrix via a tight attachment zone (sealing zone), become highly polarized, form a ruffled border and secrete lysosomal enzymes and protons to resorb bone. The adhesion of osteoclasts to bone surfaces is an important initial step in bone resorption. In the migratory or motile state, osteoclasts migrate across bone matrix and do not take part in resorption until they again attach to bone.

Integrins are involved in osteoclast attachment, activation and migration. The most abundant integrin receptor on osteoclasts (e.g., on rat, chicken, mouse and human osteoclasts) is the αvβ3 integrin receptor, which is thought to interact in bone with matrix proteins that contain the RGD sequence. Antibodies to αvβ3 block bone resorption in vitro, indicating that this integrin plays a key role in the resorptive process. There is increasing evidence to suggest that αvβ3 ligands can be used effectively to inhibit osteoclast mediated bone resorption in vivo in mammals.

The current major bone diseases of public concern are osteoporosis, hypercalcemia of malignancy, osteopenia due to bone metastases, periodontal disease, hyperparathyroidism, periarticular erosions in rheumatoid arthritis, Paget's disease, immobilization-induced osteopenia and glucocorticoid-induced osteoporosis. All of these conditions are characterized by bone loss, resulting from an imbalance between bone resorption, i.e. breakdown and bone formation, which continues throughout life at the rate of about 14% per year on the average. However, the rate of bone turnover differs from site to site; for example, it is higher in the trabecular bone of the vertebrae and the alveolar bone in the jaws than in the cortices of the long bones. The potential for bone loss is directly related to turnover and can amount to over 5% per year in vertebrae immediately following menopause, a condition that leads to increased fracture risk.

In the United States, there are currently about 20 million people with detectable fractures of the vertebrae due to osteoporosis. In addition, there are about 250,000 hip fractures per year attributed to osteoporosis. This clinical situation is associated with a 12% mortality rate within the first two years, while 30% of the patients require nursing home care after the fracture. Individuals suffering from all the conditions listed above would benefit from treatment with agents that inhibit bone resorption.

Additionally, αvβ3 ligands have been found to be useful in treating and/or inhibiting restenosis (i.e. recurrence of stenosis after corrective surgery on the heart valve), atherosclerosis, diabetic retinopathy, macular degeneration and angiogenesis (i.e. formation of new blood vessels) and inhibiting viral disease.

Moreover, it has been postulated that the growth of tumors depends on an adequate blood supply, which in turn is dependent on the growth of new vessels into the tumor; thus, inhibition of angiogenesis can cause tumor regression in animal models (Harrison's Principles of Internal Medicine, 1991, 12th ed.). Therefore, αvβ3 antagonists, which inhibit angiogenesis can be useful in the treatment of cancer by inhibiting tumor growth (Brooks et al., Cell, 1994, 79, 1157-1164). Evidence has also been presented suggesting that angiogenesis is a central factor in the initiation and persistence of arthritic disease and that the vascular integrin αvβ3 may be a preferred target in inflammatory arthritis. Therefore, αvβ3 antagonists that inhibit angiogenesis may represent a novel therapeutic approach to the treatment of arthritic disease, such as rheumatoid arthritis (C.M. Storgard, et al., Decreased Angiogenesis and Arthritic Disease in Rabbits Treated'with an αvβ3 Antagonist, J. Clin. Invest., 1999, 103, 47-54).

Inhibition of the αvβ3 integrin receptor can also prevent neovascularization. A monoclonal antibody for αvβ3 has been shown to inhibit VEGF-induced angiogenesis in rabbit cornea and the chick chorioallantoic membrane model (M.C. Friedlander, et al., Science,1995, 270, 1500-1502). Thus, αvβ3 antagonists are useful for treating and preventing macular degeneration, diabetic retinopathy, cancer and metastatic tumor growth.

Inhibition of αv integrin receptors can also prevent angiogenesis and inflammation by acting as antagonists of other β subunits, such as αvβ3 and αvβ3 (Melpo Christofidou-Solomidou, et al., Expression and Function of Endothelial Cell on Integrin Receptors in Wound-Induced Human Angiogenesis in Human Skin/SCID 25 Mice Chimeras, America Journal of Pathology, 1997, 151, 975-83; and, Xiao-Zhu Huang, et al., Inactivation of the Integrin β6 Subunit Gene Reveals a Role of Epithelial Integrins in Regulating Inflamnation in the Lungs and Skin, Journal of Cell Biology, 1996, 133, 921-28).

An antagonist to the αv integrin can act to inhibit or minimize adhesions that result from either wounding or surgical adhesions. Post-surgical adhesions result as an anomaly of the wound healing process. Cell adhesion and the migration of fibroblasts are major players in this process. Trauma caused by the wounding, a surgical procedure, normal tissue manipulation in surgery, or bleeding during a surgical procedure can act to disrupt the peritoneum and expose the underlying stroma leading to the release of inflammatory mediators and an increase in capillary permeability. Inflammatory cells are subsequently liberated and the formation of a fibrin clot ensues. Adhesions are formed and intensify as fibroblasts and inflammatory cells continue to infiltrate this extracellular matrix rich in fibrin. The extracellular matrix is composed of adhesive proteins which act as ligands for the αv integrin. To inhibit post-surgical adhesion development, application of an αv antagonist could be parenteral, subcutaneous, intravenous, oral, topical or transdermal. The av integrin antagonist can be administered before, during or after a surgical procedure. When administered during a surgical procedure the antagonists can be administered by aerosol, in a pad, gel, film, sponge, solution, suspension or similar suitable pharmaceutically acceptable carrier to the area in which surgery is performed.

An aspect of the disclosure is a composition or medicament comprising a pharmaceutically appropriate carrier and any of the compounds of the present disclosure. Illustrative of the disclosure is a composition or medicament made by mixing an instant compound and a pharmaceutically appropriate carrier. Another illustration of the disclosure is a process for making a composition or medicament comprising mixing any of the compounds described above and a pharmaceutically appropriate carrier. Further illustrative of the present disclosure are composition or medicaments comprising one or more compounds of this disclosure in association with a pharmaceutically appropriate carrier.

As used herein, the term "composition" is intended to encompass a product comprising the specified ingredients in the specified amounts, as well as any product which results, directly or indirectly, from combinations of the specified ingredients in the specified amounts for treating or ameliorating an αv integrin mediated disorder or for use as a medicament.

The compounds of the present disclosure are αv integrin inhibitors useful for treating or ameliorating an αv integrin mediated disorder. An aspect of the disclosure includes compounds that are selective inhibitors of an αv integrin receptor, or subtype thereof. In another aspect of the disclosure, the inhibitor is independently selective to the αvβ3 integrin receptor or the αvβ3 integrin receptor. An aspect of the disclosure also includes compounds that are inhibitors of a combination of αv integrin receptors, or subtypes thereof. In another aspect of the disclosure, the compounds inhibitor simultaneously antagonizes both the αvβ3 integrin and the αvβ3 integrin receptor subtypes.

An aspect of the present disclosure includes a method for treating or ameliorating an αv integrin mediated disorder in a subject in need thereof comprising administering to the subject a therapeutically effective amount of a compound of Formula (I) or composition thereof.

The term "therapeutically effective amount" or "effective amount," as used herein, means that amount of active compound or pharmaceutical agent that elicits the biological or medicinal response in a tissue system, animal or human, that is being sought by a researcher; veterinarian, medical doctor, or other clinician, which includes alleviation of the symptoms of the disease or disorder being treated.

An aspect of the present disclosure includes a prophylactic method for preventing an αv integrin mediated disorder in a subject in need thereof comprising administering to the subject a prophylactically effective amount of a compound of Formula (I) or composition thereof.

Another aspect of the present disclosure includes the preparation of a medicament comprising a therapeutically effective amount of a compound of Formula (I) for use in preventing, treating or ameliorating an αv integrin mediated disorder in a subject in need thereof.

The term "administering" is to be interpreted in accordance with the methods of the present disclosure whereby an individual compound of the present disclosure or a composition thereof can be therapeutically administered separately at different times during the course of therapy or concurrently in divided or single combination forms. Prophylactic administration can occur prior to the manifestation of symptoms characteristic of an αv integrin mediated disease or disorder such that the disease or disorder is prevented or, alternatively, delayed in its progression. The instant disclosure is therefore to be understood as embracing all such regimes of simultaneous or alternating therapeutic or prophylatic treatment.

The term "subject" as used herein, refers to an animal, preferably a mammal, most preferably a human, which has been the object of treatment, observation or experiment and is at risk of (or susceptible to) developing a disease or disorder or having a disease or disorder related to expression of an αv integrin, or subtype thereof.

The term "αv integrin mediated disorder" refers to disorders and diseases associated with pathological unregulated or disregulated cell proliferation resulting from expression of an αv integrin, or subtype thereof.

The term "unregulated" refers to a breakdown in the process of regulating cell proliferation, as in a tumor cell. The term "disregulated" refers to inappropriate cell growth as a result of pathogenesis. The term "subtype" refers to a particular αv integrin receptor selected from those receptors making up the class of αv integrins, such as an αvβ3 integrin receptor or an αvβ5 integrin receptor.

The term "disorders and diseases associated with unregulated or disregulated cell proliferation" refers to disorders wherein cell proliferation by one or more subset of cells in a multicellular organism results in harm (such as discomfort or decreased life expectancy) to the organism. Such disorders can occur in different types of animals and humans and include, and are not limited to, cancers, cancer-associated pathologies, atherosclerosis, transplantation-induced vasculopathies, neointima formation, papilloma, lung fibrosis, pulmonary fibrosis, glomerulonephritis, glomerulosclerosis, congenital multicystic renal dysplasia, kidney fibrosis, diabetic retinopathy, macular degeneration, psoriasis, osteoporosis, bone resorption, inflammatory arthritis, rheumatoid arthritis, restenosis or adhesions.

The term "cancers" refers to, and is not limited to, glioma cancers, lung cancers, breast cancers, colorectal cancers, prostate cancers, gastric cancers, esophageal cancers, leukemias, melanomas, basal cell carcinomas and lymphomas. The term "cancer-associated pathologies" refers to, and is not limited to, unregulated or disregulated cell proliferation, tumor growth, tumor vascularization, angiopathy and angiogenesis. The term "angiogenesis" refers to, and is not limited to, unregulated or disregulated proliferation of new vascular tissue including, but not limited to, endothelial cells, vascular smooth muscle cells, pericytes and fibroblasts. The term "osteoporosis" refers to, and is not limited to, formation or activity of osteoclasts resulting in bone resorption. The term "restenosis" refers to, and is not limited to, in-stent stenosis and vascular graft restenosis.

The term "αv integrin expression" refers to expression of an αv integrin, or subtypes thereof, which leads to unregulated or disregulated cell proliferation:
1. by cells which do not normally express an αv integrin, or subtype thereof,
2. by neoplastic cells,
3. in response to stimulation by a growth factor, hypoxia, neoplasia or a disease process,
4. as a result of mutations which lead to constitutive expression of an αv integrin, or subtype thereof

The expression of an αv integrin, or subtype thereof, includes selective expression of an αv integrin or subtype thereof, selective expression of the αvβ3 integrin or the αvβ5 integrin subtypes, expression of multiple αv integrin subtypes or simultaneous expression of the asp3 integrin and the αvβ5 integrin subtypes. Detecting the expression of an αv integrin, or subtype thereof, in inappropriate or abnormal levels is determined by procedures well know in the art.

Another aspect of the present disclosure includes a method for treating or ameliorating a selective αvβ3 integrin mediated disorder in a subject in need thereof comprising administering to the subject a therapeutically effective amount of a compound of Formula (1) or composition thereof.

Another aspect of the present disclosure includes a method for treating or ameliorating a selective αvβ5 integrin mediated disorder in a subject in need thereof comprising administering to the subject a therapeutically effective amount of a compound of Formula (I) or composition thereof.

Another aspect of the present disclosure includes a method for treating or ameliorating a disorder simultaneously mediated by an αvβ3 and αvβ5 integrin in a subject in need thereof comprising administering to the subject a therapeutically effective amount of a compound of Formula (I) or composition thereof.

An aspect of the present disclosure includes a method for inhibiting αv integrin mediated neoplastic activity comprising administering to a neoplasm or to the microenvironment around the neoplasm an effective amount of a compound of Formula (I) or composition thereof.

The term "neoplastic activity" refers to unregulated or disregulated cell proliferation and the process of angiogenesis or the formation of new vasculature supporting a neoplasm in the endothelial microenvironment around the neoplasm.

The term "neoplasm" refers to tumor cells are cells having unregulated or disregulated proliferation as a result of genetic instability or mutation and an endothelium wherein the endothelial cells have unregulated or disregulated proliferation as a result of a pathogenic condition. Within the scope of the present disclosure, a neoplasm is not required to express the αv integrin, or subtype thereof, by itself and is not limited to a primary tumor of origin but also to secondary tumors occurring as a result of metastasis of the primary tumor. The term "administering to a neoplasm" refers to administering a compound of Formula (I) or composition thereof to the surface of a neoplasm, to the surface of a neoplastic cell or to the endothelial microenvironment around a neoplasm.

The term "inhibiting αv integrin mediated neoplastic activity" includes attenuating a tumor's growth by limiting its blood supply and, further, preventing the formation of new supportive vasculature by preventing the process of angiogenesis.

An aspect of the present disclosure includes a method for treating or ameliorating a disease mediated by cells pathologically expressing an αv integrin, or subtype thereof.

The term "disease mediated by cells pathologically expressing an αv integrin" refers to, and is not limited to, a disorders selected from cancers, cancer-associated pathologies, diabetic retinopathy, macular degeneration, osteoporosis, bone resorption, inflammatory arthritis, rheumatoid arthritis or restenosis.

An aspect of the present disclosure includes a method for sustained neoplasm regression in a subject in need thereof comprising administering to the subject an effective amount of a compound of Formula (I) of composition thereof; wherein the compound or composition thereof is conjugated with and delivers a therapeutic agent to to a neoplasm or to the microenvironment around the neoplasm; and, wherein the therapeutic agent induces apoptosis or attenuates unregulated or disregulated cell proliferation.

The terms "conjugated with" and "delivers a therapeutic agent" refers to a compound of Formula (I) or composition thereof bound to a therapeutic agent by a conjugation means known to those skilled in the art; wherein the compound or composition thereof acts as a targeting agent for antagonizing the αv integrin receptors of a neoplasm or the microenvironment thereof; and, wherein the conjugation means facilitates and selectively delivers the therapeutic agent to the neoplasm or the microenvironment thereof.

The term "therapeutic agent," including but not limited to Technetium⁹⁹, refers to imaging agents known to those skilled in the art.

An aspect of the present disclosure includes a method for use of a compound of Formula (I) or composition thereof advantageously co administered in one or more tumor or cell anti-proliferation therapies including chemotherapy, radiation therapy, gene therapy or immunotherapy for preventing, treating or ameliorating an αv integrin mediated disorder.

The combination therapy can include:
1. co-administration of a compound of Formula (I) or composition thereof and a chemotherapeutic agent for preventing, treating or ameliorating an αv integrin mediated disorder,
2. sequential administration of a compound of Formula (I) or composition thereof and a chemotherapeutic agent for preventing, treating or ameliorating an αv integrin mediated disorder,
3. administration of a composition containing a compound of Formula (I) and a chemotherapeutic agent for preventing, treating or ameliorating an αv integrin mediated disorder, or,
4. simultaneous administration of a separate composition containing a compound of Formula (I) and a separate composition containing a chemotherapeutic agent for preventing, treating or ameliorating an αv integrin mediated disorder.

For example, the compounds of this disclosure are useful in combination therapies with at least one other chemotherapeutic agent for the treatment of a number of different cancers and advantageously appear to facilitate the use of a reduced dose of the chemotherapeutic agent that is recommended for a particular cancer or cell proliferation disorder. Therefore, it is contemplated that the compounds of this disclosure can be used in a treatment regime before the administration of a particular chemotherapeutic agent recommended for the treatment of a particular cancer, during administration of the chemotherapeutic agent or after treatment with a particular chemotherapeutic agent.

The term "chemotherapeutic agents" includes, and is not limited to, anti-angiogenic agents, anti-tumor agents, cytotoxic agents, inhibitors of cell proliferation and the like. The term "treating or ameliorating" includes, and is not limited to, facilitating the eradication of, inhibiting the progression of or promoting stasis of a malignancy. For example, an inhibitor compound of the present disclosure, acting as an anti-angiogenic agent can be administered in a dosing regimen with at least one other cytotoxic compound, such as a DNA alkylating agent.

Preferred anti-tumor agents are selected from the group consisting of cladribine (2-chloro-2'-deoxy-(beta)-D-adenosine), chlorambucil (4-(bis(2-chlorethyl)amino)benzenebutanoic acid), DTIC-Dome (5-(3,3-dimethyl-1-triazeno)-imidazole-4-carboxamide), platinum chemotherapeutics and nonplatinum chemotherapeutics. Platinum containing anti-tumor agents include, and are not limited to, cisplatin (CDDP) (cis-dichlorodiamineplatinum). Non-platinum containing anti-tumor agents include, and are not limited to, adriamycin (doxorubicin), aminopterin, bleomycin, camptothecin, carminomycin, combretastatin(s), cyclophosphamide, cytosine arabinoside, dactinomycin, daunomycin, epirubicin, etoposide (VP-16), 5-fluorouracil (5FU), herceptin actinomycin-D, methotrexate, mitomycin C, tamoxifen, taxol, taxotere, thiotepa, vinblastine, vincristine, vinorelbine and derivatives and prodrugs thereof. Each anti-tumor agent is administered in a therapeutically effective amount, which varies based on the agent used, the type of malignancy to be treated or ameliorated and other conditions according to methods well known in the art.

As will be understood by those skilled in the art, the appropriate doses of chemotherapeutic agents will be generally around those already employed in clinical therapies wherein the chemotherapeutics are administered alone or in combination with other chemotherapeutics. By way of example only, agents such as cisplatin and other DNA alkylating are used widely to treat cancer. The efficacious dose of cisplatin used in clinical applications is about 20 mg/m² for 5 days every three weeks for a total of three courses. Cisplatin is not absorbed orally and must therefore be delivered via injection intravenously, subcutaneously, intratumorally or intraperitoneally. Further useful agents include compounds that interfere with DNA replication, mitosis and chromosomal segregation. Such chemotherapeutic agents include adriamycin (doxorubicin), etoposide, verapamil or podophyllotoxin and the like and are widely used in clinical settings for tumor treatment, These compounds are administered through bolus injections intravenously at doses ranging from about 25 to about 75 mg/m² at 21 day intervals (for adriamycin) or from about 35 to about 50 mg/m² (for etoposide) intravenously or at double the intravenous dose orally. Agents that disrupt the synthesis and fidelity of polynucleotide precursors such as 5-fluorouracil (5-FU) are preferentially used to target tumors. Although quite toxic, 5-FU is commonly used via intravenous administration with doses ranging from about 3 to about 15 mg/kg/day.

Another aspect of the present disclosure includes a method for administering a compound of the present disclosure in combination with radiation therapy. As used herein, "radiation therapy" refers to a therapy that comprises exposing the subject in need thereof to radiation. Such therapy is known to those skilled in the art. The appropriate scheme of radiation therapy will be similar to those already employed in clinical therapies wherein the radiation therapy is used alone or in combination with other chemotherapeutics.

An aspect of the present disclosure includes a method for administering a compound of the present disclosure in combination, with a gene therapy or for use of a compound of the present disclosure as a gene therapy means. The term "gene therapy" refers to a therapy targeting angiogenic endothelial cells or tumor tissue during tumor development. Gene therapy strategies include the restoration of defective cancer-inhibitory genes, cell transduction or transfection with-antisense DNA (corresponding to genes coding for growth factors and their receptors) and the use of "suicide genes." The term "gene therapy means" refers to the use of a targeting vector comprising a combination of a cationic nanoparticle coupled to an αv-targeting ligand to influence blood vessel biology; whereby genes are selectively delivered to angiogenic blood vessels (as described in Hood, J.D., et al, Tumor Regression by Targeted Gene Delivery to the Neovasculature, Science, 2002, 28 June, 296, 2404-2407).

Another aspect of the present disclosure includes a method for treating or ameliorating an αv integrin mediated neoplasm in a subject in need thereof comprising administering to the subject an effective amount of a gene therapy combination product comprising a compound of Formula (I) or composition thereof and a gene therapeutic agent; wherein the product is delivered or "seeded" directly to a neoplasm or the microenvironment thereof by antagonizing the αv integrin receptors of the neoplasm or microenvironment thereof.

The term "delivered or 'seeded' directly to a neoplasm" includes using a compound of Formula (I) or composition thereof as a gene therapy means whereby the compound or composition thereof functions as a targeting agent which directs the conjugate to its intended site of action (i.e., to neoplastic vascular endothelial cells or to tumor cells). Because of the specific interaction of the αv integrin inhibitor as a targeting agent and its corresponding av integrin receptor site, a compound of this disclosure can be administered with high local concentrations at or near a targeted αv integrin receptor, or subtype thereof, thus treating the αv integrin mediated disorders more effectively.

Another aspect of the present disclosure includes a method for administering a compound of the present disclosure in combination with an immunotherapy. As used herein, "immunotherapy" refers to a therapy targeted to a particular protein involved in tumor development via antibodies specific to such protein. For example, monoclonal antibodies against vascular endothelial growth factor have been used in treating cancers.

An aspect of the present disclosure includes a method for tumor imaging in a subject in need thereof comprising advantageously coadministering to the subject an effective amount of compound of Formula (I) or composition thereof; wherein the compound or composition thereof is conjugated with and delivers a non-invasive tumor imaging agent to a tumor or to the microenvironment around the tumor.

The terms "conjugated with" and "delivers a non-invasive tumor imaging agent" refers to a compound of Formula (I) of composition thereof bound to an imaging agent by a conjugation means known to those skilled in the art; wherein the compound or composition thereof acts as a targeting agent for antagonizing the αv integrin receptors of a neoplasm or the microenvironment thereof; and, wherein the conjugation means facilitates and selectively delivers the imaging agent to the neoplasm or the microenvironment thereof (as described in PCT Application WO00/35887, WO00/35492, WO00/35488 or WO99/58162). The term "imaging agent," including but not limited to Technetium⁹⁹, refers to imaging agents known to those skilled in the art. The term "conjugation means," including but not limited to appending a compound to a linking group followed by conjugation with an imaging agent chelating group, refers to means known to those skilled in the art.

Coronary angioplasty is a highly effective procedure used to reduce the severity of coronary occlusion; however, its long-term success is limited by a high rate of restenosis. Vascular smooth muscle cell activation, migration and proliferation is largely responsible for restenosis following angioplasty (Ross, R., Nature, 1993, 362, 801-809).

An aspect of the present disclosure includes a method for use of αv integrin inhibitor compound of Formula (I) or composition thereof for treating or ameliorating arterial and venous restenosis; wherein the compound is impregnated on the surface of a therapeutic device. The term "therapeutic device" refers to, and is not limited to, an angioplasty balloon, arterial stent, venous stent, suture, artificial joint, implanted prosthesis or other like medical devices, thus targeting drug delivery to a neoplasm.

An aspect of the present disclosure includes a composition comprising a compound of Formula (I), or pharmaceutically acceptable salt thereof, in association with a pharmaceutically acceptable carrier. Compositions contemplated within this disclosure can be prepared according to conventional pharmaceutical techniques. A pharmaceutically acceptable carrier may also (but need not necessarily) be used in the composition of the disclosure.

The term "pharmaceutically acceptable" refers to molecular entities and compositions that do not produce an adverse, allergic or other untoward reaction when administered to an animal, or a human, as appropriate. Veterinary uses are equally included within the disclosure and "pharmaceutically acceptable" formulations include formulations for both clinical and/or veterinary use.

The composition may take a wide variety of forms depending on the form of preparation desired for administration including, but not limited to, intravenous (both bolus and infusion), oral, nasal, transdermal, topical with or without occlusion, and injection intraperitoneally, subcutaneously, intramuscularly, intratumorally or parenterally, all using forms well known to those of ordinary skill in the pharmaceutical arts. The composition may comprise a dosage unit such as a tablet, pill, capsule, powder, granule, sterile parenteral solution or suspension, metered aerosol or liquid spray, drop, ampoule, auto-injector device or suppository; for administration orally, parenterally, intranasally, sublingually or rectally or by inhalation or insufflation. Compositions suitable for oral administration include solid forms such as pills, tablets, caplets, capsules (each including immediate release, timed release and sustained release formulations), granules and powders; and, liquid forms such as solutions, syrups, elixirs, emulsions and suspensions. Forms useful for parenteral administration include sterile solutions, emulsions and suspensions. Alternatively, the composition may be presented in a form suitable for once-weekly or once-monthly administration; for example, an insoluble salt of the active compound, such as the decanoate salt, may be adapted to provide a depot preparation for intramuscular, injection. In preparing the compositions in oral dosage form, one or more of the usual pharmaceutical carriers may be employed, including necessary and inert pharmaceutical excipients, such as water, glycols, oils, alcohols, flavoring agents, preservatives, coloring agents, syrup and the like; in the case of oral liquid preparations, carriers such as starches, sugars, diluents, granulating agents, lubricants, binders, disintegrating agents and the like may be employed.

The dosage unit (tablet, capsule, powder, injection, suppository, measured liquid dosage and the like) containing the pharmaceutical compositions herein will contain an amount of the active ingredient necessary to deliver a therapeutically effective amount as described above. The composition may contain from about 0.001 mg to about 5000 mg of the active compound or prodrug thereof and may be constituted into any form suitable for the mode of administration selected for a subject in need.

An aspect of the present disclosure contemplates a therapeutically effective amount in a range of from about 0.001 mg to 1000 mg/kg of body weight per day. Another aspect of the present disclosure includes a range of from about 0.001 to about 500 mg/kg of body weight per day. A further aspect of the present disclosure includes a range of from about 0.001 to about 300 mg/kg of body weight per day. The compounds may be administered according to a dosage regimen of from about 1 to about 5 times per day and still more preferably 1, 2 or 3 times a day.

For oral administration, the compositions are preferably provided in the form of tablets containing, 0.01,0.05, 0.1, 0.5, 1.0, 2.5, 5.0, 10.0, 15.0, 25.0, 50.0, 100,150, 200, 250 and 500 milligrams of the active ingredient for the symptomatic adjustment of the dosage to the patient to be treated. Optimal dosages to be administered may be readily determined by those skilled in the art and will vary depending factors associated with the particular patient being treated (age, weight, diet and time of administration), the severity of the condition being treated, the compound being employed, the mode of administration and the strength of the preparation. The use of either daily administration or post-periodic dosing may be employed.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums and other pharmaceutical diluents, e.g. water, to form a solid preformulation composition containing a homogeneous mixture of a compound of the present disclosure, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.001 to about 5000 mg of the active ingredient of the present disclosure. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of material can be used for such enteric layers or coatings, such materials including a number of polymeric acids with such materials as shellac, acetyl alcohol and cellulose acetate.

For oral administration in the form of a tablet or capsule, the active drug component can be optionally combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in which the compound of formula (I) may be incorporated for administration orally or by injection include, aqueous solutions, suitably flavored syrups, aqueous or oil suspensions and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin. The liquid forms in suitably flavored suspending or dispersing agents may also include the synthetic and natural gums, for example, tragacanth, acacia, methyl-cellulose and the like. For parenteral administration, sterile suspensions and solutions are desired. Isotonic preparations that generally contain suitable preservatives are employed when intravenous administration is desired.

As is also known in the art, the compounds may alternatively be administered parenterally via injection of a formulation consisting of the active ingredient dissolved in an inert liquid carrier. The injectable formulation can include the active ingredient mixed with an appropriate inert liquid carrier. Acceptable liquid carriers include vegetable oils such as peanut oil, cottonseed oil, sesame oil and the like, as well as organic solvents such as solketal, glycerol and the like. As an alternative, aqueous parenteral formulations may also be used. For example, acceptable aqueous solvents include water, Ringer's solution and an isotonic aqueous saline solution. Further, a sterile non-volatile oil can usually be employed was a solvent or suspending agent in the aqueous formulation. The formulations are prepared by dissolving or suspending the active ingredient in the liquid carrier such that the final formulation contains from 0.005 to 10% by weight of the active ingredient. Other additives including a preservative, an isotonizer, a solubilizer, a stabilizer and a pain-soothing agent may adequately be employed.

Advantageously, compounds of Formula (I) may be administered in a single daily dose, or the total daily dosage may be administered in divided doses of two, three or four times daily. Furthermore, compounds of the present disclosure can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

Because of their ease of administration, tablets and capsules represent ah advantageous oral dosage unit form, wherein solid pharmaceutical carriers are employed. If desired, tablets may be sugarcoated or enteric-coated by standard techniques. If desired, tablets may be sugar coated or enteric coated by standard techniques. For parenterals, the carrier will usually comprise sterile water, though other ingredients, for example, for purposes such as aiding solubility or for preservation, may be included. Injectable suspensions may also be prepared, in which case appropriate liquid carriers, suspending agents and the like may be employed.

The compositions of the present disclosure also include a composition for slow release of the compound of the disclosure. The composition includes a slow release carrier (typically, a polymeric carrier) and a compound of the disclosure. In preparation for slow release, a slow release carrier, typically a polymeric carrier and a compound of the disclosure are first dissolved or dispersed in an organic solvent. The obtained organic solution is then added into an aqueous solution to obtain an oil-in-water-type emulsion. Preferably, the aqueous solution includes surface-active agent(s). Subsequently, the organic solvent is evaporated from the oil-in-water-type emulsion to obtain a colloidal suspension of particles containing the slow release carrier and the compound of the disclosure. Slow release biodegradable carriers are also well known in the art. These are materials that may form particles that capture therein an active compound(s) and slowly degrade/dissolve under a suitable environment (e.g., aqueous, acidic, basic, etc) and thereby degrade/dissolve in body fluids and release the active compound(s) therein. The particles are preferably nanoparticles (i.e., in the range of about 1 to 500 nm in diameter, preferably about 50-200 nm in diameter and most preferably about 100 nm in diameter).

The present disclosure also provides methods to prepare the pharmaceutical compositions of this disclosure. A compound of Formula (I) as the active ingredient is intimately admixed with a pharmaceutical carrier according to conventional pharmaceutical compounding techniques, which carrier may take a wide variety of forms depending on the form of preparation desired for administration. In preparing the compositions in oral dosage form, any of the usual pharmaceutical media may be employed. For solid oral dosage forms, suitable carriers and additives include starches, sugars, diluents, granulating agents, lubricants, binders, disintegrating agents and the like. For liquid oral preparations, suitable carriers and additives include water, glycols, , oils, alcohols, flavoring agents, preservatives, coloring agents and the like. Additionally, liquid forms of the active drug component can be combined in suitably flavored suspending or dispersing agents such as the synthetic and natural gums, including for example, tragacanth, acacia, methyl-cellulose and the like. Other dispersing agents that may be employed include glycerin and the like.

An antibody targeting agent includes antibodies or antigen-binding fragment thereof, that bind to a targetable or accessible component of a tumor cell, tumor vasculature or tumor stroma. The "targetable or accessible component" of a tumor cell, tumor vasculature or tumor stroma, is preferably a surface-expressed, surface-accessible or surface-localized component. The antibody targeting agents also include antibodies or antigen-binding fragments thereof, that bind to an intracellular component that is released from a necrotic tumor cell. Preferably such antibodies are monoclonal antibodies or antigen-binding fragments thereof that bind to insoluble intracellular antigen(s) present in cells that may be induced to be permeable or in cell ghosts of substantially all tumor or nominal cells, but are not present or accessible on the exterior of normal living cells of a mammal.

As used herein, the term "antibody" is intended to refer broadly to any immunologic binding agent such as IgG, IgM, IgA, IgE, F(ab')2, a univalent fragment such as Fab', Fab, Dab, as well as engineered antibodies such as recombinant antibodies, humanized antibodies, bispecific antibodies and the like. The antibody can be either the polyclonal or the monoclonal, although a monoclonal antibody is preferred. There is a very broad array of antibodies known in the art that have immunological specificity for the cell surface of virtually any solid tumor type (see a Summary Table on monoclonal antibodies for solid tumors in U.S. patent 5,855,866, Thorpe, et al). Methods are known to those skilled in the art to produce and isolate antibodies to be used as targeting agents against tumors (U.S. patent 5,855,866, Thorpe); and, U.S. patent 6,342,219 (Thorpe)).

Non-antibody targeting agents include growth factors that bind specifically to the tumor vasculature and other targeting components such as annexins and related ligands. In addition, a variety of other organic molecules can also be used as targeting agents for tumors, examples are hyaluronan oligosaccharides which specifically recognize Hyaluronan-binding protein, a cell surface protein expressed during tumor cell and endothelial cell migration and during capillary-like tubule formation (U.S. Patent 5,902,795 (Toole, et al.)) and polyanionic compounds, particularly polysulphated or polysulphonated compounds such as N- and O-sulfated polyanionic polysaccharides, polystyrene sulfonate and other polyanionic compounds (as described in U.S. Patent 5,762,918 (Thorpe) which selectively bind to vascular endothelial cells.

Techniques for conjugating a therapeutic moiety to antibodies are well known (Amon, et al., Monoclonal Antibodies For Immunotargeting Of Drugs In Cancer Therapy, Monoclonal Antibodies And Cancer Therapy, Reisfeld, et al. (eds.), pp. 243-56 (Alan R. Liss, Inc. 1985); Hellstrom, et al., Antibodies For Drug Delivery, Controlled Drug Delivery (2nd Ed.), Robinson, et al. (eds.), pp. 623-53 (Marcel Dekker, Inc. 1987); Thorpe, Antibody Carriers Of Cytotoxic Agents In Cancer Therapy: A Review, Monoclonal Antibodies '84: Biological And Clinical Applications, Pinchera, et al. (eds.), pp. 475-506 (1985**)**. Similar techniques can also be applied to attach compounds of the disclosure to non-antibody targeting agents. Those skilled in the art will know or be able to select methods in the art for forming conjugates with non-antibody targeting agents, such as oligopeptides, polysaccharides or other polyanionic compounds.

Although any linking moiety that is reasonably stable in blood can be used to link the compound of the disclosure to the targeting agent, those with biologically-releasable bonds and/or selectively cleavable spacers or linkers are preferred. "Biologically-releasable bonds" and "selectively cleavable spacers or linkers" refers to those linking moieties which have reasonable stability in the circulation and are releasable, cleavable or hydrolyzable only or preferentially under certain conditions, (i.e., within a certain environment or in contact with a particular agent). Such bonds include, for example, disulfide and trisulfide bonds and acid-labile bonds (as described in U.S. Patent 5,474,765 and 5,762,918) and enzyme-sensitive bonds, including peptide bonds, esters, amides, phosphodiesters and glycosides (as described in U.S. Patent 5,474,765 and 5,762,918). Such selective-release design features facilitate sustained release of the compounds from the conjugates at the intended target site:

The therapeutically effective amount of a compound of the disclosure conjugated to a targeting agent depends on the individual, the disease type, the disease state, the method of administration and other clinical variables. The effective amount is readily determinable using data from an animal model. Experimental animals bearing solid tumors are frequently used to optimize appropriate therapeutically effective amounts prior to translating to a clinical environment. Such models are known to be very reliable in predicting effective anti-cancer strategies. For example, mice bearing solid tumors are widely used in pre-clinical testing to determine working ranges of therapeutic agents that give beneficial anti-tumor effects with minimal toxicity.

The present disclosure further provides a composition that comprises an effective amount of the compound of the disclosure conjugated to a targeting agent and a pharmaceutically acceptable carrier. When proteins such as antibodies or growth factors, or polysaccharides are used as targeting agents, they are preferably administered in the form of injectable compositions. The injectable antibody solution will be administered into a vein, artery or into the spinal fluid over the course of from about 2 minutes to about 45 minutes, preferably from about 10 to about 20 minutes. In certain cases, intradermal and intracavitary administration are advantageous for tumors restricted to areas close to particular regions of the skin and/or to particular body cavities. In addition, intrathecal administrations may be used for tumors located in the brain.

Another aspect of the present disclosure includes a method for treating or disorders related to αv integrin expression (in particular, restenosis, intimal hyperplasia or inflammation in vessel walls) in a subject in need thereof comprising administering to the subject by controlled delivery a therapeutically effective amount of a compound of Formula (I) or composition thereof coated onto an intraluminal medical device (in particular, a balloon-catheter or stent). Such devices are useful to prevent the occurrence of restenosis by inhibiting αv integrin activity and thus preventing hyperproliferation of the endothelium.

The term "intraluminal medical device" refers to any delivery device, such as intravascular drug delivery catheters, wires, pharmacological stents and endoluminal paving. The scope of the present disclosure includes delivery devices comprising an arterial or venous stent having a coating or sheath which elutes or releases a therapeutically effective amount of an instant compound. The term "controlled delivery" refers to the release of active ingredient in a site-directed and time dependent manner. Alternatively, the delivery system for such a device may comprise a local infusion catheter that delivers the compound at a variably controlled rate.

The term "stent" refers to any device capable of being delivered by a catheter.

A stent is routinely used to prevent vascular closure due to physical anomalies such as unwanted inward growth of vascular tissue due to surgical trauma. A stent often has a tubular, expanding lattice-type structure appropriate to be left inside the lumen of a duct to relieve an obstruction. The stent has a lumen wall-contacting surface and a lumen-exposed surface. The lumen-wall contacting surface is the outside surface of the tube and the lumen-exposed surface is the inner surface of the tube. The stent material may be a polymeric, metallic or a combination polymeric-metallic material and can be optionally biodegradable.

Commonly, a stent is inserted into the lumen in a non-expanded form and are the expanded autonomously, or with the aid of a second device in situ. A typical method of expansion occurs through the use of a catheter-mounted angioplastry balloon which is inflated within the stenosed vessel or body passageway in order to shear and disrupt the obstructions associated with the wall components of the vessel and to obtain an enlarged lumen. Self-expanding stents as described in pending U.S. Patent application 2002/0016625 A1 (Falotico, et al.) may also be utilized. The combination of a stent with drugs, agents or compounds which prevent inflammation and proliferation may provide the most efficacious treatment for post-angioplastry restenosis.

Compounds of the present disclosure can be incorporated into or affixed to the stent in a number of ways. A solution of the compound of the disclosure and a biocompatible material or polymer may be incorporated into or onto a stent in a number of ways. For example, a solution of an instant compound may be sprayed onto the stent or the stent may be dipped into the solution and, in each case, allowed to dry. Another coating method electrically charges a solution of an instant compound to one polarity and charges the stent to the opposite polarity. In this manner, the solution and stent will be attracted to one another. Another method coats the stent with a solution of an instant compound using supercritical temperature and pressure conditions. Coating the stent using supercritical conditions reduces waste and allows more control over the thickness of the coat may be achieved. The compound is usually only affixed to the outer surface of the stent (the surface which makes contact with the tissue), but for some compounds, the entire stent may be coated.

A combination product comprising a therapeutically effective amount of an instant compound coated on the stent and on or in a layer or layers of a polymer coating wherein the polymer coating controls the release rate of the drug may be used when the effectiveness of the drug is affected. Accordingly, the compound may be released from the stent over a period of at least about 6 months; in another aspect, over a period of about 3 days to about 6 months; and, in another aspect over a period of about 7 to about 30 days. Any number of non-erodible, biocompatible polymeric materials may be used for the polymer coating layer or layers in conjunctipn with the compound of the disclosure.

In one illustration, the compound is directly incorporated into a polymeric matrix, such as the polymer polypyrrole and subsequently coated onto the outer surface of the stent. Essentially, the compound elutes from the matrix by diffusion through the polymer molecules. Stents and methods for coating drugs on stents are discussed in detail in PCT application WO 96/32907. In another aspect, the stent is first coated with as a base layer comprising a solution of the compound, ethylene-co-vinylacetate and polybutylmethacrylate. The stent is then further coated with an outer layer comprising polybutylmethacrylate. The outlayer acts as a diffusion barrier to prevent the compound from eluting too quickly and entering the surrounding tissues. The thickness of the outer layer or topcoat determines the rate at which the compound elutes from the matrix. Stents and methods for coating are discussed in detail in pending U.S. Patent application 2002/0016625 A1.

It is important to note that different polymers may be utilized for different stents. For example, the above-described ethylene-co-vinylacetate and polybutylmethacrylate matrix works well with stainless steel stents. Other polymers may be utilized more effectively with stents formed from other materials, including materials that exhibit superelastic properties such as alloys of nickel and titanium or shape-retentive polymeric materials that "remember" and return to their original shape upon activation at body temperature.

Methods for introducing a stent into a lumen of a body are well know. In an aspect of this disclosure, a compound-coated stent is introduced using a catheter. As will be appreciated by those of ordinary skill in the art, methods will vary slightly based on the location of stent implantation. For coronary stent implantation, the balloon catheter bearing the stent is inserted into the coronary artery and the stent is positioned at the desired site. The balloon is inflated, expanding the stent. As the stent expands; the stent contacts the lumen wall. Once the stent is positioned, the balloon is deflated and removed. The stent remains in place with the lumen-contacting surface bearing the compound directly contacting the lumen wall surface. Stent implantation may be accompanied by anticoagulation therapy as needed.

Optimum conditions for delivery of the compounds for use in the stent of the disclosure may vary with the different local delivery systems used, as well as the properties and concentrations of the compounds used. Conditions that may be optimized include, for example, the concentrations of the compounds, the delivery volume, the delivery rate, the depth of penetration of the vessel wall, the proximal inflation pressure, the amount and size of perforations and the fit of the drug delivery catheter balloon. Conditions may be optimized for inhibition of smooth muscle cell proliferation at the site of injury such that significant arterial blockage due to restenosis does not occur, as measured, for example, by the proliferative ability of the smooth muscle cells or by changes in the vascular resistance or lumen diameter. Optimum conditions can be determined based on data from animal model studies using routine computational methods.

The compounds of the present disclosure can also be administered in the form of liposome delivery systems, such as small unilamellar vesicles, large unilamellar vesicles and multilamellar vesicles. Liposomes containing delivery systems as well known in the art are formed from a variety of phospholipids, such as cholesterol, stearylamine or phosphatidylcholines.

Abbreviations used in the instant specification, particularly the Schemes and Examples, are as follows:
- Boc: *tert*-butoxycarbonyl
- BSA: Bovine Serum Albumen
- Cod: Cyclooctadiene
- d/hr/min/rt: day(s)/hour(s)/minute(s)/room temperature
- DBC: 2,6-Dichlorobenzoylchloride
- DCM: Dichloromethane
- DIEA: Diisopropylethylamine
- DMAP: Dimethylacetamide
- DMAP: Dimethylaminopyridine
- DMF: *N*,*N*-Dimethylformamide
- DMSO: Dimethyl sulfoxide
- EDC: *N*-ethyl-*N*'-dimethylaminopropylcarbodiimide hydrochloride
- Et₂O: Diethyl ether
- EtOAc: Ethyl acetate ,
- EtOH: Ethanol
- HATU: *O*-(7-azabenzotriazol-1-yl)-1,1,3,3-tetramethyluronium Hexafluorophosphate
- HBTU: *O*-Benzotriazol-1-yl-*N,N,N',N*'- tetramethyluronium Hexafluorophosphate
- HCl: Hydrochloric acid
- HOBt: 1-Hydroxybenzotriazole
- HPLC: High Performance Liquid Chromatography
- LDA: lithium diisopropylamide
- LiHMDS: lithium hexamethyldisilylamide
- Me: Methyl
- MeOH: Methanol
- MeCN: Acetonitrile
- NaHMDS: sodium hexamethyldisilylamide
- NaOH: Sodium hydroxide
- ND: Not Determined
- NMM: *N*-Methylmorpholine
- PBS: Phosphate Buffer Solution
- Ph: Phenyl
- RP-HPLC: Reverse Phase High Performance Liquid Chromatography
- rt: Room Temperature
- SDS: Sodium dodecasulfate
- TEA: Triethylamine
- TFA: Trifluoroacetic acid
- THF: Tetrahydrofuran
- Thi: Thienyl
- TMS: Tetramethylsilane
- TFA: Trifluoroacetic acid

### General Synthetic Methods

Representative compounds of the present disclosure can be synthesized in accordance with the general synthetic methods described below and are illustrated more particularly in the schemes that follow. Since the schemes are illustration whereby intermediate and target compounds of the present disclosure may be prepared, the disclosure should not be construed as being limited by the chemical reactions and conditions expressed. Additional representative compounds and stereoisomers, racemic mixtures, diastereomers and enantiomers thereof can be synthesized using the intermediates prepared in accordance with these schemes and other materials, compounds and reagents know to those skilled in the art. All such compounds, stereoisomers, racemic mixtures, diastereomers and enantiomers thereof are intended to be encompassed within the scope of the present disclosure. The preparation of the various starting materials used in the schemes is well within the skill of persons versed in the art.

### Scheme A

Scheme A describes a method for preparing a target compound of Formula (I) (wherein R₁ and W are as previously defined within the scope of the disclosure. Removal of the Boc-protective group from a Rₐ substituted (wherein Rₐ is C₁₋₄alkyl) Compound **A1** was accomplished under acidic conditions (by using an acid such as an acidic mixture of TFA and DCM or an inorganic acid in an appropriate solvent such as dioxane) and resulted in formation of a piperidine Compound **A2.** Coupling of the piperidine Compound **A2** with a carboxylic acid Compound **A3** under standard coupling conditions (by using a mixture of coupling agents such as HOBt/EDC, HOBT/HBTU or isobutyl chloroformate in the presence of a suitable base such as NMM or DIEA) afforded the ester Compound **A4.** Hydrolysis of the ester Compound **A4** under acidic or basic conditions yielded a target compound Formula (I). The individual isomers of Formula (I) can be achieved through the chiral separation of intermediate **A1 - A4,** and elaboration of the chiral intermediates to compounds of Formula (I).

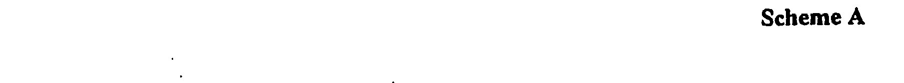

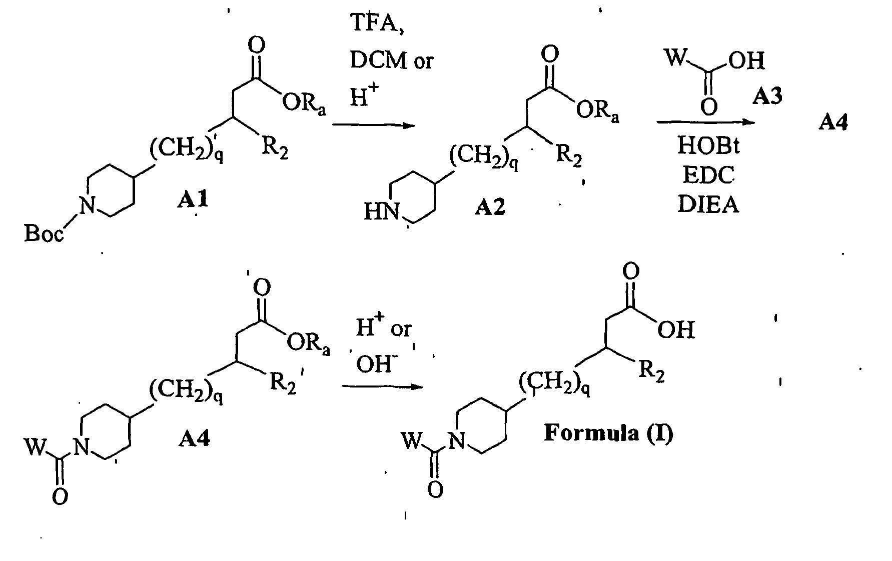

### Scheme B

Scheme B describes an alternative method for preparing a target compound of Formula (I) (wherein R₁ is -NH(R₆) and W is -(CH₂)₀₋₄alkyl-). Condensation of a Compound **A2** with a Compound **B1** (wherein R₁ is H) possessing a suitable leaving group such as a halogen or a mesylate or tosylate under standard coupling conditions (by using a mixture of coupling agents such as HOBt/EDC, HOBT/HBTU or isobutyl chloroformate in the presence of a suitable base such as NMM or DIEA) resulted in the formation of Compound **B2.** Reaction of Compound **B2** with a substituted amine Compound **B3** in the presence of an appropriate base such as LiHMDS, NaHMDS or LDA resulted in the formation of Compound **B4.** Treatment of Compound **B4** with aqueous hydrochloric acid resulted in hydrolysis of the ester to yield a target compound of Formula (I).

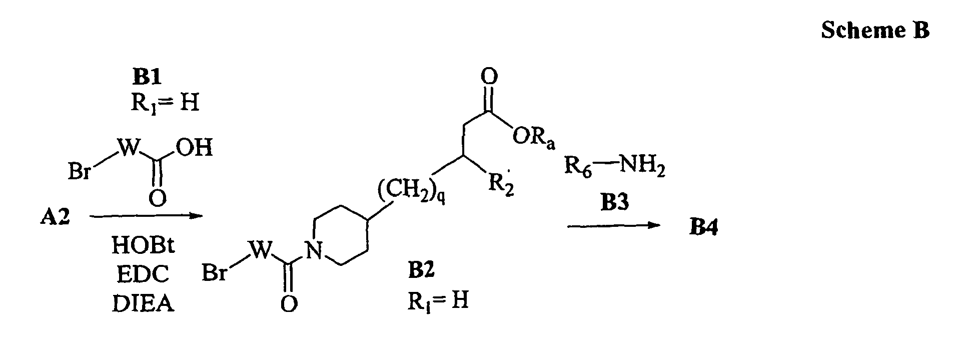

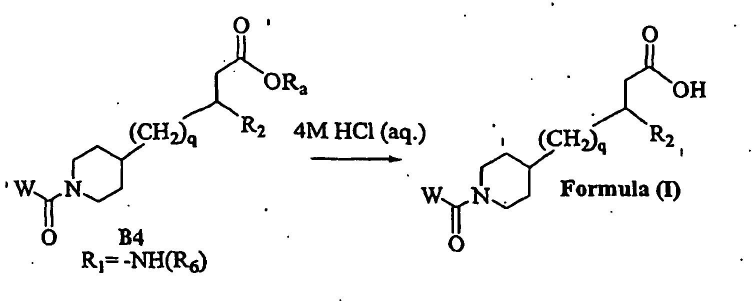

### Scheme C

Schemes C describes an alternative method whereby a Compound **A1** may be prepared. Carboxylic acid Compound **C1** was transformed into an amide Compound **C2** using *N*-methyl-*O*-methylhydroxylamine in the presence of an appropriate activating agent such as HOBt, HBTU, HATU, isobutyl chloroformate or the like. Reaction of the amide Compound **C2** with an *in situ* prepared aryl lithium species, a Grignard reagent or the like resulted in the formation of a ketone Compound **C3.** The ketone Compound **C3** was converted to a mixture of *cis* and *trans* isomers of an α,β-unsaturited ester Compound **C5** upon reaction with an appropriately substituted phosphorane or phosphonate Compound **C4** in the presence of a base such as LiHMDS, NaHMDS, LDA or the like. Conversion of Compound **C5** to Compound **A1** was accomplished under hydrogenolysis conditions (wherein a hydrogen overpressure of from about 69 to about 340 kPa (about 10 to about 50 psi) was used) in the presence of an appropriate catalyst such as 5 or 10% palladium on carbon.

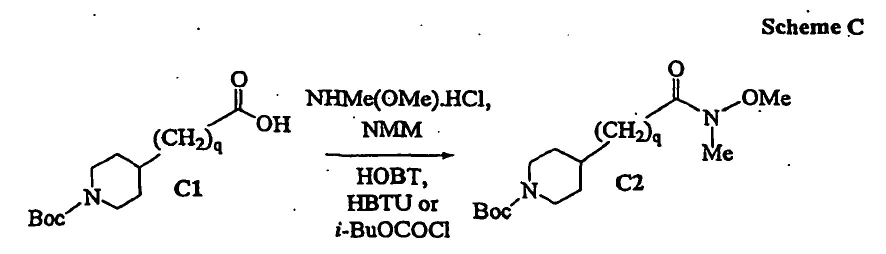

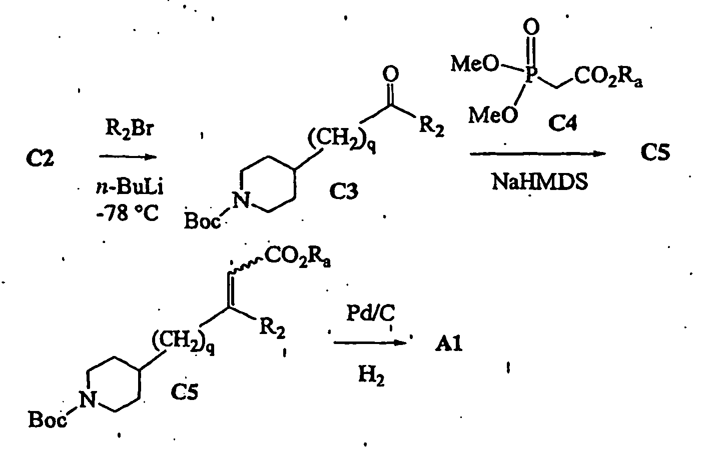

### Scheme D

Scheme D describes an alternative method for the synthesis of a Compound **A1** in which (CH₂)_{q} is (CH₂)₂₋₃. Reaction of an amide Compound **C2** with an appropriate reducing agent such as lithium aluminum hydride or the like resulted in the formation of an aldehyde Compound **D1.** Condensation of an *in situ* generated acetylide Compound **D2** with the aldehyde Compound **D1** at a low temperature resulted in formation of a propargylic alcohol Compound **D3.** The alkyne Compound **D3** was selectively reduced to a *cis*-olefin Compound **D4** under hydrogenolysis conditions using Lindlar's catalyst in pyridine. Condensation of the allylic alcohol Compound **D4** with an Rₐ substituted 3-chloro-3-oxopropionate Compound **D5** in the presence of a base such as TEA, DIEA or the like resulted in the formation of a mixed ester Compound **D6.** Treatment of Compound **D6** with chlorotrimethylsilane in the presence of a suitable base such as sodium hydride, potassium hydride, LDA or the like gave rise to an intermediate silyl ketene acetal which rearranged upon heating in a suitable solvent such as THF or Et₂O to a mixed ester Compound **D7.** Decarboxylation of the ester Compound **D7** to form Compound **D8** was accomplished upon heating Compound **D7** under vacuum. Reduction of the double bond in Compound **D8** was accomplished under standard hydrogenation conditions, applying a hydrogen overpressure (of from about 69 to about 340 kPa (about 10 to about 50 psi)) in the presence of an appropriate catalyst such as 5 or 10% palladium on carbon resulted in formation of a target compound Compound **A1** in which (CH₂)_{q} is (CH₂)₂₋₃.

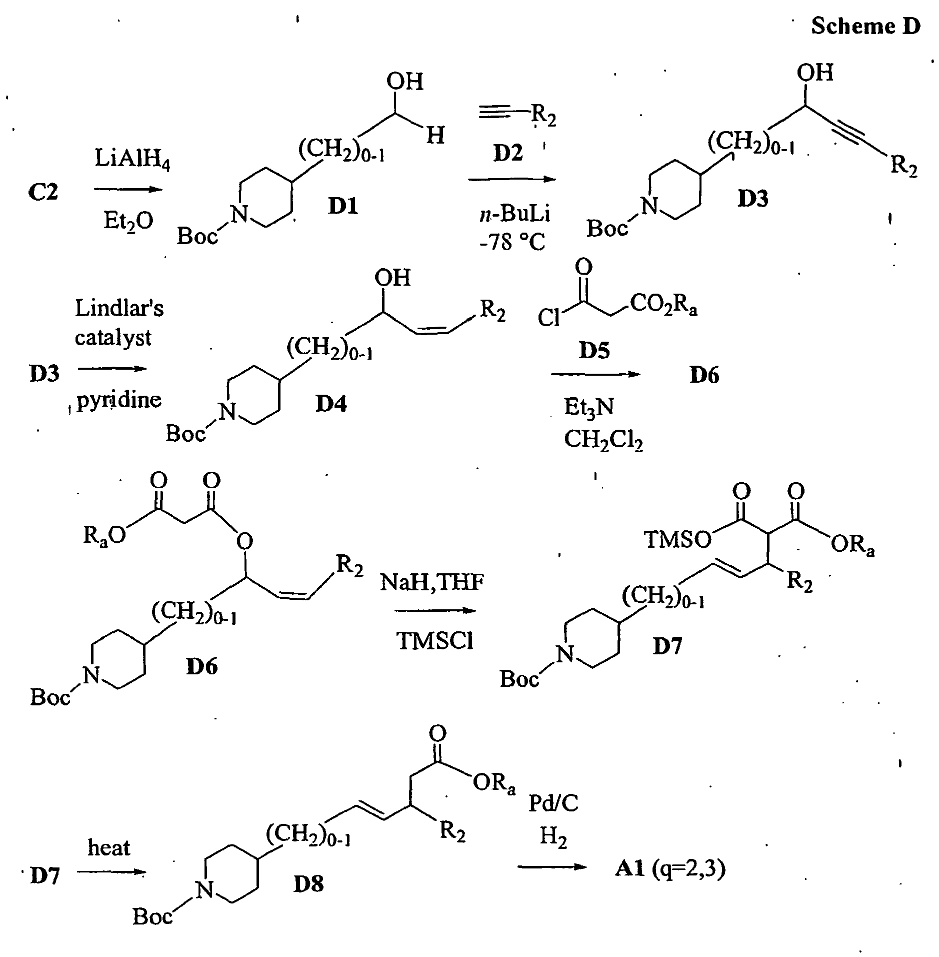

### Scheme E

Scheme E describes an alternative method for the synthesis of a target compound of Formula (I.2) (wherein R₂ for a compound of Formula (I) is hydrogen, R₁ and W are as previously defined. Condensation of an aldehyde Compound **E1** using an appropriate carbalkoxymethylene triphenylphosphorane (Wittig reaction) or a trialkyl phosphonoacetate (Homer-Emmons reaction) resulted in the formation of an α,β-unsaturated ester Compound **E2.** Treatment of Compound **E2** under acidic conditions (using an acid such as a 1:1 mixture of TFA in DCM, 4N HCl in dioxane or the like) resulted in the removal of the Boc-protective group, resulting in formation of a substituted piperidine Compound **E3.** Coupling of the piperidine Compound **E3** with a carboxylic acid Compound **A3** under standard coupling conditions (using a mixture of coupling agents such as HOBt/EDC, HOBT/HBTU or isobutyl chloroformate in the presence of a suitable base such as NMM or DIEA) resulted in an ester Compound **E4.** Hydrolysis of the ester Compound **E4** under acidic or basic conditions yielded an α,β-unsaturated acid Compound **E5.** Reduction of the double bond in Compound **E5** was accomplished under standard hydrogenation conditions, applying hydrogen overpressure (of from about 69 to about 340 kPa (about 10 to about 50 psi)) in the presence of an appropriate catalyst such as 5 or 10% palladium on carbon and resulted in the formation of a target compound of Formula (I.2).

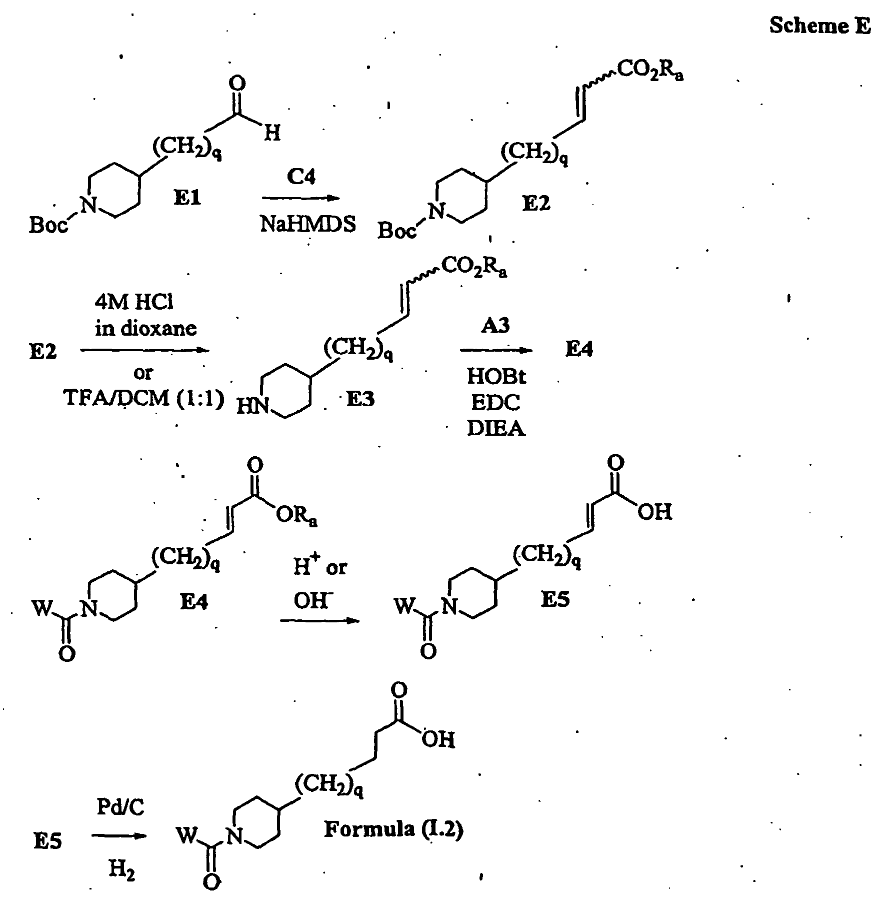

### Scheme F

Scheme F describes an alternative method whereby a target Compound **A1** may be prepared. A racemic E/Z-mixture of an α,β-unsaturated ester Compound **E2** was reacted with an R₂ substituted boronic acid Compound **F1** in the presence of an appropriate transition metal catalyst such as Rhodium or Indium to yield a target Compound **A1.**

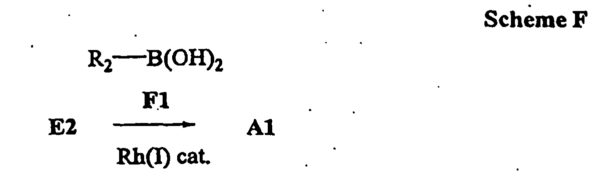

### Scheme G

Scheme G describes an alternative method for the synthesis of a target compound of Formula (I.3) (wherein (CH₂)_{q} for a compound of Formula (I) is -(CH₂)₂₋₃-, R₁ is as previously defined and W is -(CH₂)₀₋₄alkyl-). The Boc-protecting group on Compound **D8** was removed under acidic conditions (using an acid such as a 1:1 mixture of TFA in DCM, 4N HCl in dioxane for the like) to yield a substituted piperidine Compound **G1.** Coupling of the piperidine Compound **G1** with a carboxylic acid Compound **A3** under standard coupling conditions (using a mixture of coupling agents such as HOBt/EDC, HOBT/HBTU or isobutyl chloroformate in the presence of a suitable base such as NMM or DIEA) led to formation of an ester Compound **G2.** The ester Compound G2 was be converted to Compound **G3** upon exposure to strong acidic or basic aqueous conditions (in the presence of a strong acid or base such as concentrated HCl or NaOH). The double bond in Compound **G3** was reduced using standard hydrogenation conditions, applying hydrogen overpressure (of from about 69 to about 340 kPa (about 10 to about 50 psi)) in the presence of an appropriate catalyst such as 5 or 10% palladium on carbon and resulted in the formation of a target compound of Formula (I.3).

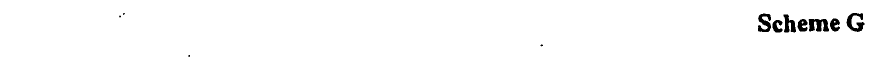

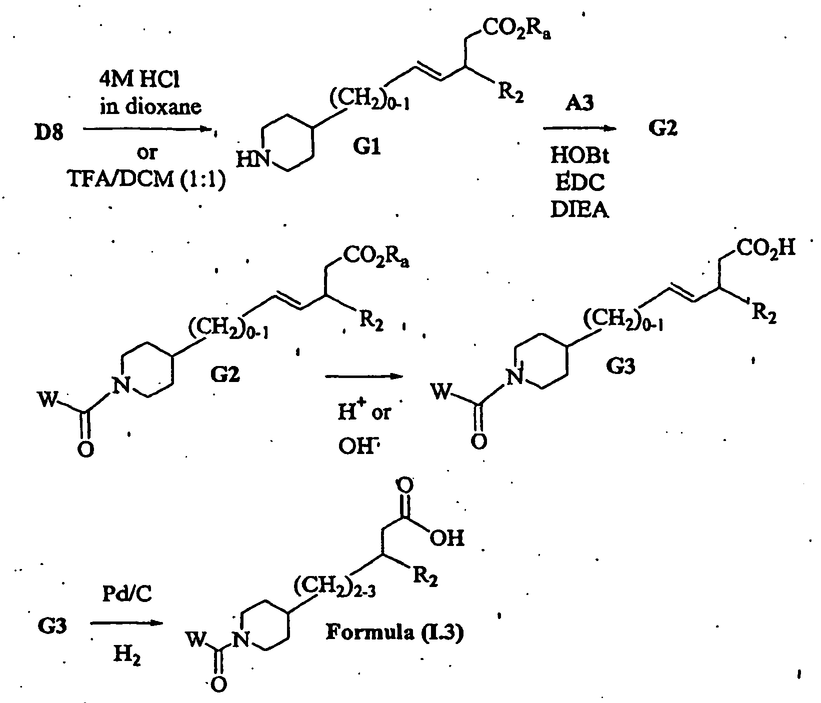

### Scheme H

Scheme H describes a method for the synthesis of a target compound of Formula (I.3a) (wherein R₁ for a compound of Formula (I.3) is -NH(R₅), W is -(CH₂)₀₋₄alkyl- and an R₅ heteroaryl subtituent is reduced to a partially unsaturated heterocyclyl substituent) by reduction of the double bond in a Compound **G3a** (wherein R₁ in a Compound G3 is -NH(R₅)) using standard hydrogenation conditions, applying hydrogen overpressure (of from about 69 to about 340 kPa (about 10 to about 50 psi)) in the presence of an appropriate catalyst such as 5 or 10% palladium on carbon, accompanied by standard reduction of R₅ to yield a target compound of Formula (I.3a).

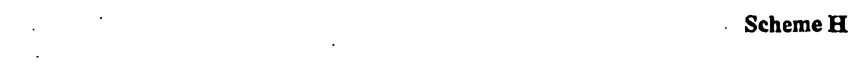

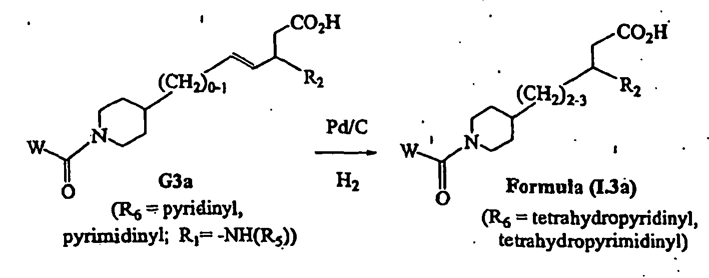

### Scheme I

Scheme I describes an alternative method for the synthesis of a target Compound **B4a** (wherein (CH₂)_{q} for the Compound **B4** is not limited to -(CH₂)₂₋₃-, R₆ is as previously defined, R₁ is H, and W is -(CH₂)₀₋₄alkyl-). Condensation of a Compound **A2** under standard coupling conditions (using a mixture of coupling agents such as HOBt/EDC, HOBT/HBTU or isobutyl chloroformate in the presence of a suitable base such as NMM or DIEA) with a protected amino acid Compound **I1** resulted in the formation of a target Compound **B4a.**

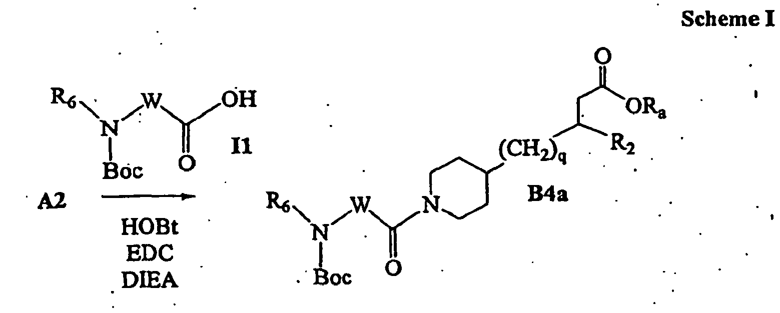

### Scheme J

Scheme J describes a method for the synthesis of a target Compound **Ala** (wherein R₂ in a Compound **A1** is a heteroaryl subtituent that has been reduced to a partially or fully unsaturated heterocyclyl substituent). The double bond in Compound **C5a** (wherein R₂ in a Compound **C5** is a unsaturated heteroaryl subtituent) was reduced under standard hydrogenation conditions, applying hydrogen overpressure (of from about 69 to about 340 kPa about 69 to about 340 kPa (about 10 to about 50 psi)) in the presence of an appropriate catalyst such as 5 or 10% palladium on carbon, accompanied by standard reduction of R₂ to yield a target Compound **Ala.** Compound A1a can be separated into its individual optical isomers by chiral chromatography at this stage. In addition, Compound **A1a** can be alkylated on the R₂ heteroatom using the appropriate alkylating agent such as iodomethane and the appropriate base such as 2,6-di-tert-butylpyridine to yield **A1b.**

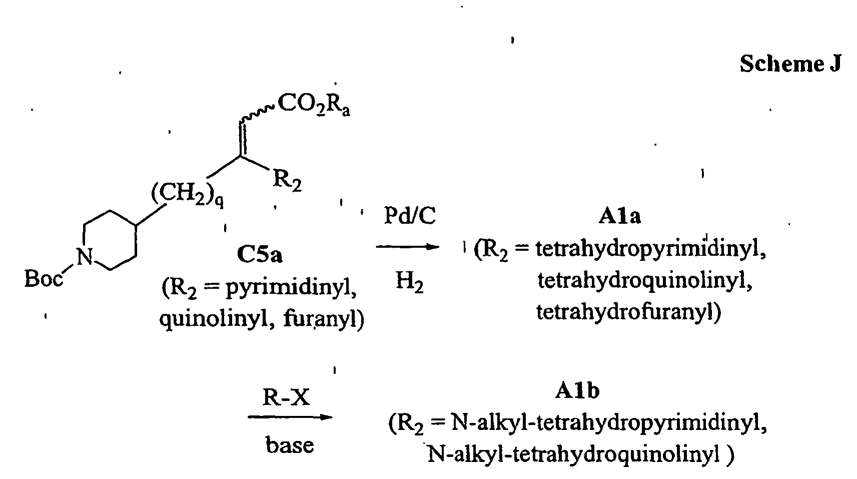

### Scheme K

Scheme K describes a method for preparing a target compound of Formula **I4.** Treatment of a compound of Formula **I** with an appropriate alcohol in the presence of a coupling agent such as 1,3-dicyclohexylcarbodiimide and an activating agent such as dimethylaminopyridine or the like resulted in the formation of target compound of Formula **(I4).** Alternatively, a compound of Formula **I** may be treated with an alkyl halide in the presence of a suitable base such as NMM or DIEA to yield a target compound of Formula **I4.**

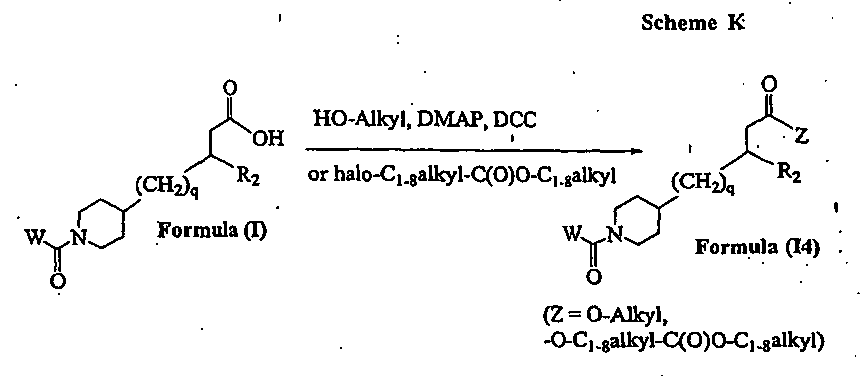

### Scheme L

Scheme L describes a method for the synthesis of a target compound of Formula **A1b** (wherein R₂ in a Compound **A1b** is a hydroxyaryl, aminoaryl, or thiophenyl substituent that has been deprotected). The double bond in Compound **C5b** (wherein R₂ in a Compound **C5** is an *O*-protected hydroxyaryl, *N*-protected anilino, or *S*-protected thioaryl substituent) was reduced under standard hydrogenation conditions, applying hydrogen overpressure (of from about 69 to about 340 kPa (about 10 to about 50 psi)) in the presence of an appropriate catalyst such as 5% or 10% palladium on carbon, accompanied by removal of the protective group to yield hydroxyaryl or anilino compound **A1b.** Alternatively, the protective group can be removed via basic or acidic hydrolysis in a subsequent step.

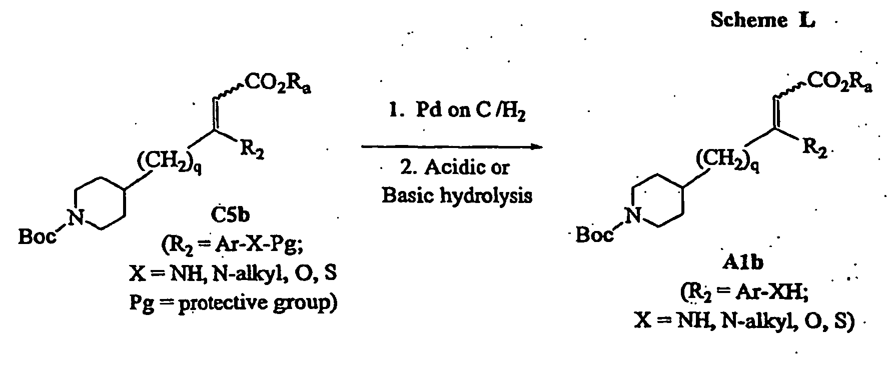

### Scheme M

Scheme M describes a method for preparing a target compound of Formula (I5) (wherein R1 and W are as previously defined). The ketone Compound **C3** was converted to a mixture of *cis* and *trans* isomers of an α,β-unsaturated nitriles Compound **M2** upon reaction with an appropriately substituted phosphorane or phosphonate Compound **M1** in the presence of a base such as LiHMDS, NaHMDS, LDA or the like. Conversion of Compound **M2** to Compound **M3** was accomplished under hydrogenolysis conditions (wherein a hydrogen overpressure of about 34 kPa (about 5 psi) was used) in the presence of an appropriate catalyst such as 5 or 10% palladium on carbon. Removal of the Boc-protective group from Compound **M3** was accomplished under acidic conditions (by using an acid such as an acidic mixture of TFA and DCM or an inorganic acid in an appropriate solvent such as dioxane) and resulted in formation of a piperidine Compound **M4.** Coupling of the piperidine Compound **M4** with a carboxylic acid Compound **A3** under standard coupling conditions (by using a mixture of coupling agents such as HOBt/EDC, HOBT/HBTU or isobutyl chloroformate in the presence of a suitable base such as NMM or DIEA) afforded the nitrile Compound **M5.** Hydrolysis of the nitrile Compound **M5** under acidic conditions yielded a target compound of Formula (I5).

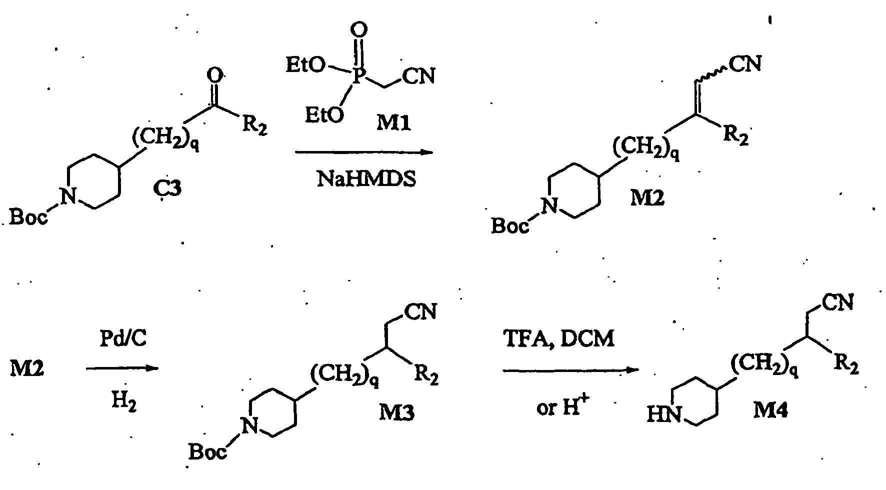

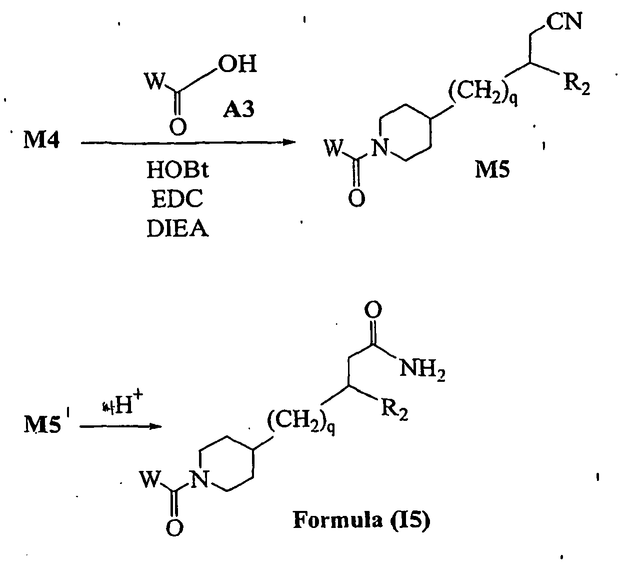

### Scheme N

Scheme N describes a method for the synthesis of a target compound of Formula (II) (wherein W is defined as C₁₋₄alkyl(R₁)). Carboxylic acid Compound **A3** was transformed into alcohol Compound **N1** using an appropriate reducing agent such as lithium aluminum hydride or the like. Alchol Compound **N1** was transformed into aldehyde Compound **N2** using an appropriate oxidizing agent such as pyridinium chlorochromate or the like. Coupling of the aldehyde Compound **N2** with a piperidine Compound **A2** under standard reductive amination conditions using a reducing agent such as sodium triacetoxyborohydride or the like afforded the ester Compound **N3.** Hydrolysis of the ester Compound **N3** under acidic or basic conditions yielded a target compound Formula **(II).**

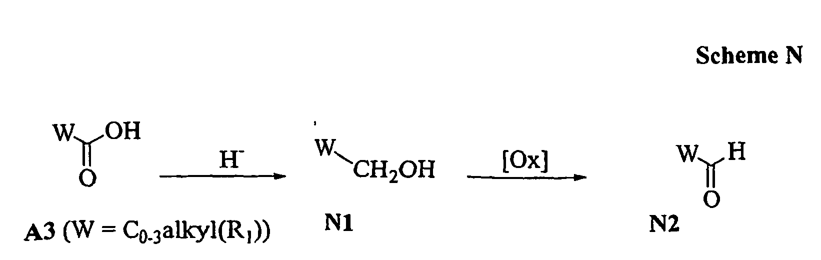

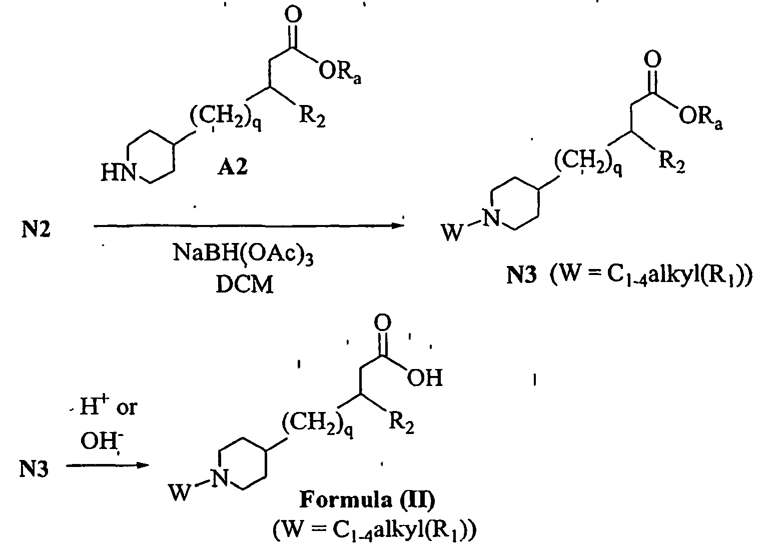

### Specific Synthetic Methods

Specific compounds which are representative of this invention were prepared as per the following examples and reaction sequences; the examples and the diagrams depicting the reaction sequences are offered by way of illustration, to aid in the understanding of the invention and should not be construed to limit in any way the invention set forth in the claims which follow thereafter. The instant compounds may also be used as intermediates in subsequent examples to produce additional compounds of the present invention. No attempt has been made to optimize the yields obtained in any of the reactions. One skilled in the art would know how to increase such yields through routine variations in reaction times, temperatures, solvents and/or reagents.

Reagents were purchased from commercial sources. Microanalyses were performed at Robertson Microlit Laboratories, Inc., Madison, New Jersey and are expressed in percentage by weight of each element per total molecular weight. Nuclear magnetic resonance (NMR) spectra for hydrogen atoms were measured in the indicated solvent with (TMS) as the internal standard on a Bruker Avance (300 MHz) spectrometer. The values are expressed in parts per million downfield from TMS. The mass spectra (MS) were determined on a Micromass Platform LC spectrometer as (ESI) *m*/*z* (M+H⁺) using an electrospray technique. Stereoisomeric compounds may be characterized as racemic mixtures or as separate diastereomers and enantiomers thereof using X-ray crystallography and other methods known to one skilled in the art. Unless otherwise noted, the materials used in the examples were obtained from readily available commercial suppliers or synthesized by standard methods known to one skilled in the art of chemical synthesis. The substituent groups, which vary between examples, are hydrogen unless otherwise noted.

### Example 1

### 1-[[3-[(1,4,5,6-Tetrahydro-2-pyrimidinyl)amino]phenyl]acetyl]-4-piperidinepropanoic acid (Cpd 1)

Methyl iodide (3.21 mL, 51.6 mmol) was added to a solution of 3,4,5,6-tetrahydro-2-pyrimidinethiol Compound **1a** (6.00 g, 51.6 mmol) in absolute ethanol (45 mL). The mixture was refluxed for 3 h, concentrated and dried *in vacuo* to yield Compound **1b** as a colorless oil. MS (ES+) m/z 172 (M+41). ¹H NMR (DMSO-*d₆*, 300 MHz) δ 1.89 (m, 2H), 2.61 (s, 3H), 3.61 (m, 4H), 9.56 (s, 1H).

Boc₂O (11.33 g, 51.91 mmol) was added to a solution of Compound **1b** (13.4 g, 51.9 mmol) and TEA (7.23 mL, 51.9 mmol) in DCM (70 mL) at 0 °C and the mixture was stirred at rt for 2 d. The organic layer was washed with water (2x75 mL), dried (Na₂SO₄) and concentrated to give Compound **1c.** MS (ES+) m/z 231 (M+H⁺).

A solution of Compound **1c** (0.91 g, 3.95 mmol) and 3-aminophenylacetic acid Compound **1d** (0.59 g, 3.95 mmol) in DMA (5 mL) was heated to 80-85 °C for 4 d. The mixture was cooled to rt and diluted with MeCN. The solid was filtered and washed with MeCN and Et₂O, then dried *in vacuo.* Water was added and the pH was adjusted to pH 1-2 by adding conc. HCl dropwise. The resulting solution was lyophilized to give Compound **1e** as a light yellow solid. MS (ES+) m/z 234 (M+H⁺).

Boc₂O (19 g, 87 mmol) and TEA (13 mL, 96 mmol) were added to a solution of 4-piperidinemethanol Compound **1f** (10 g, 87 mmol), DMAP (catalytic amount), dioxane (90 mL) and water (45 mL) at 5 °C. The reaction mixture was stirred overnight at rt and diluted with DCM (100 mL). The organic layer was washed with saturated NH₄Cl, dried (Na₂SO₄) and concentrated to give Compound **1g.** MS (ES+) m/z 216 (M+H⁺).

DMSO (4.28 mL, 60.38 mmol) was added over a 15 min period to a solution of oxalyl chloride (2.63 mL, 30.19 mmol) in DCM (110 mL) at -78 °C. After stirring at -78 °C for 30 min, a solution of Compound 1g (5.0 g, 23.2 mmol) in DCM (10 mL) was added dropwise. The resulting mixture was stirred at -78°C for 2 h. TEA (19.42 mL, 139.3 mmol) was added dropwise and the mixture was warmed to rt and quenched with water. The organic layer was separated, washed sequentially with saturated NH₄Cl (75 mL), water (75 mL), saturated NaHCO₃ (75 mL) and saturated brine (75 mL), then dried (Na₂SO₄) and concentrated to give Compound **1h.** MS (ES+) m/z 214 (M+H⁺). ¹H NMR (DMSO-*d₆*, 300 MHz) δ 1.4 (s, 9H), 1.89 (m, 4H), 2.58 (m, 1H), 3.85 (m, 4H), 9.65 (s, 1H).

A solution of Compound **1h** (2.29 g, 10.7 mmol) in DCM (15 mL) was added dropwise to a solution of carbethoxymethylene triphenylphosphorane (4.11 g, 10.7 mmol) in DCM (20 mL) at 0 °C. The resulting mixture was warmed to rt and stirred overnight. The mixture was concentrated and the residue was purified by flash chromatography (silica gel, 15-30% ethyl acetate/hexane) to give Compound **1i.** MS (ES+) m/z 284 (M+H+). ¹H NMR (DMSO-*d₆*, 300 MHz) δ 1.2 (t, *J* = 7 Hz, 3H), 1.39 (s, 9H), 1.69 (m, 2H), 2.36 (m, 1H), 2.74 (m, 2H), 3.94 (m, 2H), 4.11 (q, *J* = 7 Hz, 2H), 5.86 (d, *J* = 15 Hz, 2H), 6.82 (dd, *J* = 15, 7 Hz, 2H).

A mixture of Compound **1i** (1.6 g, 5.6 mmol), TFA (10 mL) and anisole (1 drop) in DCM (10 mL) was stirred at rt for 1.5 h. The mixture was concentrated and dried *in vacuo* to give Compound **1j** as a TFA salt. MS (ES+) m/z 184 (M+H⁺).

NMM (0.22 mL, 2.07 mmol). Compound 1e (0.29 g, 1.04 mmol), NMM (0.114 mL, 1.04 mmol), HOBT (0.07g, 0.51 mmol) and HBTU (0.46 g, 1.24 mmol) were added sequentially to a solution of Compound **1j** (0.308 g, 1.04 mmol) in MeCN (20 mL) and DMF (2 mL). The mixture was stirred at 0 °C for 1 h, then at rt overnight, quenched with saturated NH₄Cl, concentrated and extracted with EtOAc. The organic layer was dried (Na₂SO₄), filtered and concentrated *in vacuo.* The crude product was purified by flash chromatography (silica gel, 10%EtOH/1.5%NH₄OH/DCM to 16% EtOH/1.5% NH₄OH/DCM) to yield Compound **1k** as a colorless solid. MS (ES+) m/z 399 (M+H⁺).

Compound **1k** (0.27 g) was dissolved in ice cold 6N HCl (20 mL) at 0 °C and stirred at rt for 2 d. The mixture was concentrated and MeCN (3x20 mL) was used as an azeotrope. The resulting solid was triturated with Et₂O and DCM and purified by RP-HPLC (10-90% MeCN/water, 0.1% TFA) to yield Compound **1l** as a TFA salt. MS (ES+) m/z 371 (M+H⁺). ¹H NMR (DMSO-*d₆*, 300 MHz) δ 1.07 (m, 2H), 1.65 (m, 4H), 1.7 (m, 2H), 2.41 (m, 1H), 3.05 (m, 2H), 3.72 (s, 2H), 3.91 (m, 2H), 4.37 (m, 2H), 5.74 (d, *J* = 16 Hz, 1H), 6.75 (m, 1H), 7.15 (m, 3H), 7.42 (m, 1H), 8.15 (br s, 1H), 9.76 (s, 1H). Anal. Calcd for C₂₀H₂₆N₄O₃-1.57CF₃COOH-0.38H₂O: C, 49.96; H, 5.14; N, 10.08; F, 16.09; H₂O, 1.24. Found: C, 49.62; H, 5.00; N, 9.97; F, 15.98; H₂O, 1.25.

10% Palladium on carbon (85 mg) was added to a solution of Compound **1l** (0.05 g) in warm EtOH (10 mL) under argon and the mixture was hydrogenated (280 kPa) (40 psi)) in a Parr apparatus. The mixture was filtered through celite and concentrated at reduced pressure to yield Compound **1** as a sticky solid. MS (ES+) m/z 373 (M+H⁺).

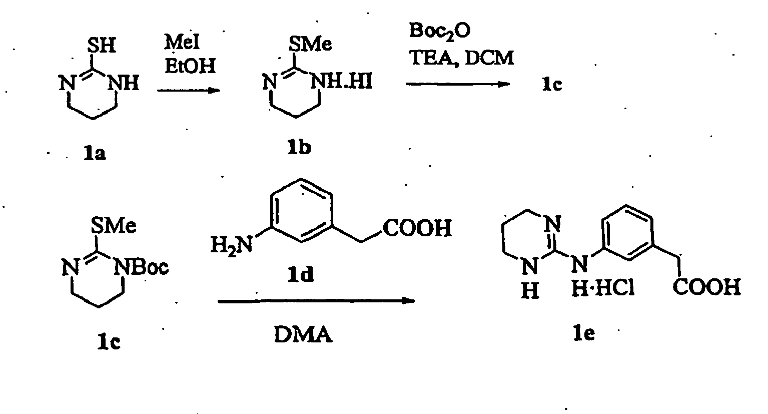

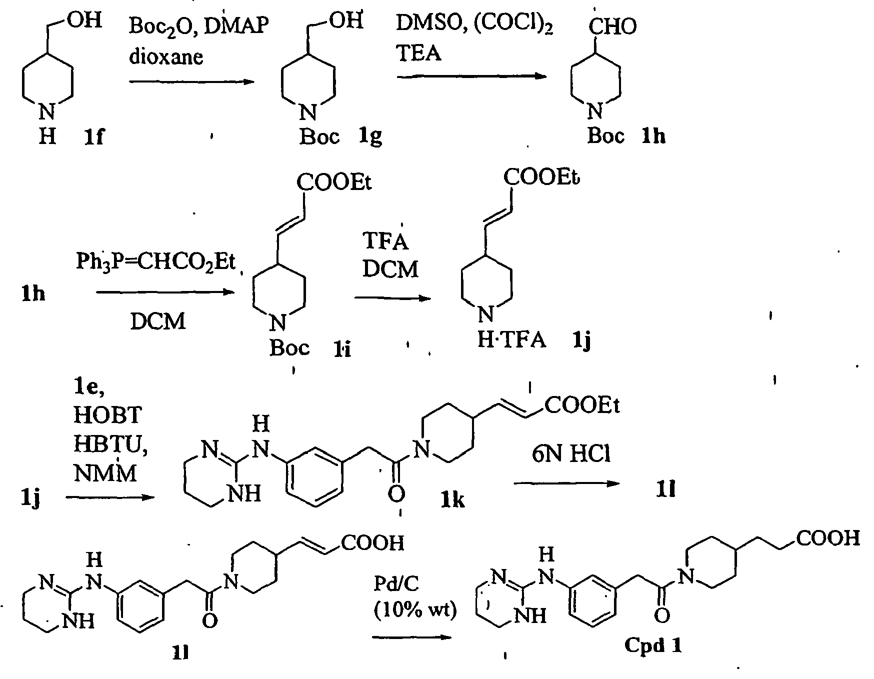

### Example 2

### 1-[1-Oxo-3-[3-[(1,4,5,6-tetrahydro-2-pyrimidinyl)amino]phenyl]propyl]-4-piperidinepropanoic acid (Cpd 2)

Compound **1c** (0.84 g, 3.65 mmol) was added to a solution of 3-(3-aminophenyl)propionic acid Compound **2a** (0.60 g, 3.65 mmol) in DMA (5 mL). The reaction mixture was stirred at 80-85 °C for 3 d, cooled to rt, diluted with MeCN (30 mL) and filtered. Water was added to the filtrate and the pH was adjusted to 1-2 by adding conc. HCl dropwise. The resulting solution was lyophilized to yield Compound **2b.** MS (ES+) m/z 248 (M+H⁺).

A solution of 4N HCl in dioxane (8 mL) was added dropwise to a solution of Compound **2c** (1.0 g, 3.9 mmol) in MeOH (20 mL) at 0 °C. The resulting mixture was stirred overnight at rt and concentrated using MeCN (3x20 mL) as an azeotrope. The solid was triturated with Et₂O and hexane, dissolved in water and lyophilized to yield Compound **2d** as a colorless solid. MS (ES+) m/z 172 (M+H⁺).

NMM (0.23 mL, 2.11 mmol) was added to a solution of Compound **2d** (0.20 g, 0.70 mmol) in MeCN (25 mL) and DMF (2 mL). Compound **2b** (0.15 g, 0.70 mmol), NMM (0.15 mL, 1.40 mmol), HOBT (0.05 g, 0.35 mmol) and HBTU (0.32 g, 0.84 mmol) were then added and the mixture was stirred for 1 h at 0 °C, followed by overnight at rt. Saturated NH₄Cl was added and the reaction mixture was concentrated and extracted with EtOAc (25 mL). The organic layer was dried (Na₂SO₄), filtered and concentrated *in vacuo.* The crude mixture was purified by RP-HPLC (10-90% MeCN/water, 0.1% TFA) to yield Compound **2e.** MS (ES+) m/z 401 (M+H⁺).

Compound 2e (0.21 g) was dissolved in 4N HCl (20 mL) at 0 °C and the mixture was stirred overnight at rt. The mixture was concentrated using MeCN (3 x 25 mL) as an azeotrope and triturated with Et₂O to yield Compound **2** as an HCl salt. MS (ES+) m/z 387 (M+H⁺). ¹H NMR (DMSO-*d₆*, 300 MHz) δ 0.93 (m, 4H), 1.46 (m, 4H), 1.67 (s, 1H), 1.88 (m, 2H), 2.25 (m, 2H), 2.66 (m, 2H), 2.82 (m, 4H), 3.39 (m; 2H), 3.82 (d, *J* = 13 Hz, 1H), 4.39 (d, *J* = 13 Hz, 1H), 7.15 (m, 3H), 7.39 (m, 1H), 7.97 (br s, 1H), 9.45 (br s, 1H). Anal. Calcd for C₂₁H₃₀N₄O₃-1.85 HCl-1.15 H₂O: C, 53.14; H, 7.26; N, 11.82; H₂O, 4.37. Found: C, 53.19; H, 7.14; N, 11.91; H₂O, 4.62.

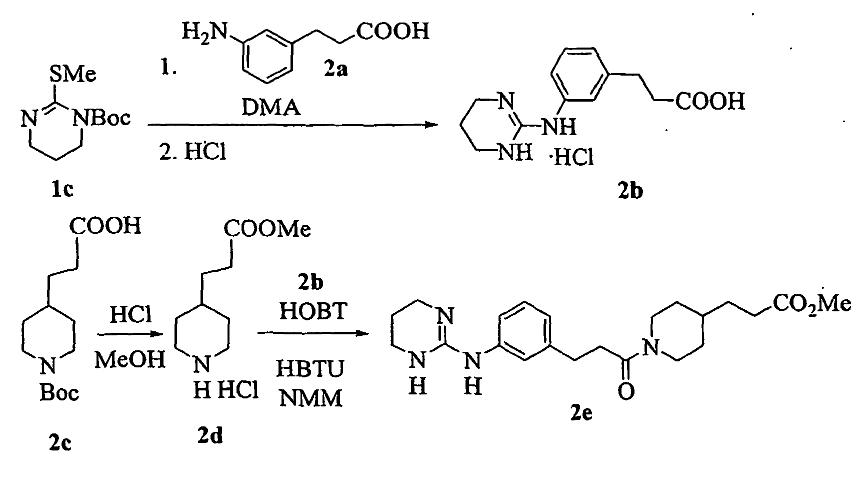

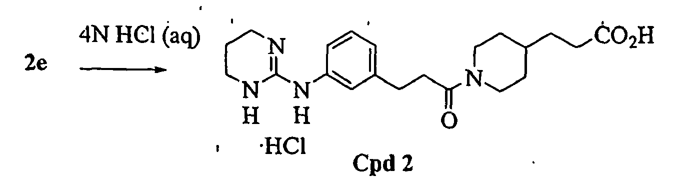

### Example 3

### β-[1-[[3-[(1,4,5,6-Tetrahydro-5-hydroxy-2-pyrimidinyl)amino]phenyl]acetyl]-4-piperidinyl]-3-quinolinepropanoic acid (Cpd 3)

*N,O*-Dimethylhydroxylamine hydrochloride (98%, 2.55 g, 26.17 mmol), NMM (14.39 mL, 130.8 mmol), HOBT (1.47 g, 10.90 mmol) and HBTU (9.83 g, 26.16 mmol) were added to a solution of Compound **3a** (5.00 g, 21.80 mmol) in MeCN (75 mL). The mixture was stirred for 1 h at 0 °C and overnight at rt, quenched with saturated NH₄Cl, concentrated and extracted with EtOAc (3x75 mL). The organic layer was dried (Na₂SO₄) and concentrated *in vacuo.* The crude product was purified by flash column chromatography (silica gel, 30-60% ethyl acetate/hexane with a few drops of TEA) to give Compound **3b** as a liquid. MS (ES+) m/z 273 (M+H⁺).

n-BuLi (2.5M in hexane, 7.34 mL, 18.35 mmol) was added dropwise to a stirred solution of 3-bromoquinoline (3.81 g, 18.35 mmol) in anhydrous Et₂O (65 mL) at -78 °C over a period of 30 min. The mixture was stirred at -78°C for 30 min and a solution of Compound **3b** (1.0 g, 3.67 mmol) in Et₂O (20 mL) was added dropwise over a period of 10 min. The resulting mixture was stirred for 30 min -78°C and allowed to warm to rt. After stirring for 2 h at rt, the mixture was quenched with a saturated NH₄Cl solution and diluted with EtOAc. The organic layer was washed with brine, dried (Na₂SO₄) and concentrated *in vacuo.* The residue was purified via chromatography (silica gel, 15-25% ethyl acetate/hexane) to give Compound **3c** as a liquid. MS (ES+) m/z 341 (M+H⁺).

A solution of NaHMDS (1M, 3.17 mL, 3.17 mmol) in THF was added over a period of 15 min to a stirred solution of trimethyl phosphonoacetate (0.51 mL, 3.17 mmol) in THF (15 mL) at 0 °C under argon. After the resulting mixture was stirred for 20 min, a solution of Compound **3c** (0.27 g, 0.79 mmol) in THF (3 mL) was added over a period of 15 min. The mixture was stirred at 0 °C for 30 min, refluxed for 2.5 h, cooled to rt, diluted with Et₂O (30 mL) and washed with a saturated NaHCO₃ solution (2x25 mL) and brine (2x25 mL). The aqueous layer was extracted with Et₂O and the combined organic layers were dried (Na₂SO₄) and concentrated *in vacuo.* The residue was purified by flash column chromatography (silica gel, 10-30% ethyl acetate/hexane) to give Compound **3d** as a mixture of *E*- and *Z*-isomers. MS (ES+) m/z 397 (M+H⁺).

A mixture of the *E*- and *Z*-isomers of Compound **3d** (0.25 g, 0.63 mmol) and 10% Pd/C (0.12 g) in MeOH (15 mL) was shaken overnight under hydrogen pressure (34 kPa (5 psi)) in a Parr apparatus. The mixture was filtered through celite and concentrated under vacuum. The crude product was purified by flash chromatography (70% ethyl acetate in hexane) to yield Compound **3e** as an oil. MS (ES+) m/z 399 (M+H⁺). ¹H NMR (DMSO-*d₆*, 300 MHz) δ 1.38 (m, 4H), 1.41 (s, 9H), 1.80 (m, 1H), 2.53 (m, 2H), 3.18 (m, 2H), 3.51 (s, 3H), 3.71 (m, 1H), 4.13 (m, 2H), 7.54 (t, *J* = 8 Hz, 1H), 7.69 (t, *J* = 8 Hz, 1H), 7.80 (d, *J* = 8 Hz, 1H), 7.89 (s, 1H), 8.09 (d, *J* = 8 Hz, 1H), 8.75 (s, 1H).

Compound 3e (0.11 g) was dissolved in dioxane (3 mL), one drop of anisole was added and 4N HCl in dioxane (3 mL) was added dropwise. The mixture was stirred at rt for 2 h and concentrated using MeCN as an azeotrope. The resulting solid was triturated witch Et₂O and hexane and dried to give Compound **3f** as a sticky solid. MS (ES+) m/z 299 (M+H⁺). ¹H NMR (DMSO-*d₆*, 300 MHz) δ 1.34 (m, 4H), 1.94 (m, 1H), 2.67 (m, 2H), 3.01 (m, 2H), 3.24 (m, 2H), 3.43 (s, 3H), 3.68 (m, 1H), 7.79 (t, *J* = 8 Hz, 1H), 7.94 (t, *J* = 8 Hz, 1H), 8.13 (d, *J* = 8 Hz, 1H), 8.23 (d, *J* = 8 Hz, 1H), 8.48 (m, 1H), 8.70 (m, 1H). Anal. Calcd for C₁₈H₂₂N₂O₂-2.2TFA-0.4H₂O: C, 48.36; H, 4.53; N, 5.04; F, 22.54. Found: C, 48.24; H, 4.42; N, 4.99; F, 22.56.

1,3-Diamino-2-hydroxypropane Compound **3i** (10.0 g, 111 mmol) was dissolved in ethanol (30 mL) and deionized water (30 mL). Carbon disulfide (6.67 mL, 110.95 mmol) was added dropwise *via* an addition funnel over a period of 35 min while the temperature was maintained at 25-33 °C to afford a milky white mixture. The resulting mixture was refluxed for 2 h to afford a yellow solution. After cooling the mixture in ice water, concentrated HCl (7 mL) was added dropwise while maintaining the mixture's temperature at 25-26 °C. The temperature of the mixture was then raised to 79°C. After stirring for 21 h, the mixture was cooled to 2 °C and filtered *via* vacuum filtration. A white solid was collected, washed three times with a 1:1 mixture of cold ethanol and water and dried *in vacuo* at 40 °C to give Compound **3j.** MS (ES⁺) m/z 174 (M⁺MeCN). ¹H NMR (DMSO-*d₆*, 300 MHz) δ 2.96 (d, *J* = 15 Hz, 2H), 3.15 (d, *J* = 13 Hz, 2H), 3.33 (m, 1H), 3.89 (m, 1H).

Methyl iodide (2.9 mL, 46 mmol) was added to a stirred solution of Compound **3j** (6.1 g, 46 mmol) in absolute ethanol (35 mL) and the mixture was refluxed for 1 h and cooled to it. After concentration, the residue was triturated with Et₂O and dried *in vacuo* to give Compound **3k** as a white solid. MS (ES⁺) m/z 188 (M+MeCN). ¹H NMR (DMSO-*d₆*, 300 MHz) δ 2.59 (s, 3H), 3.23 (d, *J* = 13 Hz, 2H), 3.43 (d, *J* = 13 Hz, 2H), 4.16 (m, 1H).

TEA (6.91 mL, 49.61 mmol) was added to a solution of Compound **3k** (13.06 g, 49.61 mmol) in DCM (50 mL) and DMA (5 mL). The mixture was cooled in an ice bath and Boc₂O (10.82 g, 49.61 mmol) was added at 4 °C. The mixture was heated at 41-43 °C for 18 h to afford a light yellow solution. The resulting solution was washed with water (3x75 mL), dried (Na₂SO₄) and concentrated *in vacuo* to yield Compound **3l** as a solid. MS (ES+) m/z 247(M+H⁺). ¹H NMR (DMSO-*d₆*, 300 MHz) δ 1.46 (s, 9H), 1.95 (s, 3H), 2.14 (m, 2H), 2.94 (m, 2H), 3.51 (m, 1H).

3-Aminophenyl acetic acid Compound **1d** (2.60 g, 17.25 mmol) was added to a solution of Compound **3l** (5.1 g, 21 mmol) in DMA (5 mL). The mixture was heated at 100 °C for 2 d, cooled to rt and diluted with MeCN (75 mL). The resulting precipitate was filtered and washed with MeCN and Et₂O, taken up in water and acidified with conc. HCl. After lyophilization, Compound **3m** was obtained as a white solid. MS (ES+) m/z 250 (M+H⁺). ¹H NMR (DMSO-*d₆*, 300 MHz) δ 3.16 (d, *J* = 13 Hz, 2H), 3.33 (d, *J* = 13 Hz, 2H), 3.59 (s, 2H), 7.12 (m, 3H), 7.35 (m, 1H), 8.14 (s, 1H).

Using the procedure described in Example 2 for converting Compound **2d** to Compound **2e**, Compound **3m** was converted to provide Compound 3n as a solid. MS (ES+) m/z 530 (M+H⁺). ¹H NMR (DMSO-*d₆*, 300 MHz) δ 0.92 (m, 4H), 1.33 (m, 2H), 1.90 (m, 1H), 2.88 (m, 4H), 3.17 (m, 3H), 3.33 (m, 2H), 3.43 (s, 3H), 4.06 (m, 2H), 4.32 (m, 1H), 6.98 (m, 3H), 7.27 (m, 1H), 7.48 (m, ¹H), 7.66 (m, 1H), 7.79 (m, 1H), 8.01 (m, 3H), 8.25 (br s, 1H), 8.83 (br s, 1H).

Using the procedure described in Example 2 for converting Compound **2e** to Compound **2,** Compound **3n** was converted to provide Compound 3 as a solid. MS (ES+) m/z 516 (M+H⁺). ¹H NMR (DMSO-*d₆*, 300 MHz) δ 0.92 (m, 4H), 1.33 (m, 1H), 1.90 (m, 2H), 2.88 (m, 4H), 3.17 (m, 1H), 3.33 (m, 4H), 4.06 (m, 2H), 4.32 (m, 1H), 6.98 (m, 3H), 7.24 (m, 1H), 7.77 (m, 1H), 7.72 (m, 1H), 8.03 (m, 1H), 8.10 (m, 1H), 8.18 (m, 1H), 8.65 (m, 1H), 9.21 (br s, 1H).

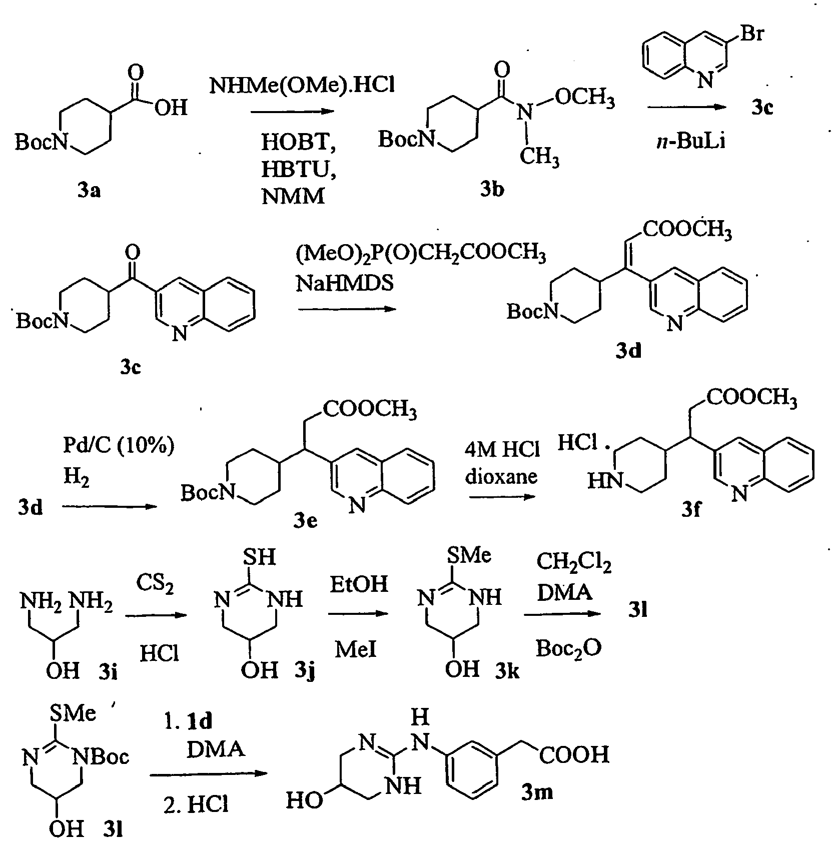

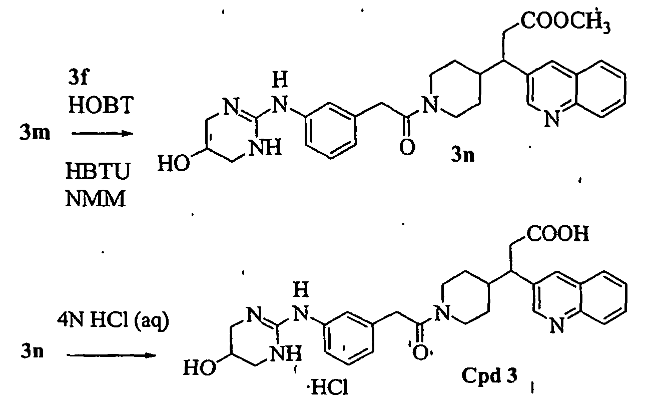

### Example 4

### β-[1-[1-Oxo-4-(5,6,7,8-tetrahydro-1,8-naphthyridin-2-yl)butyl]-4-piperidinyl]-3-quinolinepropanoic acid (Cpd 4)

Compound **4a** was prepared as described in WO 99/31061. Using the procedure described in Example 2 for converting Compound **2d** to Compound **2e,** Compound **4a** was converted and purified by RP-HPLC (10-70% acetonitrile/water, 0.1% TFA) to provide Compound **4b.** MS (ES+) m/z 501 (M+H⁺). ¹H NMR (DMSO-*d₆*, 300 MHz) δ 1.02 (m, 4H), 1.33 (m, 1H), 2.86 (m, 4H), 2.29 (m, 2H), 2.61 (m, 2H) 2.72 (m, 2H), 2.86 (m, 2H), 2.98 (m, 2H), 3.17 (m, 1H), 3.44 (s, 3H), 3.78 (m, 2H), 4.35 (m, 2H), 6.52 (d, *J* = 7 Hz, 1H), 7.56 (d, *J* = 7 Hz, 1H), 7.78 (m, 2H), 7.99 (m, 2H), 8.41 (s, 1H), 8.91 (s, 1H).

Using the procedure described in Example 2 for converting Compound **2e** to Compound **2**, Compound **4b** was converted to provide Compound **4** as a sticky solid. MS (ES+) m/z 487 (M+H⁺). ¹H NMR (DMSO-*d₆*, 300 MHz) δ 0.99 (m, 4H), 1.49 (m, 1H), 2.86 (m, 4H), 2.30 (m, 2H), 2.69 (m, 2H), 2.81 (m, 1H), 2.92 (m, 2H), 3.13 (m, 2H), 3.33 (m, 1H), 3.79 (m, 2H), 4.41 (m, 2H), 6.55 (d, *J* = 7 Hz, 1H), 7.56 (d, *J* = 7 Hz, 1H), 7.86 (m, 1H), 7.98 (m, 2H), 8.72 (m, 2H), 8.83 (s, 1H), 9.15 (s, 1H). Anal. Calcd for C₂₉H₃₄N₄O₃-3.5 HCl-H₂O: C, 55.09; H, 6.30; N, 8.86; H₂O, 3.24. Found: C, 54.83; H, 6.53; N, 9.08; H₂O, 3.24.

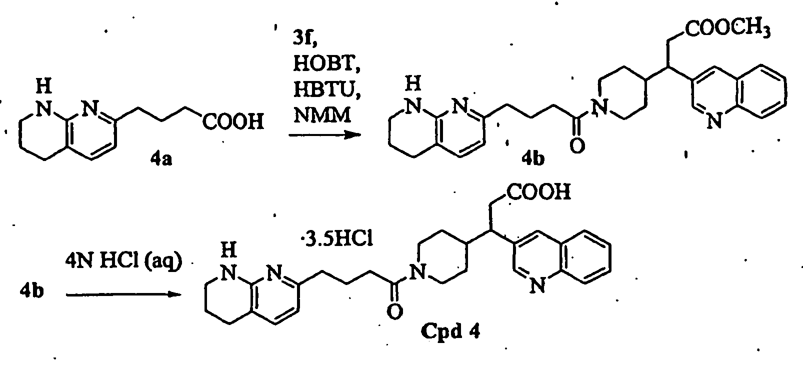

Using the procedure of Example 4 and the appropriate reagents and starting materials known to those skilled in the art, other compounds of the present invention may be prepared including, but not limited to:

| **Cpd** | **Name** | **MS (m/z)** |
|---|---|---|
| **14** | β-(1,3-benzodioxol-5-yl)-1-[1-oxo-3-(5,6,7,8-tetrahydro-1,8-naphthyridin-2-yl)propyl]-4-piperidinepropanoic acid | 466 |
| **15** | β-(1,3-benzodioxol-5-yl)-1-[1-oxo-4-(5,6,7,8-tetrahydro-1,8-naphthyridin-2-yl)butyl]-4-piperidinepropanoic acid | 480 |
| **16** | β-(1,3-benzodioxol-5-yl)-1-[(5,6,7,8-tetrahydro-1,8-naphthyridin-2-yl)acetyl]-4-piperidinepropanoic acid | 452 |
| **17** | 6-methoxy-β-[1-[1-oxo-4-(5,6,7,8-tetrahydro-1,8-naphthyridin-2-yl)butyl]-4-piperidinyl]-3-pyridinepropanoic acid | 467 |
| **82** | 3-(2,3-Dihydro-benzofuran-6-yl)-3-[1-4-(5,6,7,8-tetrahydro-1,8-naphthyridin-2-yl)butyl]-4-piperidinyl]-propanoic acid | |

and pharmaceutically acceptable salts thereof.

### Example 5

### 1,2,3,4-Tetrahydro-β-[1-[1-oxo-4-(5,6,7,8-tetrahydro-1,8-naphthyridin-2-yl)butyl]4-piperidinyl]-3-quinolinepropanoic acid (Cpd 5)

Compound **3d** (0.49 g) was combined with 10% Pd/C (0.6 g) inmethanol (40 mL) and water (1.5 mL), and hydrogenated at 340 kPa (50 psi) of H₂ for 3 d. After filtration of catalyst, the evaporated material was purified by flash chromatography (gradient 20-30% ethyl acetate in heptane with a few drops of triethylamine) to provide Compounds **5a** (0.23 g, 47%) and **5b** (0.16 g, 32%). Cpd **5a:** MS (ES+) m/z 403 (M+H⁺). ¹H NMR (CDCl₃, 300 MHz) δ 1.2-1.7 (m, 4H), 1.45 (s, 9H), 1.9-2.4 (m, 4H), 2.5-3.1 (m, 5H), 3.27 (m, 1H), 3.68 (s, 3H), 3.84 (m, 1H), 4.13 (m, 2H), 6.48 (d, *J* = 8 Hz, 1H), 6.61-6.69 (m, 1H), 6.92-6.99 (m, 2H). Cpd **5b:** MS (ES+) m/z 403.5 (M+H⁺). ¹H NMR (DMSO-*d₆*, 300 MHz) δ 0.8-1.3 (m, 4H), 1.35 (s, 9H), 1.6-1.8 (m, 4H), 2.6-2.8 (m, 10H), 3.45 (s, 3H), 3.8-4.0 (m, 2H), 7.27 (m, 1H), 8.08 (m, 1H).

Using the procedure described in Example 3 for converting Compound **3e** to Compound **3f,** Compound **5a** was converted to provide Compound **5c** as a solid. MS (ES+) m/z 303 (M+H⁺). ¹H NMR (DMSO-*d₆*, 300 MHz) δ 1.61 (m, 4H), 1.82 (m, 1H), 2.32 (m, 1H), 2.44 (m, 2H), 2.78 (m, 2H), 3.25 (m, 2H), 3.35 (m, 2H), 3.62 (s, 3H), 3.78 (m, 3H), 7.16 (m, 2H), 8.76 (m, 2H).

Using the procedure described in Example 2 for converting Compound **2d** to Compound **2e,** Compound **4a** was reacted with Compound **5c** and purified by RP-HPLC (10-70% acetonitrile/water, 0.1% TFA) to provide Compound **5d.** MS (ES+) m/z 505 (M+H⁺). ¹H NMR (DMSO-*d₆*, 300 MHz) δ 1.11 (m, 4H), 1.56 (m, 1H), 1.79 (m, 6H), 2.32 (m, 4H), 2.66 (m, 2H), 2.77 (m, 2H), 2.91 (m, 2H), 3.16 (m, 2H), 3.5 (m, 2H), 3.62 (s, 3H), 3.82 (m, 2H), 4.43 (m, 2H), 6.58 (m, 3H), 7.63 (d, *J* = 7 Hz, 1H), 7.93 (m, 2H).

Using the procedure described in Example 2 for converting Compound **2e** to Compound **2,** Compound **5d** was converted to provide Compound 5 as an HCl salt. MS (ES+) m/z 491 (M+H⁺). ¹HNMR (DMSO-*d₆*, 300 MHz) δ 1.13 (m, 4H), 1.54 (m, 2H), 1.77 (m, 4H), 2.21 (m, 4H), 2.37 (m, 1H), 2.64 (m, 2H), 2.71 (m, 2H), 2.96 (m, 2H), 3.23 (m, 2H), 3.45 (s, 2H), 3.84 (m, 2H), 4.45 (m, 2H), 6.54 (m, 3H), 6.98 (m, 2H), 7.61 (d, *J* = 8 Hz, 1H), 8.01 (br s, 1H).

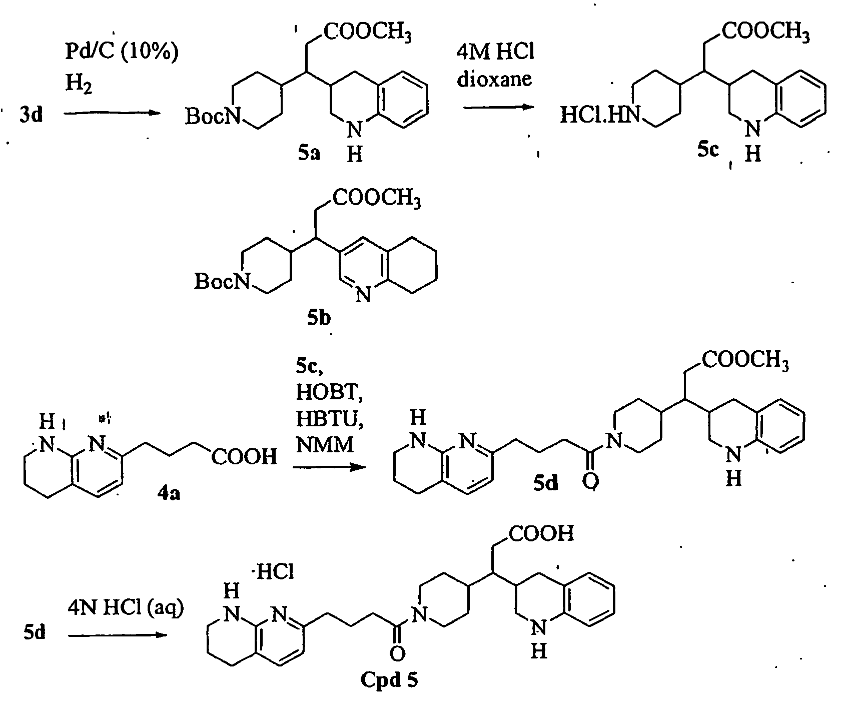

Using the procedure of Example 5 and the appropriate reagents and starting materials known to those skilled in the art, other compounds of the present invention may be prepared including, but not limited to:

| **Cpd** | **Name** | **MS (m/z)** |
|---|---|---|
| **18** | 1,4,5,6-tetrahydro-2-methyl-β-[[1-[1-oxo-3-(5,6,7,8-tetrahydro-1,8-naphthyridin-2-yl)propyl]-4-piperidinyl]methyl]-5-pyrimidinepropanoic acid | 456 |
| **19** | 1,2,3,4-tetrahydro-β-[[1-[1-oxo-3-(5,6,7,8-tetrahydro-1,8-naphthyridin-2-yl)propyl]-4-piperidinyl]methyl]-3-quinolinepropanoic acid | 491 |
| **57** | 5,6,7,8-tetrahydro-β-[[1-[1-oxo-3-(5,6,7,8-tetrahydro-1,8-naphthyridin-2-yl)propyl]-4-piperidinyl]methyl]-3-quinolinepropanoic acid | 491 |

and pharmaceutically acceptable salts thereof.

### Example 6

### β-[2-[1-[3-[(1,4,5,6-Tetrahydro-2-pyrimidinyl)amino]benzoyl]-4-piperidinyl]ethyl]-3-pyridinepropanoic acid (Cpd 6)

Using the procedure described in Example 3 for converting Compound **3a** to Compound **3b,** *N*-Boc-piperidin-4-propionic acid Compound **2c** was converted to Compound **6a** (colorless liquid; purified by flash chromatography (on silica gel, eluted with 30-50% ethyl acetate/hexane with a few drops of TEA). MS (ES+) m/z 301 (M+H⁺). ¹H NMR (DMSO-*d₆*, 300 MHz) δ 1.14 (m, 4H), 1.45 (s, 9H), 1.62 (m, 1H), 1.68 (m, 2H), 2.44 (t, *J* = 7.5 Hz, 2H), 2.63 (m, 2H), 3.18 (s, 3H), 3.68 (s, 3H), 4.08 (m, 2H).

Using the procedure described in Example 3 for converting Compound **3b** to Compound **3c,** Compound **6a** was converted to Compound **6b** (purified by flash chromatography on silica gel, eluted with 30-50% ethyl acetate/hexane with a few drops of TEA). MS (ES+) m/z 319 (M+H⁺).

Using the procedure described in Example 3 for converting Compound **3c** to Compound **3d,** Compound **6b** was converted to Compound **6c** (purified by flash chromatography on silica gel, eluted with 30-50% ethyl acetate/hexane with a few drops of TEA). MS (ES+) m/z 375 (M+H⁺).

Using the procedure described in Example 3 for converting Compound **3d** to Compound **3e,** Compound **6c** was converted to Compound **6d** (purified by flash chromatography on silica gel, eluted with 15-35% ethyl acetate/hexane with a few drops of TEA). MS (ES+) m/z 377 (M+H⁺). ¹H NMR (DMSO-*d₆*, 300 MHz) δ 0.91 (m, 4H), 1.12 (m, 2H), 1.29 (m, 1H), 1.41 (s, 9H), 1.53 (m, 3H), 2.63 (m, 2H), 3.98 (m, 2H), 3.35 (s, 3H), 3.48 (m, 1H), 3.88 (m, 2H), 7.34 (m, 1H), 7.68 (m, 1H), 8.43 (m, 2H).

Using the procedure described in Example 3 for converting Compound **3e** to Compound **3f,** Compound **6d** was converted to Compound **6e** (white solid). MS (ES+) m/z 277 (M+H⁺). ¹H NMR (DMSO-*d₆*, 300 MHz) δ 0.9 (m, 2H), 1.19 (m, 4H), 1.44 (m, 1H), 1.71 (m, 2H), 2.71 (m, 2H), 2.82 (m, 2H), 3.08 (m, 2H), 3.21 (m, 1H), 3.49 (s, 3H), 7.51 (m, 1H), 7.94 (m, 1H), 8.53 (m, 2H).

Using the procedure described in Example 1 for converting Compound **1c** to Compound **1e,** Compound **1c** was reacted with 3-aminobenzoic acid Compound **6f** to provide Compound **6g** as a white amorphous solid. MS (ES+) m/z 220 (M+H⁺). ¹H NMR (DMSO-*d₆*, 300 MHz) δ 4.13 (m, 2H); 5.42 (t, *J* = 5 Hz, 4H), 6.81 (m, 4H).

Using the procedure described in Example 1 for converting Compound **1j** to Compound **1k,** Compound **6g** was reacted with Compound 6e to produce Compound **6h** (purified via RP-HPLC: 5-50% acetonitrile/water, 0.1% TFA). MS (ES+) m/z 478 (M+H⁺).

Using the procedure described in Example 2 for converting Compound **2e** to Compound **2,** Compound **6h** was converted to Compound **6** (purified via RP-HPLC: 5-50% acetonitrile/water, 0.1% TFA). MS (ES+) m/z 464 (M+H⁺). ¹H NMR (DMSO-*d₆*, 300 MHz) δ 1.11 (m, 2H), 1.19 (m, 2H), 1.49 (m, 4H), 1.68 (m, 1H), 1.72 (m, 4H), 2.72 (m, 4H), 3.15 (m, 1H), 3.65 (m, 2H), 4.38 (m, 2H), 7.12-7.51 (m, 4H), 7.73 (m, 1H), 8.21 (m, 1H), 8.65 (m, 2H).

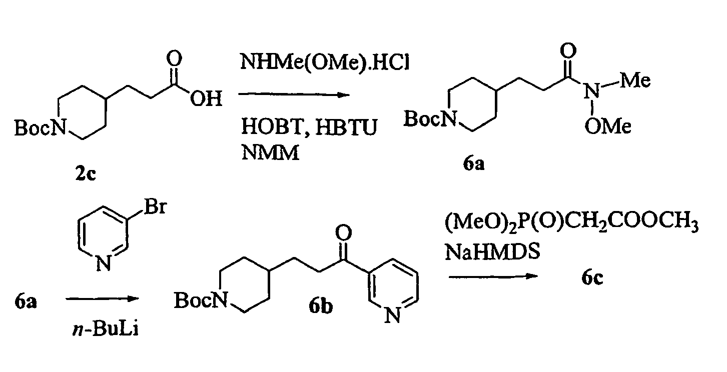

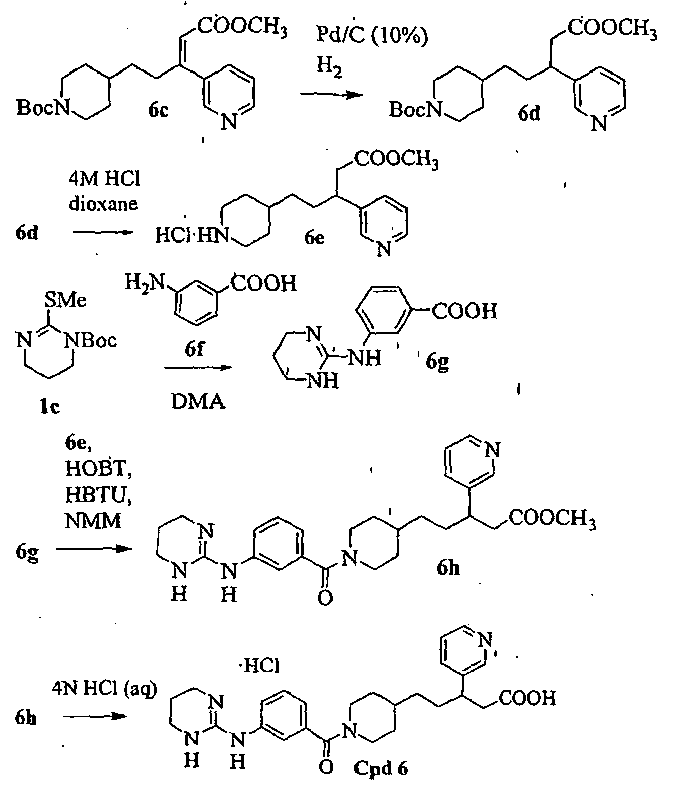

### Example 7

### β-[2-[1-[3-[(1,4,5,6-Tetrahydro-5-hydroxy 2-pyrimidinyl)amino]benzoyl]-4-piperidinyl]ethyl]-3-pyridinepropanoic acid (Cpd 7)

Using the procedure described in Example 3 for converting Compound **31** to Compound **3m,** Compound **31** was reacted with 3-aminobenzoic acid Compound **6f** to provide Compound **7a** as a white amorphous solid. MS (ES+) m/z 235 (M+H⁺). ¹H NMR (DMSO-*d₆*, 300 MHz) δ 3.18 (d, *J* = 12 Hz, 2H), 3.35 (d, *J* = 12 Hz, 2H), 4.09 (m, 1H), 7.55 (m, 2H), 7.84 (m, 2H).

Using the procedure described in Example 3 for converting Compound **3m** to Compound **3n,** Compound **7a** was reacted with Compound **6e** to produce Compound **7b** (white solid; purified by RP-HPLC: 2-30% acetonitrile/water, 0.1% TFA). MS (ES+) m/z 494 (M+H⁺).

Using the procedure described in Example 3 for converting Compound **3n** to Compound **3,** Compound **7b** was converted to provide Compound **7** as a white solid. MS (ES+) m/z 480 (M+H⁺). ¹H NMR (DMSO-*d₆*, 300 MHz) δ 1.03 (m, 2H), 2.22 (m, 4H), 1.49 (m, 1H), 1.66 (m, 2H), 2.65 (m, 2H), 2.76 (m, 2H), 3.06 (m, 2H), 3.18 (m, 4H), 3.34 (m, 1H), 4.13 (s, 1H), 7.12-8.78 (m, 8H), 9.91 (s, 1H).

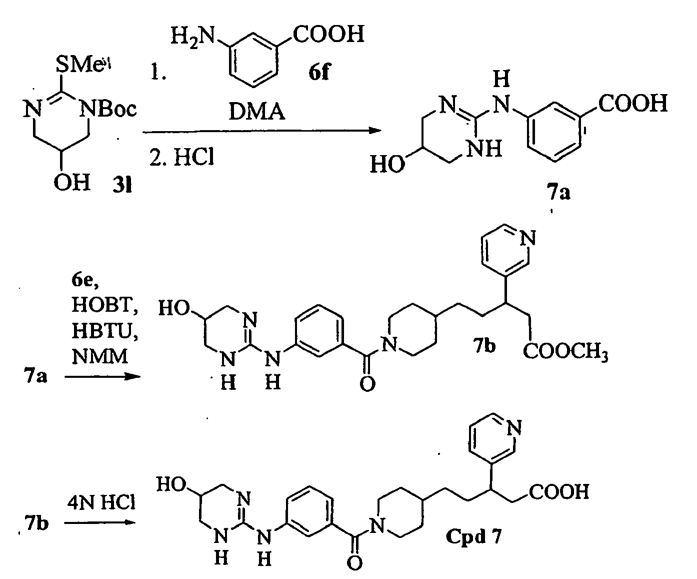

### Example 8

### β-[2-[1-[1-Oxo-3-(5,6,7,8-tetrahydro-1,8-naphthyridin-2-yl)propyl]-4-piperidinyl]ethyl]-3-pyridinepropanoic acid (Cpd 8)

The acid Compound **8a** was derived from the corresponding ethyl ester as described in WO99/31061, the synthesis of which was described in WO 00/72801.

Using the procedure described in Example 5 for converting Compound **4a** to Compound **5c,** Compound **8a** was reacted with Compound **6e** to yield Compound **8b** (purified by RP-HPLC: 10-90% acetonitrile/water, 0.1% TFA). MS (ES+) m/z 465 (M+H⁺).

Using the procedure described in Example 5 for converting Compound **5c** to Compound **5,** Compound **8b** was converted to provide Compound **8** as an HCl salt. MS (ES+) m/z 45.1 (M+H⁺). ¹H NMR (DMSO-*d₆*, 300 MHz) δ 1.03 (m, 2H), 1.19 (m, 2H), 1.49 (m, 4H), 1.68 (m, 1H), 1.72 (m, 4H), 2.72 (m, 2H), 2.98 (m, 2H), 3.18 (m, 1H), 3.65 (m, 2H), 4.33 (m, 2H), 7.25 (m, 2H), 7.51 (m, 1H), 7.73 (m, 1H), 8.21 (m, 1H), 8.31 (s, 1H), 8.65 (m, 2H).

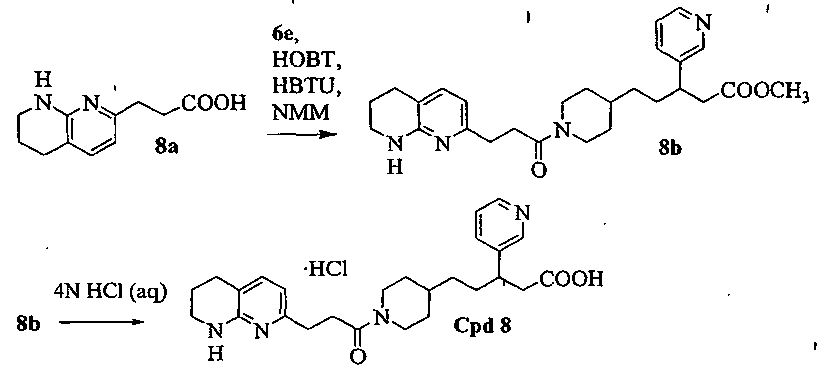

Using the procedure of Example 8 and the appropriate reagents and starting materials known to those skilled in the art, other compounds of the present invention may be prepared including, but not limited to:

| **Cpd** | **Name** | **MS (m/z)** |
|---|---|---|
| **20** | β-(1,3-benzodioxol-5-yl)-1-[1-oxo-3-(5,6,7,8-tetrahydro-1,8-naphthyridin-2-yl)propyl]-4-piperidinepentanoic acid | 494 |
| **21** | 6-methoxy-β-[2-[1-[1-oxo-3-(5,6,7,8-tetrahydro-1,8-naphthyridin-2-yl)propyl]-4-piperidinyl]ethyl]-3-pyridinepropanoic acid | 481 |

and pharmaceutically acceptable salts thereof.

### Example 9

### β-[2-[1-[1-Oxo-4-(2-pyridinylamino)butyl]-4-piperidinyl]ethyl]-3-pyridinepropanoic acid (Cpd 9)

A mixture of Compound **6e** (0.14 g, 0.44 mmol) in DCM (10 mL) and NMM (0.09 mL, 0.89 mmol) was stirred for 0.5 h at rt then cooled in an ice bath. 4-Bromobutyrylchloride Compound **9a** (0.06 mL, 0.58 mmol) and NMM (0.09 mL, 0.89 mmol) were added and the reaction mixture was stirred for 6 h at 0 °C and overnight at rt. The reaction mixture was washed with saturated NH₄Cl solution (5 mL), water (5 mL) and 1N HCl (3 x 10 mL). The organic layer was dried (Na₂SO₄) and concentrated *in vacuo* to yield Compound **9b** as a viscous oil. MS (ES+) m/z 345 (M-Br).

DIEA (0.73 mL, 4.23 mmol) was added to a stirred solution of Compound **9b** (0.60 g, 1.41 mmol) and 2-aminopyridine Compound **9c** (0.39 g, 4.23 mmol) in toluene (10 mL). The mixture was refluxed overnight and concentrated *in vacuo.* The residue was purified by RP-HPLC (2-30% acetonitrile/water, 0.1% TFA) to give Compound **9d** as an oil. MS (ES+) m/z 439 (M+H⁺).

Using the procedure described in Example 6 for converting Compound **6h** to Compound **6,** Compound **9d** was converted to Compound **9** (purified by RP-HPLC: 2-30% acetonitrile/water, 0.1 % TFA). MS (ES+) m/z 425 (M+H⁺). ¹H NMR (DMSO-*d₆*, 300 MHz) δ 1.01 (m, 2H), 1.11 (m, 4H), 1.36 (m, 1H), 1.69 (m, 4H), 2.16 (m, 2H), 2.39 (m, 2H), 3.21 (m, 2H), 3.76 (m, 2H), 4.26 (m, 2H), 4.61 (m, 1H), 7.31-8.72 (m, 8H).

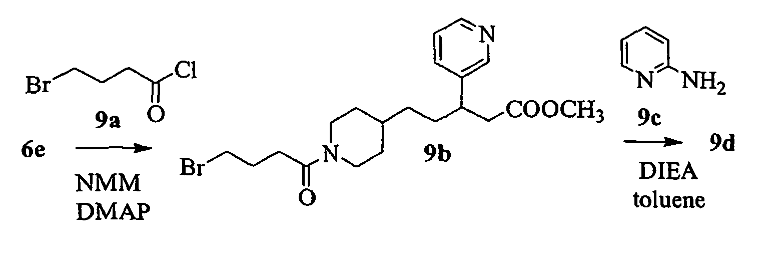

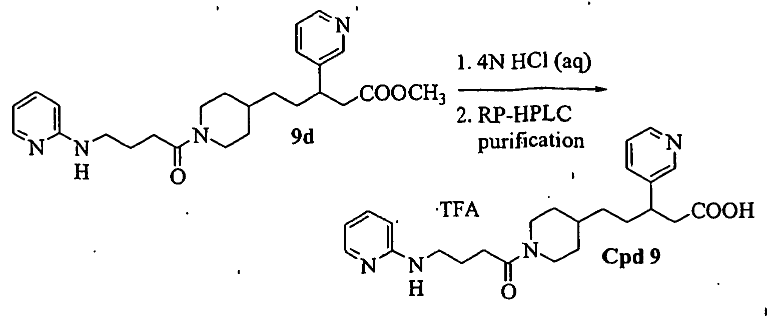

Using the procedure of Example 9 and the appropriate reagents and starting materials known to those skilled in the art, other compounds of the present invention may be prepared including, but not limited to:

| **Cpd** | **Name** | **MS (m/z)** |
|---|---|---|
| **22** | β-[2-[1-[1-oxo-4-(2-pyridinylamino)butyl]-4-piperidinyl]ethyl]-3-quinolinepropanoic acid | 475 |
| **23** | β-(1,3-benzodioxol-5-yl)-1-[1-oxo-4-(2-pyridinylamino)butyl]-4-piperidinepentanoic acid | 468 |
| **24** | β-(1,3-benzodioxol-5-yl)-1-[1-oxo-4-(2-pyridinylamino)butyl]-4-piperidinepropanoic acid | 440 |
| **25** | 6-methoxy-β-[2-[1-[1-oxo-4-(2-pyridinylamino)butyl]-4-piperidinyl]ethyl]-3-pyridinepropanoic acid | 455 |

and pharmaceutically acceptable salts thereof.

### Example 10

### 6-Methoxy-β-[2-[1-[3-[(1,4,5,6-tetrahydro-5-hydroxy-2-pyrimidinyl)amino]benzoyl]-4-piperidinyl]ethyl]-3-pyridinepropanoic acid (Cpd 10)

Using the procedure described in Example 6 for converting Compound **6c** to Compound **6d,** Compound **10a** was converted to Compound **10b** (colorless liquid; purified by flash chromatography on silica gel, 10-15% ethyl acetate/hexane with a few drops of TEA). MS (ES+) m/z 407 (M+H⁺) as a racemic mixture that was enantiomerically separated using a chiralcel OJ column eluting with hexane/ethanol (75:25). 1H-NMR (DMSO-*d₆*, 300 MHz) δ 1.04 (m, 4H), 1.19 (m, 2H), 1.47 (s, 9H), 1.61 (m, 1H), 1.73 (m, 2H), 2.66 (m, 4H), 3.02 (m, 2H), 3.61 (s, 3H), 3.92 (s, 3H), 4.01 (m, 1H), 6.81 (d, *J* = 7 Hz, 1H), 7.38 (d, *J* = 7 Hz, 1H), 8.05 (s, 1H).

Using the procedure described in Example 6 for converting Compound **6d** to Compound **6e,** Compound **10b** was converted to provide Compound **10c** as an HCl salt. MS (ES+) m/z 307 (M+H⁺). ¹H NMR (DMSO-*d₆*, 300 MHz) δ 0.98 (m, 2H), 1.18 (m, 1H), 1.53 (m, 4H), 1.81 (m, 2H), 2.62 (m, 2H), 2.81 (m, 4H), 3.22 (m, 1H), 3.53 (s, 3H), 3.83 (s, 3H), 6.76 (d, *J* = 9 Hz, 1H), 7.63 (m, 1H), 8.04 (m, 1H). Anal. Calcd for C₁₇H₂₆N₂O₃-1.63 CF₃COOH-0.2 H₂O: C, 49.08; H, 5.70; N, 5.65; H₂O, 0.73. Found: C, 49.10; H, 5.66; N, 5.65; H₂O, 0.93.

Using the procedure described in Example 7 for converting Compound **7a** to Compound **7b,** Compound **7a** was reacted with Compound **10c** to produce Compound **10d.** Using the procedure described in Example 3 for converting Compound **3n** to Compound **3**, Compound **10d** was converted to produce Compound **10** as an HCl salt (purified by RP-HPLC: 5-50% acetonitrile/water, 0.1% TFA). MS (ES+) m/z 510 (M+H⁺). ¹H NMR (DMSO-*d₆*, 300 MHz) δ 0.99 (m, 2H), 1.14 (m, 1H), 1.53 (m, 6H), 1.67 (m, 2H), 2.58 (m, 2H), 2.94 (m, 1H), 3.15 (d, *J* = 11 Hz, 2H), 3.33 (d, *J* = 12 Hz, 2H), 3.81 (s, 3H), 3.86 (m, 2H), 4.09 (m, 1H), 6.75 (d, *J* = 9 Hz, 1H), 7.12-7.29 (m, 4H), 7.63 (m, 1H), 8.03 (m, 1H).

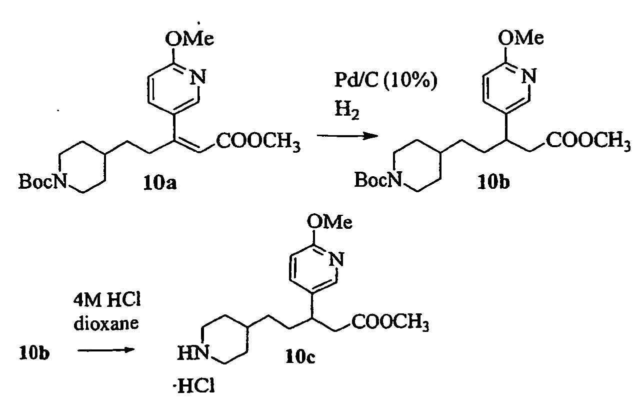

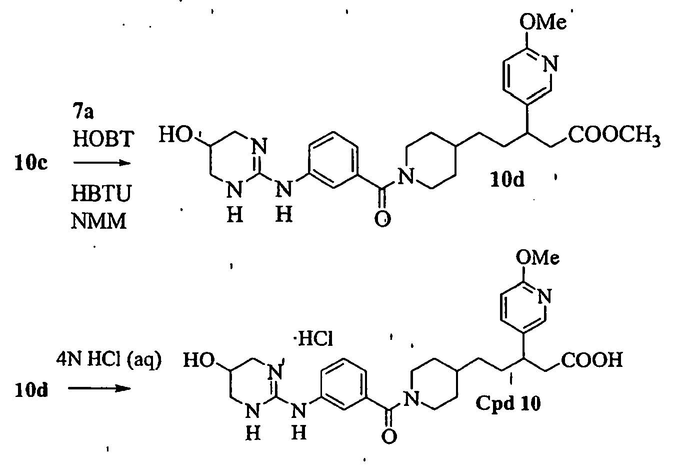

### Example 11

Using the procedures described in Examples 6 and 8 for preparing Compound **8**, the enantiomers of Compound **21** were produced from the enantiomers of **10b.**

The two pure chiral intermediates **10b-1** (isomer 1: faster eluting) and **10b-2** (isomer 2 slower eluting) were obtained by chiral HPLC chromatography (stationary phase: 500 of Chiralcel OJ; eluent: hexane/ethanol 75/25; wavelength: 220 nm). Compounds **10b-1** and **10b-2** were converted individually to **21a** and **21b,** respectively, by the same methods used to convert **6d** to **8** in Examples 6 and 8.

Using the procedure of Example 11 and the appropriate solvents, columns, reagents and starting materials known to those skilled in the art, other compounds of the present invention may be prepared including, but not limited to:

| **Cpd** | **Name** | **MS (m/z)** |
|---|---|---|
| **28a** | 6-methoxy-β-[[1-[1-oxo-3-(5,6,7,8-tetrahydro-1,8-naphthyridin-2-yl)propyl]-4-piperidinyl]methyl]-3-pyridinepropanoic acid | 467 |
| **28b** | 6-methoxy-β-[[1-[1-oxo-3-(5,6,7,8-tetrahydro-1,8-naphthyridin-2-yl)propyl]-4-piperidinyl]methyl]-3-pyridinepropanoic acid | 467 |

### Example 12

### β-(1,3-Benzodioxol-5-yl)-1-[1-oxo-3-(5,6,7,8-tetrahydro-1,8-naphthyridin-2-yl)propyl]-4-piperidinebutanoic acid (Cpd 11)

To a solution of Compound **12a** (5 g, 20.55 mmol) and NMM (4.96 mL, 45.11 mmol) in anhydrous THF (50 mL) at -20 °C under nitrogen, isobutyl chloroformate (2.67 mL, 20.58 mmol) was added *via* syringe. The mixture was stirred for 30 min and *N,O-*dimethylhydroxylamine (2 g, 20.5 mmol) was added in one portion. The mixture was warmed slowly to rt and stirred for 2 d. After concentration *in vacuo,* the residue was partitioned between EtOAc and 1N HCl. The organic phase was separated, washed with H₂O and saturated NaHCO₃, dried (Na₂SO₄) and concentrated *in vacuo* to afford Compound **12b** as an oil. Compound **12b** was used in the next reaction without further purification. Butyllithium (2.5M in hexane, 4.19 mL, 10.48 mmol) was added dropwise to a solution of 4-bromo-1,2-(methylenedioxy)benzene Compound **12c** (1.26 mL, 10.48 mmol) in THF (40 mL) at -78 °C. The mixture was stirred at -78 °C for 30 min and a solution of Compound **12b** (2 g, 6.98 mmol) in THF (10 mL) was added dropwise. After the mixture was stirred at -78 °C for 30 min, the cooling bath was removed. The mixture was stirred an additional 2 h at rt and quenched with a saturated NH₄Cl solution. The organic phase was separated, washed with brine, dried (Na₂SO₄) and concentrated. The residue was purified via RP-HPLC to yield Compound **12d** as an oil.

Sodium hexamethyldisilazide (1.0M in THF, 2.07 mL, 2.07 mmol) was added dropwise to a solution of trimethyl phosphonoacetate (0.33 mL, 2.07 mmol) in THF (10 mL) at 0 °C. The mixture was stirred at 0 °C for 30 min and a solution of Compound **12d** (0.18 g, 0.52 mmol) in THF (5 mL) was added dropwise. The mixture was heated to reflux for 16 h then stirred at rt for additional 24 h, cooled, diluted with Et₂O (30 mL) and washed with sat. NaHCO₃ and brine. The organic layer was dried (Na₂SO₄) and concentrated. The residue was purified via RP-HPLC to give Compound **12e.** A solution of Compound **12e** (0.5 g, 1.24 mmol) in MeOH (20 mL) was hydrogenated at 280 kPa (40 psi) of H₂ in the presence of 10% palladium on carbon (0.2 g) for 16 h. The catalyst was removed by filtration over celite. The filtrate was concentrated *in vacuo* to yield Compound **12f** as an oil. Compound **12f** was used in the next reaction without further purification. TFA (5 mL) was added to a solution of Compound **12f** (0.37 g, 0.91 mmol) in DCM (20 mL). The mixture was stirred at rt for 30 min, concentrated *in vacuo* and the residue was purified via RP-HPLC to give Compound **12g** as an oil.

To a solution of Compound **8a** (0.28 g, 1.15 mmol) in DMF (40 mL), 1-HOBt (0.135 g, 1.0 mmol), EDC (0.192 g, 1.0 mmol) and DIEA (0.35 mL, 2 mmol) were added under Argon at rt. The mixture was stirred at rt for 45 min. A solution of Compound **12g** (0.28 g, 0.067 mmol) and DIEA (0.35 mL, 2 mmol) in DMF (10 mL) was added to the mixture containing Compound **8a.** The resulting mixture was stirred overnight at rt. Water (2 mL) was added, followed by DCM (20 mL). The organic layer was separated, dried (Na₂SO₄) and concentrated. The resulting crude Compound **12h** was used as such in the next reaction. The crude Compound **12h** was dissolved in MeOH (20 mL) and 3N aqueous NaOH (6 mL) was added. The mixture was stirred at rt for 5 h and neutralized with 2N HCl. After the solvent was evaporated, the residue was purified via RP-HPLC to yield Compound **11.** MS (ES+) m/z 480 (M+H⁺). 1H-NMR of Compound 11: ¹HNMR (CDCL₃, 300 MHz) δ 1.09 (m, 2H,), 1.30 (m, 1H), 1.4-1.7 (m, 3H), 1.86 (m, 1H), 1.94 (m, 2H), 2.47 (m, 1H), 2.58 (d, *J*= 7.5 Hz, 2H), 2.7-3.1 (m, 7H), 3. 15 (m, 1H), 3.51 (br s, 2H), 3.99 (dd, *J* = 5.3 Hz, 14.3 Hz, 2H), 4.49 (dd, *J* = 5.3 Hz, 14.3 Hz, 2H), 5.97 (s, 2H), 6.45 (d, *J* = 7.5 Hz, 1H), 6.66 (d, *J* = 7.8 Hz, 1H), 6.69, (s, 1H), 6.75 (d, *J* = 7.8 Hz, 1H), 7.33 (d, *J* = 7.5 Hz, 1H), 9.82 (s, 2H), 15.0 (s, 1H).

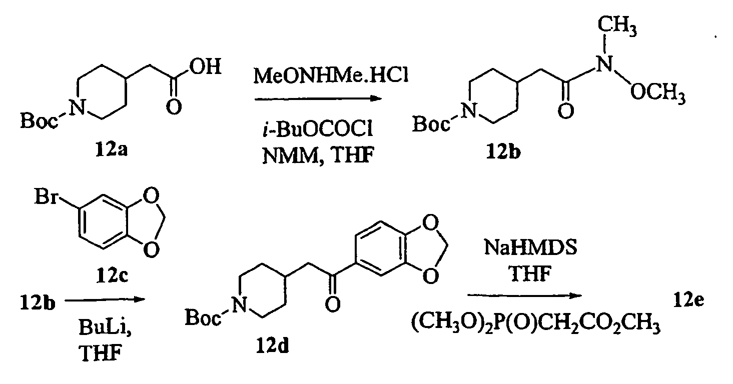

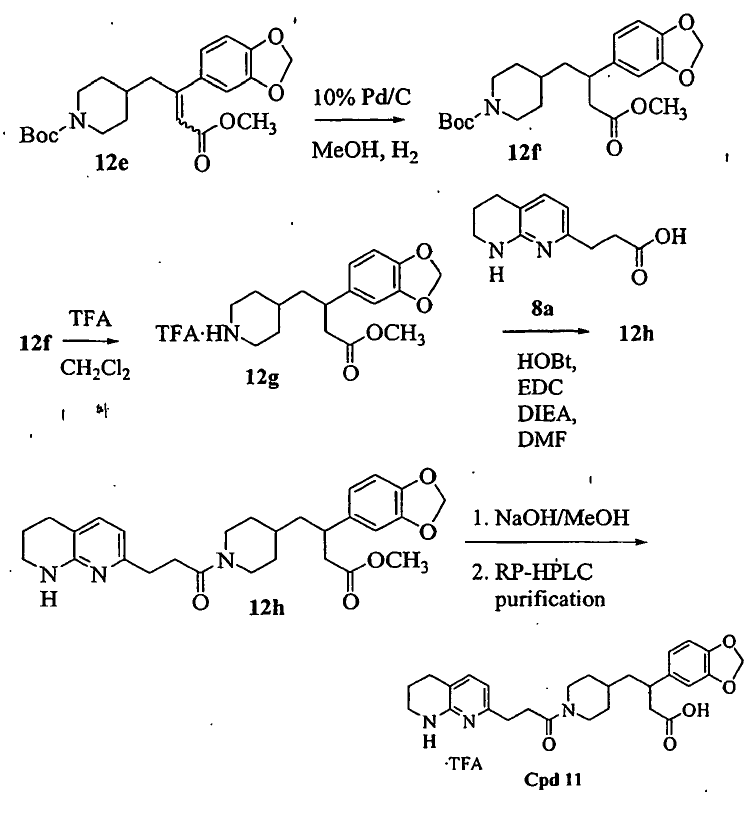

Using the procedure of Example 12 and the appropriate reagents and starting materials known to those skilled in the art, other compounds of the present invention may be prepared including, but not limited to:

| **Cpd** | **Name** | **MS (m/z)** |
|---|---|---|
| **26** | β-(1,3-benzodioxol-5-yl)-1-[1-oxo-4-(5,6,7,8-tetrahydro-1,8-naphthyridin-2-yl)butyl]-4-piperidinebutanoic acid | 494 |
| **27** | β-(1,3-benzodioxol-5-yl)-1-[3-[(1,4,5,6-tetrahydro-5-hydroxy-2-pyrimidinyl)amino]benzoyl]-4-piperidinebutanoic acid | 509 |
| **28** | 6-methoxy-β-[[1-[1-oxo-3-(5,6,7,8-tetrahydro-1,8-naphthyridin-2-yl)propyl]-4-piperidinyl]methyl]-3-pyridinepropanoic acid | 467 |
| **29** | β-[[1-[1-oxo-4-(5,6,7,8-tetrahydro-1,8-naphthyridin-2-yl)butyl]-4-piperidinyl]methyl]-3-quinolinepropanoic acid | 501 |
| **30** | β-(3-fluorophenyl)-1-[1-oxo-3-(5,6,7,8-tetrahydro-1,8-naphthyridin-2-yl)propyl]-4-piperidinebutanoic acid | 454 |
| **31** | β-(3-fluorophenyl)-1-[1-oxo-4-(5,6,7,8-tetrahydro-1,8-naphthyridin-2-yl)butyl]-4-piperidinebutanoic acid | 468 |
| **32** | β-[[1-[1-oxo-3-(5,6,7,8-tetrahydro-1,8-naphthyridin-2-yl)propyl]-4-piperidinyl]methyl]-3-quinolinepropanoic acid | 487 |
| **33** | β-(4-fluorophenyl)-1-[1-oxo-3-(5,6,7,8-tetrahydro-1,8-naphthyridin-2-yl)propyl]-4-piperidinebutanoic acid | 454 |
| **34** | β-(4-fluorophenyl)-1-[1-oxo-4-(5,6,7,8-tetrahydro-1,8-naphthyridin-2-yl)butyl]-4-piperidinebutanoic acid | 468 |
| **35** | 2-methyl-β-[[1-[1-oxo-3-(5,6,7,8-tetrahydro-1,8-naphthyridin-2-yl)propyl]-4-piperidinyl]methyl]-5-pyrimidinepropanoic acid | 452 |
| **36** | β-(2,3-dihydro-6-benzofuranyl)-1-[1-oxo-3-(5,6,7,8-tetrahydro-1,8-naphthyridin-2-yl)propyl]-4-piperidinebutanoic acid | 478 |
| **37** | β-(3,5-difluorophenyl)-1-[1-oxo-3-(5,6,7,8-tetrahydro-1,8-naphthyridin-2-yl)propyl]-4-piperidinebutanoic acid | 472 |
| **38** | β-(3,5-difluorophenyl)-1-[1-oxo-4-(5,6,7,8-tetrahydro-1,8-naphthyridin-2-yl)butyl]-4-piperidinebutanoic acid | 486 |
| **39** | 1-[1-oxo-3-(5,6,7,8-tetrahydro-1,8-naphthyridin-2-yl)propyl]-β-[3-(trifluoromethyl)phenyl]-4-piperidinebutanoic acid | 504 |
| **40** | 1-[1-oxo-3-(5,6,7,8-tetrahydro-1,8-naphthyridin-2-yl)propyl]-β-[4-(trifluoromethoxy)phenyl]-4-piperidinebutanoic acid | 520 |
| **41** | β-(2-fluoro[1,1'-biphenyl]-4-yl)-1-[1-oxo-3-(5,6,7,8-tetrahydro-1,8-naphthyridin-2-yl)propyl]-4-piperidinebutanoic acid | 530 |
| **42** | β-(3-fluoro-4-methoxyphenyl)-1-[1-oxo-3-(5,6,7,8-tetrahydro-1,8-naphthyridin-2-yl)propyl]-4-piperidinebutanoic acid | 484 |
| **43** | 1-[1-oxo-3-(5,6,7,8-tetrahydro-1,8-naphthyridin-2-yl)propyl]-β-(4-phenoxyphenyl)-4-piperidinebutanoic acid | 528 |
| **44** | β-[[1-[1-oxo-3-(5,6,7,8-tetrahydro-1,8-naphthyridin-2-yl)propyl]-4-piperidinyl]methyl]-4-isoquinolinepropanoic acid | 487 |
| **45** | β-[[1-[1-oxo-3-(5,6,7,8-tetrahydro-1,8-naphthyridin-2-yl)propyl]-4-piperidinyl]methyl]-3-pyridinepropanoic acid | 437 |
| **46** | β-(2,3-dihydro-5-benzofuranyl)-1-[1-oxo-3-(5,6,7,8-tetrahydro-1,8-naphthyridin-2-yl)propyl]-4-piperidinebutanoic acid | 478 |
| **47** | 2,4-dimethoxy-β-[[1-[1-oxo-3-(5,6,7,8-tetrahydro-1,8-naphthyridin-2-yl)propyl]4-piperidinyl]methyl]-5-pyrimidinepropanoic acid | 498 |
| **48** | 2-methoxy-β-[[1-[1-oxo-3-(5,6,7,8-tetrahydro-1,8-naphthyridin-2-yl)propyl]-4-piperidinyl]methyl]-5-pyrimidinepropanoic acid | 468 |

### Example 13

### β-[2-[1-[3-[(1,4,5,6-Tetrahydro-2-pyrimidinyl)amino]benzoyl]-4-piperidinyl]ethyl]-3-quinolinepropanoic acid (Cpd 12)

A suspension of lithium aluminum hydride (3.11 g, 0.082 mol) in Et₂O (250 mL) was cooled at -55 °C under Argon. A solution of Compound **3b** (18.5 g, 0.068 mol) in Et₂O (75 mL) was added dropwise over a period of 15 min so that the temperature did not exceed -50 °C. The cooling bath was removed and the mixture was warmed up to 5 °C, cooled again to -35 °C and celite (50 g) was added. The mixture was quenched slowly with bisulphate solution (15.30 g in 43 mL of H₂O) while the temperature was kept at -30 °C. The resulting mixture was warmed to 0 °C, filtered over celite and the solid residue on the filter was washed with EtOAc (750 mL) and H₂O (500 mL). The organic layer was separated, washed with 0.5N HCl (100 mL), saturated NaHCO₃ (100 mL) and brine (100 mL). The aqueous layer was extracted with EtOAc (500 mL) and the combined organic layers were dried, filtered and evaporated. The resulting residue was purified by Kugelrohr distillation (120-140 °C at 1.5-2 mm Hg) to yield Compound **13a** as a colorless oil.

A mixture of 3-bromoquinoline (10.40 g, 0.05 mol), trimethylsilylacetylene (8.48 mL, 0.06 mol), cuprous iodide (0.5 g) and *trans*-dichlorobis(triphenylphosphine)palladium (1 g) and TEA (15 mL) was heated at 70 °C in a sealed tube for 1 h. H₂O (150 mL) was added, followed by Et₂O (300 mL). The organic layer was separated and the aqueous layer extracted with Et₂O (200 mL). The combined organic layers were dried (Na₂SO₄) and concentrated. The residue was purified by flash column chromatography (eluent: 100% DCM) to give 3-(trimethylsilylethynyl) quinoline as a brown oil. 3-(Trimethylsilylethynyl) quinoline was dissolved in anhydrous MeOH (100 mL) and K₂CO₃ (0.69 g, 5 mmol) was added. The mixture was stirred at rt for 1 h and DCM (250 mL) was added. The mixture was filtered over celite. The filtrate was evaporated and the residue was purified by flash column chromatography to give Compound **13b** as an off-white solid.

Butyllithium (2.5M in hexane, 9.44 mL, 23.6 mmol) was added dropwise to a solution of Compound **13b** (3.62 g, 23.6 mmol) in THF (150 mL) under argon, such that the temperature did not exceed -60 °C, then the mixture was cooled to -70 °C. The mixture was stirred at -70 °C for 15 min and a solution of Compound 13a in THF (40 mL) was added dropwise while maintaining the temperature between -60 and -70 °C. After stirring at -70 °C for 30 min, the mixture was warmed to 0 °C over a period of 20 min and H₂O (1 mL) was added. The resulting mixture was dried over K₂CO₃, filtered and evaporated. The residue was purified by flash column chromatography (eluent gradient: DCM/MeOH: 100:0 to 95:5) to yield Compound **13c** as an oil: A mixture of Compound **13c** (6.05 g) in pyridine (100 mL) was hydrogenated in the presence of Lindlar's catalyst (1 g) at 7 kPa (1 psi) of hydrogen for 7 h. The catalyst was removed by filtration over celite and the solvent was evaporated. The residue was purified by flash column chromatography (eluent gradient: hexane/EtOAc: 9:1 to 1:1) to yield Compound **13d** as a solid.

A solution of methyl 3-chloro-3-oxopropionate (1.24 mL, 11.53 mmol) in DCM (20 mL) was added dropwise over a period of 30 min to a solution of Compound **13d** (4.25 g, 11.53 mmol) and TEA (1.81 mL, 13 mmol) in DCM (80 mL) at 0 °C under argon. The mixture was stirred overnight at rt. Aqueous NH₄Cl solution (50 mL) and DCM (150 mL) were added. The organic layer was separated and washed with sat. NaHCO₃ (100 mL) and brine (100 mL), dried (Na₂SO₄), filtered and evaporated. The residue was purified by flash column chromatography (eluent gradient: hexane/EtOAc: 4:1 to 1:1) to yield Compound **13e** as an oil.

A solution of Compound **13e** (4.45 g, 9.5 mmol) in THF (20 mL) was added dropwise to a flask containing sodium hydride (60% in mineral oil, 0.57 g, 14.25 mmol, triple washed with hexane (3 x 25 mL)) at 60 °C under argon. The mixture was heated to 60 °C for 15 min. Chlorotrimethylsilane (2.41 g, 19 mmol) was added via syringe and the mixture was heated for 4 h at 60 °C. H₂O (0.5 mL) was added and the mixture was stirred overnight at rt. The reaction mixture was evaporated, DCM (250 mL) was added and the mixture was dried (Na₂SO₄). After filtration and evaporation, the residue was heated at 130 °C for 2 h under vacuum. Purification by flash column chromatography (eluent: 1% MeOH in DCM) gave Compound **13f** as a yellow oil.

A solution of Compound **13f** (0.375 g, 0.88 mmol) in MeOH (50 mL) was hydrogenated in the presence of 10% palladium on carbon (120 mg) at 7 kPa (1 psi) of hydrogen for 2 h. The catalyst was removed by filtration over celite and the solvent was evaporated to give a crude Compound **13g,** which was used as such for the next reaction. TFA (10 mL) was added to a solution of Compound **13g** (0.35 g, 0.82 mmol) in DCM (10 mL). The mixture was stirred at rt for 1 h and concentrated under vacuum to give crude Compound **13h**, which was used as such for the next reaction.

Isobutyl chloroformate (0.118 mL, 0.90 mmol) was added to a solution of Compound **6g** (230 mg, 0.90 mmol) and NMM (0.385 mL, 3.5 mmol) in DMF (8 mL) under argon at 0 °C. The mixture was stirred at 0 °C for 5 min and a solution of Compound **13h** (0.455 g, 0.82 mmol) in DMF (7 mL) was added dropwise. After the addition was complete, the cooling bath was removed. The mixture was stirred at rt overnight. H₂O (0.5 mL) was added and the mixture was concentrated under high vacuum at 80 °C. The residue was purified by RP-HPLC to yield Compound **13i** as a white powder.

1N aqueous NaOH (10 mL) was added to a solution of Compound **13i** (0.15 g, 0.2 mmol) in 1,4-dioxane (10 mL). The reaction mixture was stirred for 20 h at rt and neutralized with 1N HCl (10 mL). Purification by RP-HPLC yielded Compound 12 as a white powder after lyophilization. MS (ES+) m/z 514 (M+H⁺). 1H-NMR of Compound 12: ¹HNMR (DMSO-*d₆*, 300 MHz) δ 0.97-1.86 (m, 18H), 2.66 (m, 2H), 2.90 (m, 1H), 3.55 (m, 1H), 7.14 (s, 1H), 7. 18 (d, *J* = 8.5 Hz, 1H), 7. 24 (d, *J*=8.5 Hz, 1H), 7.44 (t, *J* = 7.6 Hz, 1H), 7.65 (t, *J* = 7.6 Hz, 1H), 7.78 (t, *J* = 7.6 Hz, 1H), 8.01 (t, *J* = 8.5 Hz, 2H), 8.19 (s, 1H), 8.35 (s, 1H), 8.91 (s, 1H).

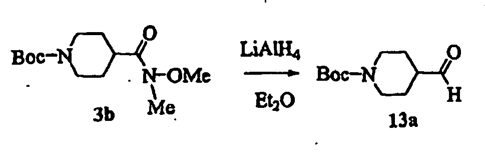

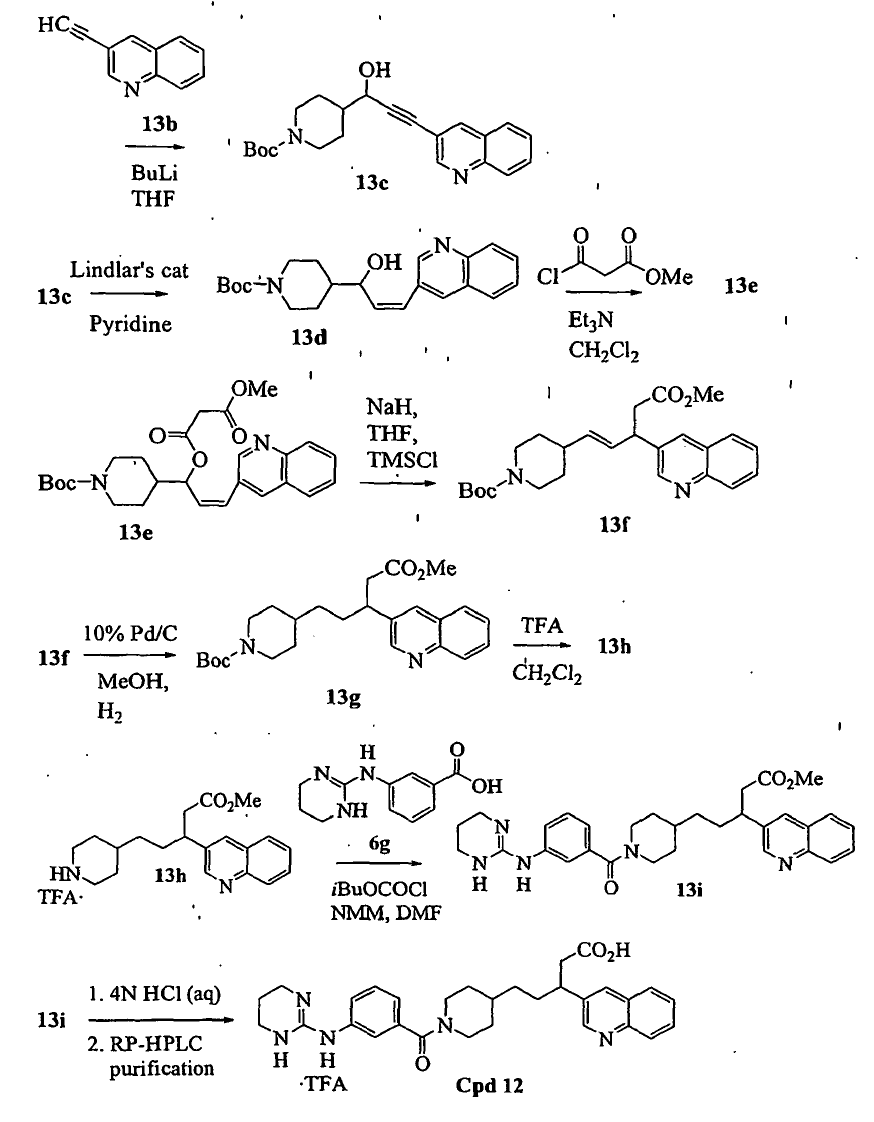

Using the procedure of Example 13 and the appropriate reagents and starting materials known to those skilled in the art, other compounds of the present invention may be prepared including, but not limited to:

| **Cpd** | **Name** | **MS (m/z)** |
|---|---|---|
| **49** | β-[2-[1-[3-[(1,4,5,6-tetrahydro-5-hydroxy-2-pyrimidinyl)amino]benzoyl]-4-piperidinyl]ethyl]-3-quinolinepropanoic acid | 530 |
| **50** | β-[2-[1-[3-[(3,4,5,6-tetrahydro-2-pyridinyl)amino]benzoyl]-4-piperidinyl]ethyl]-3-quinolinepropanoic acid | 513 |
| **51** | β-[2-[1-[1-oxo-3-(5,6,7,8-tetrahydro-1,8-naphthyridin-2-yl)propyl]-4-piperidinyl]ethyl]-3-quinolinepropanoic acid | 501 |
| **5**2 | β-[2-[1-[1-oxo-3-(5,6,7,8-tetrahydro-1,8-naphthyridin-2-yl)propyl]-4-piperidinyl]ethyl]-3-quinolinepropanoic acid | 507 |
| **53** | β-(1,3-benzodioxol-5-yl)-1-[3-[(3,4,5,6-tetrahydro-2-pyridinyl)amino]benzoyl]-4-piperidinepentanoic acid | 506 |
| **54** | β-(1,3-benzodioxol-5-yl)-1-[3-[(1,4,5,6-tetrahydro-5-hydroxy-2-pyrimidinyl)amino]benzoyl]-4-piperidinepentanoic acid | 523 |
| **55** | β-(1,3-benzodioxol-5-yl)-1-[(5,6,7,8-tetrahydro-1,8-naphthyridin-2-yl)acetyl]-4-piperidinepentanoic acid | 480 |

### Example 14

### 1-[1-oxo-3-(5,6,7,8-tetrahydro-1,8-naphthyridin-2-yl)propyl]-β-phenyl-4-piperidinebutanoic acid (Cpd 13)

Di-*tert*-butyl dicarbonate (41.25g, 189 mmol) was added in one portion to a solution of 4-(2-hydroxyethyl)piperidine Compound **14a** (24.42g, 189 mmol) in DMF (200 mL) at 0 °C. After 1 hour, the cooling bath was removed and the reaction mixture was allowed to stir for 20 h at RT. The reaction mixture was treated with Et₂O (200 mL) and H₂O (500 mL). The organic layer was separated, washed with sat NH₄Cl (200 mL) and brine (200 mL) and dried MgSO₄). After filtration and evaporation, Compound **14b** was obtained as a transparent oil and used as such without further purification.

A solution of DMSO (14g, 179 mmol) in DCM (80 mL) was added dropwise over a period of 1.5 h to a 2M solution of oxalyl chloride (62.8mL, 125.6 mmol) in dry DCM (200 mL) at -78 °C, such that the temperature did not exceed -60 °C. A solution of Compound **14a** in DCM (30 mL) was added dropwise at -78°C over a 50 min period. After stirring 30 min at -78 °C, the cooling bath was removed and the temperature of the reaction mixture was allowed to rise to -30 °C over a 30 min period. TEA (25.41 g, 251 mmol) was added and the reaction mixture was allowed to stir for 1h at rt. The solid precipitate that had formed was removed by filtration and the filtrate was washed with 0.3N HCl (2 x 100 mL) and brine (200 mL). The organic phase was dried (Na₂SO₄), evaporated and the residue was purified via flash column chromatography (eluent gradient: hexane/EtOAc 100/0 to 70/30) to yield Compound **14c.**

A 1M solution of LiHMDS (73 mL, 73 mmol) was added via syringe to a solution of trimethyl phosphonoacetate (13.29g, 73 mmol) in THF (200 mL) at -78°C under argon. The reaction mixture was then stirred for 20 min at -78°C and a solution of Compound **14c** (8.3g, 36.5 mmol) in THF (50 mL) was added over a 30 min period. After stirring for 15 min at -78 °C., the cooling bath was remover and the reaction mixture was heated to reflux for 2. The reaction mixture was allowed to cool to room temperature and a saturated NH₄Cl solution (40 mL) was added. Et₂O (200 mL) was added, the organic layer was separated and washed with brine (140 mL) and dried (Na₂SO₄). After filtration and evaporation, the residue was purified via flash column chromatography (eluent gradient: hexane/EtOAc: 100/0 to 85/15), yielding a mixture of *E*- and *Z-*isomers of Compound **14d.**

Compound **14d,** phenyl boronic acid (1.55g, 12.32 mmol), [RhCl(Cod)]2 (0.1g, 0.227 mmol) and Cod (0.557g, 5.15 mmol) were combined in H₂O (15mL) and heated to 100 °C for 3 h under a nitrogen atmosphere. Phenylboronic acid (1.0g, 8.2 mmol) was added again and the reaction mixture was heated to 100 °C for another 6 h. The reaction mixture was allowed to cool to rt, Et₂O (100 mL) was added and the organic layer was separated. The aqueous layer was washed with Et₂O (2 x 100 mL) and the combined organic layers were dried (Na₂SO₄), filtered and evaporated. The residue was purified via flash column chromatography, yielding Compound **14e.**

TFA (6 mL) was added to a solution of Compound **14e** (1.48 g, 4.09 mmol) in DCM (14 mL). The mixture was stirred at rt for 20 min, concentrated under vacuum and purified via RP-HPLC to yield Compound **14f** as a trifluoroacetate salt.

HOBt (0.333 g, 2.46 mmol), EDC (0.47 g, 2.46 mmol) and NMM (0.68 g, 5.28 mmol) were added to a solution of Compound **8a** (0.64 g, 2.64 mmol) in DMF (30 mL) under argon. The mixture was stirred at rt for 1 h, then a solution of Compound **14f** (0.66 g, 1.76 mmol) and NMM (0.68 g, 5.28 mmol) in DMF (10 mL) was added. The resulting mixture was stirred overnight at rt. Water (2 mL) was added, followed by DCM (20 mL). The organic layer was separated, dried (Na₂SO₄) and concentrated. The resulting crude Compound **14g** was used as such in the next reaction. To a solution of Compound **14g** in dioxane (2 mL) and H₂O (1 mL) was added NaOH (0.78g, 19.5 mmol). The mixture was stirred at rt for 5 h and neutralized with 2N HCl. After the solvent was evaporated, the residue was purified by RP-HPLC to give Compound **13** after lyophilization.

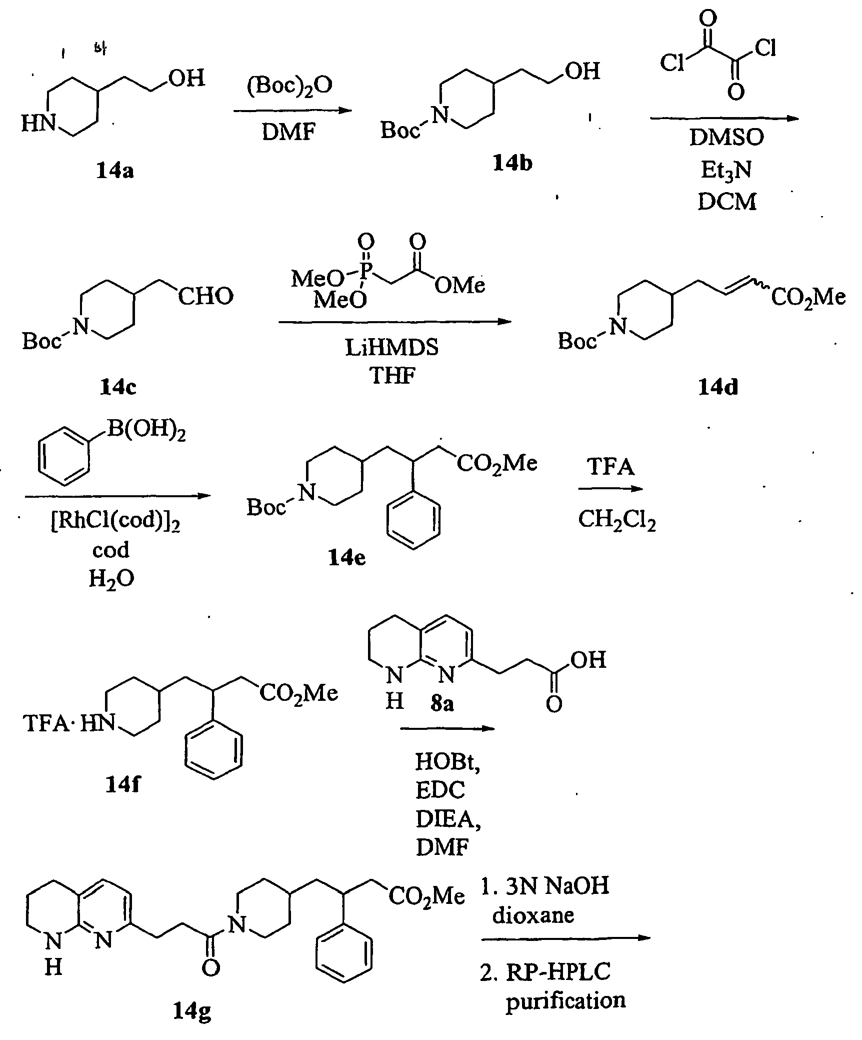

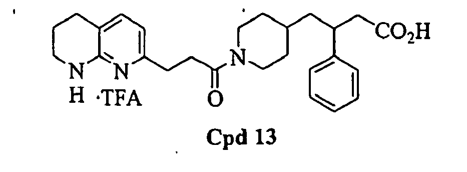

Using the procedure of Example 14 and the appropriate reagents and starting materials known to those skilled in the art, other compounds of the present invention may be prepared including but not limited to:

| **Cpd** | **Name** | **MS (m/z)** |
|---|---|---|
| **56** | β-(2-naphthalenyl)-1-[1-oxo-3-(5,6,7,8-tetrahydro-1,8-naphthyridin-2-yl)propyl]-4-piperidinebutanoic acid | 486 |

and pharmaceutically acceptable salts thereof.

### Example 15

### Isomers 1, 2, 3, and 4 of 1,2,3,4-tetrahydro-β-[[1-[1-oxo-3-(5,6,7,8-tetrahydro-1,8-naphthyridin-2-yl)propyl]-4-piperidinyl]methyl]-3-quinolinepropanoic acid (Cpd 19-1, 19-2, 19-3, 19-4)

To a stirred solution of the Weinreb amide **12b** (3.00 g, 10.48 mmol) and 3-bromoquinoline Compound **15a** (10.9 g, 52.38 mmol) in THF (120 mL) were added dropwise *n*-BuLi (2.5 M solution in hexane; 21.0 mL, 52.38 mmol) over a period of 20 min at -78°C. The reaction mixture was kept below -74 °C during the addition. After the addition, the mixture was stirred for 30 min at -78 °C, and then the cooling bath was removed. The reaction mixture was allowed to warm up to rt over a period of 1 h. The reaction mixture was quenched by the addition of saturated NH₄Cl in water (50 mL), and it was extracted with EtOAc (100 mL). The organic layer was washed with brine (10 mL), and dried over MgSO₄, filtered and concentrated under reduced pressure. The residue was purified by flash column chromatography (30% EtOAc/hexane) to give the ketone Compound **15b** as an amber foam. MS (ES+) m/z 355.4 (M+H⁺). ¹H-NMR (CDCl₃, 300 MHz) δ 1.26 (m, 2H), 1.46 (s, 9H), 1.78 (m, 2H), 2.22 (m, 1H), 2.77 (m, 2H), 3.02 (d, *J* = 7 Hz, 2H), 4.08-4.18 (m, 2H), 7.64 (t, *J* = 7 Hz, 1H), 7.85 (t, *J* = 8 Hz, 1H), 7.96 (d, *J*= 8 Hz,1H), 8.17 (d, *J* = 8 Hz, 1H), 8.70 (br s, I H), 9.42 (br s, 1H).

To a THF (166 mL) solution of trimethyl phosphonoacetate (11.65 mL, 80.58 mmol) was added dropwise NaHMDS (1.0M in THF; 67.2 mL, 67.15 mmol) over a period of 10 min at -78 °C. The resulting partially solidified mixture was stirred at -50°C for 20 min. To the resulting thick solidified mixture, a THF (119 mL) solution of the ketone Compound **15b** (4.76 g, 13.43 mmol) was added at -50°C over a period of 5 min. After the addition, the cooling bath was changed to a water bath and it was stirred for 15 min. The reaction mixture was then refluxed for 2.5 h. The reaction was monitored by HPLC. After cooling to rt, the mixture was diluted with EtOAc (400 mL) and it was washed with saturated NaHCO₃ (50 mLx2), and brine (50 mL). The organic layer was dried over MgSO₄, filtered, and concentrated under reduced pressure. The residue was purified by flash column chromatography (100 g, 6.5x5 cm, 20% to 30% EtOAc/hexane) to give the olefin Compound **15c** as an amber-red syrup, mixture of E,Z-isomers. MS (ES+) m/z 411.3 (M+H⁺).

A MeOH (150 mL) solution of the olefin Compound **15c** (2.76 g, 6.72 mmol) was added to 10% Pd/C (5.52 g as is, 50% water wet). The solution was vacuum/N₂ , degassed and then pressurized to 41 kPa (60 psi) H₂ pressure. The reaction was agitated at rt for 22 h. The reaction mixture was filtered and the filtrates were concentrated under reduced pressure. The residue was purified by flash column chromatography (70 g, 3x25 cm column, eluting with 30% EtOAc/hexane) to afford the hydraquinoline Compound **15d** as a light yellow gum) and Compound **15e** as a minor product

Alternatively, toluene can be used as the solvent. A solution of Compound **15c** (17.14 g, mmol), was combined with 10% Pd/C (8.6 g) in toluene (210 mL) with TEA (2.1 mL). The reaction mixture was shaken on a Parr apparatus at 50 °C and 340 kPa (50 psi) for about 28 h. It was stopped when the hydrogen uptake slowed. After chromatography Compound 15d was isolated. MS (ES+) m/z 417.1 (M+H⁺). ¹NMR (CDCl₃, 300 MHz) δ 1.0-1.6 (m, 6H), 1.45 (s, 9H), 2.0-2.7 (m, 8H), 3.00 (m, 1H), 3.26 (m, 1H), 3.67 (s, 3H), 3.83 (m, 1H), 4.11 (m, 2H), 6.49 (d, *J* = 8Hz, 1H), 6.62 (t, *J*= 7Hz, 1H), 6.97 (m, 2H).

The individual enantiomers of Compound **19** were prepared by separating the isomers **of 15d** and taking them to final product Compounds **19-1, 19-2, 19-3,** and **19-4,** by the same method that Compound **5a** was converted to Compound 5 in Example 5, but using the tetrahydronaphthyridine Compound **8a** instead of **4a.**

The four isomers of Compound **15d** were separated by sequential chiral chromatography. The UV triggered preparative HPLC work was accomplished using a Dynamic Axial Compression type Prochrom LC50 column, which was filled with 500 grams of stationary phase. A Prep LC 4000 (Waters) quaternary gradient low pressure mixing pump, a K-2500 UV detector (KNAUER), a 233 XL auto injector (Gilson), a 402 Syringe pump (Gilson), a 202 fraction collector (Gilson), an rh.7030L fraction collector valve (Gilson), and Unipoint control software (Gilson) were utilized. Isomers (numbered based on elution order: isomer 1 first, eluting) **15d-1** and **15d-2** were separated from isomers **15d-3** and **15d-4** using a Chiralpak® OD column: Cellulose tris-(3,5-dimethylphenylcarbamate) coated on a 20 µm silica-gel, 5 cm ID; 41 cm length ; using methanol as eluent: 100 vol% at 80 mL/min. and a wavelength 220 nM. This resulted in **15d-1** and **15d-2** as a mixture and **15d-3** and **15d-4** as a mixture. The isomers **15d-1** and **15d-2** were separated on a chiral column: Chiralpak® AD: Amylose tris-(3,5-dimethylphenylcarbamate) coated on a 20 µm silica-gel, 5 cm ID, 41 cm length; using ethanol as eluent: 100 vol% at 80 mL/min.; wavelength 220 nM. This results in two pure isomers **15d-1** and **15d-2,** which were individually converted to **19-1** and **19-2,** respectively, by the methods described in Example 5 with the appropriate reagents and starting materials.

The isomers **15d-3** and **15d-4** were separated on a chiral column: Chiralpak® AD, Amylose tris-(3,5-dimethylphenylcarbamate) coated on a 20 µm silica-gel, 500 gr; 5 cm ID; 41 cm length and as eluent using ethanol: 100 vol% at 80 mL/min.; wavelength 220 nM. This resulted in two pure isomers **15d-3** and **15d-4,** which were individually converted to **19-3** and **19-4,** respectively, by the methods described in Example 5 with the appropriate reagents and starting materials.

**Cpds 19-1,19-2,19-3,19-4:** ¹H-NMR (DMSO-*d₆*, 300 MHz) 8 0.86-2.95 (m, 24H), 3.22 (br d, 1H), 3.41 (br s, 2H), 3.82 (br d, 1H), 4.37 (br d, 1H), 6.65 (m, 3H), 6.95 (m, 2H), 7.61 (d, *J*= 7Hz, 1H), 7.95 (br s, 1H).

| Compound No. | Optical Rotation of **15d** (in MeOH) | Compound No. | Optical Rotation of **19** (in MeOH) |
|---|---|---|---|
| **15d-1** | +30° | **19-1** | +15.85° |
| **15d-2** | +62.03° | **19-2** | +24.15° |
| **15d-3** | -64.57° | **19-3** | -24.78° |
| **15d-4** | -30.99° | **19-4** | -14.57° |

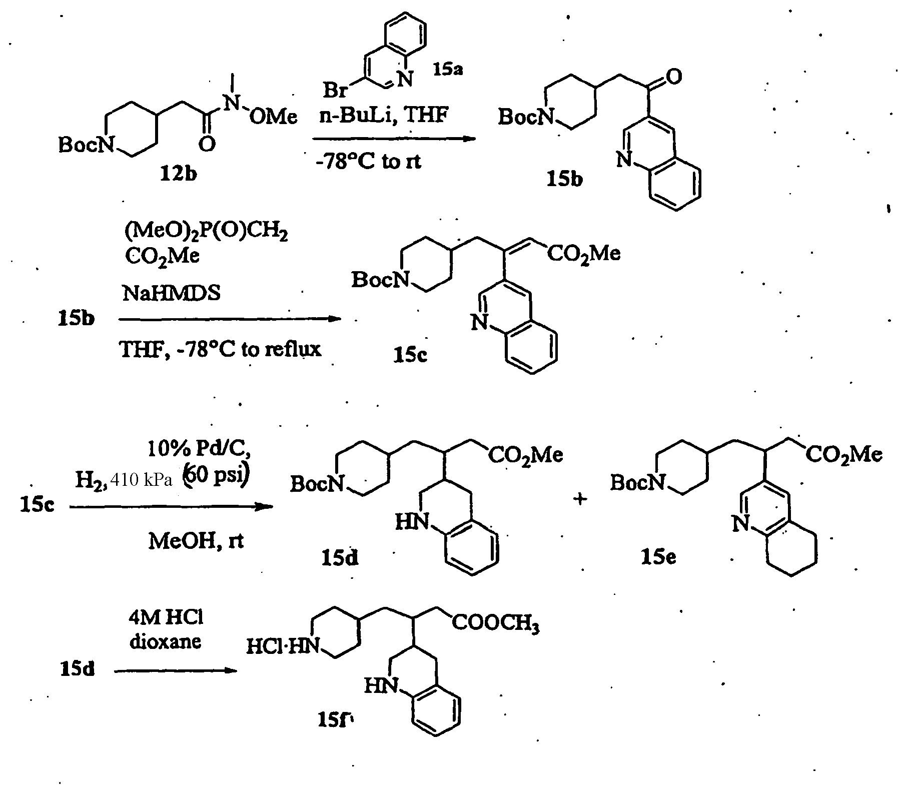

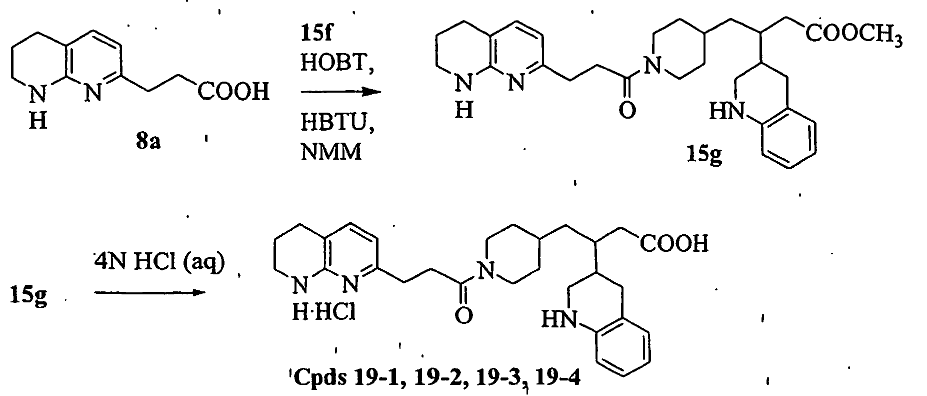

Using the procedures of Example 19 and the appropriate solvents and starting materials known to those skilled in the art, other individual isomers of the compounds of the present invention may be prepared including, but not limited to:

| **Cpd** | **Name** | **MS (m/z)** |
|---|---|---|
| **5-1,** **5-2,** **5-3,** **5-4** | 1,2,3,4-Tetrahydro-β-[1-[1-oxo-4-(5,6,7,8-tetrahydro-1,8-naphthyridin-2-yl)butyl]-4-piperidinyl]-3-quinolinepropanoic acid | 491 |
| | | |
| | | |
| | | |
| **58a** | 5,6,7,8-Tetrahydro-β-[1-[1-oxo-4-(5,6,7,8-tetrahydro-1,8-naphthyridin-2-yl)butyl]-4-piperidinyl]-3-quinolinepropanoic acid | 491 |
| **58b** | 5,6,7,8-Tetrahydro-β-[1-[1-oxo-4-(5,6,7,8-tetrahydro-1,8-naphthyridin-2-yl)butyl]-4-piperidinyl]-3-quinolinepropanoic acid | 491 |

and pharmaceutically acceptable salts thereof.

| Compound No. | Optical Rotation of **5a** (in MeOH) | Compound No. | Optical Rotation of **5** (in MeOH) |
|---|---|---|---|
| **5a-3** | -62° | **5-3** | -26.41 ° |
| **5a-4** | -46° | 5-4 | -19.57° |

### Example 16

### N-Methyl-1,2,3,4-tetrahydro-β-[[1-[1-oxo-3-(5,6,7,8-tetrahydro-1,8-naphthyridin-2-yl)propyl]-4-piperidinyl]methyl]-3-quinolinepropanoic acid (Cpd 67)

Compound **67** was prepared by the same method used to convert Compound **15d** to Compound **19** as described in Example 15, except in this case the intermediate Compound **15d** was alkylated prior to the Boc deprotection step. The alkylated product Compound **16a** was converted to Compound **67** in the same manner Compound **15d** was converted to Compound **19.** Compound **15d** (280 mg, 0.67 mmol) was dissolved in anhydrous DMF (10 mL) and treated with 2,6-di-*tert*-butylpyridine (0.181 mL, 0.81 mmol) and iodomethane (0.050 mL, 0.81 mmol) and left at rt for 20 h. The crude reaction mixture was evaporated and then purified by flash chromatography (20% EtOAc in hexane, few drops of triethyl amine) to yield **16a** (90 mg, 31 %) as a glassy solid. MS (ES+) m/z 431 (M+H⁺). ¹H NMR (DMSO-*d₆*, 300 MHz) δ 1.0-1.7 (m, 7H), 1.45 (s, 9H), 2.0-2.7 (m, 8H), 2.88 (s, 3H), 3.01 (m, 1H), 3.09 (m, 1H), 3.67 (s, 3H), 4.01 (m, 2H), 6.4-6.6 (m, 2H), 6.96 (d, *J* = 7 Hz, 1H), 7.08 (t, *J* = 8 Hz, 1H).

| **Cpd** | **Name** | **MS (m/z)** |
|---|---|---|
| **67** | *N*-Methyl-1,2,3,4-tetrahydro-β-[[1-[1-oxo-3-(5,6,7,8-tetrahydro-1,8-naphthyridin-2-yl)propyl]-4-piperidinyl]methyl]-3-quinolinepropanoic acid | 505 |

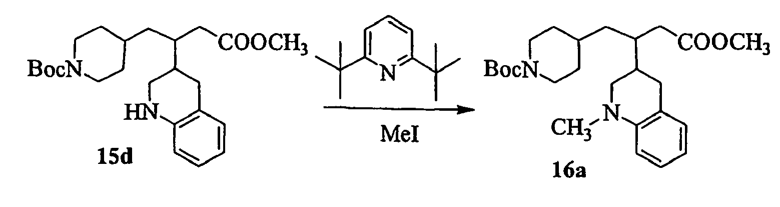

### Example 17

### 4-[1-(3-5,6,7,8-Tetrahydro-[1,8]naphthyridin-2-yl-propionyl)-piperidin-4-yl]-butyric acid tert-butyl ester (Cpd 70)

Using the procedure described in Example 3 for converting Compound **3d** to Compound **3e,** Compound **14d** was converted to Compound **17a.** MS (ES+) m/z 286 (M+H⁺).

Using the procedure described in Example 3 for converting Compound **3e** to Compound **3f,** Compound **17a** was converted to Compound **17b.** MS (ES+) m/z 186 (M+H⁺).

Using the procedure described in Example 14 for converting Compound **14f** to Compound **14g,** Compound **17b** was reacted with Compound **8a** to yield Compound **17c.** MS (ES+) m/z 374.2 (M+H⁺).

3N NaOH (3.21 mL, 9.63 mmol) was added to a solution of Compound **17c** (1.8g, 4.82 mmol) in MeOH (9 mL). The resulting mixture was stirred for 4.5 h at rt. 2N HCl (4.82 mL, 9.64 mmol) was added, and the mixture was concentrated under reduced pressure. DCM was added to the residue, and the solid was removed via filtration. The filtrate was evaporated to yield Compound **17d.** MS (ES+) m/z 360.3 (M+H⁺).

*t*-Butanol (0.476 mL, 4.98 mmol), 1,3-dicyclohexylcarbodiimide (1M in DCM; 1 mL, 1 mmol), and DMAP (1M in DCM; 0.11 mL, 0.11 mmol) were added to a solution of Compound **17d** (0.3g, 0.83 mmol) in DCM (2 mL). The resulting mixture was stirred overnight at rt. The mixture was filtered and concentrated at reduced pressure and the residue was purified by RP-HPLC (10-90% MeCN/water, 0.1% TFA) to yield C Compound **70.** MS (ES+) m/z 388.4 (M+H⁺). ¹H NMR (CDCl₃, 300 MHz) δ 0.98-1.86 (m, 9H), 1.42 (s, 9H), 1.93 (m, 2H), 2.20 (t, *J*= 7.5 Hz, 2H), 2.58 (t, *J*= 7:5 Hz, 1H), 2.68-3.10 (m, 7H), 3.50 (t, *J*= 5.4 Hz, 2H), 4.05 (d, *J*= 12.3 Hz, 1H), 4.54 (d, *J* = 12.3 Hz, 1H), 6.49 (d, *J* = 6.9 Hz, 1H), 7.33 (d, *J*= 6.9 Hz, 1H).

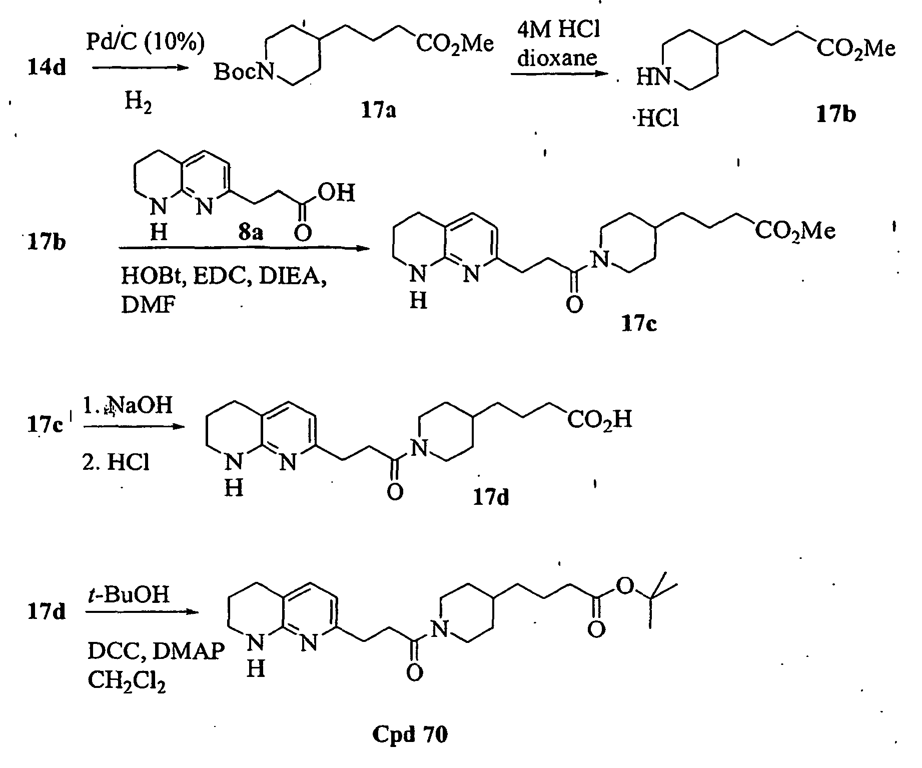

Using the procedure of Example 17 and the appropriate reagents and starting materials know to those skilled in the art, other compounds of the present invention may be prepared including, but not limited to:

| **Cpd** | **Name** | **MS (m/z)** |
|---|---|---|
| **68** | 4-[1-(3-5,6,7,8-Tetrahydro-[1,8]naphthyridin-2-yl-propionyl)-piperidin-4-yl]-butyric acid ethyl ester | 388.4 |
| **69** | 4-[1-(3-5,6,7,8-Tetrahydro-[1,8]naphthyridin-2-yl-propionyl)-piperidin-4-yl]-butyric acid isopropyl ester | 402.3 |
| **71** | 4-[1-(3-5,6,7,8-Tetrahydro-[1,8]naphthyridin-2-yl-propionyl)-piperidin-4-yl]-butyric acid octyl ester | 472.5 |
| **72** | 4-[1-(3-5,6,7,8-Tetrahydro-[1,8]naphthyridin-2-yl-propionyl)-piperidin-4-yl]-butyric acid isobutyl ester | 416.4 |
| **73** | 4-[1-(3-5,6,7,8-Tetrahydro-[1,8]naphthyridin-2-yl-propionyl)-piperidin-4-yl]-butyric acid methyl ester | 374.2 |

### Example 18

### 4-[1-(3-5,6,7,8-Tetrahydro-[1,8]naphthyridin-2-yl-propionyl)-piperidin-4-yl]-butyric acid 2,2-dimethyl-propionyloxymethyl ester (Cpd 74)

3N NaOH (3.21 mL, 9.63 mmol) was,added to a solution of Compound **17c** (1.8g, 4.82 mmol) in MeOH (10 mL). The resulting mixture was stirred for 4 h at rt and concentrated at reduced pressure to yield **18a**. MS (ES+) m/z 360.3 (M+H⁺).

Chloromethyl pivalate (0.21 mL, 1.46 mmol) and 25% aqueous Nal (0.13 mL) were added to a suspension of Compound **18a** (0.5g, 1.3 mmol) in acetone (10 mL) and the resulting mixture was heated to reflux for 5 h. The solvent was removed at reduced pressure and the residue was purified by RP-HPLC (10-90% MeCN/water, 0.1% TFA) to yield Compound **74.** MS (ES+) m/z 474.3 (M+H⁺). ¹H NMR (CDCl₃, 300 MHz) δ 1.05 (m, 2H), 1.20 (s, 9H), 1.27 (m, 2H), 1.50 (m, 1H), 1.67 (m, 2H), 1.77 (m, 2H), 1. 95 (m, 2H), 2.37 (t, *J* = 7.8 Hz, 2H), 2.57 (t, *J* = 13.2 Hz, 1H), 2.75 (t, *J* = 7.5 Hz, 2H), 2.82 (m, 2H), 2.95-3.10 (m, 3H), 3.51 (t, *J* = 6 Hz, 2H), 4.05 (d, *J* = 13.2 Hz, 1H), 4.56 (d, *J* = 13.2 Hz, 1H), 5.76 (s, 2H), 6.50 (d, *J* = 7.5 Hz, 1H), 7.33 (d, *J* = 7.5 Hz, 1H).

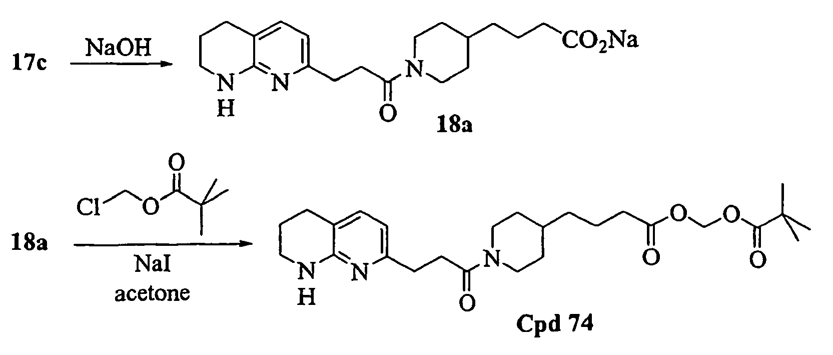

### Example 19

### 3-(2,3-Dihydro-benzofuran-6-yl)-4-[1-(3-5,6,7,8-tetrahydro-[1,8]naphthyridin-2-yl-propionyl)-piperidin-4-yl]-butyric acid (Cpd 36a)

Using the procedure described in Example 12 for converting Compound **12b** to Compound **12d,** Compound **12b** was converted to Compound **19b** upon reaction with *n*-BuLi and 6-bromo-2,3-dihydrobenzofuran **19a** (Compound **19a** was obtained in three steps from 1,4-dibromo-2-fluorobenzene as described in Organic Letters (2001), 3(21), 3357-3360). MS (ES+) m/z 368.4 (M+Na⁺).

Using the procedure described in Example 12 for converting Compound **12d** to Compound **12e,** Compound **19b** was converted to Compound **19c.** MS (ES+) m/z 424.4 (M+Na⁺).

Using the procedure described in Example 12 for converting Compound **12e** to Compound **12f,** Compound **19c** was converted to Compound **19d.** MS (ES+) m/z 426.5 (M+Na⁺).

Racemic Compound **19d** was separated into the two enantiomerically pure Compounds **19e** and **19f on** a chiral column using methanol as eluent (Stationary phase: Chiralpak AD 20 µm (Daicel); eluent: methanol; column diameter: 50 mm; detector: 0.5 mm Knauer superpreparative cell; wavelength: 225 nm). Compound **19f** (second eluting isomer): [a]²⁰_{D} -24.3 (c 0.717, MeOH). Compound **19e** (first eluting isomer): [a]²⁰D +24.8 (*c* 0.775, MeOH).

Using the procedure described in Example 12 for converting Compound **12f** to Compound **12g,** Compound **19f** was converted to Compound **19g.** MS (ES+) m/z 304.4 (M+H⁺).

Using the procedure described in Example 12 for converting Compound **12g** to Compound **12h,** Compound **19g** was converted to Compound **19h.** MS (ES+) m/z 492 (M+H⁺).

The crude Compound **19h** was dissolved in MeOH (20 mL) and 3N aqueous NaOH (6 mL) was added. The mixture was stirred at rt for 5 h and neutralized with 2N HCl. After the solvent was evaporated, the residue was purified via RP-HPLC to yield Compound **36a.** MS (ES+) m/z 478.8 (M+H⁺). ¹HNMR (CDCl₃, 300 MHz) δ 1.09 (1.07 (m, 2H), 1.27 (m, 1H), 1.40-1.86 (m, 3H), 1.73-2.0 (m, 3H), 2.42 (t, *J*= 12.5 Hz, *J*= 4.4 Hz, 1H), 2.55 (d, *J* = 7.3, Hz, 2H), 2.67-3.24 (m, 10H), 3.5 (br s, 2H), 3.93 (dd, *J* =19.8 Hz, *J* =16.2 Hz, 1H), 4.43 (dd, *J* = 16.2 Hz, *J* = 14.7 Hz, 1H), 4.57 (t, *J* = 7.5 Hz, 1H), 6.62 (s, 1H), 6.67 (d, *J* = 8.1 Hz, 1H), 7.10 (d, *J* = 8.1 Hz, 1H), 7.33 (d, *J* = 7.5 Hz, 1H), 8.41 (br s, 1H). Anal. Calcd for C₂₈H₃₅N₃O₄-1.05 HCl-0.6 H₂O: C, 63.86; H, 7.13; N, 7.98; Cl, 7.07; H₂O, 2.06. Found: C, 63.67; H, 7.32; N, 8.12; Cl, 6.94; H₂O, 1.91. [α]²⁰_{D}-31.1 (c0.675, MeOH).

Enantiomer **36b** was obtained from the fast moving enantiomer Compound **19e** using procedures described for converting **19f** to Compound **36a.**

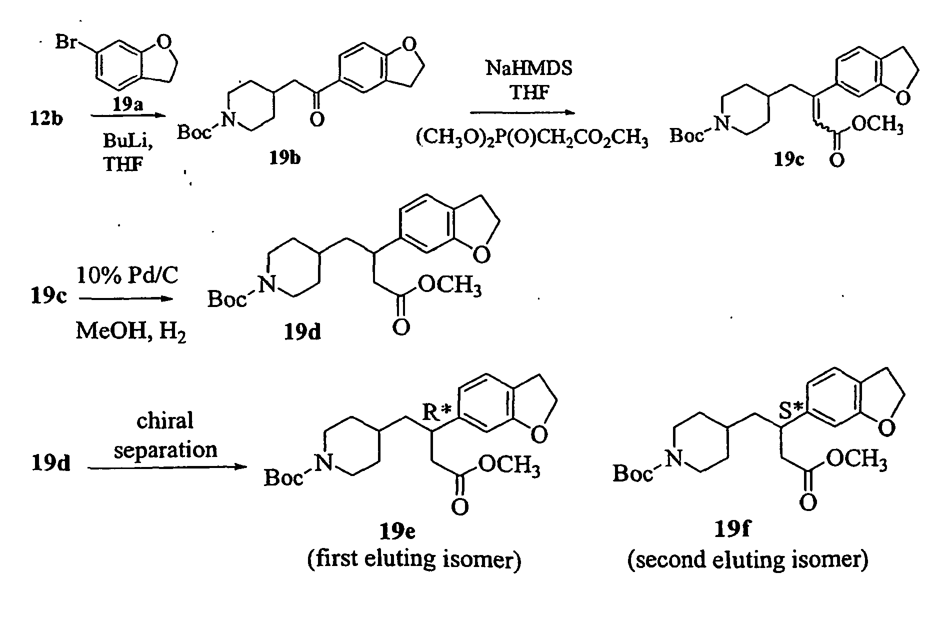

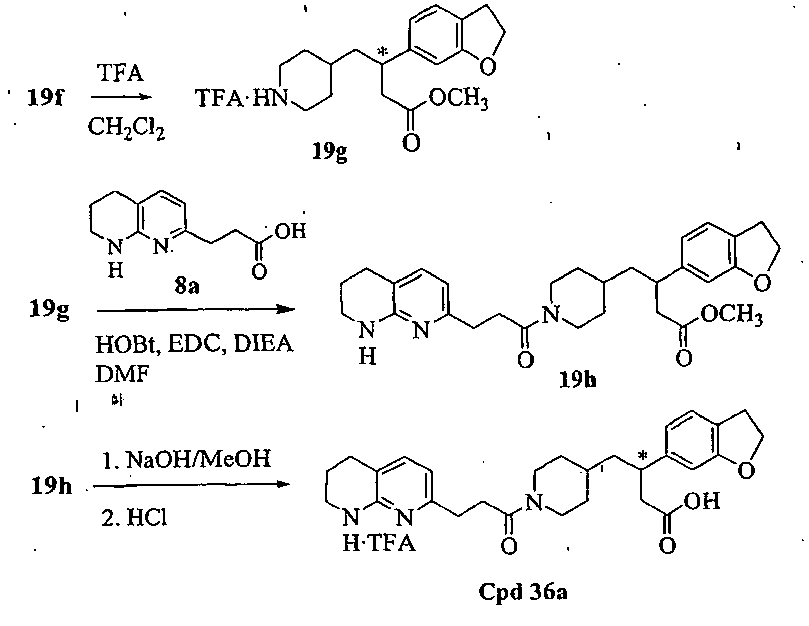

### Example 20

### 3-(4-Hydroxy-3-methoxy-phenyl)-4-[1-(3-5,6,7,8-tetrahydro-[1,8]naphthyridin-2-yl-propionyl)-piperidin-4-yl]-butyric acid (Cpd 76)

To a solution of bromo-methoxyphenol Compound **20a** (10g, 49.2 mmol) and *N,N-*diethyl-N-diisopropylamine (0.7g, 54.2 mmol) in dry DCM (100 mL) was added 2-(trimethylsilyl)ethoxymethyl chloride (9.03g, 54.2 mmol). The resulting mixture was stirred for 2 h at rt, and water and brine were added. The organic layer was separated and dried over Na₂SO_{4.} The solvent was removed under reduced pressure and the residue was purified via flash column chromatography (silica gel; eluent:hexane:EtOAc; 9:1) to yield Compound **20b.** MS (ES+) m/z 396/398 (M+H⁺).

Using the procedure described in Example 12 for converting Compound **12b** to Compound **12d,** Compound **12b** was converted to Compound **20c.** MS (ES+) m/z 502.2 (M+Na⁺).

Using the procedure described in Example 12 for converting Compound **12d** to Compound **12e,** Compound **20c** was converted to Compound **20d.** MS (ES+) m/z 558.2 (M+Na⁺).

Using the procedure described in Example 12 for converting Compound **12e** to Compound **12f,** Compound **20d** was converted to Compound **20e.** MS (ES+) m/z 408.3 (M+H⁺).

Using the procedure described in Example 12 for converting Compound **12f** to Compound **12g,** Compound **20e** was converted to Compound **20f.** MS (ES+) m/z 308.1 (M+H⁺).

Using the procedure described in Example 12 for converting Compound **12g** to Compound **12h,** Compound **20f** was converted to Compound **20g.** MS (ES+) m/z 496.8 (M+H⁺).

Using the procedure described in Example 12 for converting Compound **12h** to Compound **11,** Compound **20g** was converted to Compound **76.** MS (ES+) m/z 482.4 (M+H⁺). ¹HNMR (DMSO-*d₆*, 300 MHz) δ 0.93 (m, 2H), 1.25 (m, 1H), 1.5 (m, 3H), 1.8 (m, 3H), 2.4.7 (m, 6H), 2.72 (m, 3H), 2.83 (d, *J* = 7.3 Hz, 2H), 2.99 (m, 1H), 3.40 (br s, 2H), 3.74 (s, 3H), 3.77 (dd, *J* = 14.7 Hz, *J* = 14.3 Hz, 1H), 4.28 (dd, *J* = 14.7 Hz, *J* = 14.3 Hz, 1H), 6.60 (d, *J* = 8.1 Hz, 1H), 6.63 (d, *J* = 7.2 Hz, 1H), 6.66 d, *J* = 8.1 Hz, 1H), 6. 77 (br s, 1H), 7.59 (d, *J* = 7.2 Hz, 1H), 8.04 (br s, 1H).

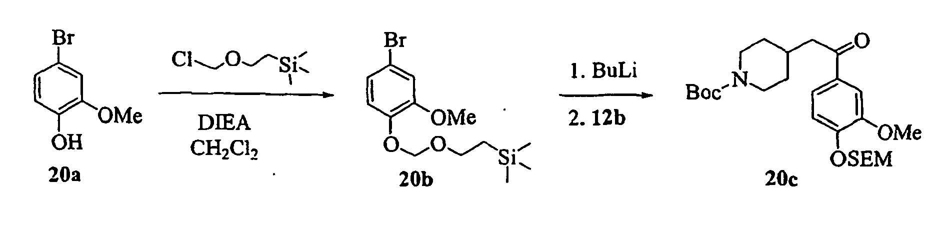

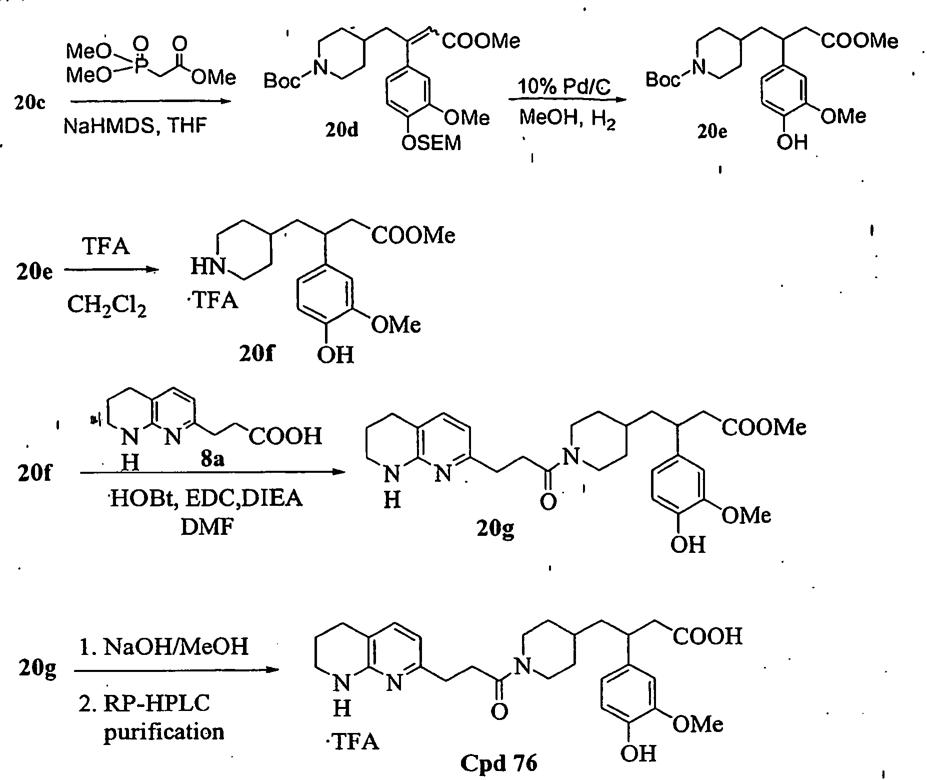

Derivatives in which the hydroxyl substituent of Compound 76 is alkylated or acylated can be made using general methods, starting materials, and reagents known to one killed in the art.

### Example 21

### 3-(3-Methylamino-phenyl)-4-[1-(3-5,6,7,8-tetrahydro-[1,8]naphthyridin-2-yl-propionyl)-piperidin-4-yl]-butyric acid (Cpd 79)

A solution of 3-bromoaniline Compound **21a** (2 mL, 18.4 mmol), di-tert-butyl dicarbonate (4.05g, 18.6 mmol) in THF (20 mL) was heated to reflux for 30 h under N₂. The mixture was evaporated under reduced pressure, and the residue was dissolved in EtOAc. The solution was washed with saturated NaHCO₃ solution and brine. The organic layer was dried over MgSO₄, filtered, and evaporated, to yield Compound **21b.** MS (ES+) m/z 256.8/258.8 (M-CH₃).

Sodium hydride (60% in oil; 0.78g, 19.5 mmol) was added in small portions to a solution of Compound **21b** (4.18g, 15.4 mmol) and methyl iodide (1.21 mL, 19.5 mmol) in DMF (50 mL) at 0 °C. The resulting mixture was allowed to warm to rt and stirred for 1 h. The mixture was poured in ice-water and extracted with EtOAc. The organic layer was separated, dried over MgSO₄, filtered, and evaporated under reduced pressure to yield Compound **21c.** MS (ES+) m/z 270.9/272.9 (M-CH₃).

Using the procedure described in Example 12 for converting Compound **12b** to Compound **12d,** Compound **21c** was converted to Compound **21d.** MS (ES+) m/z 455.0 (M+Na⁺).

Using the procedure described in Example 12 for converting Compound **12d** to Compound **12e,** Compound **21d** was converted to Compound **21e.** MS (ES+) m/z 510.9 (M+Na⁺).

Using the procedure described in Example 12 for converting Compound **12e** to Compound **12f,** Compound **21e** was converted to Compound **21f.** MS (ES+) m/z 512.8 (M+Na⁺).

Using the procedure described in Example 12 for converting Compound **12f** to Compound **12g,** Compound **21f was** converted to Compound **21g.** MS (ES+) m/z 291.0 (M+H⁺).

Using the procedure described in Example 12 for converting Compound **12g** to Compound **12h,** Compound **21g** was converted to Compound **21h.** MS (ES+) m/z 479.0 (M+H⁺).

Using the procedure described in Example 12 for converting Compound **12h** to Compound **11,** Compound **21h** was converted to Compound **79.** MS (ES+) m/z 465.0 (M+H⁺). ¹HNMR (DMSO-*d₆*, 300 MHz) δ 0.99 (m, 2H), 1.21 (m, 1H), 1.4-1.65 (m, 3H), 1.72 (m, 1H), 1.86 (m; 2H), 2.3-3.0 (m, 13H), 3.17 (m, 1H), 3.42 (m, 2H), 3.87 (dd, *J* = 17.7 Hz, *J* = 15.2 Hz, 1H), 4.40 (dd, *J* = 15.2 Hz, *J* = 11.6 Hz, 1H), 6.41 (d, *J* = 7.5 Hz, 1H), 7.1-7.4 (m, 5H).

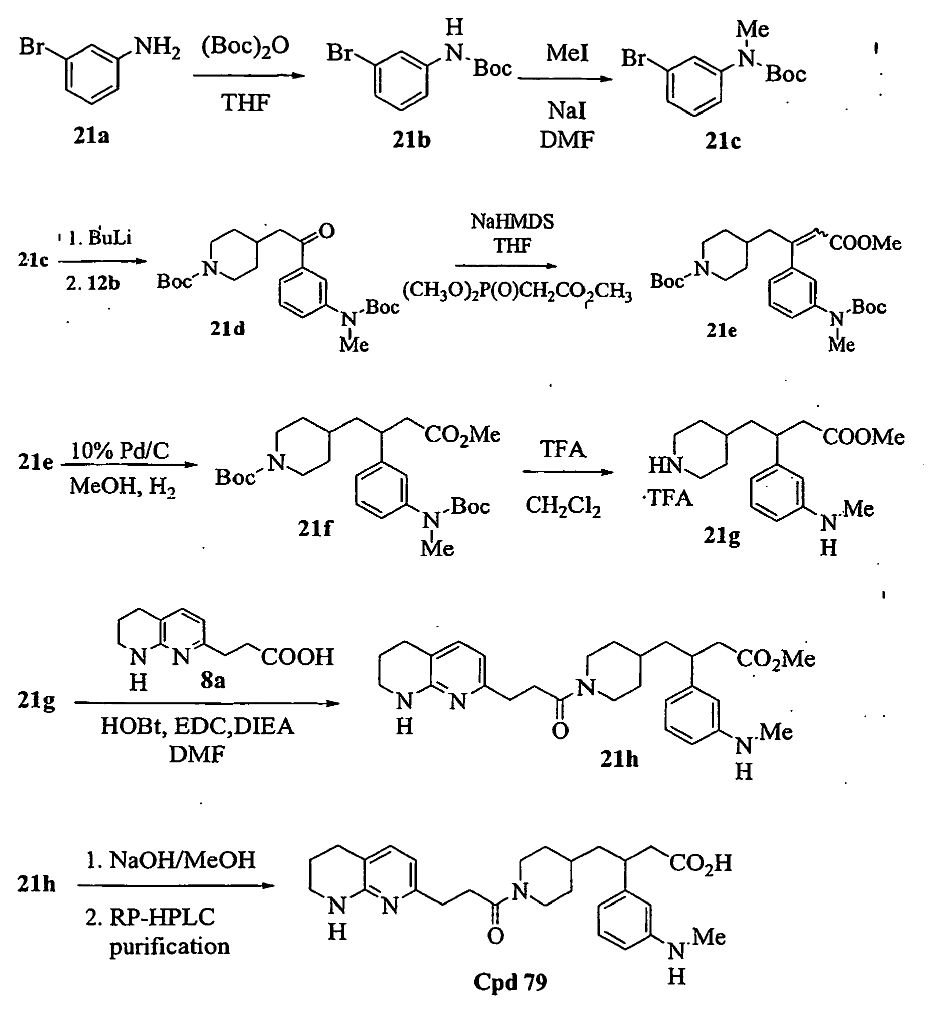

Using the procedure of Example 21 and the appropriate reagents and starting materials known to those skilled in the art, other compounds of the present invention may be prepared including, but not limited to:

| **Cpd** | **Name** | **MS (m/z)** |
|---|---|---|
| **78** | 3-(3-Ethylamino-phenyl)-4-[1-(3-5,6,7,8-tetrahydro-[1,8]naphthyridin-2-yl-propionyl)-piperidin-4-yl]-butyric acid | 479.0 |

### Example 22

### 3-Naphthalen-2-yl-4-[1-(3-5 ,6,7 ,8-tetrahydro-[1,8]naphthyridin-2-yl-propionyl)-piperidin-4-yl]-butyric acid (Cpd 56a)

Using the procedure described in Example 19 for converting Compound **12b** to Compound **19b,** Compound **12b** was converted to Compound **22a** upon reaction with 2-bromonaphthalene. MS (ES+) m/z 376 (M+Na⁺).

Using the procedure described in Example 19 for converting Compound **19b** to Compound **19c,** Compound **22a** was converted to Compound **22b.** MS (ES+) m/z 432.1 (M+Na⁺).

Using the procedure described in Example 19 for converting Compound **19c** to Compound **19d,** Compound **22b** was converted to Compound **22c.** MS (ES+) m/z 434.1 (M+Na⁺).

Racemic Compound **22c** was separated into the two enantiomerically pure Compounds **22d** and **22e** on a chiral column using ethanol as eluent (Stationary phase: Chiralpak AD 20 µm (Daicel); column diameter: 50 mm; detector: 0.5 mm Knauer superpreparative cell; wavelength: 225 nm). **22d** (first eluting isomer): [α]²⁰_{D} +0.177 (c 0.75, MeOH). **22e** (second eluting isomer): [α]²⁰_{D} -0.167 (c 0.653, MeOH).

Using the procedure described in Example 19 for converting Compound **19f to** Compound **19g,** Compound **22e** was converted to Compound **22f.** MS (ES+) m/z 312.0 (M+H⁺).

Using the procedure described in Example 19 for converting Compound **19g** to Compound **19h,** Compound **22f** was reacted with Compound **8a** to yield Compound **22g.** MS (ES+) m/z 500.0 (M+H⁺).

Using the procedure described in Example 19 for converting Compound **19h** to Compound **36a,** Compound **22g** was converted to Compound **56a .** MS (ES+) m/z : 486.0(M+H⁺). ¹HNMR (CDCl₃, 300 MHz) δ 0.95-1.35 (m, 3H), 1.44-2.0 (m, 6H), 2.35 (t, *J* = 12.7 Hz, 1H), 2.55-3.1 (m, 9H), 3.40 (m, 3H), 3.89 (m, 1H), 4.42 (m, 1H), 6.45 (d, *J* = 7.4 Hz, 1H), 7.24 (d, *J* = 7.4 Hz, 1H), 7.35 (d, *J* = 8.1 Hz, 1H), 7.45 (m, 2H), 7.65 (s, 1H), 6.45 (d, *J* = 7.4 Hz, 1H), 7.7-7.85 (m, 3H). Anal. Calcd for C₃₀H₃₅N₃O₃-1.1 HCl-0.75 H₂O: C, 66.83; H, 7.03; N, 7.80; Cl, 7.24; H₂O, 2.51. Found: C, 66.53; H, 7.26; N, 8.15; Cl, 7.27; H₂O, 2.39. [α]²⁰_{D} -0.193 (c 0.717, MeOH).

Enantiomer **56b** was obtained from the fast moving enantiomer **22d** using procedures described for converting **22e** to Compound **56a.**

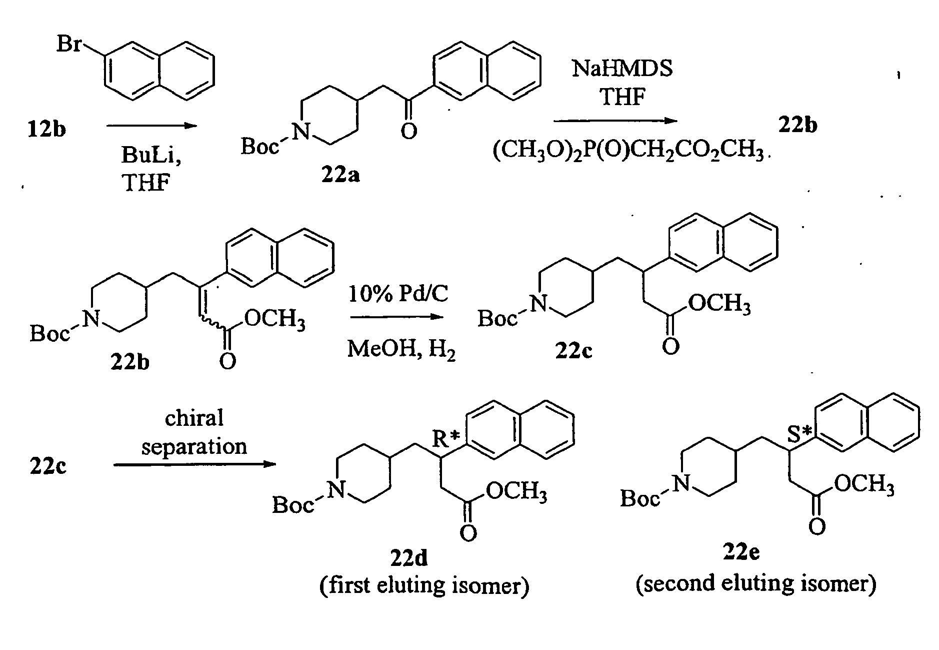

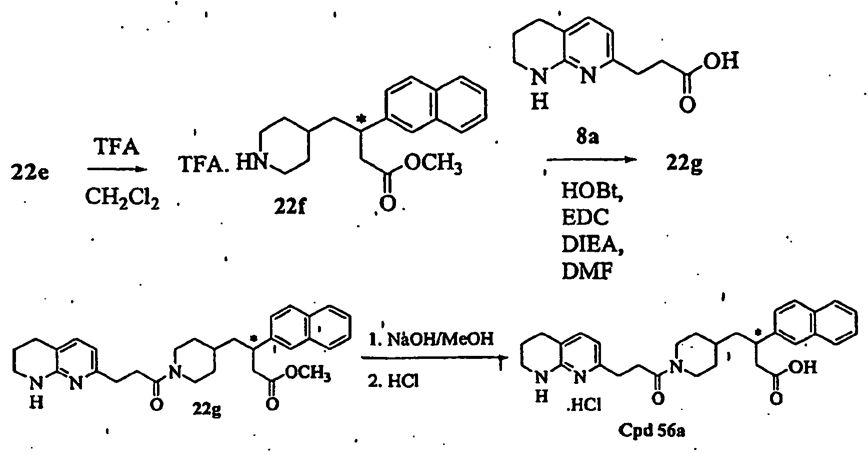

### Example 23

### 3-(3-Fluoro-phenyl)-4-[1-(3-5,6,7,8-tetrahydro-[1,8]naphthyridin-2-yl-propionyl)-piperidin-4-yl]-butyramide (Cpd 64)

Using the procedure described in Example 12 for converting Compound **12b** to Compounds **12d**, Compound **12b** was converted to Compound **23a** upon reaction with 1-bromo-3-fluorobenzene. MS (ES+) m/z 344 (M+Na⁺).

Using the procedure described in Example 12 for converting Compound **12d** to Compound **12e**, Compound **23a** was converted to Compound **23b** upon reaction with Diethyl cyanomethylphosphonate. MS (ES+) m/z 367.4 (M+Na⁺).

A solution of of Compound **23b** (2.06g, 5.98 mmol) in EtOH (50 mL) was hydrogenated a 34 kPa (5 psi) in the presence of 10% palladium on carbon (200 mg) for 40 h. The catalyst was removed by filtration over celite. The filtrate was concentrated in vacuo to yield Compound **23c**. MS (ES+) m/z 369.5 (M+Na⁺).

Using the procedure described in Example 12 for converting Compound **12f** to Compound **12g**, Compound **23c** was converted to Compound **23d**. MS (ES+) m/z 247 (M+H⁺).

Using the procedure described in Example 12 for converting Compound **12g** to Compound **12h,** Compound **23d** was reacted with Compound **8a** to yield Compound **23e.** MS (ES+) m/z 435 (M+H⁺).

A mixture of Compound **23e** (150 mg, 0.345 mmol) and 12N HCl (10 mL) was heated to 40 °C for 3 h. The mixture was evaporated to dryness and further dried by lyophilization to yield Compound **64.** MS (ES+) m/z 453.5 (M+Na⁺). ¹HNMR (DMSO-*d₆*, 300 MHz) δ 0.8-1.1 (m, 2H), 1.25 (m, 1H), 1.4-1.65 (m, 3H), 1.7-1.9 (m, 4H), 2.25-2.5 (m, 4H), 2.7-2.9 (m, 8H), 3.21 (m, 1H), 3.82 (t, *J* = 13.6 Hz, 1H), 4.31 (t, *J*=13.6 H, 1H), 6.66 (d,*J*= 7.3Hz, 1H), 6.71 (br s, 1R), 6.95-7.15 (m, 3H), 7.25 (br s, 1H), 7.36 (dd, *J* = 15.1 Hz, *J* = 7.3 Hz, 1H), 7.63 (d, *J* = 7.3 Hz, 1H), 7.98 (br s, 1H), 13.77 (br s, 1H).

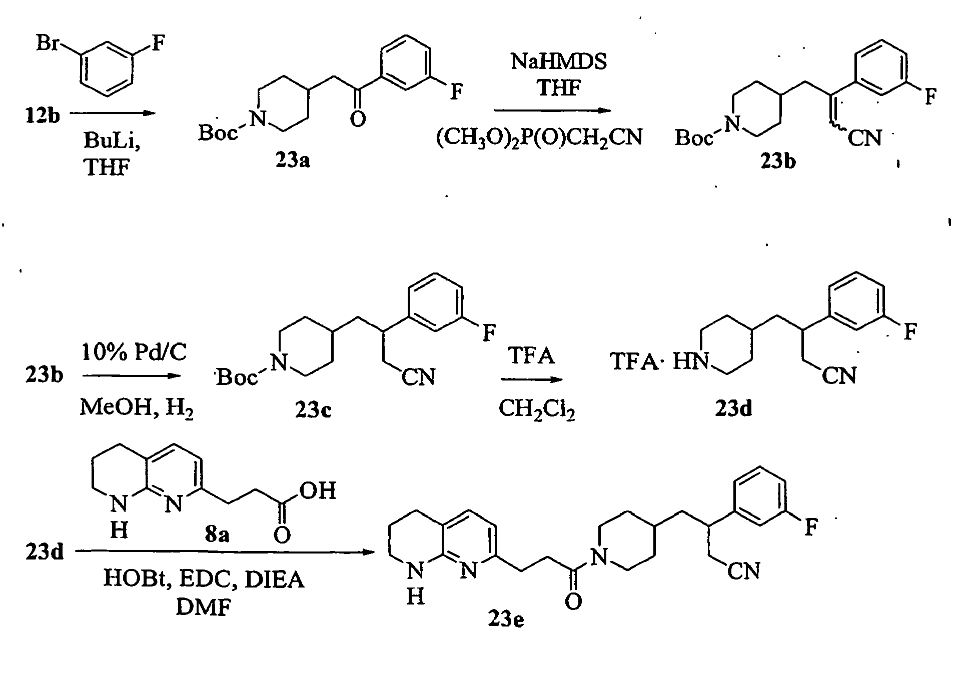

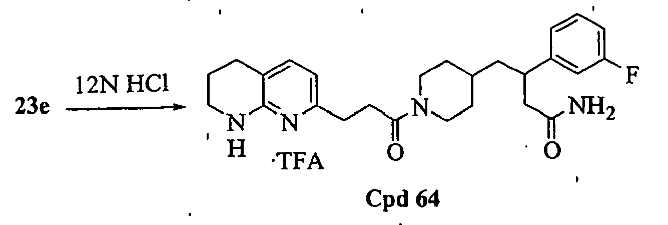

### Example 24

### 3-(3-Fluoro-phenyl)-4-[1-(3-5,6,7,8-tetrahydro-[1,8]naphthyridin-2-yl-propyl]-piperidin-4-yl]-butyric acid (Cpd 81)

Lithium aluminum hydride (1.0M in THF; 16.5 mL, 16.5 mmol) was added slowly to a suspension of Compound **8a** (2.0g, 8.2 mmol) in dry THF (60 mL) at 0 °C. The cooling bath was removed, and the mixture was stirred for 24 hr at rt. The mixture was quenched with water and celite was added. The mixture was extracted with Et₂O and EtOAc. The organic phase was dried over Na₂SO₄, filtered, and concentrated under reduced pressure, yielding Compound **24a.** MS (ES+) m/z 193.2 (M+H+).

Compound **24a** (0.5g, 2.6 mmol) was added to a suspension of pyridinium chlorochromate (0.67g, 3.12 mmol) in DCM (5 mL). The mixture was stirred overnight at rt. Diethyl ether was added, and the mixture was filtered. The filtrate was dried over Na₂SO₄. After removal of the drying agent via filtration, the solvent was removed under reduced pressure, yielding a mixture of **24a** and **24b** that was used as such for the next reaction. Compound **24b:** MS (ES+) m/z 191.1 (M+H⁺).

Sodium triacetoxyborohydride (25.6 mg, 0.074 mmol) was added to a mixture of **24a** and **24b** (0.01g, 0.05 mmol) and piperidine Compound **24c** (0.01g, 0.05 mmol; obtained using the procedure described in Example 12 for converting Compound **12a** to Compound **12g,** and wherein bromo-3-fluorobenzene was substituted for the 4-bromo-1,2-(methylenedioxy)benzene (Compound **12c**) and was reacted to form a 3-fluorophenyl compound analogous to compound **12f**) in DCM (0.2 mL) and the mixture was stirred for 4 hr at rt. Diethyl ether was added, and the organic layer was separated and dried over Na₂SO₄. The drying agent was removed by filtration, and the solvent was removed under reduced pressure. The residue was purified via column chromatography (eluent gradient: DCM:MeOH:NH₄OH; 1.00:0:0 to 90:9:1) to yield Compound **24d.** MS (ES+) m/z 454.4 (M+H⁺).

Using the procedure described in Example 12 for converting Compound **12h** to Compound **11,** Compounds **24d** was converted to Compound **81.** MS (ES+) m/z 440.5 (M+H⁺).

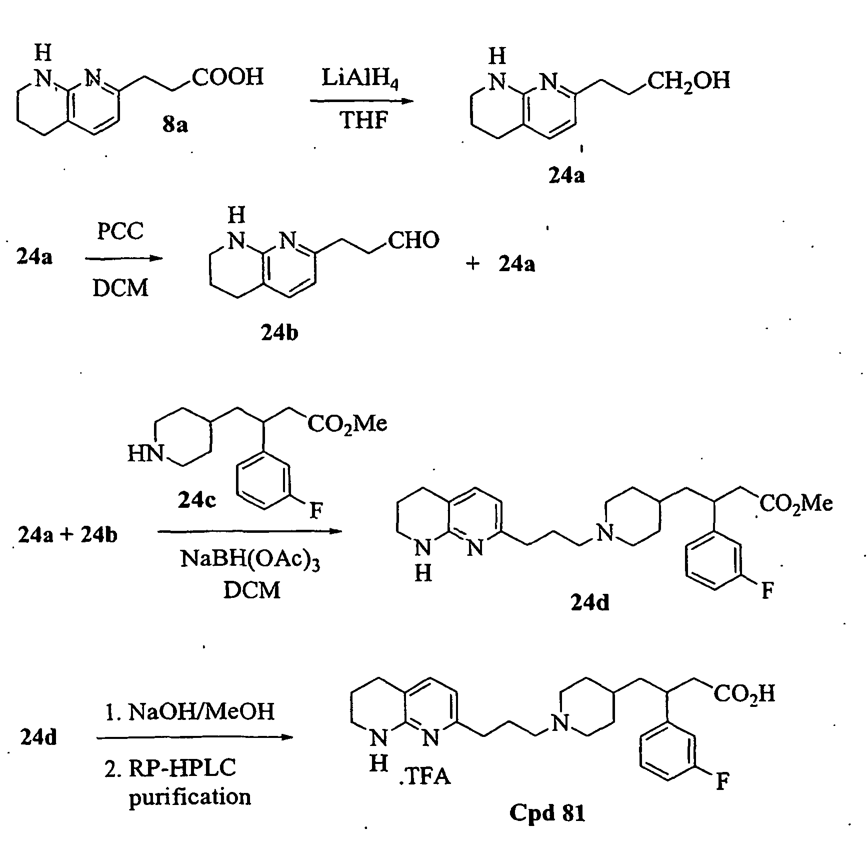

### Example 25

### β-(3-fluorophenyl)-1-[1-oxo-3-(5 ,6,7,8-tetrahydro-1, 8-naphthyridin-2-yl)propyl]-4-piperidinebutanoic acid (Cpd 30a and 30b)

Compound **30** was synthesized following the process set forth in Example 12 wherein bromo-3-fluorobenzene was substituted for the 4-bromo-1,2-(methylenedioxy)benzene (Compound **12c**) and was reacted to form a 3-fluorophenyl compound analogous to compound **12f.**

Additional Compound **30** was resolved into two isomers (Cpd **30a** and Cpd **30b**) by generally following the procedure set in Example 19, wherein the stationary phase was Chiralcel OD; eluent: hexane/EtOH: 95/5; wavelength: 220 nm. The isomer of most interest was the second eluting isomer. The separated isomers were converted into Compounds **30a** and **30b** by completion of the synthesis from Compound **12f** on as set forth in Examples 12 to yield Compounds **30a** and **30b.**

### Prospective Example 26

### 3-(2,3-Dihydro-benzofuran-6-yl)-4-[1-(3-5,6,7,8-tetrahydro-[1,8]naphthyridin-2-yl-butyl)-piperidin-4-yl]-propanoic acid (Cpd 80)

Using the procedure described in Example 3 for converting Compound **3b** to Compound **3c**, Compound **3b** may be converted to provide Compound **26a** when reacted with 6-bromo-2,3-dihydrobenzofuran.

Using the procedure described in Example 3 for converting Compound **3c** to Compound **3d,** Compound **26a** may be converted to provide Compound **26b.**

Using the procedure described in Example 3 for converting Compound **3d** to Compound **3e,** Compound **26b** may be converted to provide Compound **26c.**

Using the procedure described in Example 3 for converting Compound **3e** to Compound **3f,** Compound **26c** may be converted to provide Compound **26d.**

Using the procedure described in Example 3 for converting Compound **3f** to Compound **3g,** Compound **26d** may be converted to provide Compound **26e.**

Using the procedure described in Example 4 for converting Compound **4a** to Compound **4b,** Compound **26e** may be converted to provide Compound **26f.**

Using the procedure described in Example 4 for converting Compound **4b** to Compound **4,** Compound **26f** may be converted to provide Compound **80.**

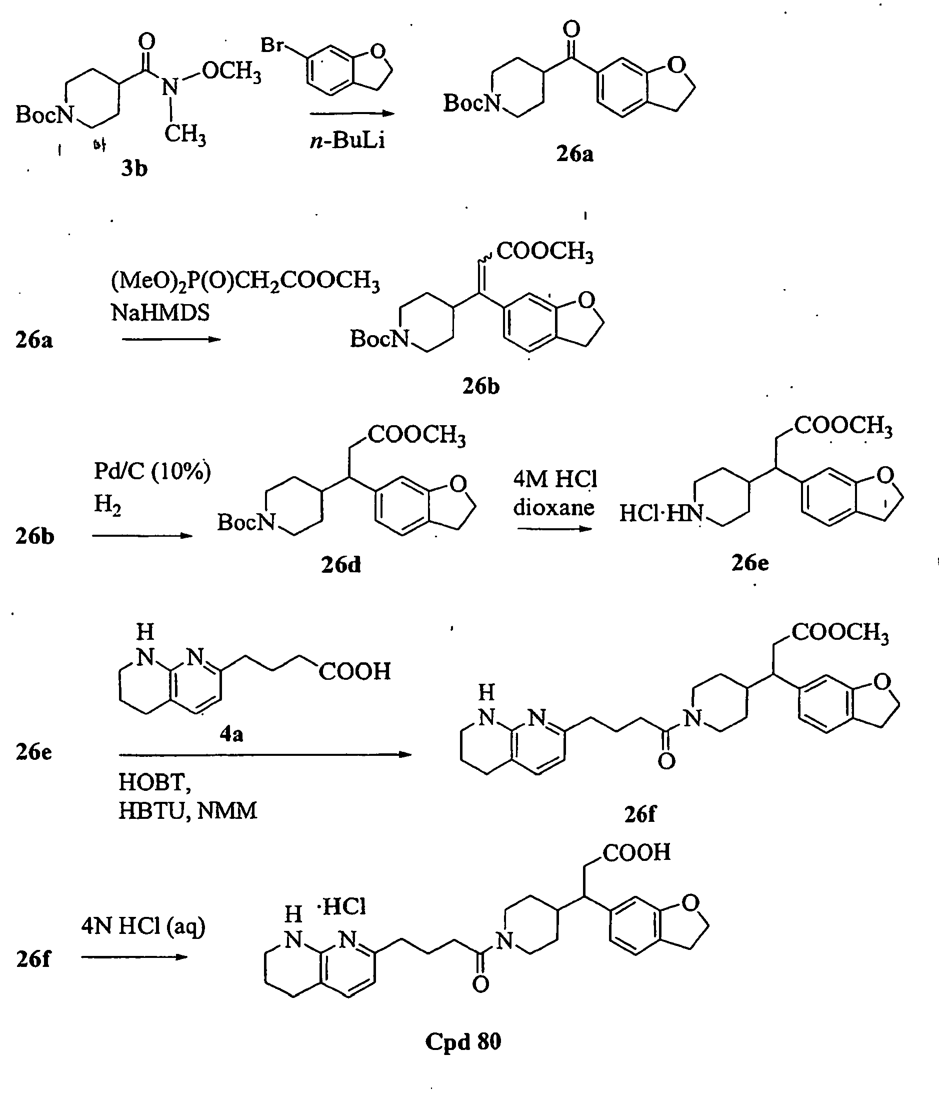

### Biological Experimental Examples

As demonstrated by biological studies described hereinafter, as shown in Table I, the compounds of the present invention are αvβ3 and αvβ5 integrin receptor antagonists useful in treating an integrin mediated disorder.

### Example 1

### In Vitro Solid Phase Purified αυβ3 Binding Assay

The vitronectin/αvβ3 binding assay methods were derived from Mehta et al. (Biochem J,. 1998, 330, 861). Human αᵥβ₃ (Chemicon International Inc., Temecula, CA), at a concentration of 1 µg/ml dissolved in Tris buffer (20 mM Tris, 1 mM CaCl₂, 1mM MgCl₂, 10 µM MuCl₂, 150 mM NaCl), was immobilized on Immulon 96 well plates (Dynex Technologies, Chantilly, VA) overnight at 4 °C. Plates were washed and treated with blocking buffer (3 % BSA in Tris buffer) for 2 h at 37 °C. Plates were then rinsed 2 times with assay buffer comprised of Tris buffer. Synthesized compounds were added to wells in duplicate immediately prior to the addition of 2 nM vitronectin (Sigma, St. Louis, MO). Following a 3 hour incubation at 37 °C, plates were washed 5 times in assay buffer. An anti-human vitronectin IgG rabbit polyclonal antibody (Calbiochem, San Diego, CA) was added (1:2000) and plates were incubated for 1 hour at room temperature. VectaStain ABC peroxidase kit reagents (Vector Laboratories, Burlingame, CA) employing a biotin labeled anti-rabbit IgG, were utilized for detection of bound antibody. Plates were read at 490 nm on a Molecular Devices (Sunnyvale, CA) microplate reader. Table 1 shows the results of the in vitro solid phase purified αvβ3 binding assay for representative compounds of the present invention.

### Example 2

### In Vitro Solid Phase Purified GP IIb/IIIa Blinding Assay

A 96 well Immulon-2 microtiter plate (Dynatech-Immulon) was coated with 50 µL/well of RGD-affinity purified GP IIb/IIIa (effective range 0.5-10 µg/mL) in 10 mM HEPES, 150 mM NaCl, 1 mM MgCl₂ at pH 7.4. The plate was covered and incubated overnight at 4°C. The GP IIb/IIIa solution was discarded and 150 µL, of 5% BSA was added and incubated at RT for 1-3 h. The plate was washed extensively with modified Tyrodes buffer. Biotinylated fibrinogen (25 µL/well) at 2 x final concentration was added to the wells that contain the test compounds (25 µL/well). The plate was covered and incubated at RT for 2-4 h. Twenty minutes prior to incubation completion, one drop of Reagent A (VectaStain ABC Horseradish Peroxidase kit, Vector Laboratories, Inc.) and one drop Reagent B were added with mixing to 5 mL modified Tyrodes buffer mix and let stand. The ligand solution was discarded and the plate washed (5 x 200 µL/well) with modified Tyrodes buffer. Vecta Stain HRP-Biotin-Avidin reagent (50 µL/well, as prepared above) was added and incubated at RT for 15 min. The Vecta Stain solution was discarded and the wells washed (5 x 200 µL/well) with modified Tyrodes buffer. Developing buffer (10 mL of 50 mM citrate/phosphate buffer @ pH 5.3, 6 mg *o*-phenylenediamine, 6 µL 30% H₂O₂; 50 µL/well) was added and incubated at RT for 3-5 min and then 2 N H₂SO₄ (50 µL/well) was added. The absorbance was read at 490 nM. Table 1 shows the results of the in-vitro solid phase purified GP IIb/IIIa binding assay for representative compounds of the present invention.

### Example 3

### In Vitro Solid Phase Purified αvβ5 Binding Assay

The vitronectin/αᵥβ₅ binding assay method was performed in the same manner as the vitronectin/αᵥβ₃ binding assay of Example 2, with the difference that 1 µg/mL of human purified αᵥβ₅ (Chemicon International, Inc.) was immobilized onto Immulon 96 well plates (Dynex Technologies) instead of αᵥβ₃. All other aspects of the assay including buffers, reagents and incubation times remain unchanged.

**Table 1**

| **Cpd** | **αᵥ**β**₃IC₅₀ (uM)** | | **αᵥ**β**₅ IC₅₀ (uM)** | | **α_{IIb}**β**3 IC₅₀ (uM)** | |
|---|---|---|---|---|---|---|
| **1** | 0.0560 ± 0.007 | N=2 | >5 | ND | 4.33 ± 0.15 | N=2 |
| **2** | 5.4000± | N=1 | | | 4.78 ± 1.013 | N=2 |
| **3** | 0.0036 ± 0.0004 | N=5 | 2.5 | | 0.21 | N=1 |
| **4** | 0.0005 ± 0.0001 | N=3 | 0.0355 ± 0.0089 | N=4 | 0.87 ± 0.19 | N=2 |
| **5** | 0.0037 ±0.0014 | N=3 | 0.2607 ± 0.0569 | N=3 | 14.84 ± 0.68 | N=2 |
| **5-3** | 0.1613 | N=1 | >5 | N=1 | ND | |
| **5-4** | 0.0054 ± 0.0002 | N=3 | 0.1616 ± 0.0627 | N=3 | 9.82 | N=1 |
| **6** | 0.0076 ±0.0021 | N=2 | 0.54 | N=1 | 1.62 ± 0.05 | N=2 |
| **7** | 0.0082 ± 0.0014 | N=2 | 0.0395 ± 0.0085 | N=2 | 1.67 ± 0.74 | N=2 |
| **8** | 0.0179 ± 0.0034 | N=4 | 0.253 | N=1 | 1.36 ±0.43 | N=2 |
| **9** | >1 | N=1 | ND | | 8.51 ± 2.36 | N=2 |
| **10** | 0.0024 ±0.0013 | N=2 | 0.0335 ± 0.0075 | N=2 | 1.67 | N=1 |
| **11** | 0.0011 ± 0.0002 | N=3 | 0.0023 ± 0.0009 | N=3 | 2.52 ± 0.30 | N=2 |
| **12** | 0.0042 ± 0.0014 | N=3 | 0.078 ± 0.017 | N=2 | 0.136 ± 0.003 | N=2 |
| **13** | 0.0032 ± 0.0006 | N=2 | 0.036 ± 0.0133 | N=2 | 11.09 ± 3.40 | N=2 |
| **14** | 0.0361 ± 0.0001 | N=2 | 0.108 ± 0.034 | N=1 | 5.04 | N=1 |
| **15** | 0.0019 ± 0.0002 | N=4 | 0.0334 ± 0.0063 | N=4 | 4.03 ± 0.43 | N=2 |
| **16** | 0.2810 | N=1 | 0.775 | N=1 | 25.38 | N=1 |
| **17** | 0.0008 ± 0.0001 | N=4 | 0.0313 ± 0.0060 | N=4 | 6.60 ± 1.42 | N=2 |
| **18** | >5 | N=1 | >5 | N=1 | >50 | N=1 |
| **19** | 0.0025 ± 0.0004 | N=3 | 0.0171 ± 0.0025 | N=3 | 13.77 ± 9.69 | N=2 |
| **19-1** | 0.0367 | N=1 | 1.12 | N=1 | >50 | N=1 |
| **19-2** | 0.0013 ± 0.0001 | N=2 | 0.0092 ± 0.0004 | N=2 | 12.9 | N=1 |
| **19-3** | 0.0447 ± 0.0204 | N=2 | 1.17 ± 0.02 | N=2 | ND | |
| **19-4** | 0.0013 ± 0.0007 | N=3 | 0.0075 ± 0.0018 | N=3 | 4.86 | N=1 |
| **20** | 0.1417 ± 0.027 | N=3 | 0.995 | N=1 | 1.80 | N=1 |
| **21** | 0.0280 ± 0.0031 | N=3 | 0.78 | N=1 | 1.80 ± 0.63 | N=2 |
| **21b** | 0.405 | N=1 | 0.28 | N=1 | 1.97 | N=1 |
| **21a** | 0.0213 ± 0.0019 | N=3 | 0.8413 ± 0.4054 | N=3 | 5.31 | N=1 |
| **22** | 0.0046 ± 0.0008 | N=3 | 0.195 | N=1 | 0.43 ± 0.07 | N=2 |
| **23** | 0.2980 ± 0.1460 | N=2 | 2.010 | N=1 | 4.93 | N=1 |
| **24** | 0.3070 | N=1 | 0.387 | N=1 | 19.30 | N=1 |
| **25** | 0.0456 ± 0.0066 | N=2 | 0.773 ± 0.118 | N=2 | 8.67 ± 1.72 | N=2 |
| **26** | 0.0277 ± 0.0053 | N=2 | 0.5 | N=1 | 5.92 | N=1 |
| **27** | 0.0480 | N=1 | 0.81 | N=1 | 1.62 ± 0.56 | N=2 |
| **28** | 0.0007 ± 0.0002 | N=3 | 0.0027 ± 0.0008 | N=4 | 6.10 ± 2.44 | N=2 |
| **28a** | 0.0003 ± 0.0002 | N=2 | 0.0042 ± 0.0018 | N=2 | 1.83 ± 0.57 | N=2 |
| **28b** | 0.0208 ± 0.0053 | N=2 | 0.1262 ± 0.0448 | N=2 | 24.26 | N=1 |
| **29** | 0.0022 ± 0.0008 | N=3 | 0,119 ± 0.0150 | N=3 | 1.74 ± 0.89 | N=2 |
| **30** | 0.0010 ± 0.0002 | N=3 | 0.0028 ± 0.0001 | N=3 | 14.39 ± 5.98 | N=2 |
| **30a** | 0.0004 ± 0.0002 | N=3 | 0.0019 ± 0.0004 | N=3 | 2.93 ± 1.86 | N=2 |
| **30b** | 0.0317 ± 0.0147 | N=2 | 0.0482 ± 0.0028 | N=2 | >50 | N=1 |
| **31** | 0.0330 | N=1 | 0.3 | N=1 | 21.57 ± 4.87 | N=2 |
| **32** | 0.0008 ± 0.0002 | N=3 | 0.0022 ± 0.0007 | N=3 | 1.055 ± 0.56 | N=2 |
| **33** | 0.0013 ± 0.0004 | N=3 | 0.0226 ± 0.0052 | N=3 | >50 | N=1 |
| **34** | 0.1476 ± 0.1004 | N=2 | 1.041 ± 0.109 | N=2 | >50 | N=1 |
| **35** | 0.0007 ± 0.0004 | N=2 | 0.0007 ± 0.0002 | N=3 | 0.965 ± 0.07 | N=2 |
| **36** | 0.0008 ± 0.00006 | N=4 | 0.0007 ± 0.0002 | N=3 | 3.11 ± 0.04 | N=2 |
| **36a** | 0.0004 | N=3 | 0.0009 ± 0.0006 | N=2 | 0.79 ± 0.05 | N=3 |
| **36b** | 0.084 | N=1 | 0.129 | N=1 | >50 | N=1 |
| **37** | 0.0158 ± 0.0043 | N=2 | 0.0897 ± 0.0116 | N=3 | >50 | N=1 |
| **38** | 0.4840 | N=1 | 2.11 | N=1 | >50 | N=1 |
| **39** | 0.0066 ± 0.0018 | N=2 | 0.0287 ± 0.0133 | N=2 | >50 | N=1 |
| **40** | 0.0052 ± 0.0002 | N=2 | 0.308 ± 0.0630 | N=2 | 23.95 ± 9.89 | N=2 |
| **41** | 0.0018 ± 0.0010 | N=2 | 0.8725 ± 0.1575 | N=2 | 19.3 ± 2.60 | N=2 |
| **42** | 0.0007 ± 0.0003 | N=3 | 0.0189 ± 0.0046 | N=3 | 5 ± 0.74 | N=2 |
| **43** | 0.0079 ± 0.0007 | N=2 | 0.2225 ± 0.0885 | N=2 | 28.82 ± 15.8 | N=2 |
| **44** | 0.0022 ± 0.0009 | N=3 | 0.002 ± 0.0006 | N=3 | 5.44 ± 1.1 | N=2 |
| **45** | 0.0008 ± 0.0001 | N=3 | 0.0017 ± 0.0003 | N=3 | 6.61 ± 2.85 | N=2 |
| **46** | 0.0035 ± 0.0006 | N=2 | 0.0659 ± 0.0171 | N=2 | 13.64 ± 1.33 | N=2 |
| **47** | 0.0014 ± 0.0007 | N=3 | 0.0046 ± 0.0017 | N=3 | 1.47 ± 0.37 | N=2 |
| **48** | 0.0010 ± 0.0005 | N=3 | 0.0033 ± 0.0014 | N=3 | 1.21 ± 0.20 | N=2 |
| **49** | 0.0018 ± 0.0005 | N=3 | 0.0895 ± 0.0255 | N=2 | 0.16 ± 0.02 | N=3 |
| **50** | 0.0156 ± 0.0044 | N=4 | 0.676 | N=1 | 0.19 ± 0.04 | N=2 |
| **51** | 0.0030 ± 0.0006 | N=4 | 0.169 ± 0.019 | N=2 | 0.48 ± 0.01 | N=2 |
| **52** | 0.0064 ± 0.0014 | N=4 | >50 | N=1 | 0.57 ± 0.04 | N=2 |
| **53** | 0.0298 ± 0.0137 | N=5 | 0.1375 ± 0.0415 | N=2 | 0.94 ± 0.05 | N=2 |
| **54** | 0.0017 ± 0.0005 | N=3 | 0.0347 ± 0.0117 | N=3 | 0.24 | N=1 |
| **55** | 0.0950 | N=1 | 0.737 | N=1 | 15.59 | N=1 |
| **56** | 0.0019 ± 0.0006 | N=3 | 0.0245 ± 0.0065 | N=2 | 39.12 ± 0.785 | N=2 |
| **56a** | 0.0005 ± 0.0002 | N=3 | 0.0265 ± 0.0034 | N=3 | 14.66 | N=1 |
| **56b** | 0.3263 ± 0.0894 | N=3 | 0.8096 ± 0.1045 | N=3 | ND | |
| **57** | 0.0016 ± 0.0007 | N=3 | 0.0109 ± 0.0042 | N=3 | 3.04 ± 0.55 | N=2 |
| **58a** | 0.0004 ± 0.0003 | N=3 | 0.0323 ± 0.0082 | N=3 | 1.44 ± 0.39 | N=2 |
| **58b** | 0.083 ± 0.020 | N=2 | 0.5760 ± 0.1490 | N=2 | 35.5 | N=1 |
| **59** | 0.0026 ± 0.0014 | N=2 | 0.0096 ± 0.0038 | N=2 | 7.805 ± 4.67 | N=2 |
| **60** | 0.0010 ± 0.0008 | N=2 | 0.0309 ± 0.0006 | N=2 | 4.53 ± 2.47 | N=2 |
| **61** | 0.0045 ± 0.0007 | N=3 | 0.0253 ± 0.0073 | N=3 | 37.45± 31.58 | N=2 |
| **62** | 0.0900 ± 0.0020 | N=2 | 0.1700 ± 0.0810 | N=2 | >50 | N=1 |
| **63** | 0.0018 ± 0.0008 | N=3 | 0.0070 ± 0.0008 | N=3 | 10.23 ± 6.41 | N=2 |
| **64** | 0.0615 ± 0.0055 | N=2 | 0.1473 ± 0.0847 | N=2 | >50 | N=1 |
| **65** | 0.0008 | N=2 | 0.0346 ± 0.0002 | N=2 | 3.84 | N=1 |
| **66** | 0.0012 ± 0.0001 | N=3 | 0.0103 ± 0.0014 | N=3 | 28.27 | N=1 |
| **67** | 0.048 ± 0.0030 | N=2 | 0.176 ± 0.0350 | N=2 | 7.82 | N=1 |
| **68** | 0.413 | N=1 | >1 | N=1 | 35.6 | N=1 |
| **69** | >0.5 | N=1 | >1 | N=1 | >50 | N=1 |
| **70** | >0.5 | N=1 | >1 | N=1 | >50 | N=1 |
| **71** | >0.5 | N=1 | >1 | N=1 | >50 | N=1 |
| **72** | >0.5 | N=1 | >1 | N=1 | >50 | N=1 |
| **73** | >0.5 | N=1 | >1 | N=1 | >50 | N=1 |
| **74** | 0.193 | N=1 | >1 | N=1 | >50 | N=1 |
| **75** | 0.0053 ± 0.0010 | N=2 | 0.0419 ± 0.0052 | N=3 | >50 | N=1 |
| **76** | 0.0018 ± 0.0003 | N=2 | 0.0397 ± 0.0121 | N=2 | 5.38 | N=1 |
| **76a** | 0.0011 + 0.0002 | N=2 | 0.0169 ± 0.0021 | N=2 | 10.38 | N=1 |
| **77** | 0.138 | N=1 | 0.789 ± 0.065 | N=2 | ND | |
| **78** | 0.0057 ± 0.0001 | N=2 | 0.0260 ± 0.0030 | N=2 | 24.72 | N=1 |
| **79** | 0.0035 ± 0.0015 | N=3 | 0.025 ± 0.0060 | N=2 | 40.23 | N=1 |
| **81** | 0.0067 ± 0.0002 | N=3 | 0.0101 ± 0,0017 | N=3 | 22.73 | N=1 |

While the foregoing specification teaches the principles of the present invention, with examples provided for the purpose of illustration, it will be understood that the practice of the invention encompasses all of the usual variations, adaptations and/or modifications as come within the scope of the following claims.

## Claims

1. A compound of Formula (I): 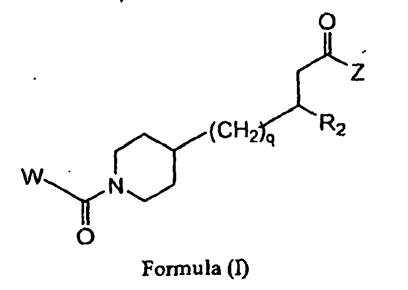 wherein
W is selected from the group consisting of -C₀₋₄alkyl(R₁) and -C₀₋₄alkyl-phenyl (R₁,R₈);
R₁ is selected from the group consisting of -N(R₄)(R₆),-dihydro-1*H*-pyrrolo[2,3-b]pyridinyl(R₈), -tetrahydropyrimidinyl(R₈), -tetrahydro-1,8-naphthyridinyl(R₈), -tetrahydro-1*H*-azepino[2,3-b]pyridinyl(R₈) and -pyridinyl(Rₛ) ;
R₄ is selected from the group consisting of hydrogen and -C₁₋₈alkyl(R₇);
R₆ R₆ is -dihydroimidazolyl(R₄),-tetrahydropyridinyl(R₈), is -dihydroimidazolyl(R₄), -tetrahydropyridinyl(R₈), -tetrahydropyrimidinyl(R₈) or -pyridinyl(R₈);
R₇ is one to two substituents independently selected from the group consisting of hydrogen, -C₁₋₈alkoxy(R₉), -NH₂, -NH-C₁₋₈alkyl(R₉), -N(C₁₋₈alkyl(R₉))₂, -C(=O)H, -C(=O)-C₁₋₈alkyl(R₉), -C(=O)-NH₂, -C(=O)-NH-C₁₋₈alkyl(R₉), -C(=O)-N(C₁₋₈alkyl(R₉))₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₈alkyl(R₉), -CO₂-aryl(R₁₀), -C(=NH)-NH₂, -SH, -S-C₁₋₈alkyl(R₉), -S-C₁₋₈alkyl-S-C₁₋₈alkyl(R₉), -S-C₁₋₈alkyl-C₁₋₈alkoxy(R₉), -S-C₁₋₈alkyl-NH-C₁₋₈alkyl(R₉), -SO₂-C₁₋₈alkyl(R₉), -SO₂-NH₂, -SO₂-NH-C₁₋₈alkyl(R₉), -SO₂-N(C₁₋₈alkyl(R₉))₂, -SO₂-aryl(R₁₀), cyano, (halo)₁₋₃, hydroxy, nitro, oxo, -cycloalkyl(R₁₀), -heterocyclyl(R₁₀), -aryl(R₁₀) and -heteroaryl(R₁₀);
R₈ is one to four substituents independently selected from the group consisting of hydrogen, -C₁₋₈alkyl(R₉), -C(=O)H, -C(=O)-C₁₋₈alkyl(R₉), -C(=O)-NH₂, -C(=O)-NH-C₁₋₈alkyl(R₉), -C(=O)-N(C₁₋₈alkyl(R₉))₂, -C(=O)-NH-aryl(R₁₀), -C(=O)-cycloalkyl(R₁₀)', -C(=O)-heterocyclyl(R₁₀), -C(=O)-aryl(R₁₀), -C(=O)-heteroaryl(R₁₀), -CO₂H, -CO₂-C₁₋₈alkyl(R₉), -CO₂-aryl(R₁₀), -C(=NH)-NH₂, -SO₂-C₁₋₈alkyl(R₉), -SO₂-NH₂, -SO₂-NH-C₁₋₈alkyl(R₉), -SO₂₋N(C₁₋₈alkyl(R₉))₂, -SO₂-aryl(R₁₀), -cycloalkyl(R₁₀) and -aryl(R₁₀) when attached to a nitrogen atom; and, wherein R₈ is one to four substituents independently selected from the group consisting of hydrogen,-C₁₋₈alkyl(R₉),-C₁₋₈alkoxy(R₉), -O-cycloalkyl(R₁₀), -O-aryl(R₁₀), -C(=O)H, -C(=O)-C₁₋₈alkyl(R₉), -C(=O)-NH₂, -C(=O)-NH-C₁₋₈alkyl(R₉), -C(=O)-N(C₁₋₈alkyl(R₉))₂, -C(=O)-NH-aryl(R₁₀), -C(=O)-cycloalkyl(R₁₀), -C(=O)-heterocyclyl(R₁₀), -C(=O)-aryl(R₁₀), -C(=O)-heteroaryl(R₁₀), -CO₂H, -CO₂-C₁₋₈alkyl(R₉), -CO₂-aryl(R₁₀), -C(=NH)-NH₂, -SO₂-C₁₋₈alkyl(R₉), -SO₂-NH₂, -SO₂-NH-C₁₋₈alkyl(R₉), -SO₂-N(C₁₋₈alkyl(R₉))₂, -SO₂-aryl(R₁₀), -SH, -S-C₁₋₈alkyl(R₉), -S-C₁₋₈alkyl-S-C₁₋₈alkyl(R₉), -S-C₁₋₈alkyl-C₁₋₈alkoxy(R₉),-S-C₁₋₈alkyl-NH-C₁₋₈alkyl(R₉), -NH₂, -NH-C₁₋₈alkyl(R₉), -N(C₁₋₈alkyl(R₉))₂, cyano, halo, hydroxy, nitro, oxo, -cycloalkyl(R₁₀), -hetorocyclyl(R₁₀), -aryl(R₁₀) and -heteroaryl(R₁₀) when attached to a carbon atom;
R₉ is selected from the group consisting of hydrogen, -C₁₋₈alkoxy; -NH₂, -NH-C₁₋₈alkyl, -N(C₁₋₈alkyl)₂, -C(=O)H, -C(=O)-NH₂, -C(=O)-NH-C₁₋₈alkyl, -C(=O)-N(C₁₋₈alkyl)₂, -CO₂H, -CO₂-C₁₋₈alkyl, -SO₂-C₁₋₈alkyl, -SO₂-NH₂, -SO₂-NH-C₁₋₈alkyl, -SO₂-N(C₁₋₈alkyl)₂, cyano, (halo)₁₋₃, hydroxy, nitro and oxo;
R₁₀ is one to four substituents independently selected from the group consisting of hydrogen, -C₁₋₈alkyl, -C(=O)H, -C(=O)-C₁₋₈alkyl, -C(=O)-NH₂, -C(=O)-NH-C₁₋₈alkyl, -C(=O)-N(C₁₋₈alkyl)₂, -CO₂H, -CO₂- C₁₋₄alkyl, -SO₂-C₁₋₈alkyl, -SO₂-NH₂, -SO₂-NH-C₁₋₈alkyl and -SO₂-N(C₁₋₈alkyl)₂ when attached to a nitrogen atom; and, wherein R₁₀ is one to four substituents independently selected from the group consisting of hydrogen, -C₁₋₈alkyl, -C₁₋₈alkoxy, -C(=O)H, -C(=O)-C₁₋₈alkyl, -C(=O)-NH₂, -C(=O)-NH-C₁₋₈alkyl, -C(=O)-N(C₁₋₈alkyl)₂, -CO₂H, -CO₂- C₁₋₄alkyl, -SO₂-C₁₋₈alkyl, -SO₂-NH₂, -SO₂-NH-C₁₋₈alkyl, -SO₂-N(C₁₋₈alkyl)₂, -NH₂, -NH-C₁₋₈alkyl, -N(C₁₋₈alkyl)₂, cyano, halo, hydroxy, nitro and oxo when attached to a carbon atom;
R₂ is hydrogen, -tetrahydropyrimidinyl(R₈), -1,3-benzodioxolyl(R₈), -dihydrobenzofuranyl(R₈), -tetrahydroquinolinyl(R₈), -phenyl(R₈), -naphthalenyl(R₈), -pyridinyl(R₈), -pyrimidinyl(R₈) or -quinolinyl(R₈);
q is selected from the group consisting of 0,1, 2 or 3;
Z is selected from the group consisting of hydroxy, -NH₂, -NH-C₁₋₈alkyl, -N(C₁₋₈alkyl)₂, -O-C₁₋₈alkyl,-O-C₁₋₈alkyl-OH, -O-C₁₋₈alkylC₁₋₈alkoxy, -O- C₁₋₈alkylcarbonylC₁₋₈alkyl, -O-C₁₋₈alkyl-CO₂H, -O-C₁₋₈alkyl-C(O)O-C₁₋₈alkyl,-O- C₁₋₈alkyl-O-C(O)C₁₋₈alkyl,-O-C₁₋₈alkyll-NH₂,-O-C₁₋₈alkyl-NH-C₁₋₈alkyl,-O- C₁₋₈alkyl-N(C₁₋₈alkyl)₂, -O-C₁₋₈alkylamide, -O-C₁₋₈alkyl-C(O)-NH-C₁₋₈alkyl, -O-C₁₋₈alkyl-C(O)-N(C₁₋₈alkyl)₂ and -NHC(O)C₁₋₈alkyl;
and pharmaceutically acceptable salts, racemic mixtures and enantiomers thereof.

2. The compound of claim 1 wherein R₁ is selected from the group consisting of -N(R₄)(R₆), -tetrahydropyrimidinyl(R₈) and -tetrahydro-1,8-naphthyridinyl(R₈).

3. A compound of Formula (I): 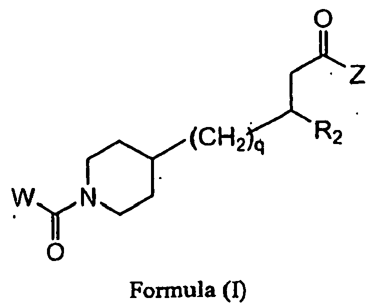 wherein W, R₁, R₂, q and Z are selected from:
| W | R₁ | R₂ | q | Z |
|---|---|---|---|---|
| -CH₂-Ph(3-R₁) | -NH-1,4,5,6-tetrahydro-pyrimidin-2-yl | H | 0 | OH |
| -(CH₂)₂-Ph(3-R₁) | -NH-1,4,5,6-tetrahydro-pyrimidin-2-yl | H | 0 | OH |
| -CH₂-Ph(3-R₁) | -NH-1,4,5,6-tetrahydro-5-OH-pyrimidin-2-yl | quinolin-3-yl | 0 | OH |
| -(CH₂)₃-R₁ | 5,6,7,8-tetrahydro-[1,8]naphthyridin-2-yl | quinolin-3-yl | 0 | OH |
| -(CH₂)₃- R₁ | 5,6,7,8-tetrahydro-[1,8]naphthyridin-2- yl | 1,2,3,4-tetrahydro-quinolin-3-yl | 0 | OH |
| Ph(3-R₁) | -NH-1,4,5,6-tetrahydro-pyrimidin-2-yl | pyridin-3-yl | 2 | OH |
| Ph(3-R₁) | -NH-1,4,5,6-tetrahydro-5-OH-pyrimidin-2-yl | pyridin-3-yl | 2 | OH |
| -(CH₂)₂-R₁ | 5,6,7,8-tetrahydro-[1,8]naphthyridin-2-yl | pyridin-3-yl | 2 | OH |
| -(CH₂)₃-R₁ | -NH-pyridin-2-yl | pyridin-3-yl | 2 | OH |
| Ph(3-R₁) | -NH-1,4,5,6-tetrahydro-5-OH-pyrimidin-2-yl | (6-OCH₃)-pyridin-3-yl | 2 | OH |
| -(CH₂)₂-R₁ | 5,6,7,8-tetrahydro-[1,8]naphthyridin-2-yl | 1,3-benzodioxol-5-yl | 1 | OH |
| Ph(3-R₁) | -NH-1,4,5,6-tetrahydro-pyrimidin-2-yl | quinolin-3-yl | 2 | OH |
| -(CH₂)₂-R₁ | 5,6,7,8-tetrahydro-[1,8]naphthyridin-2-yl | phenyl | 1 | OH |
| -(CH₂)₂-R₁ | 5,6,7,8-tetrahydro-[1,8]naphthyridin-2-yl | 1,3-benzodioxol-5-yl | 0 | OH |
| -(CH₂)₃-R₁ | 5,6,7,8-tetrahydro-[1,8]naphthyridih-2-yl | 1,3-benzodioxol-5-yl | 0 | OH |
| -CH₂-R₁ | 5,6,7,8-tetrahydro-[1,8]naphthyridin-2-yl | 1,3-benzodioxol-5-yl | 0 | OH |
| -(CH₂)₃-R₁ | 5,6,7,8-tetrahydro-[1,8]naphthyridin-2-yl | (6-OCH₃)-pyridin-3-yl | 0 | OH |
| -(CH₂)₂-R₁ | 5,6,7,8-tetrahydro-[1,8]naphthyridin-2-yl | 1,4,5,6-tetrahydro-2-Me-pyrimidin-5-yl | 1 | OH |
| -(CH₂)₂-R₁ | 5,6,7,8-tehahydro-[1,8]naphthyridin-2-yl | 1,2,3,4-tetrahydro-quinolin-3-yl | 1 | OH |
| -(CH₂)₂-R₁ | 5,6,7,8-tetrahydro-[1,8]naphthyridin-2-yl | 1,3-benzodioxol-5-yl | 2 | OH |
| -(CH₂)₂-R₁ | 5,6,7,8-tetrahydro-[1,8]naphthyridin-2-yl | (6-OCH₃)pyridin-3-yl | 2 | OH |
| -(CH₂)₂-R₁ | -NH-1,4,5,6-tetrahydro-5-OH-pyrimidin-2-yl | (6-OCH₃)-pyridin-3-yl | 2 | OH |
| -(CH₂)₂-R₁ | -NH-1,4,5,6-tetrahydro-5-OH-pyrimidin-2-yl | (6-OCH₃)-pyridin-3-yl | 2 | OH |
| -(CH₂)₃-R₁ | -NH-pyridin-2-yl | quinolin-3-yl | 2 | OH |
| -(CH₂)₃-R₁ | -NH-pyridin-2-yl | 1,3-benzodioxol-5-yl | 2 | OH |
| -(CH₂)₃-R₁ | -NH-pyridin-2-yl | 1,3-benzodioxol-5-yl | 0 | OH |
| -(CH₂)₃-R₁ | -NH-pyridin-2-yl | (6-OCH₃)-pyridin-3-yl | 2 | OH |
| -(CH₂)₃-R₁ | 5,6,7,8-tetrahydro-[1,8]naphthyridin-2-yl | 1,3-benzodioxol-5-yl | 1 | OH |
| Ph(3-R₁) | -NH-1,4,5,6-tetrahydro-5-OH-pyrimidin-2-yl | 1,3-benzodioxol-5-yl | 1 | OH |
| -(CH₂)₂-R₁ | 5,6,7,8-tetrahydro-[1,8]naphthyridin-2-yl | (6-OCH₃)-pyridin-3-yl | 1 | OH |
| -(CH₂)₃-R₁ | 5,6,7,8-tetrahydro-[1,8]naphthyridin-2-yl | quinolin-3-yl | 1 | OH |
| -(CH₂)₂-R₁ | 5,6,7,8-tetrahydro-[1,8]naphthyridin-2-yl | (3-F)phenyl | 1 | OH |
| -(CH₂)₃-R₁ | 5,6,7,8-tetrahydro-[1,8]naphthyridin-2-yl | (3-F)phenyl | 1 | OH |
| -(CH₂)₂-R₁ | 5,6,7,8-tetrahydro-[1,8]naphthyridin-2-yl | quinolin-3-yl | 1 | OH |
| -(CH₂)₂-R₁ | 5,6,7,8-tetrahydro-[1,8]naphthyridin-2-yl | (4-F)phenyl | 1 | OH |
| -(CH₂)₃-R₁ | 5,6,7,8-tetrahydro-[1,8]naphthyridin-2-yl | (4-F)phenyl | 1 | OH |
| -(CH₂)₂-R₁ | 5,6,7,8-tetrahydro-[1,8]paphthyridin-2-yl 5-yl | (2-CH₃)pyrimidin- | 1 | OH |
| -(CH₂)₂-R₁ | 5,6,7,8-tetrahydro-[1,8]naphthyridin-2-yl | 2,3-dihydro-benzofuran-6-yl | 1 | OH |
| -(CH₂)₂-R₁ | 5,6,7,8-tetrahydro-[1,8]naphthyridin-2-yl | (3,5-difluoro)-phenyl | 1 | OH |
| -(CH₂)₃-R₁ | 5,6,7,8-tetrahydro-[1,8]naphthyridin-2-yl | (3,5-difluoro)-phenyl | 1 | OH |
| -(CH₂)₂-R₁ | 5,6,7,8-tetrahydro-[1,8]naphthyridin-2-yl | (3-CF₃)-phenyl | 1 | OH |
| -(CH₂)₂-R₁ | 5,6,7,8-tetrahydro-[1,8]naphthyridin-2-yl | (4-OCF₃)-phenyl | 1 | OH |
| -(CH₂)₂-R₁ | 5,6,7,8-tetrahydro-[1,8]naphthyridin-2-yl | (3-F-4-Ph)-phenyl | 1 | OH |
| -(CH₂)₂-R₁ | 5,6,7,8-tetrahydro-[1,8]naphthyridin-2-yl | (3-F-4-OCH₃)-phenyl | 1 | OH |
| -(CH₂)₂-R₁ | 5,6,7,8-tetrahydro-[1,8]naphthyridin-2-yl | (4-OPh)-phenyl | 1 | OH |
| -(CH₂)₂-R₁ | 5,6,7,8-tetrahydro-[1,8]naphthyridin-2-yl | isoquinolin-4-yl | 1 | OH |
| -(CH₂)₂-R₁ | 5,6,7,8-tetrahydro-[1,8]naphthyridin-2-yl | pyridin-3-yl | 1 | OH |
| -(CH₂)₂-R₁ | 5,6,7,8-tetrahydro-[1,8]naphthyridin-2-yl | dihydrobenzofuran -5-yl | 1 | OH |
| -(CH₂)₂-R₁ | 5,6,7,8-tetrahydro-[1,8]naphthyridin-2-yl | (2,4-OCH₃)-pyrimidin-5-yl | 1 | OH |
| -(CH₂)₂-R₁ | 5,6,7,8-tetrahydro-[1,8]naphthyridin-2-yl | (2-OCH₃)-pyrimidin-5-yl | 1 | OH |
| Ph(3-R₁) | -NH-1,4,5,6-tetrahydro-5-OH-pyrimidin-2-yl | quinolin-3-yl | 2 | OH |
| Ph(3-R₁) | -NH-1,4,5,6-tetrahydro-pyridin-2-yl | quinolin-3-yl | 2 | OH |
| -(CH₂)₂-R₁ | 5,6,7,8-tetrahydro-[1,8]naphthyridin-2-yl | quinolin-3-yl | 2 | OH |
| Ph(3-R₁) | -NH-3,4,5,6-tetrahydro-pyrimidin-2-yl | 1,3-benzodioxol-5-yl | 2 | OH |
| Ph(3-R₁) | -NH-3,4,5,6-tetrahydro-pyridin-2-yl | 1,3-benzodioxol-5-yl | 2 | OH |
| Ph(3-R₁) | NH-1,4,5,6-tetrahydro-5-OH-pyrimidin-2-yl | 1,3-benzodioxol-5-yl | 2 | OH |
| -CH₂-R₁ | 5,6,7,8-tetrahydro-[1,8]naphthyridin-2-yl | 1,3-benzodioxol-5-yl | 2 | OH |
| -(CH₂)₂-R₁ | 5,6,7,8-tetrahydro-[1,8]naphthyridin-2-yl | naphthalene-2-yl | 1 | OH |
| -(CH₂)₂-R₁ | 5,6,7,8-tetrahydro-[1,8]naphthyridin-2-yl | 5,6,7,8-tetrahydro-quinolin-3-yl | 1 | OH |
| -(CH₂)₃-R₁ | 5,6,7,8-tetrahydro-[1,8]naphthyridin-2-yl | 5,6,7,8-tetrahydro-quinolin-3-yl | 0 | OH |
| -(CH₂)₂-R | 5,6,7,8-tetrahydro-[1,8]paphthyridin-2-yl | (3-OCH₃)phenyl | 1 | OH |
| -(CH₂)₂-R₁ | 5,6,7,8-tetrahydro-[1,8]naphthyridin-2-yl | (4-OCH₃)phenyl | 1 | OH |
| (CH₂)₂-R₁ | 5,6,7,8-tetrahydro-[1,8]naphthyridin-2-yl | H | 1 | OH |
| -(CH₂)₂-R₁ | 5,6,7,8-tetrahydro-[1,8]naphthyridin-2-yl | tetrahydrofuran-3-yl | 1 | OH |
| -(CH₂)₂-R₁ | 5,6,7,8-tetrahydro-[1,8]naphthyridin-2-yl | thiophen-2-yl | 1 | OH |
| -(CH₂)₂-R₁ | 5,6,7,8-tetraydro-[1,8]naphthyridin-2-yl | (3-F)phenyl | 1 | NH₂ |
| -(CH₂)₂-R₁ | 5,6,7,8-tetrahydro-[1,8]naphthyridin-2-yl | 2,3-dihydro-benzo[1,4]-dioxin-6-yl | 1 | OH |
| -(CH₂)₂-R₁ | 5,6,7,8-tetrahydro-[1,8]naphthyridin-2-yl | (3-SCH₃)phenyl | 1 | OH |
| -(CH₂)₂-R₁ | 5,6,7,8-tetrahydro-[1,8]naphthyridin-2- yl | *N*-methyl-1,2,3,4-tetrahydro-quinolin-3-yl | 1 | OH |
| -(CH₂)₂-R₁ | 5,6,7,8-tetrahydro-[1,8]naphthyridin-2-yl | H | 1 | -O-ethyl |
| -(CH₂)₂-R₁ | 5,6,7,8-tetrahydro- [1,8]naphthyridin-2-yl | H | 1 | -O-2-propyl |
| -(CH₂)₂-R₁ | 5,6,7,8-tetrahydro-[1,8]naphthyridin-2-yl | H | 1 | -*O*-*t*-butyl |
| -(CH₂)₂-R₁ | 5,6,7,8-tetrahydro-[1,8]naphthyridin-2-yl | H | 1 | -*O*-*n*-octyl |
| -(CH₂)₂-R₁ | 5,6,7,8-tetrahydro-[1,8]naphthyridin-2-yl | H | 1 | -*O*-*s*-butyl |
| -(CH₂)₂-R₁ | 5,6,7,8-tetrahydro-[1,8]naphthyridin-2-yl | H | 1 | -O-methyl |
| -(CH₂)₂-R₁ | 5,6,7,8-tetrahydro-[1,8]naphthyridin-2-yl | H | 1 | -O-CH₂-OC(O)-*t*-butyl |
| -(CH₂)₂-R₁ | 5,6,7,8-tetrahydro-[1,8]naphthyridin-2-yl | (3-(NMe₂)phenyl | 1 | OH |
| -(CH₂)₂-R₁ | 5,6,7,8-tetrahydro-[1,8]naphthyridin-2-yl | (3-OMe-4-OH)phenyl | 1 | OH |
| Ph(3-R₁) | -NH-4,5-dihydro-1*H*-imidazol-2-yl | (3-F)phenyl | 1 | OH |
| -(CH₂)₂-R₁ | 5,6,7,8-tetrahydro-[1,8]naphthyridin-2-yl | (3-NHEt)phenyl | 1 | OH |
| -(CH₂)₂-R₁ | 5,6,7,8-tetrahydro-[1,8]naphthyridin-2- yl | (3-NHMe)phenyl | 1 | OH |
| -(CH₂)₃-R₁ | 5,6,7,8-tetrahydro-[1,8]naphthyridin-2-yl | dihydrobenzofuran -6-yl | 0 | OH |
and pharmaceutically acceptable salts, racemic mixtures and enantiomers thereof.

4. A composition comprising a compound of claim 1 or claim 3 wherein the compound is selected from the group consisting of:
a compound of Formula (I) wherein W is -CH₂-Ph(3-R₁); R₁ is -NH-1,4,5,6-tetrahydro-pyrimidin-2-yl; R₂ is H, q is 0, and 2 is OH;
a compound of Formula (I) wherein W is -(CH₂)₂-Ph(3-R₁); R₁ is -NH-1,4,5,6-tetrahydro-pyrimidin-2-yl; R₂ is H, q is 0 and Z is OH;
a compound of Formula (I) wherein W is -CH₂-Ph(3-R₁); R₁ is -NH-1,4,5,6-tetrahydro-5-OH-pyrimidin-2-yl; R₂ is -3-quinolinyl, q is 0 and Z is OH;
a compound of Formula (I) wherein W is -(CH₂)₃-R₁; R₁ is -5,6,7,8-tetrahydro-1,8-naphthyridin-2-yl; R₂ is -3-quinolinyl, q is 0 and Z is OH;
a compound of Formula (I) wherein W is -(CH₂)₃-R₁; R₁ is -5,6,7,8-tetrahydro-1,8-naphthyridin-2-yl; R₂ is -1,2,3,4-tetrahydro-3-quinolinyl, q is 0 and Z is OH;
a compound of Formula (I) wherein W is -Ph(3-R₁); R₁ is -NH-1,4,5,6-tetrahydro-pyrimidin-2-yl; R₂ is -3-pyridinyl, q is 2 and Z is OH;
a compound of Formula (I) wherein W is -Ph(3-R₁); R₁ is -NH-1,4,5,6-tetrahydro-5-OH-pyrimidin-2-yl, R₂ is -3-pyridinyl, q is 2 and Z is OH;
a compound of Formula (I) wherein W is -(CH₂)₂-R₁; R₁ is -5,6,7,8-tetrahydro-1,8-naphthyridin-2-yl; R₂ is -3-pyridinyl, q is 2 and Z is OH;
a compound of Formula (I) wherein W is -(CH₂)₃-R₁; R₁ is -NH-pyridin-2-yl; R₂ is -3-pyridinyl, q is 2 and Z is OH;
a compound of Formula (I) wherein W is -Ph(3-R₁); R₁ is -NN-1,4,5,6-tetrahydro-5-OH-pyrimidin-2-yl; R₂ is -(6-MeO)pyridin-3-yl, q is 2 and Z is OH;
a compound of Formula (I) wherein W is -(CH₂)₂-R₁; R₁ is -5,6,7,8-tetrahydro-1,8-naphthyridin-2-yl; R₂ is -1,3-benzodioxol-5-yl, q is 1 and Z is OH;
a compound of Formula (I) wherein W is -Ph(3-R₁); R₁ is -NH-1,4,5,6-tetrahydro-pyrimidin-2-yl; R₂ is -3-quinolinyl, q is 2 and Z is OH;
a compound of Formula (I) wherein W is -(CH₂)₂-R₁; R₁ is -5,6,7,8-tetrahydro-1,8-naphthyridin-2-yl; R₂ is -Ph, q is 1 and Z is OH; a compound of Formula (I) wherein W is -(CH₂)₂-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -(CH₂)₃-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -CH₂-R₁; R₁ is -5,6,7,8-tetrahydro-1,8-naphthyridin-2-yl; R₂ is -1,3-benzodioxol-5-yl, q is 0 and Z is OH;
a compound of Formula (I) wherein W is -(CH₂)₃-R₁; R₁ is -5,6,7,8-tetrahydro-1,8-naphthyridin-2-yl ; R₂ is -(6-MeO)pyridin-3-yl, q is 0, and Z is OH;
a compound of Formula (I) wherein W is -(CH₂)₂-R₁; R₁ is -5,6,7,8-tetrahydro-1,8-naphthyridin-2-yl; R₂ is -1,4,5,6-tetrahydro-2-Me-pyrimidin-5-yl, q is 1 and Z is OH;
a compound of Formula (I) wherein W is -(CH₂)₂-R₁; R₁ is -5,6,7,8-tetrahydro-1,8-naphthyridin-2-yl; R₂ is -1,2,3,4-tetrahydro-3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -1,3-benzodioxol-5-yl, q is 2 and Z is OH;
a compound of Formula (I) wherein W is -(CH₂)₂-R₁; R₁ is -5,6,7,8-tetrahydro-1,8-naphthyridin-2-yl; R₂ is -(6-MeO)pyridin-3-yl, q is 2 and Z is OH;
a compound of Formula (1) wherein W is -(CH₂)₃-R₁; R₁ is -NH-pyridin-2-yl; R₂ is -3-quinolinyl, q is 2 and Z is OH;
a compound of Formula (I) wherein W is -(CH₂)₃-R₁; R₁ is -NH-pyridin-2-yl; R₂ is -1,3-benzodioxol-5-yl, q is 2 and Z is OH;
a compound of Formula (I) wherein W is -(CH₂)₃-R₁; R₁ is -NH-pyridin-2-yl; R₂ is -1,3-benzodioxol-5-yl, q is 0, and Z is OH;
a compound of Formula (I) wherein W is -(CH₂)₃-R₁; R₁ is -NH-pyridin-2-yl; R₂ is -(6-MeO)pyridin-3-yl, q is 2 and Z is OH;
a compound of Formula (I) wherein W is -(CH₂);-R₁; R₁ is -5,6,7,8-tetrahydro-1,8-napthyridin-2-yl; R₂ is -1,3-benzodioxol-5-yl, q is 1 and Z is OH;
a compound of Formula (I) wherein W is -Ph(3-R₁); R₁ is -NH-1,4,5,6-tetrahydro-5-OH-2-pyrimidinyl; R₂ is -1,3-benzodioxol-5-yl, q is 1 and Z is OH;
a compound of Formula (1) wherein W is -(CH₂)₂-R₁; R₁ is -5,6,7,8-tetrahydro-1,8-naphthyridin-2-yl; R₂ is -(6-MeO)pyridin-3-yl, q is 1 and Z is OH;
a compound of Formula (I) wherein W is -(CH₂)₃-R₁; R₁ is -5,6,7,8-tetrahydro-1,8-naphthyridin-2-yl; R₂ is -3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -(3-F)Ph, q is 1 and Z is OH;
a compound of Formula (I) wherein W is -(CH₂)₃-R₁; R₁ is -5,6,7,8-tetrahydro-1,8-naphthyridin-2-yl; R₂ is -(3-F)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -3-quinolinyl, q is 1 and Z is OH;
a compound of Formula (I) wherein W is -(CH₂)₂-R₁; R₁ is -5,6,7,8-tetrahydro-1,8-naphthyridin-2-yl; R₂ is -(4-F)Ph, q is 1, and Z is OH;
a compound of Formula (I) wherein W is -(CH₂)₃-R₁; R₁ is -5,6,7,8-tetrahydro-1,8-naphthyridin-2-yl; R₂ is -(4-F)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -(2-Me)pyrimidin-5-yl, q is 1 and Z is OH;
a compound of Formula (I) wherein W is -(CH₂)₂-R₁; R₁ is -5,6,7,8-tetrahydro-1,8-naphthyridin-2-yl; R₂ is -2,3-dihydro-benzofuran-6-yl, q is 1, and Z is OH;
a compound of Formula (I) wherein W is -(CH₂)₂-R₁; R₁ is -5,6,7,8-tetrahydro-1,8-naphthyridin-2-yl; R₃ is -(3,5-F₂)Ph, q is 1, and Z is OH;
a compound of Formula (I) wherein W is -(CH₂)₃-R₁; R₁ is -5,6,7,8-tetrahydro-1,8-naphthyridin-2-yl; R₂ is -(3,5-F₂)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -(3-CF₃)Ph, q is 1, and Z is OH;
a compound of Formula (I) wherein W is -(CH₂)₂-R₁; R₁ is -5,6,7,8-tetrahydro-7,8-naphthyridin-2-yl; R₂ is -(4-OCF₃)Ph, q is 1 and Z is OH;
a compound of Formula (I) wherein W is -(CH₂)₂-R₁; R, is -5,6,7,8-tetrahydro-1,8-naphthyridin-2-yl; R₂ is -(3-F-4-Ph)Ph, q is 1, and Z is OH;
a compound of Formula (I) wherein W is -(CH₂)₂-R₁; R₁ is -5,6,7,8-tetrahydro-1,8-naphthyridin-2-yl; R₂ is -(3-F-4-OMe)Ph; q is 1, and Z is OH;
a compound of Formula (I) wherein W is -(CH₂)₂-R₁; R₁ is -5,6,7,8-tetrahydro-1,8-naphthyridin-2-yl; R₂ is -(4-OPh)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -4-isoquinolinyl, q is 1 and 2 is OH;
a compound of Formula (I) wherein W is -(CH₂)₂-R₁; R₁ is -5,6,7,8-tetrahydro-1,8-naphthyridin-2-yl; R₂ is -3-pyridinyl, q is 1 and Z is OH;
a compound of Formula (I) wherein W is -(CH₂)₂-R₁; R₁ is -5,6,7,8-tetrahydro-1,8-naphthyridin-2-yl; R₂ is -5-dihydrobenzofuranyl, q is 1 and Z is OH;
a compound of Formula (I) wherein W is -(CH₂)₂-R₁; R₁ is -5,6,7,8-tetrahydro-1,8-naphthyridin-2-yl; R₂ is -2,4-(OMe)₂-pyrimid-5-yl, q is 1 and Z is OH;
a compound of Formula (I) wherein W is -(CH₂)₂-R₁; R₁ is -5,6,7,8-tetrahydro-1,8-naphthyridin-2-yl; R₂ is -(2-OMe)pyrimidin-5-yl, q is 1, and Z is OH;
a compound of Formula (I) wherein W is -Ph(3-R₁); R₁ is -NH-1,4,5,6-tetrahydro-5-OH-pyrimidin-2-yl; R₂ is -3-quinolinyl, q is 2 and Z is OH;
a compound of Formula (I) wherein W is -Ph(3-R₁); R₁ is -NH-3,4,5,6-tetrahydro-pyridin-2-yl; R₂ is -3-quinolinyl, q is 2 and Z is OH;
a compound of Formula (I) wherein W is -(CH₂)₂-R₁; R₁ is -5,6,7,8-tetrahydro-1,8-naphthyridin-2-yl; R₂ is -3-quinolinyl, q is 2, and Z is OH;
a compound of Formula (I) wherein W is -Ph(3-R₁); R₁ is -NH-3,4,5,6-tetrahydro-pyrimidin-2-yl; R₂ is -1,3-benzodioxol-5-yl, q is 2 and Z is OH;
a compound of Formula (I) wherein W is -Ph(3-R₁); R₁ is -NH-3,4,5,6-tetrahydro-pyridin-2-yl; R₂ is -1,3-benzodioxol-5-yl, q is 2 and Z is OH;
a compound of Formula (I) wherein W is -Ph(3-R₁); R₁ is -NH-1,4,5,6-tetrahydro-5-OH-pyrimidin-2-yl; R₂ is -1,3-benzodioxol-5-yl, q is 2 and Z is OH;
a compound of Formula (I) wherein W is -CH₂-R₁; R₁ is -5,6,7,8-tetrahydro-1,8-naphthyridin-2-yl; R₂ is -1,3-benzodioxol-5-yl, q is 2, and Z is OH; and,
a compound of Formula (I) wherein W is -(CH₂)₂-R₁; R₁ is -5,6,7,8-tetrahydro-1,8-naphthyridin-2-yl; R₂ is -2-naphthalenyl, q is 1 and Z is OH.

5. The composition of claim 4 wherein the compound is selected from the group consisting of:
a compound of Formula (I) wherein W is -(CH₂)₃-R₁; R₁ is -5,6,7,8-tetrahydro-1,8-naphthyridin-2-yl; R₂ is -1,2,3,4-tetrahydro-3-quinolinyl, q is 0, and Z is OH;
a compound of Formula (I) wherein W is -(CH₂)₃-R₁; R₁ is -5,6,7,8-tetrahydro-1,8-naphthyridin-2-yl; R₂ is -1,3-benzodioxol-5-yl, q is 0, and Z is OH;
a compound of Formula (I) wherein W is -(CH₂)₂-R₁; R₁ is -5,6,7,8-tetrahydro-1,8-naphthyridin-2-yl; R₂ is -1,2,3,4-tetrahydro-3-quinolinyl, q is 1 and Z is OH;
a compound of Formula (I) wherein W is p(CH₂)₂-R₁; R₁ is -5,6,7,8-tetrahydro-1,8-naphthyidin-2-yl; R₂ is -(6-MeO)pyridin-3-yl, q is 1 and Z is OH;
a Compound of formula (I) wherein W is -(CH₂)₂-R₁; R₁ is -5,6,7,8 tetrahydro-1,8-naphthyridin-2-yl; R₂ is -(3-F)Ph, q is 1 and Z is OH;
a compound of Formula (I) wherein W is -(CH₂)₂-R₁; R₁ is -5,6,7,8-tetrahydro-1,8-naphthyridin-2-yl; R₂ is -3-quinoliny), q is 1 and Z is OH;
a compound of Formula (I) wherein W is -(CH₂)₂-R₁; R₁ is -5,6,7,8-tetrahydro-1,8-naphthyridin-2-yl; R₂ is -(2-Me)pyrimidin-5-yl, q is 1 and Z is OH;
a compound of Formula (I) wherein W is -(CH₂)₂-R₁; R₁ is -5,6,7,8-tetrahydro-1,8-naphlhyridin-2-yl; R₂ is -2,3-dihydro-benzofuran-6-yl, q is 1 and Z is OH;
a compound of Formula (I) wherein W is -(CH₂)₂-R₁; R₁ is -5,6,7,8-tetrahydro-1,8-naphthyridin-2-yl; R₂ is -4-isoquinolinyl, q is 1 and Z is OH;
a compound of Formula (I) wherein W is -(CH₂)₂-R₁; R₁ is -3,6,7,8-tetrahydro-1,8-naphthyridin-2-yl; R₂ is -3-pyridinyl, q is 1 and Z is OH;
a compound of Formula (I) wherein W is -(CH₂)₂-R₁; R₁ is -5,6,7,8-tetrahydro-1,8-naphthyridin-2-yl; R₂ is -2,4-(OMe)₂-pyrimid-5-yl, q is 1, and Z is OH; and,
a compound of Formula (I) wherein W is -(CH₂)₂-R₁ ; R₁ is -5,6,7,8-tetrahydro-1,8-naphthyridin-2-yl; R₂ is -(2-OMe)pyrimidin-5-yl, q is 1, and Z is OH.

6. The compound of claim 1 wherein W is -(CH₂)₃-R₁; R₁ is -5,6,7,8-tetrahydro-1,8-naphthyridin-2-yl; R₂ is -1,2,3,4-tetrahydro-ß-quinolinyl, q is 0 and Z is OH.

7. The compound of claim 1 wherein W is -(CH₂)₃-R₁; R₁ is -5,6,7,8-tetrahydro-1,8-naphthyridin-2-yl; R₂ is -1,3-benzodioxol-5-yl, q is 0 and Z is OH.

8. The compound of claim 1 wherein W is -(CH₂)₂-R₁; R₁ is -5,6,7,8-tetrahydro-1,8-naphthyridin-2-yl; R₂ is -1,2,3,4-tetrahydro-3-quinolinyl, q is 1 and Z is OH.

9. The compound of claim 1 wherein W is -(CH₂)₂-R₁; R₁ is -5,6,7,8-tetrahydro-1,8-naphthyridin-2-yl; R₂ is -(6-MeO)pyridin-3-yl, q is 1 and Z is OH.

10. The compound of claim 1 wherein W is -(CH₂)₂-R₁; R₁ is -5,6,7,8-tetrahydro-1,8-naphthyridin-2-yl; R₂ is -(3-F)Ph, q is 1 and Z is OH.

11. The compound of claim 1 wherein W is -(CH₂)₂-R₁; R₁ is -5,6,7,8-tetrahydro-1,8-naphthyridin-2-yl; R₂ is -3-quinolinyl, q is 1 and Z is OH.

12. The compound of claim 1 wherein W is -(CH₂)₂-R₁; R₁ is -5,6,7,8-tetrahydro-1,8-naphthyridin-2-yl; R₂ is -(2-Me)pyrimidin-5-yl, q is 1 and Z is OH.

13. The compound of claim 1 wherein W is -(CH₂)₂-R₁; R₁ is -5,6,7,8-tetrahydro-1,8-naphthyridin-2-yl; R'₂ is -2,3-dihydro-benzofuran-6-yl, q is 1 and Z is OH.

14. The compound of claim 3 wherein W is -(CH₂)₂-R₁; R₁ is -5,6,7,8-tetrahydro-1,8-naphthyridin-2-yl; R₂ is -4-isoquinolinyl, q is 1 and Z is OH.

15. The compound of claim 1 wherein W is -(CH₂)₂-R₁; R₁ is -5,6,7,8-tetrahydro-1,8-naphthyridin-2-yl; R₂ is -3-pyridinyl, q is 1, and Z is OH.

16. The compound of claim 1 wherein W is -(CH₂)₂-R₁; R₁ is -5,6,7,8-tetrahydro-1,8-naphthyridin-2-yl; R₂ is -2,4-(OMe)₂-pyrimid-5-yl,q is 1 and Z is OH.

17. The compound of claim 1 wherein W is -(CR₂)₂-R₁; R₁ is -5,6,7,8-tetrahydro-1,8-naphthyridin-2-yl; R₂ is -(2-OMe)pyrimidín-5-yl, q is 1 and Z is OH.

18. The compound of claim 1 wherein W is -(CH₂)₃-R₁; R₁ is -5,6,7,8-tetrahydro1,8-naphthyridin-2-yl; R₂ is 2,3-dihydro-benzofuran-6-yl, q is 0 and Z is OH.

19. A compound of Formula (I): 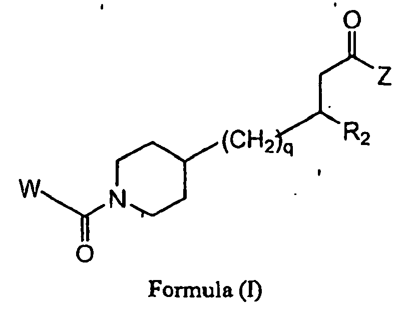 wherein
W is selected from the group consisting of -C₀₋₄alkyl(R₁) and -C₀₋₄alkyl-phenyl(R₁,R₈);
R₁ is -NH(R₆);
R₂ is selected from the group consisting of hydrogen, -tetrahydropyrimidinyl(R₈), -1,3-benzodioxolyl(R₈), -dihydrobenzofuranyl(R₈), -tetrahydroquinolinyl(R₈), -phenyl(R₈), -naphthalenyl(R₈), -pyridinyl(R₈), -pyrimidinyl(R₈) and -quinolinyl(R₈);
R₆ is selected from the group consisting of -dihydroimidazolyl(R₈), -tetrahydropyridinyl(R₈), -tetrahydropyrimidinyl(R₈) and -pyridinyl(R₈);
R₈ is one to four substituents independently selected from the group consisting of hydrogen and -C₁₋₄alkyl(R₉) when attached to a nitrogen atom; and, wherein R₈ is one to four substituents independently selected from the group consisting of hydrogen, -C₁₋₄alkyl(R₉), -C₁₋₄alkoxy(R₉), -O-aryl(R₁₀) and hydroxy when attached to a carbon atom;
R₉ is selected from the group consisting of hydrogen, -C₁₋₄alkoxy, -NH₂, -NH-C₁₋₄alkyl, -N(C₁₋₄alkyl)₂, (halo)₁₋₃ and hydroxy;
R₁₀ is independently selected from the group consisting of hydrogen, -C₁₋₄alkyl, -C₁₋₄alkoxy, -C(=O)H, -C(=O)-C₁₋₄alkyl, -CO₂H, -CO₂-C₁₋₄alkyl, -NH₂, -NH-C₁₋₄alkyl, -N(C₁₋₄alkyl)₂, halo, hydroxy, nitro and oxo when attached to a carbon atom;
q is 1, 2 or 3;
Z is slected from the group consisting hydroxy, -NH₂, -NH-C₁₋₈alkyl, -N(C₁₋₈alkyl)₂, O-C₁₋₈alkyl-OH, -O-C₁₋₈alkylC₁₋₈alkoxy, -O-C₁₋₈alkylcarbonylC₁₋₈alkyl, -O- C₁₋₈alkyl-CO₂H, -O-C₁₋₈alkyl-C(O)O-C₁₋₈alkyl, -O-C₁₋₈alkyl-O-C(O)C₁₋₈alkyl; -O- C₁₋₈akyl-NH₂, -O-C₁₋₈alkyl, -O-C₁₋₈alkyl-N(C₁₋₈alkyl)₂, -O- C₁₋₈alkylamide, -O-C₁₋₈alkyl-C(O)-NH-C₁₋₈alkyl, -O-C₁₋₈alkyl-C(O)-N(C₁₋₈alkyl)₂ and-NH(O)C₁₋₈alkyl;
and pharmaceutically acceptable salts, racemic mixtures and enantiomers thereof.

20. A compound according to claim 1 of Formula (I.2): 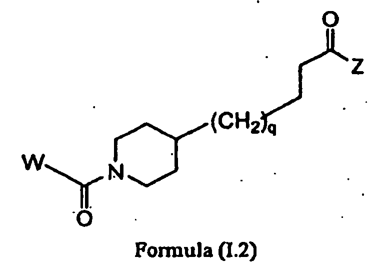 wherein
W is selected from the group consisting of -C₀₋₄alkyl(R₁) and -C₀₋₄alkyl-phenyl(R₁,R₈);
R₁ is selected from the group consisting of -NH(R₆), -dihydro-1Hpyrrolo[2,3-b]pyridinyl(R₈), -tetrahydropyrimidinyl(R₈), -tetrahydro-1,8-naphthyridinyl(R₈), -tetrahydro-1H-azepino[2,3-b]pyridinyl(R₈) and -pyridinyl (R₈);
R₆ is selected from the group consisting of -dihydroimidazolyl(R₈), -tetrahydropyridinyl(R₈), -tetrahydropyrimidinyl(R₈) and -pyridinyl(R₈);
R₈ is one to four substituents independently selected from the group consisting of hydrogen and -C₁₋₄alkyl(R₉) when attached to a nitrogen atom; and, wherein R₈ is one to four substituents independently selected from the group consisting of hydrogen, -C₁₋₄alkyl(R₉), -C₁₋₄alkoxy(R₉), -O-aryl(R₁₀) and hydroxy when attached to a carbon atom;
R₉ is selected from the group consisting of hydrogen, -C₁₋₄alkoxy, -NH₂, -NH-C₁₋₄alkyl, -N(C₁₋₄alkyl)₂, (halo)₁₋₃ and hydroxy; R₁₀ is one to four substituents independently selected from the group consisting of hydrogen, -C₁₋₄alkyl, -C₁₋₄alkoxy, -C(=O)H, -C(=O)-C₁₋₄alkyl, -CO₂H, -CO₂-C₁₋₄alkyl, -NH₂, -NH-C₁₋₄alkyl, -N(C₁₋₄alkyl)₂, halo, hydroxy, nitro and oxo when attached to a carbon atom;
q is 1, 2 or 3;
Z is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈alkoxy, -O- C₁₋₈alkylcarbonylC₁₋₈alkyl, -O-C₁₋₈-alkyl-CO₂H, -O-C₁₋₈alkyl-C(O)O-C₁₋₈alkyl, -O- C₁₋₈alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(C₁₋₈alkyl)_{2,} -O-C₁₋₈alkylamide, -O-C₁₋₈alkyl-C(O)-NH-C₁₋₈alkyl, -O-C₁₋₈alkyl-C(O)-N(C₁₋₈alkyl)₂ and -NHC(O)C₁₋₈alkyl;
and pharmaceutically acceptable salts, racemic mixtures and enantiomers thereof.

21. A compound according to claim 1 of Formula (I.3): 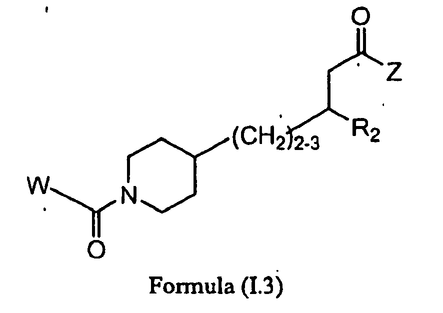 wherein
W is selected from the group consisting of -C₀₋₄alkyl(R₁) and -C₀₋₄alkyl-phenyl(R₁,R₈);
R₁ is selected from the group consisting of -NH(R₆), -dihydro-1*H*-pyrrolo[2,3-b]pyridinyl(R₈),-tetrahydropyrimidinyl(R₈), -tetrahydro-1,8-naphthyridinyl(R₈), -tetrahydro-1*H-*azepino[2,3-b]pyridinyl(R₈) and -pyridinyl(R₈);
R₂ is selected from the group consisting of hydrogen, -tetrahydropyrimidinyl(R₈), -1,3-benzodioxolyl(R₈), -dihydrobenzofuranyl(R₈), -tetrahydroquinolinyl(R₈), -phenyl(R₈), -naphthalenyl(R₈), -pyridinyl(R₈), -pyrimidinyl(R₈) and -quinolinyl(R₈);
R₆ is -dihydroimidazolyl(R₈), -tetrahydropyridiny(R₈), -tetrahydropyrimidinyl(R₈) or -pyridinyl(R₈);
R₈ is one to four substituents independently selected from the group consisting of hydrogen and -C₁₋₄alkyl(R₉) when attached to a nitrogen atom; and, wherein R₈ is one to four substituents independently selected from the group consisting of hydrogen, -C₁₋₄alkyl(R₉), -C₁₋₄alkoxy(R₉), -O-aryl(R₁₀) and hydroxy when attached to a carbon atom; and,
R₉ is selected from the group consisting of hydrogen, -C₁₋₄alkoxy, -NH₂, -NH-C₁₋₄alkyl, -N(C₁₋₄alkyl)₂, (halo)₁₋₃ and hydroxy;
R₁₀ is one to four substituents independently selected from the group consisting of hydrogen, -C₁₋₄alkyl, -C₁₋₄alkoxy, -C(=O)H, -C(=O)-C₁₋₄alkyl, -CO₂H, -CO₂-C₁₋₄alkyl, -NH₂, -NH-C₁₋₄alkyl, -N(C₁₋₄alkyl)₂, halo, hydroxy, nitro and oxo when attached to a carbon atom;
Z is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈alkoxy, -O- C₁₋₈alkylcarbonylC₁₋₈alkyl, -O-C₁₋₈alkyl-CO₂H, -O-C₁₋₈alkyl-C(O)O-C₁₋₈alkyl, -O- C₁₋₈alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(C₁₋₈alkyl)₂, -O-C₁₋₈alkylamide, -O-C₁₋₈alkyl-C(O)-NH-C₁₋₈alkyl, -O-C₁₋₈akyl-C(O)-N(C₁₋₈akyl)₂ and -NHC(O)C₁₋₈alkyl;
and pharmaceutically acceptable salts, racemic mixtures and enantiomers thereof.

22. The compound of claim 20 or 21 wherein R₁ is selected from the group consisting of -NH(R₆), -tetrahydropyrimidinyl(R₈) and-tetrahydro-1,8-naphthyridinyl(R₈); and, all other variables are as previously defined.

23. A compound of Formula (I.4): 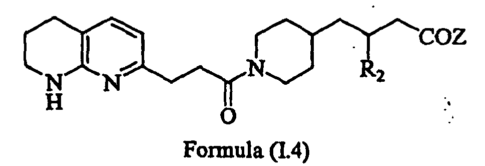
wherein R₂ is is selected from the group consisting of -2-benzofuranyl, -3-benzofuranyl, -4-benzofuranyl, -5-benzofuranyl, -6-benzofuzanyl, -7-benzofuranyl, -benzo[*b*]thien-2-yl, -benzo[*b*]thien-3-yl, -benzo[*b*]thien-4-yl, -benzo[*b*]thien-5-yl, -benzo[*b*]thien-6-yl, -benzo[*b*]thien-7-yl, -1*H*-indol-2-yl, -1*H*-indol-3-yl, -1*H*-indol-4-yl, -1*H*-indol-5-yl, -1*H*-indol-6-yl, -1*H*-indol-7-yl, -2-benzoxazolyl, -4-benzoxazolyl, -5-benzoxazolyl, -6-benzoxazolyl, -7-benzoxazolyl, -2-behzothiazolyl, -3-benzothiazolyl, -4-benzothiazolyl, -5-benzothiazolyl, -6-benzothiazolyl, -7-benzothiazolyl, -1*H*-benzimidazolyl-2-yl, -1*H*-benzimidazolyl-4-yl, -1*H*-benzimidazolyl-5-yl, -1*H*-benzimidazolyl-6-yl, -1*H*-benzimidazolyl-7-yl, -2-quinolinyl, -3-quinolinyl,-4-quinolinyl, -5-quinolinyl, -6-quinolinyl, -7-quinolinyl, -8-quinolinyl, -2*H*-1-benzopyran-2-yl, -2*H*-1-benzopyran-3-yl,-2*H*-1-benzopyran-4-yl, -2*H*-1-benzopyran-5-yl, -2*H*-1-benzopyran-6-yl, -2*H*-1-benzopyran-7-yl, -2*H*-1-benzopyran-8-yl, -4*H*-1-benzopyran-2-yl, -4*H*-1-benzopyran-3-yl, -4*H*-1-benzopyran-4-yl, -4*H*-1-benzopyraran-5-yl, -4*H*-1-benzopyran-6-yl, -4*H*-1-benzopyran-7-yl, -4*H*-1-benzopyran-8-yl, -1*H*-2-benzopyran-1-yl, -1*H*-2-benzopyran-3-yl, -1*H*-2-benzopyran-3-yl, -1*H*-2-benzopyran-5-yl, -1*H*-2-benzopyran-6-yl, -1*H*-2-benzopyran-7-yl, -1*H*-2-benzopyran-8-yl, -1,2,3,4-tetrahydro-1-naphthalenyl, -1,2,3,4-tetrahydro-2-naphthalenyl, -1,2,3,4-tetrahydro-5-naphthalenyl, -1,2,3,4-tetrahydro-6-naphthalenyl, -2,3-dihydro-2-benzofuranyl, -2,3-dihydro-3-benzofuranyl, -2,3-dihydro-4-benzofuranyl, -2,3-dihydro-5-benzofuranyl, -2,3-dihydro-6-benzofuranyl, -2,3-dihydro-7-benzofuranyl, -2,3-dihydrobenzo[*b*]thien-2-yl, -2,3-dihydrobenzo[*b*]thien-3-yl, -2,3-dihydrobenzo[*b*]thien-4-yl, -2,3-dihydrobenzo[*b*]thien-5-yl, -2,3-dihydrobenzo[*b*]thien-6-yl, -2,3-dihydrobenzo[*b*]thien-7-yl, -2,3-dihydro-1*H*-indol-2-yl, -2,3-dihydro-1*H*-indol-3-yl, -2,3-dihydro-1*H*-indol-4-yl, -2,3-dihydro-1*H*-indol-5-yl, -2,3-dihydro-1*H*-indol-6-yl, -2,3-dihydro-1*H*-indol-7-yl, -2,3-dihydro-2-benzoxazolyl, -2,3-dihydro-4-benzoxazolyl, -2,3-dihydro-5-benzoxazolyl, -2,3-dihydro-6-benzoxazolyl, -2,3-dihydro-7-benzoxazolyl, -2,3-dihydro-1*H*-benzimidazol-2-yl, -2,3-dihydro-1*H*-benzimidazol-4-yl, -2,3-dihydro-1*H*-benzimidazol-5-yl, -2,3-dihydro-1*H*-benzimidazol-6-yl, -2,3-dihydro-1*H*-benzimidazol-7-yl, -3,4-dihydro-1(2*H*)-quinolinyl, -1,2,3,4-tetrahydro-2-quinolinyl, -1,2,3,4-tetrahydro-3-quinolinyl, -1,2,3,4-tetrahydro-4-quinolinyl, -1,2,3,4-tetrahydro-5-quinolinyl, -1,2,3,4-tetrahydro-6-quinolinyl, -1,2,3,4-tetrahydro-7-quinolinyl, -1,2,3,4-tetrahydro-8-quinolinyl, -3,4-dihydro-2*H*-1-benzopyran-2-yl, -3,4-dihydro-2*H*-1-benzopyran-3-yl, -3,4-dihydro-2*H*-1-benzopyran-4-yl, -3,4-dihydro-2*H*-1-benzopyran-5-yl, -3,4-dihydro-2*H*-1-benzopyran-6-yl, -3,4-dihydro-2*H*-1-benzopyran-7-yl, -3,4-dihydro-2*H*-1-benzopyran-8-yl, -3,4-dihydro-4*H*-1-benzopyran-2-yl, -3,4-dihydro-4*H*-1-benzopyran-3-yl, -3,4-dihydro-4*H*-1-benzopyran-4-yl, -3,4-dihydro-4*H*-1-benzopyran-5-yl, -3,4-dihydro-4*H*-1-benzopyran-6-yl, -3,4-dihydro-4*H*-1-benzopyran-7-yl, -3,4-dihydro-4*H*-1-benzopyran-8-yl, -3,4-dihydro-1*H*-2-benzopyran-2-yl, -3,4-dihydro-1*H*-2-benzopyran-3-yl, -3,4-dibydro-1*H*-2-benzopyran-4-yl, -3,4-dihydro-1*H*-2-benzopyran-5-yl, -3,4-dihydro-1*H*-2-benzopyran-6-yl, -3,4-dihydro-1*H*-2-benzopyran-7-yl and -3,4-dihydro-1*H*-2-benzopyran-8-yl optionally substituted when allowed by available valences with up to 7 substituents independently selected from methyl when attached to a nitrogen atom; and, independently selected from methyl, methoxy or fluoro when attached to a carbon atom;
Z is selected from the group consisting of hydroxy, -NH₂, -NH-C₁₋₈alkyl, -N(C₁₋₈alkyl)₂, -O-C₁₋₈alkyl, -O-C₁₋₈alkyl-OH, -O-C₁₋₈alkylC₁₋₈alkoxy, -O- C₁₋₈alkyloarbonylC₁₋₈alkyl, -O-C₁₋₈alkyl-CO₂H, -O-C₁₋₈alkyl-C(O)O-C₁₋₈alkyl, -O- C₁₋₈alkyl-O-C(O)C₁₋₈alkyl, -O-C₁₋₈alkyl-NH₂, -O-C₁₋₈alkyl-NH-C₁₋₈alkyl, -O- C₁₋₈alkyl-N(₁₋₈alkyl)₂. -O-C₁₋₈alkylamide, -O-C₁₋₈alkyl-C(O)-NH-C₁₋₈alkyl, -O-C₁₋₈alkyl-C(O)-N(C₁₋₈alkyl)₂ and -NHC(O)C₁₋₈ alkyl;
and, pharmaceutically acceptable salts, racemic mixtures and enantiomers thereof.

24. A composition comprising the compound of any of claims 1 to 3 or 6 to 23, or a compound described in claim 4 or 5, and a pharmaceutically acceptable carrier.

25. A compound of any of claims 1 to 3 or 6 to 23, or a compound described in claim 4 or 5, for use in medicine.

26. A compound of any of claims 1 to 3 or 6 to 23, or a compound described in claim 4 or 5 for use in preventing treating or ameliorating an αv integrin mediated disorder wherein the av integrin mediated disorder is selected from the group consisting of cancers, cancer-associated pathologies, atherosclerosis, transplantation-induced vasculopathies, neointima formation, papilloma, lung fibrosis, pulmonary fibrosis; glomerulonephritis, glomerulosclerosis, congenital multicystic renal dysplasia, kidney fibrosis, diabetic retinopathy, macular degeneration, psoriasis, osteoporosis, bone resorption, inflammatory arthritis, rheumatoid arthritis, restenosis and adhesions and wherein the compound is administered in a therapeutically effective amount to a subject in need thereof.

27. The compound of claim 26 wherein the therapeutically effective amount is from about 0.001 mg/kg/day to about 1000mg/kg/day.

28. The compound of claim 26 or claim 27 wherein the disease mediated by cells pathologically expressing an αv integrin is selected from the group consisting of cancers, cancer-associated pathologies, diabetic retinopathy, macular degeneration, osteoporosis, bone resorption, inflammatory arthritis, rheumatoid arthritis and restenosis,

## Patentansprüche

1. Verbindung der Formel (I) 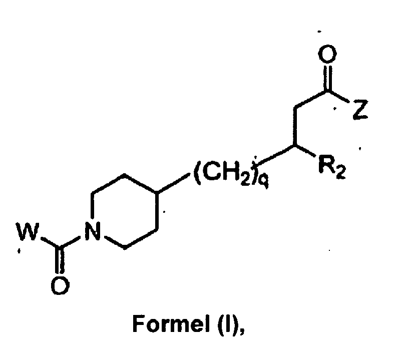 wobei
W von der Gruppe, bestehend aus -C₀₋₄Alkyl (R₁) und -C₀₋₄Alkyl- phenyl (R₁, R₈), ausgewählt ist;
R₁ von der Gruppe, bestehend aus -N(R₄) (R₆), -Dihydro-1H- pyrrolo[2,3-b]pyridinyl(R₈₎, -Tetrahydropyrimidinyl (R₈₎, -Tetrahydro-1,8 naphthyridinyl (R₈), -Tetrahydro-1H-azepino[2,3-b]pyridinyl (R₈) und -Pyridinyl (R₈), ausgewählt ist;
R₄ von der Gruppe, bestehend aus Wasserstoff und -C₁₋₈Alkyl(R₇) ausgewählt ist;
R₆ -Dihydroimidazolyl (R₈), -Tetrahydropyridinyl (R₈), -Tetrahydropyrimidinyl (R₈) oder -Pyridinyl (R₈) ist;
R₇ einen bis zwei Substituenten darstellt, welche unabhängig von der Gruppe, bestehend aus Wasserstoff, -C₁₋₈Alkoxy(R₉), -NH₂, -NH-C₁₋₈Alkyl(R₉), -N(C₁₋₈Alkyl (R₉))₂, -C(=O)H, -C(=O)-C₁₋₈Alkyl(R₉), -C(=O)-NH₂, -C(=O)-NH-C₁₋₈Alkyl(R₉), -C(=O)-N(C₁₋₈Alkyl(R₉))₂, -C(=O)-NH-Aryl(R₁₀), -C(=O)-Cycloalkyl(R₁₀), -C(=O)-Heterocyclyl(R₁₀), -C(=O)-Aryl(R₁₀), -C(=O)-Heteroaryl(R₁₀), -CO₂H, -CO₂-C₁₋₈Alkyl (R₉), -CO₂-Aryl(R₁₀) , -C(=NH) -NH₂, -SH, -S-C₁₋₈Alkyl(R₉), -S-C₁₋₈Alkyl-S-C₁₋₈Alkyl(R₉), -S-C₁₋₈Alkyl-C₁₋₈alkoxy(R₉), -S-C₁₋₈Alkyl-NH-C₁₋₈alkyl(R₉), -SO₂-C₁₋₈Alkyl(R₉), -SO₂-NH₂, -SO₂-NH-C₁₋₈Alkyl(R₉), -SO₂-N(C₁₋₈Alkyl(R₉))₂, -SO₂-Aryl(R₁₀), Cyano, (Halo)₁₋₃, Hydroxy, Nitro, Oxo, -Cycloalkyl(R₁₀), Heterocyclyl(R₁₀), -Aryl(R₁₀) und -Heteroaryl(R₁₀), ausgewählt sind;
R₈ einen bis vier Substituenten darstellt, welche unabhängig von der Gruppe, bestehend aus Wasserstoff, -C₁₋₈Alkyl (R₉), -C(=O)H, -C(=O)-C₁₋₈Alkyl(R₉), -C(=O)-NH₂, -C(=O)-NH-C₁₋₈Alkyl(R₉), -C(=O)-N(C₁₋₈Alkyl(R₉))₂, -C(=O)-NH-Aryl(R₁₀), -C(O)-Cycloalkyl(R₁₀), -C (=O) -Heterocyclyl (R₁₀), -C (=O) -Aryl (R₁₀), -C- (=O) -Heteroaryl (R₁₀), -CO₂H, -CO₂-C₁₋₉Alkyl(R₉), -CO₂-Aryl(R₁₀), -C(=NH)-NH₂, -SO₂-C₁₋₈Alkyl(R₉), -SO₂-NH₂, -SO₂-NH-C₁₋₈Alkyl(R₉), -SO₂-N(C₁₋₈Alkyl(R₉))₂, -SO₂-Aryl(R₁₀), -Cycloalkyl(R₁₀) und -Aryl(R₁₀), ausgewählt sind, wenn diese an ein Stickstoffatom gebunden sind; und wobei R₈ einen bis vier Substituenten darstellt, welche unabhängig von der Gruppe, bestehend aus -C₁₋₈Alkyl(R₉), -C₁₋₈Alkoxy(R₉), -O-Cycloalkyl(R₁₀), -O-Aryl(R₁₀), -C(=O)H, -C(=O)-C₁₋₈Alkyl(R₉), -C=)-NH₂, -C(=O)-NH-C₁₋₈Alkyl(R₉), -C(=O)-N(C₁₋₈Alkyl(R₉))₂, -C(=O)-NH-Aryl(R₁₀), -C(=O)-Cycloalkyl(R₁₀), -C(=O)-Heterocyclyl(R₁₀), -C(=O)-Aryl(R₁₀), -C(=O)-Heteroaryl(R₁₀), -CO₂H, -CO₂-C₁₋₈Alkyl(R₉), -CO₂-Aryl(R₁₀), -C(=NH)-NH₂,-SO₂-C₁₋₈Alkyl(R₉),-SO₂-NH₂, -SO₂-NH-C₁₋₈Alkyl(R₉), -SO₂-N(C₁₋₈Alkyl(R₉))₂, -SO₂-Aryl(R₁₀), -SH, -S-C₁₋₈Alkyl (R₉), -S-C₁₋₈Alkyl-S-C₁₋₈alkyl (R₉), -S-C₁₋₈Alkyl-C₁₋₈alkoxy (R₉₎, -S-C₁₋₈Alkyl-NH-C₁₋₈alkyl (R₉) , -NH₂, -NH-C₁₋₈Alkyl (R₉), -N(C₁₋₈Alkyl(R₉))₂, Cyano, Halo, Hydroxy, Nitro, Oxo, -Cycloalkyl (R₁₀), -Heterocyclyl (R₁₀), -Aryl(R₁₀) und -Heteroaryl (R₁₀), ausgwählt sind, wenn diese an ein Kohlenstoffatom gebunden sind;
R₉ von der Gruppe, bestehend aus Wasserstoff, -C₁₋₈Alkoxy, -NH₂, -NH-C₁₋₈Alkyl, -N (C₁₋₈Alkyl) ₂, -C(=O)H, -C (=O) -NH₂, -C(=O)-NH-C₁₋₈Alkyl,-C(=O)-N(C₁₋₈Alkyl)₂, -CO₂H, -CO₂-C₁₋₈Alkyl, -SO₂-C₁₋₈Alkyl, -SO₂-NH₂, -SO₂-NH-C₁₋₈Alkyl, -SO₂-N(C₁₋₈Alkyl)₂, Cyano, (Halo) ₁₋₃, Hydroxy, Nitro und Oxo, ausgewählt ist;
R₁₀ einen bis vier Substituenten darstellt, welche unabhängig von der Gruppe, bestehend aus Wasserstoff, -C₁₋₈Alkyl, -C(=O)H, -C(=)-C₁₋₈Alkyl, -C(=O)-NH₂, -C(=O)-NH-C₁₋₈Alkyl, -C(=O)-N(C₁₋₈Alkyl)₂, -CO₂H, -CO₂-C₁₋₄Alkyl, -SO₂-C₁₋₈Alkyl, -SO₂-NH₂, -SO₂-NH-C₁₋₈Alkyl und -SO₂-N(C₁₋₈Alkyl)₂, ausgewählt sind, wenn diese an ein Stickstoffatom gebunden sind; und wobei R₁₀ einen bis vier Substituenten darstellt, welche unabhängig von der Gruppe, bestehend aus Wasserstoff, -C₁₋₈Alkyl, -C₁₋₈Alkoxy, -C(=O)H, -C(=O)-C₁₋₈Alkyl, -C(=O)-NH₂,-C(=O)-NH-C₁₋₈Alkyl, -C(=O)-N(C₁₋₈Alkyl)₂,-CO₂H, -CO₂-C₁₋₄Alkyl, -SO₂-C₁₋₈Alkyl, -SO₂-NH₂, -SO₂-NH-C₁₋₈Alkyl, -SO₂-N(C₁₋₈Alkyl)₂, -NH₂, -NH-C₁₋₈Alkyl, -N(C₁₋₈Alkyl)₂, Cyano, Halo, Hydroxy, Nitro und Oxo, ausgewählt sind, wenn diese an ein Kohlenstoffatom gebunden sind;
R₂ Wasserstoff, -Tetrahydropyrimidinyl (R₈₎, -1,3-Benzodioxolyl(R₈₎, -Dihydrobenzofuranyl (R₈), -Tetrahydrochinolinyl(R₈),-Phenyl(R₈), -Naphthalinyl (R₈), -Pyridinyl(R₈),-Pyrimidinyl(R₈)oder -Chinolinyl(R₈) ist;
q von der aus 0, 1, 2 oder 3 bestehenden Gruppe ausgewählt ist;
Z von der Gruppe, bestehend aus Hydroxy, -NH₂, -NH-C₁₋₈Alkyl, -N(C₁₋₈Alkyl)₂, -O-C₁₋₈Alkyl, -O-C₁₋₈Alkyl-OH, -O-C₁₋₈Alkyl C₁₋₈alkoxy, -O-C₁₋₈AlkylcarbonylC₁₋₈alkyl, -O-C₁₋₈Alkyl-CO₂H, -O-C₁₋₈Alkyl-C(O)O-C₁₋₈alkyl, -O-C₁₋₈Alkyl-O-C(O)C₁₋₈alkyl, -O-C₁₋₈Alkyl-NH₂, -O-C₁₋₈Alkyl-NH-C₁₋₈alkyl, -O-C₁₋₈Alkyl-N(C₁₋₈Alkyl)₂, -O-C₁₋₈Alkylamid, -O-C₁₋₈Alkyl-C(O)-NH-C₁₋₈alkyl, -O-C₁₋₈Alkyl-C (O) -N (C₁₋₈Alkyl)₂ und -NHC(O) C₁₋₈Alkyl, ausgewählt ist; und pharmazeutisch annehmbare Salze, racemische Gemische und Enantiomere hievon.

2. Verbindung nach Anspruch 1, wobei R₁ von der Gruppe, bestehend aus -N(R₄)(R₆), -Tetrahydropyrimidinyl (R₈) und -Tetrahydro-1,8-naphthyridinyl (R₈₎, ausgewählt ist.

3. Verbindung der Formel (I): 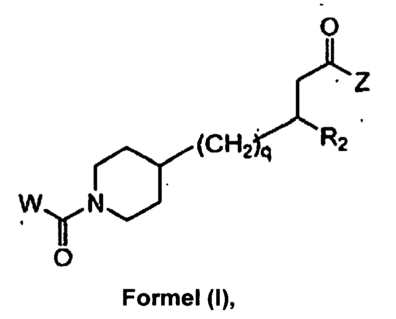 wobei W, R₁, R₂, q und Z ausgewählt sind unter
| W | R₁ | R₂ | q | Z |
|---|---|---|---|---|
| -CH₂-Ph(3-R₁) | -NH-1,4,5,6-Tetrahydro-pyrimidin-2-yl | H | 0 | OH |
| -(CH₂) ₂-Ph(3-R₁) | -NH-1,4,5,6-Tetrahydro-pyrimidin-2-yl | H | 0 | OH |
| -CH₂-Ph(3-R₁) | -NH-1,4,5,6-Tetrahydro-5-OH-pyrimidin-2-yl | Chinolin-3-yl | 0 | OH |
| -(CH₂)₃-R₁ | 5,6,7,8-Tetrahydro-[1,8]naphthyridin-2-yl | Chinolin-3-yl | 0 | OH |
| -(CH₂)₃-R₁ | 5,6,7,8-Tetrahydro-[1,8]naphthyridin-2-yl | 1,2,3,4-Tetrahydro-chinolin-3-yl | 0 | OH |
| Ph(3-R₁) | -NH-1,4,5,6-Tetrahydro-pyrimidin-2-yl | Pyridin-3-yl | 2 | OH |
| Ph(3-R₁) | -NH-1,4,5,6-Tetrahydro-5-OH-pyrimidin-2-yl | Pyridin-3-yl | 2 | OH |
| -(CH₂)₂-R₁ | 5,6,7,8-Tetrahydro-[1,8]-naphthyridin-2-yl | Pyridin-3-yl | 2 | OH |
| -(CH₂)₃-R₁ | -NH-Pyridin-2-yl | Pyridin-3-yl | 2 | OH |
| Ph(3-R₁) | -NH-1,4,5,6-Tetrahydro-5-OH-pyrimidin-2-yl | (6-OCH₃)-Pyridin-3-yl | 2 | OH |
| -(CH₂)₂-R₁ | 5,6,7,8-Tetrahydro-[1,8]naphthyridin-2-yl | 1,3-Benzodioxol-5-yl | 1 | OH |
| Ph(3-R₁) | -NH-1,4,5,6-Tetrahydro-pyrimidin 2-yl | Chinolin-3-yl | 2 | OH |
| -(CH₂)₂-R₁ | 5,6,7,8-Tetrahydro-[1,8]naphthyridin-2-yl | Phenyl | 1 | OH |
| -(CH₂)₂-R₁ | 5,6,7,8-Tetrahydro-[1,8]naphthyridin-2-yl | 1,3-Benzodioxol-5-yl | 0 | OH |
| -(CH₂)₃-R₁ | 5,6,7,8-Tetrahydro-[1,8]naphthyridin-2-yl | 1,3-Benzodioxol-5-yl | 0 | OH |
| -CH₂-R₁ | 5,6,7,8-Tetrahydro-[1,8]naphthyridin-2-yl | 1,3-Benzodioxol-5-yl | 0 | OH |
| -(CH₂)₃-R₁ | 5,6,7,8-Tetrahydro-[1,8]naphthyridin-2-yl | (6-OCH₃)-Pyridin-3-yl | 0 | OH |
| -(CH₂)₂-R₁ | 5,6,7,8-Tetrahydro-[1,8]naphthyridin-2-yl | 1,4,5,6,-Tetrahydro-2-Me-pyrimidin-5-yl | 1 | OH |
| -(CH₂)₂-R₁ | 5,6,7,8-Tetrahydro-[1,8]naphthyridin-2-yl | 1,2,3,4-Tetrahydro-chinolin-3-yl | 1 | OH |
| -(CH₂)₂-R₁ | 5,6,7,8-Tetrahydro-[1,8]naphthyridin-2-yl | 1,3-Benzodioxol-5-yl | 2 | OH |
| -(CH₂)₂-R₁ | 5,6,7,8-Tetrahydro-[1,8]naphthyridin-2-yl | (6-OCH₃)-Pyridin-3-yl | 2 | OH |
| -(CH₂)₂-R₁ | -NH-1,4,5,6-Tetrahydro-5-OH pyrimidin-2-yl | (6-OCH₃)-Pyridin-3-yl | 2 | OH |
| -(CH₂)₂-R₁ | -NH-1,4,5,6-Tetrahydro-5-OH pyrimidin-2-yl | (6-OCH₃)-Pyridin-3-yl | 2 | OH |
| -(CH₂)₃-R₁ | -NH-Pyridin-2-yl | Chinolin-3-yl | 2 | OH |
| -(CH₂)₃-R₁ | -NH-Pyridin-2-yl | 1,3-Benzodioxol-5-yl | 2 | OH |
| -(CH₂)₃-R₁ | -NH-Pyridin-2-yl | 1,3-Benzodioxol-5-yl | 0 | OH |
| -(CH₂)₃-R₁ | -NH-Pyridin-2-yl | (6-OCH₃)-Pyridin-3-yl | 2 | OH |
| -(CH₂)₃-R₁ | 5,6,7,8-Tetrahydro-[1,8]naphthyridin-2-yl | 1,3- Benzodioxol-5-yl | 1 | OH |
| Ph(3-R₁) | -NH-1,4,5,6-Tetrahydro-5-OH-pyrimidin-2-yl | 1,3- Benzodioxol-5-yl | 1 | OH |
| -(CH₂)₂-R₁ | 5,6,7,8-Tetrahydro-[1,8]naphthyridin-2-yl | (6-OCH₃)-Pyridin-3-yl | 1 | OH |
| -(CH₂)₃-R₁ | 5,6,7,8-Tetrahydro-[1,8]naphthyridin-2-yl | Chinolin-3-yl | 1 | OH |
| -(CH₂)₂-R₁ | 5,6,7,8-Tetrahydro-[1,8]naphthyridin-2-yl | (3-F)Phenyl | 1 | OH |
| -(CH₂)₃-R₁ | 5,6,7,8-Tetrahydro-[1,8]naphthyridin-2-yl | (3-F)Phenyl | 1 | OH |
| -(CH₂)₂-R₁ | 5,6,7,8-Tetrahydro-[1,8]naphthyridin-2-yl | Chinolin-3-yl | 1 | OH |
| -(CH₂)₂-R₁ | 5,6,7,8-Tetrahydro-[1,8]naphthyridin-2-yl | (4-F)Phenyl | 1 | OH |
| -(CH₂)₃-R₁ | 5,6,7,8-Tetrahydro-[1,8]naphthyridin-2-yl | (4-F)Phenyl | 1 | OH |
| -(CH₂)₂-R₁ | 5,6,7,8-Tetrahydro-[1,8]naphthyridin-2-yl | (2-CH₃)Pyrimidin-5-yl | 1 | OH |
| -(CH₂)₂-R₁ | 5,6,7,8-Tetrahydro-[1,8]naphthyridin-2-yl | 2,3-Dihydro- benzofuran-6-yl | 1 | OH |
| -(CH₂)₂-R₁ | 5,6,7,8-Tetrahydro-[1,8]naphthyridin-2-yl | (3,5-Difluoro)-phenyl | 1 | OH |
| -(CH₂)₃-R₁ | 5,6,7,8-Tetrahydro- [1,8]naphthyridin-2-yl | (3,5-Difluoro)-phenyl | 1 | OH |
| -(CH₂)₂-R₁ | 5,6,7,8-Tetrahydro-[1,8]naphthyridin-2-yl | (3-CF₃)-Phenyl | 1 | OH |
| -(CH₂)₂-R₁ | 5,6,7,8-Tetrahydro-[1,8]naphthyridin-2-yl | (4-OCF₃)-Phenyl | 1 | OH |
| -(CH₂)₂-R₁ | 5,6,7,8-Tetrahydro-[1,8]naphthyridin-2-yl | (3-F-4-Ph)-Phenyl | 1 | OH |
| -(CH₂)₂-R₁ | 5,6,7,8-Tetrahydro-[1,8]naphthyridin-2-yl | (3-F-4-OCH₃)-Phenyl | 1 | OH |
| -(CH₂)₂-R₁ | 5,6,7,8-Tetrahydro-[1,8]naphthyridin-2-yl | (4-OPh)-Phenyl | 1 | OH |
| -(CH₂)₂-R₁ | 5,6,7,8-Tetrahydro-[1,8]naphthyridin-2-yl | Isochinolin-4-yl | 1 | OH |
| -(CH₂)₂-R₁ | 5,6,7,8-Tetrahydro-[1,8]naphthyridin-2-yl | Pyridin-3-yl | 1 | OH |
| -(CH₂)₂-R₁ | 5,6,7,8-Tetrahydro-[1,8]naphthyridin-2-yl | Dihydrobenzofuran -5-yl | 1 | OH |
| -(CH₂)₂-R₁ | 5,6,7,8-Tetrahydro-[1,8]naphthyridin-2-yl | (2,4-OCH₃)-Pyrimidin-5-yl | 1 | OH |
| -(CH₂)₂-R₁ | 5,6,7,8-Tetrahydro-[1,8]naphthyridin-2-yl | (2-OCH₃)-Pyrimidin-5-yl | 1 | OH |
| Ph(3-R₁) | -NH-1,4,5,6-Tetrahydro-5-OH-pyrimidin-2-yl | Chinolin-3-yl | 2 | OH |
| Ph(3-R₁) | -NH-1,4,5,6-Tetrahydro-pyridin-2-yl | Chinolin-3-yl | 2 | OH |
| -(CH₂)₂-R₁ | 5,6,7,8-Tetrahydro-[1,8]naphthyridin-2-yl | Chinolin-3-yl | 2 | OH |
| Ph(3-R₁) | -NH-3,4,5,6-Tetrahydro-pyrimidin-2-yl | 1,3-Benzodioxol-5-yl | 2 | OH |
| Ph(3-R₁) | -NH-3,4,5,6-Tetrahydro-pyridin-2-yl | 1,3-Benzodioxol-5-yl | 2 | OH |
| Ph(3-R₁) | -NH-1,4,5,6-Tetrahydro-5-OH-pyrimidin-2-yl | 1,3-Benzodioxol-5-yl | 2 | OH |
| -CH₂-R₁ | 5,6,7,8-Tetrahydro-[1,8]naphthyridin-2-yl | 1,3-Benzodioxol-5-yl | 2 | OH |
| -(CH₂)₂-R₁ | 5,6,7,8-Tetrahydro-[1,8]naphthyridin-2-yl | Naphthalin-2-yl | 1 | OH |
| -(CH₂)₂-R₁ | 5,6,7,8-Tetrahydro-[1,8]naphthyridin-2-yl | 5,6,7,8-Tetrahydro-chinolin-3-yl | 1 | OH |
| -(CH₂)₃-R₁ | 5,6,7,8-Tetrahydro-[1,8]naphthyridin-2-yl | 5,6,7,8-Tetrahydro-chinolin-3-yl | 0 | OH |
| -(CH₂)₂-R₁ | 5,6,7,8-Tetrahydro-[1,8]naphthyridin-2-yl | (3-OCH₃)-Phenyl | 1 | OH |
| -(CH₂)₂-R₁ | 5,6,7,8-Tetrahydro-[1,8]naphthyridin-2-yl | (4-OCH₃)-Phenyl | 1 | OH |
| -(CH₂)₂-R₁ | 5,6,7,8-Tetrahydro-[1,8]naphthyridin-2-yl | H | 1 | OH |
| -(CH₂)₂-R₁ | 5,6,7,8-Tetrahydro-[1,8]naphthyridin-2-yl | Tetrahydrofuran-3-yl | 1 | OH |
| -(CH₂)₂-R₁ | 5,6,7,8-Tetrahydro-[1,8]naphthyridin-2-yl | Thiophen-2-yl | 1 | OH |
| -(CH₂)₂-R₁ | 5,6,7,8-Tetrahydro-[1,8]naphthyridin-2-yl | (3-F)Phenyl | 1 | NH₂ |
| -(CH₂)₂-R₁ | 5,6,7,8-Tetrahydro-[1,8]naphthyridin-2-yl | 2,3-Dihydro-benzol[1,4]-dioxin-6-yl | 1 | OH |
| -(CH₂)₂-R₁ | 5,6,7,8-Tetrahydro-[1,8]naphthyridin-2-yl | (3-SCH₃)-Phenyl | 1 | OH |
| -(CH₂)₂-R₁ | 5,6,7,8-Tetrahydro-[1,8]naphthyridin-2-yl | N-Methyl-1,2,3,4-tetrahydro-chinolin-3-yl | 1 | OH |
| -(CH₂)₂-R₁ | 5,6,7,8-Tetrahydro-[1,8]naphthyridin-2-yl | H | 1 | -O-Ethyl |
| -(CH₂)₂-R₁ | 5,6,7,8-Tetrahydro-[1,8]naphthyridin-2-yl | H | 1 | -O-2-Propyl |
| -(CH₂)₂-R₁ | 5,6,7,8-Tetrahydro-[1,8]naphthyridin-2-yl | H | 1 | -O-t-Butyl |
| -(CH₂)₂-R₁ | 5,6,7,8-Tetrahydro-[1,8]naphthyridin-2-yl | H | 1 | -O-n-Octyl |
| -(CH₂)₂-R₁ | 5,6,7,8-Tetrahydro-[1,8]naphthyridin-2-yl | H | 1 | -O-s-Butyl |
| -(CH₂)₂-R₁ | 5,6,7,8-Tetrahydro-[1,8]naphthyridin-2-yl | H | 1 | -O-Methyl |
| -(CH₂)₂-R₁ | 5,6,7,8-Tetrahydro-[1,8]naphthyridin-2-yl | H | 1 | -O-CH₂-OC(O)-t-Butyl |
| -(CH₂)₂-R₁ | 5,6,7,8-Tetrahydro-[1,8]naphthyridin-2-yl | (3-(NMe₂)Phenyl | 1 | OH |
| -(CH₂)₂-R₁ | 5,6,7,8-Tetrahydro-(1,8)naphthyridin-2-yl | (3-OMe-4-OH)Phenyl | 1 | OH |
| Ph(3-R₁) | -NH-4,5-dihydro-1*H* imidazol-2-yl | (3-F)Phenyl | 1 | OH |
| -(CH₂)₂-R₁ | 5,6,7,8-Tetrahydro-[1,8]naphthyridin-2-yl | (3-NHEt)Phenyl | 1 | OH |
| -(CH₂)₂-R₁ | 5,6,7,8-Tetrahydro-[1,8]naphthyridin-2- yl | (3-(NHMe)Phenyl | 1 | OH |
| -(CH₂)₃-R₁ | 5,6,7,8-Tetrahydro-[1,8]naphthyridin-2-yl | Dihydrobenzofuran -6-yl | 0 | OH |
und pharmazeutisch annehmbare Salze, racemische Gemische und Enantiomere hievon.

4. Zusammensetzung, umfassend eine Verbindung nach Anspruch 1 oder Anspruch 3, wobei die Verbindung ausgewählt ist von der Gruppe, bestehend aus:
einer Verbindung der Formel (I), wobei W -CH₂-Ph(3-R₁) ist; R₁ -NH-1,4,5,6-Tetrahydro-pyrimidin-2-yl ist; R₂ H ist, q 0 ist, und Z OH ist;
einer Verbindung der Formel (I), wobei W -(CH₂)₂-Ph(3-R₁) ist; R₁ -NH-1,4,5,6-Tetrahydro-pyrimidin-2-yl ist; R₂ H ist, q 0 ist, und Z OH ist;
einer Verbindung der Formel (I), wobei W -CH₂-Ph(3-R₁) ist; R₁ -NH-1,4,5,6-Tetrahydro-5-OH-pyrimidin-2-yl ist; R₂ -3-Chinolinyl ist, q 0 ist, und Z OH ist;
einer Verbindung der Formel (I), wobei W -(CH₂)₃-R₁ ist; R₁ -5,6,7,8-Tetrahydro-1,8-naphthyridin-2-yl ist; R₂ -3-Chinolinyl ist, q 0 ist, und Z OH ist;
einer Verbindung der Formel (I), wobei W -(CH₂)₃-R₁ ist; R₁-5,6,7,8-Tetrahydro-1,8-naphthyridin-2-yl ist; R₂-1,2,3,4-Tetrahydro-3-chinolinyl ist, q 0 ist, und Z OH ist;
einer Verbindung der Formel (I), wobei W -Ph(3-R₁) ist; R₁ -NH-1,4,5,6-Tetrahydro-pyrimidin-2-yl ist; R₂ -3-Pyridinyl ist, q 2 ist, und Z OH ist;
einer Verbindung der Formel (I), wobei W -Ph(3-R₁) ist; R₁-NH-1,4,5,6-Tetrahydro-5-OH-pyrimidin-2-yl ist; R₂-3-Pyridinyl ist, q 2 ist, und Z OH ist;
einer Verbindung der Formel (I), wobei W-(CH₂)₂-R₁ ist; R₁-5,6,7,8-Tetrahydro-1,8-naphthyridin-2-yl ist; R₂-3-Pyridinyl ist, q 2 ist, und Z OH ist;
einer Verbindung der Formel (I), wobei W -(CH₂)₃-R₁ ist; R₁ -NH-Pyridin-2-yl ist; R₂ -3-Pyridinyl ist, q 2 ist, und Z OH ist;
einer Verbindung der Formel (I), wobei W -Ph(3-R₁) ist; R₁ -NH-1,4,5,6-Tetrahydro-5-OH-pyrimidin-2-yl ist; R₂ -(6-MeO)Pyridin-3-yl ist, q 2 ist, und Z OH ist;
einer Verbindung der Formel (I), wobei W-(CH₂)₂-R₁ ist; R₁ -5,6,7,8-Tetrahydro-1,8-naphthyridin-2-yl ist; R₂ -1,3-Benzodioxol-5-yl, q 1 ist, und Z OH ist;
einer Verbindung der Formel (I), wobei W -Ph(3-R₁) ist; R₁ -NH-1,4,5,6-Tetrahydro-pyrimidin-2-yl ist; R₂ -3-Chinolinyl ist, q 2 ist, und Z OH ist;
einer Verbindung der Formel (I), wobei W-(CH₂)₂-R₁ ist; R₁ -5,6,7,8-Tetrahydro-1,8-naphthyridin-2-yl ist; R₂ -Ph ist,
q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -1,3-Benzodioxol-5-yl ist, q 0 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -1,3-Benzodioxol-5-yl ist, q 0 ist und Z OH ist;
einer Verbindung der Formel (I), wobei W -CH₂R₁ ist; R₁ -5,6,7,8-Tetrahydro-1,8-naphthyridin-2-yl ist; R₂ -1,3-Benzodioxol ist, q 0 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -(6-MeO)Pyridin-3-yl ist, q 0 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -1,4,5,6-Tetrahydro-2-Me-pyrimidin-5-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -1,2,3,4-Tetrahydro-3-chinolinyl, q 1 ist und Z OH ist;
einer Verbindung der Formel (I), wobei W-(CH₂)₂-R₁ ist; R₁ -5,6,7,8-Tetrahydro-1,8-naphthyridin-2-yl ist; R₂ -1,3-benzodioxol-5-yl ist, q 2 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(6-MeO)Pyridin-3-yl ist, q 2 ist und Z OH ist;
einer Verbindung der Formel (I), wobei W -(CH₂)₃-R₁ ist; R₁ -NH-Pyridin-2-yl ist; R₂ -3-Chinolinyl ist, q 2 ist und Z OH ist;
einer Verbindung der Formel (I), wobei W -(CH₂)₃-R₁ ist; R₁ -NH-Pyridin-2-yl ist; R₂ -1,3-Benzodioxol-5-yl ist, q 2 ist und Z OH ist;
einer Verbindung der Formel (I), wobei W -(CH₂)₃-R₁ ist; R₁ -NH-Pyridin-2-yl ist; R₂ -1,3-Benzodioxol-5-yl ist, q 0 ist und Z OH ist;
einer Verbindung der Formel (I), wobei W -(CH₂)₃-R₁ ist; R₁ -NH-Pyridin-2-yl ist; R₂-(6-MeO)Pyridin-3-yl ist, q 2 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -1,3-Benzodioxol-5-yl ist, q 1 ist und Z OH ist;
einer Verbindung der Formel (I), wobei W -Ph(3-R₁) ist; R₁ -NH-1,4,5,6-Tetrahydro-5-OH-2-pyrimidinyl ist; R₂ -1,3-Benzodioxol-5-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(6-MeO)Pyridin-3-yl ist, q 1 ist und Z OH ist;
einer Verbindung der Formel (I), wobei W -(CH₂)₃-R₁ ist; R₁ -5,6,7,8-Tetrahydro-1,8-naphthyridin-2-yl ist; R₂-3-Chinolinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(3-F)Ph ist, q 1 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -(3-F)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -3-Chinolinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(4-F)Ph ist, q 1 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -(4-F)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(2-Me)Pyrimidin-5-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -2,3-Dihydro-benzofuran-6-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(3,5-F₂)Ph ist, q 1 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -(3,5-F₂)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(3-CF₃)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(4-OCF₃) Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(3-F-4-Ph)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(3-F-4-OMe)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(4-OPh)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -4-Isochinolinyl ist, q 1 ist und Z OH ist;
einer Verbindung der Formel (I), wobei W - (CH₂)₂-R₁ ist; R₁ -5,6,7,8-Tetrahydro-1,8-naphthyridin-2-yl ist; R₂ -3-Pyridinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -5-Dihydrobenzofuranyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -2,4-(OMe)₂-Pyrimid-5-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(2-(OMe)Pyrimidin-5-yl ist, q 1 ist und Z OH ist;
einer Verbindung der Formel (I), wobei W -Ph(3-R₁) ist; R₁ -NH-1,4,5,6-Tetrahydro-5-OH-pyrimidin-2-yl ist; R₂ -3-Chinolinyl ist, q 2 ist und Z OH ist;
einer Verbindung der Formel (I), wobei W -Ph(3-R₁) ist; R₁ -NH-3,4,5,6-Tetrahydro-pyridin-2-yl ist; R₂ -3-Chinolinyl ist, q 2 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -3-Chinolinyl ist, q 2 ist und Z OH ist;
einer Verbindung der Formel (I), wobei W -Ph(3-R₁) ist; R₁ -NH-3,4,5,6-Tetrahydro-pyrimidin-2-yl ist; R₂ -1,3-Benzodioxol-5-yl ist, q 2 ist und Z OH ist;
einer Verbindung der Formel (I), wobei W -Ph(3-R₁) ist; R₁ -NH-3,4,5,6-Tetrahydro-pyridin-2-yl ist; R₂ -1,3-Benzodioxol-5-yl ist, q 2 ist und Z OH ist;
einer Verbindung der Formel (I), wobei W -Ph(3-R₁) ist; R₁ -NH-1,4,5,6-Tetrahydro-5-OH-pyrimidin-2-yl ist; R₂ -1,3-Benzodioxol-5-yl ist, q 2 ist und Z OH ist;
einer Verbindung der Formel (I), wobei W -CH₂-R₁ ist; R₁ -5,6,7,8-Tetrahydro-1,8-naphthyridin-2-yl ist; R₂ -1,3-Benzodioxol-5-yl ist, q 2 ist und Z OH ist; und
einer Verbindung der Formel (I), wobei W -(CH₂)₂-R₁ ist; R₁ -5,6,7,8-Tetrahydro-1,8-naphthyridin-2-yl ist; R₂ -2-Naphthalinyl ist, q 1 ist und Z OH ist.

5. Zusammensetzung nach Anspruch 4, wobei die Verbindung von der Gruppe ausgewählt ist, bestehend aus:
einer Verbindung der Formel (I), wobei W -(CH₂)₃-R₁ ist; R₁ -5,6,7,8-Tetrahydro-1,8-naphthyridin-2-yl ist; R₂ -1,2,3,4-Tetrahydro-3-chinolinyl ist, q 0 ist und Z OH ist;
einer Verbindung der Formel (I), wobei W -(CH₂)₃-R₁ ist; R₁ -5,6,7,8-Tetrahydro-1,8-naphthyridin-2-yl ist; R₂ -1,3-Benzodioxol-5-yl ist, q 0 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -1,2,3,4-Tetrahydro-3-chinolinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(6-MeO)-Pyridin-3-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(3-F)Ph ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -3-Chinolinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -(2-Me)-Pyrimidin-5-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -2,3-Dihydro-benzofuran-6-yl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -4-Isochinolinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -3-Pyridinyl ist, q 1 ist und Z OH ist;
einer Verbindung der Formel (I), wobei W -(CH₂)₂-R₁ ist; R₁ -5,6,7,8-Tetrahydro-1,8-naphthyridin-2-yl ist; R₂ -2,4-(OMe)₂-Pyrimid-5-yl ist, q 1 ist und Z OH ist; und
einer Verbindung der Formel (I), wobei W -(CH₂)₂-R₁ ist; R₁ -5,6,7,8-Tetrahydro-1,8-naphthyridin-2-yl ist; R₂ -(2-OMe)Pyrimidin-5-yl ist, q 1 ist und Z OH ist.

6. Verbindung nach Anspruch 1, wobei W -(CH₂)₃-R₁ ist; R₁ -5,6,7,8-Tetrahydro-1,8-naphthyridin-2-yl ist; R₂ -1,2,3,4-Tetrahydro-3-chinolinyl ist, q 0 ist und Z OH ist.

7. Verbindung nach Anspruch 1, wobei W -(CH₂)₃-R₁ ist; R₁ -5,6,7,8-Tetrahydro-1,8-naphthyridin-2-yl ist; R₂ -1,3-Benzodioxol-5-yl ist, q 0 ist und Z OH ist.

8. Verbindung nach Anspruch 1, wobei W -(CH₂)₂-R₁ ist; R₁ -5,6,7,8-Tetrahydro-1,8-naphthyridin-2-yl ist; R₂ -1,2,3,4-Tetrahydro-3-chinolinyl ist, q 1 ist und Z OH ist.

9. Verbindung nach Anspruch 1, wobei W -(CH₂)₂-R₁ ist; R₁ -5,6,7,8-Tetrahydro-1,8-naphthyridin-2-yl ist; R₂ -(6-MeO)Pyridin-3-yl ist, q 1 ist und Z OH ist.

10. Verbindung nach Anspruch 1, wobei W -(CH₂)₂-R₁ ist; R₁ -5,6,7,8-Tetrahydro-1,8-naphthyridin-2-yl ist; R₂ -(3-F)Ph ist, q 1 ist und Z OH ist.

11. Verbindung nach Anspruch 1, wobei W -(CH₂)₂-R₁ ist; R₁ -5,6,7,8-Tetrahydro-1,8-naphthyridin-2-yl ist; R₂ -3-Chinolinyl ist, q 1 ist und Z OH ist.

12. Verbindung nach Anspruch 1, wobei W -(CH₂)₂-R₁ ist; R₁ -5,6,7,8-Tetrahydro-1,8-naphthyridin-2-yl ist; R₂ -(2-Me)Pyrimidin-5-yl ist, q 1 ist und Z OH ist.

13. Verbindung nach Anspruch 1, wobei W -(CH₂)₂-R₁ ist; R₁ -5,6,7,8-Tetrahydro-1,8-naphthyridin-2-yl ist; R₂ -2,3-Dihydro-benzofuran-6-yl ist, q 1 ist und Z OH ist.

14. Verbindung nach Anspruch 3, wobei W -(CH₂)₂-R₁ ist; R₁ -5,6,7,8-Tetrahydro-1,8-naphthyridin-2-yl ist; R₂ -4-Isochinolinyl ist, q 1 ist und Z OH ist.

15. Verbindung nach Anspruch 1, wobei W -(CH₂)₂-R₁ ist; R₁ -5,6,7,8-Tetrahydro-1,8-naphthyridin-2-yl ist; R₂ -3-Pyridinyl ist, q 1 ist und Z OH ist.

16. Verbindung nach Anspruch 1, wobei W -(CH₂)₂-R₁ ist; R₁ -5,6,7,8-Tetrahydro-1,8-naphthyridin-2-yl ist; R₂ -2,4-(OMe)₂-Pyrimid-5-yl ist, q 1 ist und Z OH ist.

17. Verbindung nach Anspruch 1, wobei W-(CH₂)₂-R₁ ist; R₁ -5,6,7,8-Tetrahydro-1,8-naphthyridin-2-yl ist; R₂ -(2-OMe)Pyrimidin-5-yl ist, q 1 ist und Z OH ist.

18. Verbindung nach Anspruch 1, wobei W -(CH₂)₃-R₁ ist; R₁ -5,6,7,8-Tetrahydro-1,8-naphthyridin-2-yl ist; R₂ -2,3-Dihydro-benzofuran-6yl ist, q 0 ist und Z OH ist.

19. Verbindung der Formel (I): 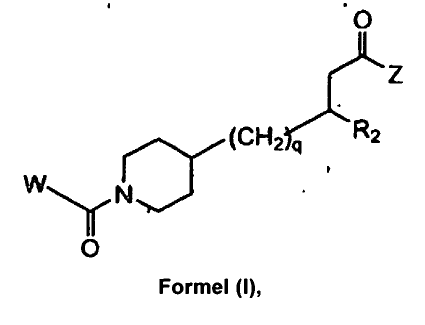 wobei
W von der Gruppe, bestehend aus -C₀₋₄Alkyl (R₁) und -C₀₋₄Alkyl-phenyl (R₁, R₈) ausgewählt ist;
R₁ -NH (R₆) ist;
R₂ von der Gruppe, bestehend aus Wasserstoff, -Tetrahydropyrimidinyl(R₈), -1,3-Benzodioxolyl(R₈), -Dihydrobenzofuranyl(R₈), -Tetrahydrochinolinyl(R₈), -Phenyl(R₈), -Naphthalinyl(R₈), -Pyridinyl(R₈), -Pyrimidinyl(R₈) und -Chinolinyl(R₈), ausgewählt ist;
R₆ von der Gruppe, bestehend aus -Dihydroimidazolyl(R₈), -Tetrahydropyridinyl(R₈), -Tetrahydropyrimidinyl(R₈) und -Pyridinyl(R₈), ausgewählt ist;
R₈ einen bis vier Substituenten darstellt, welche unabhängig von der Gruppe, bestehend aus Wasserstoff und -C₁₋₄Alkyl(R₉), ausgewählt sind, wenn diese an ein Stick- stoffatom gebunden sind; und wobei R₈ einen bis vier Substituenten darstellt, welche unabhängig von der Gruppe, bestehend aus Wasserstoff, -C₁₋₄Alkyl(R₉), -C₁₋₄Alkoxy(R₉), -O-Aryl(R₁₀) und Hydroxy, ausgewählt sind, wenn diese an ein Kohlenstoffatom gebunden sind;
R₉ von der Gruppe, bestehend aus Wasserstoff, -C₁₋₄Alkoxy, -NH₂, -NH-C₁₋₄Alkyl, -N (C₁₋₄Alkyl)₂, (Halo)₁₋₃ und Hydroxy, ausgewählt ist;
R₁₀ unabhängig von der Gruppe, bestehend aus Wasserstoff, -C₁₋₄Alkyl, -C₁₋₄Alkoxy, -C (=O) H, -C (=O) -C₁₋₄Alkyl, -CO₂H, -CO₂-C₁₋₄Alkyl, -NH₂, -NH-C₁₋₄Alkyl, -N(C₁₋₄Alkyl)₂, Halo, Hydroxy, Nitro und Oxo, ausgewählt ist, wenn dieser an ein Kohlenstoffatom gebunden ist;
q 1, 2 oder 3 ist;
Z von der Gruppe, bestehend aus Hydroxy, -NH₂, -NH-C₁₋₈Alkyl, -N(C₁₋₈Alkyl)₂, O-C₁₋₈Alkyl-OH, O-C₁₋₈AlkylC₁₋₈alkoxy, -O-C₁₋₈AlkylcarbonylC₁₋₈alkyl, -O-C₁₋₈Alkyl-CO₂H, -O-C₁₋₈Alkyl-C(O)O-C₁₋₈alkyl, -O-C₁₋₈Alkyl-O-C(O)C₁₋₈alkyl, -O-C₁₋₈Alkyl-NH₂, -O-C₁₋₈Alkyl-NH-C₁₋₈alkyl, -O-C₁₋₈Alkyl-N(C₁₋₈alkyl)₂, -O-C₁₋₈Alkylamid, -O-C₁₋₈Alkyl-C(O)-NH-C₁₋₈alkyl, -O-C₁₋₈Alkyl-C(O)-N(C₁₋₈alkyl)₂ und -NHC(O)C₁₋₈Alkyl, ausgewählt ist;
und pharmazeutisch annehmbare Salze, racemische Gemische und Enantiomere hievon.

20. Verbindung nach Anspruch 1 der Formel (I.2): 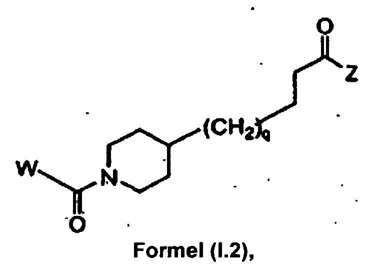 wobei
W von der Gruppe, bestehend aus -C₀₋₄Alky1 (R₁) und -C₀₋₄Alkyl-phenyl (R₁, R₈), ausgewählt ist;
R₁ von der Gruppe, bestehend aus -NH(R₆), -Dihydro-1*H*-pyrrolo[2,3-*b*]pyridinyl(R₈), -Tetrahydropyrimidinyl(R₈), -Tetrahydro-1,8-naphthyridinyl(R₈), -Tetrahydro-1*H*-azepino[2,3-*b*]pyridinyl(R₈) und -Pyridinyl(R₈), ausgewählt ist;
R₆ von der Gruppe, bestehend aus -Dihydroimidazolyl(R₈), -Tetrahydropyridinyl(R₈), -Tetrahydropyrimidinyl(R₈) und -Pyridinyl(R₈), ausgewählt ist;
R₈ einen bis vier Substituenten darstellt, welche unabhängig von der Gruppe, bestehend aus Wasserstoff und -C₁₋₄Alkyl(R₉), ausgewählt sind, wenn diese an ein Stick- stoffatom gebunden sind; und wobei R₈ einen bis vier Substituenten darstellt, welche unabhängig von der Gruppe, bestehend aus Wasserstoff, -C₁₋₄Alkyl(R₉), -C₁₋₄Alkoxy(R₉), -O-Aryl(R₁₀) und Hydroxy, ausgewählt sind, wenn diese an ein Kohlenstoffatom gebunden sind;
R₉ von der Gruppe, bestehend aus Wasserstoff, -C₁₋₄A1koxy, -NH₂, -NH-C₁₋₄Alkyl, -N (C₁₋₄Alkyl)₂, (Halo)₁₋₃ und Hydroxy, ausgewählt ist;
R₁₀ einen bis vier Substituenten darstellt, welche unabhängig von der Gruppe, bestehend aus Wasserstoff, -C₁₋₄Alkyl, -C₁₋₄Alkoxy, -C(=O)H, -C(=O)-C₁₋₄Alkyl, -CO₂H, -CO₂-C₁₋₄Alkyl, -NH₂, -NH-C₁₋₄Alkyl, -N(C₁₋₄Alkyl)₂, Halo, Hydroxy, Nitro und Oxo, ausgewählt sind, wenn diese an ein Kohlenstoffatom gebunden sind;
q 1, 2 oder 3 ist;
Z von der Gruppe, bestehend aus Hydroxy, -NH₂, -NH-C₁₋₈Alkyl, -N(C₁₋₈Alkyl)₂, O-C₁₋₈Alkyl, O-C₁₋₈Alkyl-OH, O-C₁₋₈AlkylC₁₋₈alkoxy, -O-C₁₋₈AlkylcarbonylC₁₋₈alkyl, -O-C₁₋₈Alkyl-CO₂H, -O-C₁₋₈Alkyl-C(O)O-C₁₋₈alkyl, -O-C₁₋₈Alkyl-O-C(O)C₁₋₈alkyl, -O-C₁₋₈Alkyl-NH₂, -O-C₁₋₈Alkyl-NH-C₁₋₈alkyl, -O-C₁₋₈Alkyl-N(C₁₋₈alkyl)₂, -O-C₁₋₈Alkylamid, -O-C₁₋₈Alkyl-C(O)-NH-C₁₋₈alkyl, -O-C₁₋₈Alkyl-C(O)-N(C₁₋₈alkyl)₂ und -NHC(O)C₁₋₈Alkyl, ausgewählt ist;
und pharmazeutisch annehmbare Salze, racemische Gemische und Enantiomere hievon.

21. Verbindung nach Anspruch 1 der Formel (I.3): 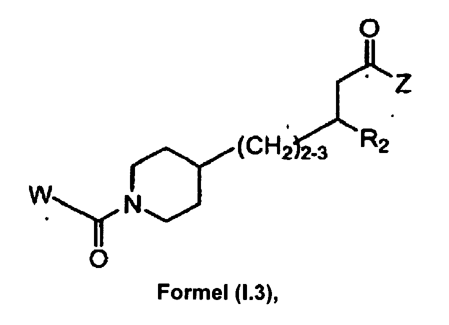 wobei
W von der Gruppe, bestehend aus -C₀₋₄Alkyl (R₁) und -C₀₋₄Alkyl-phenyl(R₁, R₈), ausgewählt ist;
R₁ von der Gruppe, bestehend aus -NH(R₆), -Dihydro-1*H*-pyrrolo[2,3-*b*]pyridinyl(R₈), -Tetrahydropyrimidinyl(R₈), -Tetrahydro-1,8-naphthyridinyl(R₈), -Tetrahydro-1*H*-azepino[2,3-*b*]pyridinyl(R₈) und -Pyridinyl(R₈), ausgewählt ist;
R₂ von der Gruppe, bestehend aus Wasserstoff, -Tetrahydropyrimidinyl(R₈), -1,3-Benzodioxolyl(R₈), -Dihydrobenzofuranyl(R₈), -Tetrahydrochinolinyl(R₈), -Phenyl(R₈), -Naphthalinyl(R₈), -Pyridinyl(R₈), -Pyrimidinyl(R₈) und -Chinolinyl(R₈), ausgewählt ist;
R₆ -Dihydroimidazolyl(R₈), -Tetrahydropyridinyl(R₈), -Tetrahydropyrimidinyl(R₈) und -Pyridinyl(R₈) ist;
R₈ einen bis vier Substituenten darstellt, welche unabhängig von der Gruppe, bestehend aus Wasserstoff und -C₁₋₄Alkyl(R₉), ausgewählt sind, wenn diese an ein Stick- stoffatom gebunden sind; und wobei R₈ einen bis vier Substituenten darstellt, welche unabhängig von der Gruppe, bestehend aus Wasserstoff, -C₁₋₄Alkyl(R₉), -C₁₋₄Alkoxy(R₉), -O-Aryl(R₁₀) und Hydroxy, ausgewählt sind, wenn diese an ein Kohlenstoffatom gebunden sind;
R₉ von der Gruppe, bestehend aus Wasserstoff, -C₁₋₄Alkoxy, -NH₂, -NH-C₁₋₄Alkyl, -N(C₁₋₄Alkyl)₂, (Halo)₁₋₃ und Hydroxy, ausgewählt ist;
R₁₀ einen bis vier Substituenten darstellt, welche unabhängig von der Gruppe, bestehend aus Wasserstoff, -C₁₋₄Alkyl, -C₁₋₄Alkoxy, -C (=O) H, -C(=O)-C₁₋₄Alkyl, -CO₂H, -CO₂-C₁₋₄Alkyl, -NH₂, -NH-C₁₋₄Alkyl, -N(C₁₋₄Alkyl)₂, Halo, Hydroxy, Nitro und Oxo, ausgewählt sind, wenn diese an ein Kohlenstoffatom gebunden sind;
Z von der Gruppe, bestehend aus Hydroxy, -NH₂, -NH-C₁₋₈Alkyl, -N(C₁₋₈Alkyl)₂, O-C₁₋₈Alkyl, O-C₁₋₈Alkyl-OH, O-C₁₋₈AlkylC₁₋₈alkoxy, -O-C₁₋₈AlkylcarbonylC₁₋₈alkyl, -O-C₁₋₈Alkyl-CO₂H, -O-C₁₋₈Alkyl-C (O) O-C₁₋₈alkyl, -O-C₁₋₈Alkyl-O-C (O) C₁₋₈alkyl, -O-C₁₋₈Alkyl-NH₂, -O-C₁₋₈Alkyl-NH-C₁₋₈alkyl, -O-C₁₋₈Alkyl-N (C₁₋₈alkyl)₂, -O-C₁₋₈Alkylamid, -O-C₁₋₈Alkyl-C(O)-NH-C₁₋₈alkyl, -O-C₁₋₈Alkyl-C(O)-N(C₁₋₈alkyl)₂ und -NHC(O)C₁₋₈Alkyl, ausgewählt sind;
und pharmazeutisch annehmbare Salze, racemische Gemische und Enantiomere hievon.

22. Verbindung nach Anspruch 20 oder 21, wobei R₁ von der Gruppe, bestehend aus -NH(R₆), -Tetrahydropyrimidinyl(R₈) und -Tetrahydro-1,8-naphthyridinyl(R₈), ausgewählt ist; und alle anderen Variablen wie zuvor definiert sind.

23. Verbindung der Formel (I.4): 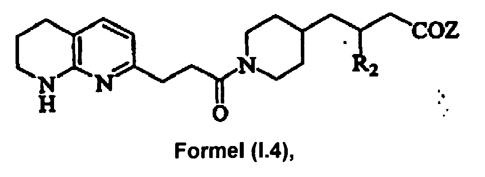 wobei
R₂ von der Gruppe, bestehend aus -2-Benzofuranyl, -3-Benzofuranyl, -4-Benzofuranyl, -5-Benzofuranyl, -6-Benzofuranyl, -7-Benzofuranyl, -Benzo[*b*]thien-2-yl, -Benzo[*b*]thien-3-yl, -Benzo[*b*]thien-4-yl, -Benzo[*b*]thien-5-yl, -Benzo[*b*]thien-6-yl, -Benzo[*b*]thien-7-yl, -1*H*-Indol-2-yl, -1*H*-Indol-3-yl, -1*H*-Indol-4-yl, -1*H*-Indol-5-yl, -1*H*-Indol-6-yl, -1*H*-Indol-7-yl, -2-Benzoxazolyl, -4-Benzoxazolyl, -5-Benzoxazolyl, -6-Benzoxazolyl, -7-Benzoxazolyl, -2-Benzothiazolyl, -3-Benzothiazolyl, -4-Benzothiazolyl, -5-Benzothiazolyl, -6-Benzothiazolyl, -7-Benzothiazolyl, -1*H*-Benzimidazolyl-2-yl, -1*H*-Benzimidazolyl-4-yl, -1*H*-Benzimidazolyl-5-yl, -1*H*-Benzimidazolyl-6-yl, -1*H*-Benzimidazolyl-7-yl, -2-Chinolinyl, -3-Chinolinyl, -4-Chinolinyl, -5-Chinolinyl, -6-Chinolinyl, -7-Chinolinyl, -8-Chinolinyl, -2*H*-1-Benzopyran-2-yl, -2*H*-1-Benzopyran-3-yl, -2*H*-1-Benzopyran-4-yl, -2*H*-1-Benzopyran-5-yl, -2*H*-1-Benzopyran-6-yl, -2*H*-1-Benzopyran-7-yl, -2*H*-1-Benzopyran-8-yl, -4*H*-1-Benzopyran-2-yl, -4*H*-1-Benzopyran-3-yl, -4*H*-1-Benzopyran-4-yl, -4*H*-1-Benzopyran-5-yl, -4*H*-1-Benzopyran-6-yl, -4*H*-1-Benzopyran-7-yl, -4*H*-1-Benzopyran-8-yl, -1*H*-2-Benzopyran-1-yl, -1*H*-2-Benzopyran-3-yl, -1*H*-2-Benzopyran-3-yl, -1*H*-2-Benzopyran-5-yl, -1*H*-2-Benzopyran-6-yl, -1*H*-2-Benzopyran-7-yl, -1*H*-2-Benzopyran-8-yl, -1,2,3,4-Tetrahydro-1-naphthalinyl, -1,2,3,4-Tetrahydro-2-naphthalinyl, -1,2,3,4-Tetrahydro-5-naphthalinyl, -1,2,3,4-Tetrahydro-6-naphthalinyl, -2,3-Dihydro-2-benzofuranyl, -2,3-Dihydro-3-benzofuranyl, -2,3-Dihydro-4-benzofuranyl, -2,3-Dihydro-5-benzofuranyl, -2,3-Dihydro-6-benzofuranyl, -2,3-Dihydro-7-benzofuranyl, -2,3-Dihydrobenzo[*b*]thien-2-yl, -2,3-Dihydrobenzo[*b*]thien-3-yl, -2,3-Dihydrobenzo[*b*]thien-4-yl, -2,3-Dihydrobenzo[*b*]thien-5-yl, -2,3-Dihydrobenzo[*b*]thien-6-yl, -2,3-Dihydrobenzo[*b*]thien-7-yl, -2,3-Dihydro-1*H*-indol-2-yl, -2,3-Dihydro-1*H*-indol-3-yl, -2,3-Dihydro-1*H*-indol-4-yl, -2,3-Dihydro-1*H*-indol-5-yl, -2,3-Dihydro-1*H*-indol-6-yl, -2,3-Dihydro-1*H*-indol-7-yl, -2,3-Dihydro-2-benzoxazolyl, -2,3-Dihydro-4-benzoxazolyl, -2,3-Dihydro-5-benzoxazolyl, -2,3-Dihydro-6-benzoxazolyl, -2,3-Dihydro-7-benzoxazolyl, -2,3-Dihydro-1*H*-benzimidazol-2-yl, -2,3-Dihydro-1*H*-benzimidazol-4-yl, -2,3-Dihydro-1*H*-benzimidazol-5-yl, -2,3-Dihydro-1*H*-benzimidazol-6-yl, -2,3-Dihydro-1*H*-benzimidazol-7-yl, -3,4-Dihydro-1(*2H*)-chinolinyl, -1,2,3,4-Tetrahydro-2-chinolinyl, -1,2,3,4-Tetrahydro-3-chinolinyl, -1,2,3,4-Tetrahydro-4-chinolinyl, -1,2,3,4-Tetrahydro-5-chinolinyl, -1,2,3,4-Tetrahydro-6-chinolinyl, -1,2,3,4-Tetrahydro-7-chinolinyl, -1,2,3,4-Tetrahydro-8-chinolinyl, -3,4-Dihydro-2*H*-1-benzopyran-2-yl, -3,4-Dihydro-2*H*-1-benzopyran-3-yl, -3,4-Dihydro-2*H*-1-benzopyran-4-yl, -3,4-Dihydro-2*H*-1-benzopyran-5-yl, -3,4-Dihydro-2*H*-1-benzopyran-6-yl, -3,4-Dihydro-2*H*-1-benzopyran-7-yl, -3,4-Dihydro-2*H*-1-benzopyran-8-yl, -3,4-Dihydro-4*H*-1-benzopyran-2-yl, -3,4-Dihydro-4*H*-1-benzopyran-3-yl, -3,4-Dihydro-4*H*-1-benzopyran-4-yl, -3,4-Dihydro-4*H*-1-benzopyran-5-yl, -3,4-Dihydro-4*H*-1-benzopyran-6-yl, -3,4-Dihydro-4*H*-1-benzopyran-7-yl, -3,4-Dihydro-4*H*-1-benzopyran-8-yl, -3,4-Dihydro-1*H*-2-benzopyran-2-yl, -3,4-Dihydro-1*H*-2-benzopyran-3-yl, -3,4-Dihydro-1*H*-2-benzopyran-4-yl, -3,4-Dihydro-1*H*-2-benzopyran-5-yl, -3,4-Dihydro-1*H*-2-benzopyran-6-yl, -3,4-Dihydro-1*H*-2-benzopyran-7-yl und -3,4-Dihydro-1*H*-2-benzopyran-8-yl, ausgewählt ist, wahlweise substituiert, wenn durch dies aufgrund verfügbarer Valenzen zulässig ist, mit bis zu 7 Substituenten, welche unabhängig ausgewählt sind unter Methyl, wenn sie an ein Stickstoffatom gebunden sind; und welche unabhängig ausgewählt sind unter Methyl, Methoxy oder Fluor, wenn sie an ein Kohlenstoffatom gebunden sind;
Z von der Gruppe, bestehend aus Hydroxy, -NH₂, -NH-C₁₋₈Alkyl, -N(C₁₋₈Alkyl)₂, -O-C₁₋₈Alkyl, -O-C₁₋₈Alkyl-OH, -O-C₁₋₈AlkylC₁₋₈alkoxy, -O-C₁₋₈AlkylcarbonylC₁₋₈alkyl, -O-C₁₋₈Alkyl-CO₂H, -O-C₁₋₈Alkyl-C(O)O-C₁₋₈alkyl, -O-C₁₋₈Alkyl-O-C(O)C₁₋₈alkyl, -O-C₁₋₈Alkyl-NH₂, -O-C₁₋₈Alkyl-NH-C₁₋₈alkyl, -O-C₁₋₈Alkyl-N(C₁₋₈alkyl)₂, -O-C₁₋₈Alkylamid, -O-C₁₋₈Alkyl-C(O)-NH-C₁₋₈alkyl, -O-C₁₋₈Alkyl-C(O)-N(C₁₋₈alkyl)₂ und -NHC(O)C₁₋₈Alkyl, ausgewählt ist;
und pharmazeutisch annehmbare Salze, racemische Gemische und Enantiomere hievon.

24. Zusammensetzung, umfassend die Verbindung nach einem der Ansprüche 1 bis 3 oder 6 bis 23, oder eine Verbindung, welche in Anspruch 4 oder 5 beschrieben ist, und einen pharmazeutisch annehmbaren Träger.

25. Verbindung nach einem der Ansprüche 1 bis 3 oder 6 bis 23, oder eine Verbindung, welche in Anspruch 4 oder 5 beschrieben ist, zur Verwendung in der Medizin.

26. Verbindung nach einem der Ansprüche 1 bis 3 oder 6 bis 23, oder eine Verbindung, welche in Anspruch 4 oder 5 beschrieben ist, zur Prävention, Behandlung oder Verbesserung einer durch αv-Integrin mediierten Störung, wobei die durch αv-Integrin mediierte Störung von der Gruppe, bestehend aus Krebs, mit Krebs assoziierten Pathologien, Atherosklerose, durch Transplantation hervorgerufenen Vaskulopathien, Neointimabildung, Papillom, Lungenfibrose, pulmonarer Fibrose, Glomerulonephritis, Glomerulosklerose, angeborener multizystischer renaler Dysplasie, Nierenfibrose, diabetischer Retinopathie, Makuladegeneration, Psoriasis, Osteoporose, Knochenresorption, entzündlicher Arthritis, rheumatoider Arthritis, Restenose und Adhäsionen, ausgewählt ist, und wobei die Verbindung in einer therapeutisch wirksamen Menge an einen Patienten, welcher einer solchen bedarf, verabreicht wird.

27. Verbindung nach Anspruch 26, wobei die therapeutisch wirksame Menge von etwa 0,001 mg/kg/Tag bis etwa 1000 mg/kg/Tag beträgt.

28. Verbindung nach Anspruch 26 oder 27, wobei die Krankheit, welche durch Zellen mediiert wird, welche pathologisch ein αv-Integrin exprimieren, von der Gruppe, bestehend aus Krebs, mit Krebs assoziierten Pathologien, diabetischer Retinopathie, Makuladegeneration, Osteoporose, Knochenresorption, entzündlicher Arthritis, rheumatoider Arthritis und Restenose, ausgewählt ist.

## Revendications

1. Composé de formule (I) 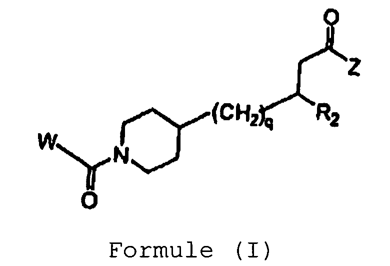 dans laquelle
W est choisi dans le groupe constitué par un groupe -alkyl en C_{0 à 4} (R₁) et -alkyl en C_{0 à 4}-phényl(R₁, R₈) ;
R₁ est choisi dans le groupe constitué par
-N(R₄)(R₆), -dihydro-1H-pyrrolo[2,3-b]pyridinyl(R₈),
-tétrahydropyrimidinyl(R₈), -tétrahydro-1,8-naphtyridinyl(R₈),
-tétrahydro-1H-azépino[2,3-b]pyridinyl(R₈) et -pyridinyl(R₈) ;
R₄ est choisi dans le groupe constitué par un atome d'hydrogène et un groupe -alkyl en C_{1 à 8}(R₇) ;
R₆ est un groupe -dihydroimidazolyl(R₈), -tétrahydropyridinyl(R₈), -tétrahydropyrimidinyl(R₈) ou -pyridinyl(R₈) ;
R⁷ est un à deux substituants indépendamment choisis dans le groupe constitué par
un atome d'hydrogène, un groupe -alcoxy en C_{1 à 8}(R₉), -NH₂, -NH-alkyl en C_{1 à 8} (R₉), -N (alkyl en C_{1 à 8}(R₉))₂, -C(=O)H, -C(=O)-alkyl en C_{1 à 8}(R₉), -C(=O)-NH₂, -C(=O)-NH-alkyl en C_{1 à 8}(R₉), -C(=O)-N(alkyl en C_{1 à 8}) (R₉))₂, C-(=O)NH-aryl(R₁₀), -C(=O)-cycloalkyl(R₁₀), -C(=O)-hétérocyclyl(R₁₀), -C(=O)-aryl(R₁₀), -C(=O)-hétéroaryl(R₁₀), -CO₂H, -CO₂-alkyl en C_{1 à 8} (R₉), -CO₂-aryl (R₁₀), -C (=NH) -NH₂, -SH, -S-alkyl en C_{1 à 8} (R₉), -S-alkyl en C_{1 à 8}-S-alkyl en C_{1 à 8} (R₉), -S-alkyl en C_{1 à 8}-alcoxy en C_{1 à 8} (R₉), -S-alkyl en C_{1 à 8}-NH-alkyl en C_{1 à 8} (R₉), -SO₂-alkyl en C_{1 à 8} (R₉), -SO₂-NH₂, -SO₂-NH-alkyl en C_{1 à 8} (R₉), -SO₂-N-(alkyl en C_{1 à 8} (R₉))₂, -SO₂-aryl (R₁₀), cyano, (halogéno)_{1 à 3}, hydroxy, nitro, oxo, -cycloalkyl (R₁₀), -hétérocyclyl (R₁₀), -aryl (R₁₀) et -hétéroaryl (R₁₀) ;
R⁸ est un à quatre substituants indépendamment choisis dans le groupe constitué par
un atome d'hydrogène, un groupe -alkyl en C_{1 à 8}(R₉), -C(=O)H, -C(=O)-alkyl en C_{1 à 8} (R₉), -C(=O)-NH₂, -C(=O)-NH-alkyl en C_{1 à 8} (R₉), -C(=O)-NH-(alkyl en C_{1 à 8} (R₉))₂, -C(=O)-NH-aryl(R₁₀), -C(=O)-cycloalkyl(R₁₀), -C(=O)-hétérocyclyl(R₁₀), -C(=O)-aryl(R₁₀), -C(=O)-hétéroaryl (R₁₀), -CO₂H, -CO₂-alkyl en C_{1 à 8} (R₉), -CO₂-aryl (R₁₀), -C(=NH)-NH₂, -SO₂-alkyl en C_{1 à 8} (R₉), -SO₂-NH₂, -SO₂-NH-alkyl en C_{1 à 8} (R₉), -SO₂-N-(alkyl en C_{1 à 8} (R₉))₂, -SO₂-aryl (R₁₀), -cycloalkyl (R₁₀) et -aryl (R₁₀) lorsqu'il est attaché à un atome d'azote ; et où R⁸ est un à quatre substituants indépendamment choisis dans le groupe constitué par un atome d'hydrogène, un groupe -alkyl en C_{1 à 8} (R₉), alcoxy en C_{1 à} (R₉), -O-cycloalkyl (R₁₀), -O-aryl (R₁₀), -C(=O)H, -C(=O)-alkyl en C_{1 à 8} (R₉), -C(=O)-NH₂, -C(=O)-NH-alkyl en C_{1 à 8} (R₉), -C(=O)-NH-(alkyl en C_{1 à 8} (R₉))₂, -C(=O)-NH-aryl(R₁₀), -C(=O)-cycloalkyl (R₁₀), -C(=O)-hétérocyclyl (R₁₀), -C(=O)-aryl(R₁₀), -C(=O)-hétéroaryl (R₁₀), -CO₂H, -CO₂-alkyl en C_{1 à 8} (R₉), -CO₂-aryl(R₁₀), -C(=NH)-NH₂, -SO₂-alkyl en C_{1 à 8}(R₉), -SO₂-NH₂, -SO₂-NH-alkyl en C_{1 à 8}(R₉), -SO₂-N-(alkyl en C_{1 à 8}(R₉))₂, -SO₂-aryl (R₁₀), -SH, -S-alkyl en C_{1 à 8} (R₉) , -S-alkyl en C_{1 à 8}-S-alkyl en C_{1 à 8}(R₉), -S-alkyl en C_{1 à 8}-alcoxy en C_{1 à 8}(R₉) , -S-alkyl en C_{1 à 8}-NH-alkyl en C_{1 à 8}(R₉), -NH₂, -NH-alkyl en C_{1 à 8}(R₉), -N-(alkyl en C_{1 à 8}(R₉))₂, cyano, halogéno, hydroxy, nitro, oxo, -cycloalkyl(R₁₀). -hétérocyclyl (R₁₀), -aryl (R₁₀) et -hétéroaryl (R₁₀) lorsqu'il est attaché à un atome de carbone ;
R₉ est choisi dans le groupe constitué par un atome d'hydrogène, un groupe -alcoxy en C_{1 à 8} ; -NH₂, -NH-alkyle en C_{1 à 8}, -N(alkyle en C_{1 à 8})₂, -C(=O)H, -C(=O)-NH₂, -C (=O) -NH-alkyle en C_{1 à 8}, -C (=O) -N (alkyle en C_{1 à 8})₂, -CO₂H, -CO₂-alkyle en C_{1 à 8}, -SO₂-alkyle en C_{1 à 8}, -SO₂-NH₂, -SO₂-NH-alkyle en C_{1 à 8}, -SO₂-N(alkyle en C_{1 à 8})₂, cyano, (halogéno)_{1 à 3}, hydroxy, nitro et oxo ;
R₁₀ est un à quatre substituants indépendamment choisis dans le groupe constitué par
un atome d'hydrogène, un groupe -alkyle en C_{1 à 8}, -C(=O)H, -C(=O)-alkyle en C_{1 à 8}, -C(=O)-NH₂, -C(=O)NH-alkyle en C_{1 à 8}, -C (=O) -N (alkyle en C_{1 à 8})₂, -CO₂H, -CO₂-alkyle en C_{1 à 4}, -SO₂-alkyle en C_{1 à 8}, -SO₂-NH₂, -SO₂-NH-alkyle en C_{1 à 8} et -SO₂-N (alkyle en C_{1 à 8})₂ lorsqu' il est attaché à un atome d'azote ; et où R₁₀ est un à quatre substituants indépendamment choisis dans le groupe constitué par un atome d'hydrogène, un groupe -alkyle en C_{1 à 8}, -alcoxy en C_{1 à 8}, -C (=O) H, -C (=O) -alkyle en C_{1 à 8}, -C(=O)-NH₂, -C(=O)NH-alkyle en C_{1 à 8}, -C(=O)-N (alkyle en C_{1 à 8})₂, -CO₂H, -CO₂-alkyle en C_{1 à 4}, -SO₂-alkyle en C_{1 à 8}, -SO₂-NH₂, -SO₂-NH-alkyle en C_{1 à 8}, -SO₂-N(alkyle en C_{1 à 8})₂, NH₂, -NH-alkyle en C_{1 à 8}, -N(alkyle en C_{1 à 8})₂, cyano, halogéno, hydroxy, nitro et oxo lorsqu'il est attaché à un atome de carbone ;
R₂ est un atome d'hydrogène, un groupe -tétrahydropyrimidinyl(R₈), -1,3-benzodioxolyl(R₈), -dihydrobenzofuranyl(R₈), -tétrahydroquinoléinyl(R₈), -phényl(R₈), naphtalényl(R₈), -pyridinyl(R₈), -pyrimidinyl (R₈) ou -quinoléinyl (R₈) ;
q est choisi dans le groupe constitué par 0, 1, 2 ou 3 ;
Z est choisi dans le groupe constitué par un groupe hydroxy, -NH₂, -NH-alkyle en C_{1 à 8}, -N(alkyle en C_{1 à 8})₂, -O-alkyle en C_{1 à 8}, -O-alkyl en C_{1 à 8}-OH, -O-alkyl en C_{1 à 8}-alcoxy en C_{1 à 8}, -O-alkylcarbonyl en C_{1 à 8}-alkyle en C_{1 à 8}, -O-alkyl en C_{1 à 8}-CO₂H, -O-alkyl en C_{1 à 8}-C(O)O-alkyle en C_{1 à 8}, -O-alkyl en C_{1 à 8}-O-C(O)alkyle en C_{1 à 8}, -O-alkyl en C_{1 à 8}-NH₂, -O-alkyl en C_{1 à 8}-NH-alkyle en C_{1 à 8}, -O-alkyl en C_{1 à 8}-N(alkyle en C_{1 à 8})₂, -O-alkylamide en C_{1 à 8}, -O-alkyl en C_{1 à 8}-C(O)-NH-alkyle en C_{1 à 8}, -O-alkyl en C_{1 à 8}-C(O)-N(alkyle en C_{1 à 8})₂ et -NHC(O)alkyle en C_{1 à 8} ;
et sels, mélanges racémiques et énantiomères pharmaceutiquement acceptables de celui-ci.

2. Composé selon la revendication 1, dans lequel R₁ est choisi dans le groupe constitué par
un groupe -N(R₄)(R₆), -tétrahydropyrimidinyl(R₈₎ et -tétrahydro-1,8-naphtyridinyl (R₈).

3. Composé de formule (I) : 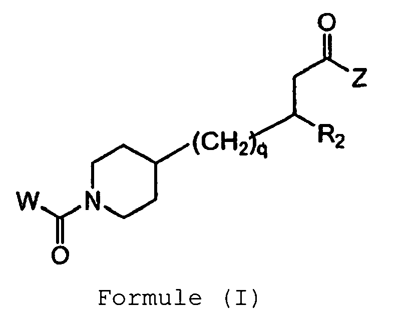 dans laquelle W, R₁, R₂, q et Z sont choisis parmi :
| W | R₁ | R₂ | q | Z |
|---|---|---|---|---|
| -CH₂-Ph(3-R₁) | -NH-1,4,5,6-tétrahydro-pyrimidin-2-yle | H | 0 | OH |
| -(CH₂)₂-Ph(3-R₁) | -NH-1,4,5,6-tétrahydro-pyrimidin-2-yle | H | 0 | OH |
| -CH₂-Ph(3-R₁) | -NH-1,4,5,6-tétrahydro-5-OH-pyrimidin-2-yle | quinoléin-3-yle | 0 | OH |
| -(CH₂)₃₋R₁ | 5,6,7,8-tétrahydro-[1,8]naphtyridin-2-yle | quinoléin-3-yle | 0 | OH |
| -(CH₂)₃-R₁ | 5,6,7,8-tétrahydro-[1,8]naphtyridin-2-yle | 1,2,3,4-tétrahydro-quinoléin-3-yle | 0 | OH |
| Ph(3-R₁) | -NH-1,4,5,6-tétrahydro-pyrimidin-2-yle | pyridin-3-yle | 2 | OH |
| Ph(3-R₁) | -NH-1,4,5,6-tétrahydro-5-OH-pyrimidin-2-yle | pyridin-3-yle | 2 | OH |
| -(CH₂)₂-R₁ | 5,6,7,8-tétrahydro-[1,8]naphtyridin-2-yle | pyridin-3-yle | 2 | OH |
| -(CH₂)₃-R₁ | -NH-pyridin-2-yle | pyridin-3-yle | 2 | OH |
| Ph(3-R₁) | -NH-1,4,5,6-tétrahydro-5-OH-pyrimidin-2-yle | (6-OCH₃)-pyridin-3-yle | 2 | OH |
| -(CH₂)₂-R₁ | 5,6,7,8-tétrahydro-[1,8]naphtyridin-2-yle | 1,3-benzodioxol-5-yle | 1 | OH |
| Ph(3-R₁) | -NH-1,4,5,6-tétrahydro-pyrimidin-2-yle | quinoléin-3-yle | 2 | OH |
| -(CH₂)₂-R₁ | 5,6,7,8-tétrahydro-[1,8]naphtyridin-2-yle | phényle | 1 | OH |
| -(CH₂)₂-R₁ | 5,6,7,8-tétrahydro-[1,8]naphtyridin-2-yle | 1,3-benzodioxol-5-yle | 0 | OH |
| -(CH₂)₃-R₁ | 5,6,7,8-tétrahydro-[1,8]naphtyridin-2-yle | 1,3-benzodioxol-5-yle | 0 | OH |
| -CH₂-R₁ | 5,6,7,8-tétrahydro-[1,8]naphtyridin-2-yle | 1,3-benzodioxol-5-yle | 0 | OH |
| -(CH₂)₃-R₁ | 5,6,7,8-tétrahydro-[1,8]naphtyridin-2-yle | (6-OCH₃)-pyridin-3-yle | 0 | OH |
| -(CH₂)₂-R₁ | 5,6,7,8-tétrahydro-[1,8]naphtyridin-2-yle | 1,4,5,6-tétrahydro-2-Me-pyrimidin-5-yle | 1 | OH |
| -(CH₂)₂-R₁ | 5,6,7,8-tétrahydro- [1,8]naphtyridin-2-yle | 1,2,3,4-tétrahydro- quinoléin-3-yle | 1 | OH |
| -(CH₂)₂-R₁ | 5,6,7,8-tétrahydro-[1,8]naphtyridin-2-yle | 1,3-benzodioxol-5-yle | 2 | OH |
| -(CH₂)₂-R₁ | 5,6,7,8-tétrahydro-[1,8]naphtyridin-2-yle | (6-OCH₃)-pyridin-3-yle | 2 | OH |
| -(CH₂)₂-R₁ | -NH-1,4,5,6-tétrahydro-5-OH-pyrimidin-2-yle | (6-OCH₃)-pyridin-3-yle | 2 | OH |
| -(CH₂)₂-R₁ | -NH-1,4,5,6-tétrahydro-5-OH-pyrimidin-2-yle | (6-OCH₃)-pyridin-3-yle | 2 | OH |
| -(CH₂)₃-R₁ | -NH-pyridin-2-yle | quinoléin-3-yle | 2 | OH |
| -(CH₂)₃-R₁ | -NH-pyridin-2-yle | 1,3-benzodioxol-5-yle | 2 | OH |
| -(CH₂)₃-R₁ | -NH-pyridin-2-yle | 1,3-benzodioxol-5-yle | 0 | OH |
| -(CH₂)₃-R₁ | -NH-pyridin-2-yle | (6-OCH₃)-pyridin-3-yle | 2 | OH |
| -(CH₂)₃-R₁ | 5,6,7,8-tétrahydro-[1,8]naphtyridin-2-yle | 1,3-benzodioxol-5-yle | 1 | OH |
| Ph(3-R₁) | -NH-1,4,5,6-tétrahydro-5-OH-pyrimidin-2-yle | 1,3-benzodioxol-5-yle | 1 | OH |
| -(CH₂)₂-R₁ | 5,6,7,8-tétrahydro-[1,8]naphtyridin-2-yle | (6-OCH₃)-pyridin-3-yle | 1 | OH |
| -(CH₂)₃-R₁ | 5,6,7,8-tétrahydro-[1,8]naphtyridin-2-yle | quinoléin-3-yle | 1 | OH |
| -(CH₂)₂-R₁ | 5,6,7,8-tétrahydro-[1,8]naphtyridin-2-yle | (3-F)phényle | 1 | OH |
| -(CH₂)₃-R₁ | 5,6,7,8-tétrahydro-[1,8]naphtyridin-2-yle | (3-F) phényle | 1 | OH |
| -(CH₂)₂-R₁ | 5,6,7,8-tétrahydro-[1,8]naphtyridin-2-yle | quinoléin-3-yle | 1 | OH |
| -(CH₂)₂-R₁ | 5,6,7,8-tétrahydro-[1,8]naphtyridin-2-yle | (4-F)phényle | 1 | OH |
| -(CH₂)₃-R₁ | 5,6,7,8-tétrahydro-[1,8]naphtyridin-2-yle | (4-F)phényle | 1 | OH |
| -(CH₂)₂-R₁ | 5,6,7,8-tétrahydro-[1,8]naphtyridin-2-yle | (2-CH₃)pyrimidin-5-yle | 1 | OH |
| -(CH₂)₂-R₁ | 5,6,7,8-tétrahydro-[1,8]naphtyridin-2-yle | 2,3-dihydro-benzofuran-6-yle | 1 | OH |
| -(CH₂)₂-R₁ | 5,6,7,8-tétrahydro-[1,8]naphtyridin-2-yle | (3,5-difluoro)-phényle | 1 | OH |
| -(CH₂)₃-R₁ | 5,6,7,8-tétrahydro-[1,8]naphtyridin-2-yle | (3,5-difluoro)-phényle | 1 | OH |
| -(CH₂)₂-R₁ | 5,6,7,8-tétrahydro-[1,8]naphtyridin-2-yle | (3-CF₃)-phényle | 1 | OH |
| -(CH₂)₂-R₁ | 5,6,7,8-tétrahydro-[1,8]naphtyridin-2-yle | (4-OCF₃)-phényle | 1 | OH |
| -(CH₂)₂-R₁ | 5,6,7,8-tétrahydro-[1,8]naphtyridin-2-yle | (3-F-4-Ph)-phényle | 1 | OH |
| -(CH₂)₂-R₁ | 5,6,7,8-tétrahydro-[1,8]naphtyridin-2-yle | (3-F-4-OCH₃)-phényle | 1 | OH |
| -(CH₂)₂-R₁ | 5,6,7,8-tétrahydro-[1,8]naphtyridin-2-yle | (4-OPh)-phényle | 1 | OH |
| -(CH₂)₂-R₁ | 5,6,7,8-tétrahydro-[1,8]naphtyridin-2-yle | isoquinoléin-4-yle | 1 | OH |
| - (CH₂)₂-R₁ | 5,6,7,8-tétrahydro-[1,8]naphtyridin-2-yle | pyridin-3-yle | 1 | OH |
| -(CH₂)₂-R₁ | 5,6,7,8-tétrahydro-[1,8]naphtyridin-2-yle | dihydrobenzofuran-5-yle | 1 | OH |
| -(CH₂)₂-R₁ | 5,6,7,8-tétrahydro-[1,8]naphtyridin-2-yle | (2,4-O-CH₃)-pyrimidin-5-yle | 1 | OH |
| -(CH₂)₂-R₁ | 5,6,7,8-tétrahydro-[1,8]naphtyridin-2-yle | (2-OCH₃)-pyrimidin-5-yle | 1 | OH |
| Ph(3-R₁) | -NH-1,4,5,6-tétrahydro-5-OH-pyridin-2-yle | quinoléin-3-yle | 2 | OH |
| Ph(3-R₁) | -NH-1,4,5,6-tétrahydro-5-OH-pyrimidin-2-yle | quinoléin-3-yle | 2 | OH |
| -(CH₂)₂-R₁ | 5,6,7,8-tétrahydro-[1,8]naphtyridin-2-yle | quinoléin-3-yle | 2 | OH |
| Ph(3-R₁) | -NH-1,4,5,6-tétrahydro-5-pyridin-2-yle | 1,3-benzodioxol-5-yle | 2 | OH |
| Ph(3-R₁) | -NH-1,4,5,6-tétrahydro-5-pyrimidin-2-yle | 1,3-benzodioxol-5-yle | 2 | OH |
| Ph(3-R₁) | -NH-1,4,5,6-tétrahydro-5-OH-pyrimidin-2-yle | 1,3-benzodioxol-5-yle | 2 | OH |
| -CH₂-R₁ | 5,6,7,8-tétrahydro-[1,8]naphtyridin-2-yle | 1,3-benzodioxol-5-yle | 2 | OH |
| -(CH₂)₂-R₁ | 5,6,7,8-tétrahydro-[1,8]naphtyridin-2-yle | naphtalène-2-yle | 1 | OH |
| -(CH₂)₂-R₁ | 5,6,7,8-tétrahydro-[1,8]naphtyridin-2-yle | 5,6,7,8-tétrahydro-quinoléin-3-yle | 1 | OH |
| -(CH₂)₃-R₁ | 5,6,7,8-tétrahydro-[1,8]naphtyridin-2-yle | 5,6,7,8-tétrahydro-quinoléin-3-yle | 0 | OH |
| -(CH₂)₂-R | 5,6,7,8-tétrahydro-[1,8]naphtyridin-2-yle | (3-OCH₃)phényle | 1 | OH |
| -(CH₂)₂-R₁ | 5,6,7,8-tétrahydro-[1,8]naphtyridin-2-yle | (4-OCH₃)phényle | 1 | OH |
| -(CH₂)₂-R₁ | 5,6,7,8-tétrahydro-[1,8]naphtyridin-2-yle | H | 1 | OH |
| -(CH₂)₂-R₁ | 5,6,7,8-tétrahydro-[1,8]naphtyridin-2-yle | tétrahydrofuran-3-yle | 1 | OH |
| -(CH₂)₂-R₁ | 5,6,7,8-tétrahydro-[1,8]naphtyridin-2-yle | thiophén-2-yle | 1 | OH |
| -(CH₂)₂-R₁ | 5,6,7,8-tétrahydro-[1,8]naphtyridin-2-yle | (3-F)phényle | 1 | NH₂ |
| -(CH₂)₂-R₁ | 5,6,7,8-tétrahydro-[1,8]naphtyridin-2-yle | 2,3-dihydrobenzo[1,4]-dioxin-6-yle | 1 | OH |
| -(CH₂)₂-R₁ | 5,6,7,8-tétrahydro-[1,8]naphtyridin-2-yle | (3-SCH₃)phényle | 1 | OH |
| -(CH₂)₂-R₁ | 5,6,7,8-tétrahydro-[1,8]naphtyridin-2-yle | N-méthyl-1,2,3,4-tétrahydro-quinoléin-3-yle | 1 | OH |
| -(CH₂)₂-R₁ | 5,6,7,8-tétrahydro-[1,8]naphtyridin-2-yle | H | 1 | -O-éthyle |
| -(CH₂)₂-R₁ | 5,6,7,8-tétrahydro-[1,8]naphtyridin-2-yle | H | 1 | -O-propyle |
| -(CH₂)₂-R₁ | 5,6,7,8-tétrahydro-[1,8]naphtyridin-2-yle | H | 1 | -O-t-butyle |
| -(CH₂)₂-R₁ | 5,6,7,8-tétrahydro-[1,8]naphtyridin-2-yle | H | 1 | -O-n-octyle |
| -(CH₂)₂-R₁ | 5,6,7,8-tétrahydro-[1,8]naphtyridin-2-yle | H | 1 | -O-s-butyle |
| -(CH₂)₂-R₁ | 5,6,7,8-tétrahydro-[1,8]naphtyridin-2-yle | H | 1 | -O-méthyle |
| -(CH₂)₂-R₁ | 5,6,7,8-tétrahydro-[1,8]naphtyridin-2-yle | H | 1 | -O-CH₂-OC(O)-t-butyle |
| -(CH₂)₂-R₁ | 5,6,7,8-tétrahydro-[1_{,}8]naphtyridin-2-yle | (3-(NMe₂)phényle | 1 | OH |
| -(CH₂)₂-R₁ | 5,6,7,8-tétrahydro-[1,8]naphtyridin-2-yle | (3-OMe-4-OH)phényle | 1 | OH |
| Ph(3-R₁) | -NH-4,5-dihydro-1H-imidazol-2-yle | (3-F)phényle | 1 | OH |
| -(CH₂)₂-R₁ | 5,6,7,8-tétrahydro-[1,8]naphtyridin-2-yle | (3-NHEt)phényle | 1 | OH |
| -(CH₂)₂-R₁ | 5,6,7,8-tétrahydro-[1,8]naphtyridin-2-yle | (3-NHMe)phényle | 1 | OH |
| -(CH₂)₃-R₁ | 5,6,7,8-tétrahydro-[1,8]naphtyridin-2-yle | dihydrobenzofuran-6-yle | 0 | OH |
et sels, mélanges racémiques et énantiomères pharmaceutiquement acceptables de celui-ci.

4. Composition comprenant un composé selon la revendication 1 ou la revendication 3, dans laquelle le composé est choisi dans le groupe constitué par :
un composé de formule (I) dans laquelle W est -CH₂-Ph(3-R₁) ; R₁ est -NH-1,4,5,6-tétrahydro-pyrimidin-2-yle ; R₂ est H, q vaut 0 et Z est OH ;
un composé de formule (I) dans laquelle W est -(CH₂)₂-Ph(3-R₁) ; R₁ est -NH-1, 4, 5, 6-tétrahydro-pyrimidin-2-yle ; R₂ est H, q vaut 0 et Z est OH ;
un composé de formule (I) dans laquelle W est -CH₂-Ph(3-R₁) ; R₁ est -NH-1,4,5,6-tétrahydro-5-OH-pyrimidin-2-yle ; R₂ est -3-quinoléinyle, q vaut 0 et Z est OH ;
un composé de formule (I) dans laquelle W est -(CH₂)₃-R₁ ; R₁ est -5,6,7,8-tétrahydro-1,8-naphtyridin-2-yle ; R₂ est -3-quinoléinyle, q vaut 0 et Z est OH ;
un composé de formule (I) dans laquelle W est - (CH₂)₃-R₁ ; R₁ est -5, 6, 7, 8-tétrahydro-1,8-naphtyridin-2-yle ; R₂ est -1,2,3,4-tétrahydro-3-quinoléinyle, q vaut 0 et Z est OH ;
un composé de formule (I) dans laquelle W est -Ph(3-R₁) ; R₁ est
-NH-1,4,5,6-tétrahydro-pyrimidin-2-yle ; R₂ est -3-pyridinyle, q vaut 2 et Z est OH ;
un composé de formule (I) dans laquelle W est -Ph(3-R₁) ; R₁ est
-NH-1,4,5,6-tétrahydro-5-OH-pyrimidin-2-yle ; R₂ est -3-pyridinyle, q vaut 2 et Z est OH ;
un composé de formule (I) dans laquelle W est -(CH₂)₂-R₁ ; R₁ est
-5,6,7,8-tétrahydro-1,8-naphtyridin-2-yle ; R₂ est -3-pyridinyle, q vaut 2 et Z est OH ;
un composé de formule (I) dans laquelle W est - (CH₂) ₃-R₁ ; R₁ est -NH-pyridin-2-yle ; R₂ est -3-pyridinyle, q vaut 2 et Z est OH ;
un composé de formule (I) dans laquelle W est -Ph(3-R₁); R₁ est
-NH-1,4,5,6-tétrahydro-5-OH-pyrimidin-2-yle ; R₂ est -(6-MeO)pyridin-3-yle, q vaut 2 et Z est OH ;
un composé de formule (I) dans laquelle W est -(CH₂)₂-R₁; R₁ est -5,6,7,8-tétrahydro-1,8-naphtyridin-2-yle ; R₂ est -1,3-benzodioxol-5-yle, q vaut 1 et Z est OH ;
un composé de formule (I) dans laquelle W est -Ph(3-R₁) ; R₁ est -NH-1,4,5,6-tétrahydro-pyrimidin-2-yle ; R₂ est -3-quinoléinyle, q vaut 2 et Z est OH ;
un composé de formule (I) dans laquelle W est - (CH₂) ₂-R₁ ; R₁ est -5, 6, 7, 8-tétrahydro-1,8-naphtyridin-2-yle ; R₂ est -Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -1,3-benzodioxol-5-yle, q vaut 0 et Z est OH ;
un composé de formule (I) dans laquelle W est -(CH₂)₃-R₁ ; R₁ est -5,6,7,8-tétrahydro-1,8-naphtyridin-2-yle ; R₂ est -1,3-benzodioxol-5-yle, q vaut 0 et Z est OH ;
un composé de formule (I) dans laquelle W est -CH₂-R₁ ; R₁ est -5,6,7,8-tétrahydro-1,8-naphtyridin-2-yle ; R₂ est -1,3-benzodioxol-5-yle, q vaut 0 et Z est OH ;
un composé de formule (I) dans laquelle W est - (CH₂) ₃-R₁ ; R₁ est -5,6,7,8-tétrahydro-1,8-naphtyridin-2-yle ; R₂ est -(6-MeO)pyridin-3-yle, q vaut 0 et Z est OH ;
un composé de formule (I) dans laquelle W est - (CH₂) ₂-R₁ ; R₁ est -5,6,7,8-tétrahydro-1,8-naphtyridin-2-yle ; R₂ est -1,4,5,6-tétrahydro-2-Me-pyrimidin-5-yle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -1,4,5,6-tétrahydro-3-quinoléinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -1,3-benzodioxol-5-yle, q vaut 2 et Z est OH ;
un composé de formule (I) dans laquelle W est -(CH₂)₂-R₁ ; R₁ est -5,6,7,8-tétrahydro-1,8-naphtyridin-2-yle ; R₂ est -(6-MeO)pyridin-3-yle, q vaut 2 et Z est OH ;
un composé de formule (I) dans laquelle W est -(CH₂)₃-R₁ ; R₁ est -NH-pyridin-2-yle ; R₂ est 3-quinoléinyle, q vaut 2 et Z est OH ;
un composé de formule (I) dans laquelle W est -(CH₂)₃-R₁ ; R₁ est -NH-pyridin-2-yle ; R₂ est -1,3-benzodioxol-5-yle, q vaut 2 et Z est OH ;
un composé de formule (I) dans laquelle W est -(CH₂)₃-R₁ ; R₁ est -NH-pyridin-2-yle ; R₂ est -1,3-benzodioxol-5-yle, q vaut 0 et Z est OH ;
un composé de formule (I) dans laquelle W est -(CH₂)₃-R₁ ; R₁ est -NH-pyridin-2-yle ; R₂ est -(6-MeO)pyridin-3-yle, q vaut 2 et Z est OH ;
un composé de formule (I) dans laquelle W est -(CH₂)₃-R₁ ; R₁ est -5,6,7,8-tétrahydro-1,8-naphtyridin-2-yle ; R₂ est -1,3-benzodioxol-5-yle, q vaut 1 et Z est OH ;
un composé de formule (I) dans laquelle W est -Ph(3-R₁); R₁ est -NH-1,4,5,6-tétrahydro-5-OH-2-pyrimidinyle ; R₂ est -1,3-benzodioxol-5-yle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(6-MeO)pyridin-3-yle, q vaut 1 et Z est OH ;
un composé de formule (I) dans laquelle W est -(CH₂)₃-R₁ ; R₁ est -5,6,7,8-tétrahydro-1,8-naphtyridin-2-yle ; R₂ est -3-quinoléinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(3-F)Ph, q vaut 1 et Z est OH ;
un composé de formule (I) dans laquelle W est -(CH₂)₃-R₁ ; R₁ est -5,6,7,8-tétrahydro-1,8-naphtyridin-2-yle ; R₂ est - (3-F) Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -3-quinoléinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(4-F) Ph, q vaut 1 et Z est OH ;
un composé de formule (I) dans laquelle W est - (CH₂)₃-R₁ ; R₁ est -5,6,7,8-tétrahydro-1,8-naphtyridin-2-yle ; R₂ est -(4-F) Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(2-Me)pyrimidin-5-yle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -2,3-dihydro-benzofuran-6-yle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(3,5-F₂) Ph, q vaut 1 et Z est OH ;
un composé de formule (I) dans laquelle W est -(CH₂)₃-R₁ ; R₁ est -5,6,7,8-tétrahydro-1,8-naphtyridin-2-yle ; R₂ est -(3,5-F₂)Ph, q vaut 1 et Z est OH ;
un composé de formule (I) dans laquelle W est - (CH₂)₂-R₁ ; R₁ est -5,6,7,8-tétrahydro-1,8-naphtyridin-2-yle ; R₂ est -(3-CF₃)Ph, q vaut 1 et Z est OH ;
un composé de formule (I) dans laquelle W est - (CH₂) ₂-R₁ ; R₁ est -5,6,7,8-tétrahydro-1,8-naphtyridin-2-yle ; R₂ est -(4-OCF₃) Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(3-F-4-Ph)Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(3-F-4-OMe)Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(4-OPh)Ph, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -4-isoquinoléinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -3-pyridinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -5-dihydrobenzofuranyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -2,4-(OMe)₂-pyrimid-5-yle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(2-Ome)pyrimidin-5-yle, q vaut 1 et Z est OH ;
un composé de formule (I) dans laquelle W est -Ph(3-R₁) ; R₁ est -NH-1,4,5,6-tétrahydro-5-OH-pyrimidin-2-yle ; R₂ est -3-quinoléinyle, q vaut 2 et Z est OH ;
un composé de formule (I) dans laquelle W est -Ph(3-R₁) ; R₁ est -NH-3,4,5,6-tétrahydro-pyridin-2-yle ; R₂ est -3-quinoléinyle, q vaut 2 et Z est OH ;
un composé de formule (I) dans laquelle W est - (CH₂) ₂-R₁ ; R₁ est -5,6,7,8-tétrahydro-1,8-naphtyridin-2-yle ; R₂ est -3-quinoléinyle, q vaut 2 et Z est OH ;
un composé de formule (I) dans laquelle W est -Ph(3-R₁) ; R₁ est -NH-3,4,5,6-tétrahydro-pyrimidin-2-yle ; R₂ est -1,3-benzodioxol-5-yle, q vaut 2 et Z est OH ;
un composé de formule (I) dans laquelle W est -Ph(3-R₁) ; R₁ est -NH-3,4,5,6-tétrahydro-pyridin-2-yle ; R₂ est -1,3-benzodioxol-5-yle, q vaut 2 et Z est OH ;
un composé de formule (I) dans laquelle W est -Ph(3-R₁) ; R₁ est -NH-1,4,5,6-tétrahydro-5-OH-pyrimidin-2-yle ; R₂ est -1,3-benzodioxol-5-yle, q vaut 2 et Z est OH ;
un composé de formule (I) dans laquelle W est -(CH₂)-R₁ ; R₁ est -5,6,7,8-tétrahydro-1,8-naphtyridin-2-yle ; R₂ est -1,3-benzodioxol-5-yle, q vaut 2 et Z est OH ; et
un composé de formule (I) dans laquelle W est -(CH₂)₂-R₁ ; R₁ est -5,6,7,8-tétrahydro-1,8-naphtyridin-2-yle ; R₂ est -2-naphtalényle, q vaut 1 et Z est OH.

5. Composition selon la revendication 4 dans laquelle le composé est choisi dans le groupe constitué par
un composé de formule (I) dans laquelle W est -(CH₂)₃-R₁ ; R₁ est -5,6,7,8-tétrahydro-1,8-naphtyridin-2-yle ; R₂ est -1,2,3,4-tétrahydro-3-quinoléinyle, q vaut 0 et Z est OH ;
un composé de formule (I) dans laquelle W est -(CH₂)₃-R₁ ; R₁ est -5,6,7,8-tétrahydro-1,8-naphtyridin-2-yle ; R₂ est -1,3-benzodioxol-5-yle, q vaut 0 et Z est OH ;
un composé de formule (I) dans laquelle W est -(CH₂)₂-R₁ ; R₁ est -5,6,7,8-tétrahydro-1,8-naphtyridin-2-yle ; R₂ est -1,2,3,4-tétrahydro-3-quinoléinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -(6-MeO)pyridin-3-yle, q vaut 1 et Z est OH ;
un composé de formule (I) dans laquelle W est - (CH₂) ₂-R₁ ; R₁ est -5,6,7,8-tétrahydro-1,8-naphtyridin-2-yle ; R₂ est -(3-F) Ph, q vaut 1 et Z est OH ;
un composé de formule (I) dans laquelle W est -(CH₂)₃-R₁ ; R₁ est -5,6,7,8-tétrahydro-1,8-naphtyridin-2-yle ; R₂ est -1,3-quinoléinyle, q vaut 1 et Z est OH ;
un composé de formule (I) dans laquelle W est -(CH₂)₃-R₁ ; R₁ est -5,6,7,8-tétrahydro-1,8-naphtyridin-2-yle ; R₂ est -(2-Me)pyrimidin-5-yle, q vaut 1 et Z est OH ;
un composé de formule (I) dans laquelle W est - (CH₂) ₂-R₁ ; R₁ est -5,6,7,8-tétrahydro-1,8-naphtyridin-2-yle ; R₂ est -2,3-dihydro-benzofuran-6-yle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -4-isoquinoléinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -3,4-pyridinyle, q vaut 1 et Z est OH ;
un composé de formule (I) dans laquelle W est -(CH₂)₂-R₁ ; R₁ est -5,6,7,8-tétrahydro-1,8-naphtyridin-2-yle ; R₂ est -2,4-(OMe)₂-pyrimid-5-yle, q vaut 1 et Z est OH ; et
un composé de formule (I) dans laquelle W est -(CH₂)₂-R₁ ; R₁ est -5,6,7,8-tétrahydro-1,8-naphtyridin-2-yle ; R₂ est -(2-OMe)-pyrimidin-5-yle, q vaut 1 et Z est OH.

6. Composé selon la revendication 1, dans lequel W est -(CH₂)₃-R₁ ; R₁ est -5,6,7,8-tétrahydro-1,8-naphtyridin-2-yle ; R₂ est -1,2,3,4-tétrahydro-3-quinoléinyle, q vaut 0 et Z est OH.

7. Composé selon la revendication 1, dans lequel W est - (CH₂)₃-R₁ ; R₁ est -5,6,7,8-tétrahydro-1,8-naphtyridin-2-yle ; R₂ est -1,3-benzodioxol-5-yle, q vaut 0 et Z est OH.

8. Composé selon la revendication 1, dans lequel W est - (CH₂) ₂-R₁ ; R₁ est -5,6,7,8-tétrahydro-1,8-naphtyridin-2-yle ; R₂ est -1,2,3,4-tétrahydro-3-quinoléinyle, q vaut 1 et Z est OH.

9. Composé selon la revendication 1, dans lequel W est - (CH₂)₂-R₁ ; R₁ est -5,6,7,8-tétrahydro-1,8-naphtyridin-2-yle ; R₂ est -(6-MeO)pyridin-3-yle, q vaut 1 et Z est OH.

10. Composé selon la revendication 1, dans lequel W est -(CH₂)₂-R₁ ; R₁ est -5,6,7,8-tétrahydro-1,8-naphtyridin-2-yle ; R₂ est -(3-F)Ph, q vaut 1 et Z est OH.

11. Composé selon la revendication 1, dans lequel W est -(CH₂)₂-R₁ ; R₁ est -5,6,7,8-tétrahydro-1,8-naphtyridin-2-yle ; R₂ est -3-quinoléinyle, q vaut 1 et Z est OH.

12. Composé selon la revendication 1, dans lequel W est -(CH₂)₂-R₁ ; R₁ est -5,6,7,8-tétrahydro-1,8-naphtyridin-2-yle ; R₂ est -(2-Me)pyrimidin-5-yle, q vaut 1 et Z est OH.

13. Composé selon la revendication 1, dans lequel W est -(CH₂)₂-R₁ ; R₁ est -5,6,7,8-tétrahydro-1,8-naphtyridin-2-yle ; R₂ est -2,3-dihydro-benzofuran-6-yle, q vaut 1 et Z est OH.

14. Composé selon la revendication 3, dans lequel W est - (CH₂) ₂-R₁ ; R₁ est -5,6,7,8-tétrahydro-1,8-naphtyridin-2-yle ; R₂ est -4-isoquinoléinyle, q vaut 1 et Z est OH.

15. Composé selon la revendication 1, dans lequel W est - (CH₂) ₂-R₁ ; R₁ est -5,6,7,8-tétrahydro-1,8-naphtyridin-2-yle ; R₂ est -3-pyridinyle, q vaut 1 et Z est OH.

16. Composé selon la revendication 1, dans lequel W est - (CH₂) ₂-R₁ ; R₁ est -5,6,7,8-tétrahydro-1,8-naphtyridin-2-yle ; R₂ est -2,4(OMe)₂-pyrimid-5-yle, q vaut 1 et Z est OH.

17. Composé selon la revendication 1, dans lequel W est -(CH₂)₂-R₁ ; R₁ est -5,6,7,8-tétrahydro-1,8-naphtyridin-2-yle ; R₂ est -(2-OMe)-pyrimidin-5-yle, q vaut 1 et Z est OH.

18. Composé selon la revendication 1, dans lequel W est -(CH₂)₃-R₁ ; R₁ est -5,6,7,8-tétrahydro-1,8-naphtyridin-2-yle ; R₂ est -2,3-dihydro-benzofuran-6-yle, q vaut 0 et Z est OH.

19. Composé de formule (I) : 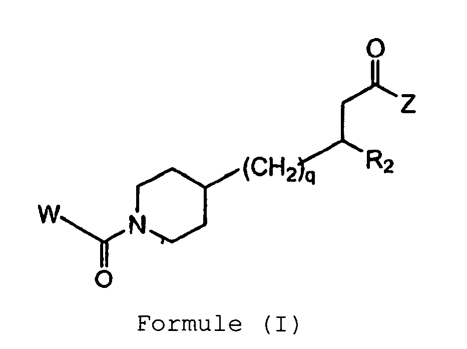 dans laquelle
W est choisi dans le groupe constitué par un groupe -alkyl en C_{0 à 4} (R₁) et -alkyl en C_{0 à 4}-phényl(R₁, R₈) ;
R₁ est -NH (R₆) ;
R₂ est choisi dans le groupe constitué par un atome d'hydrogène, un groupe -tétrahydropyrimidinyl(R₈), -1,3-benzodioxolyl(R₈), -dihydrobenzofuranyl(R₈), -tétrahydroquinoléinyl(R₈), -phényl(R₈), -naphtalényl(R₈), -pyridinyl(R₈), -pyrimidinyl(R₈) et -quinoléinyl(R₈) ;
R₆ est choisi dans le groupe constitué par un groupe -dihydroimidazolyl(R₈), -tétrahydropyridinyl(R₈), -tétrahydropyrimidinyl(R₈) et -pyridinyl (R₈) ;
R⁸ est un à quatre substituants indépendamment choisis dans le groupe constitué par un atome d'hydrogène et un groupe -alkyl en C_{1 à 4}(R₉) lorsqu' il est attaché à un atome d'azote ; et, où R₈ est un à quatre substituants indépendamment choisis dans le groupe constitué par un atome d'hydrogène, un groupe -alkyl en C_{1 à 4}(R₉), -alcoxy en C_{1 à 4}(R₉), -O-aryl(R₁₀), et hydroxy lorsqu'il est attaché à un atome de carbone ;
R₉ est choisi dans le groupe constitué par un atome d'hydrogène, un groupe -alcoxy en C_{1 à 4}, -NH₂, -NH-alkyle en C_{1 à 4}, -N (alkyle en C_{1 à 4})₂, (halogéno) _{1 à 3} et hydroxy ;
R₁₀ est indépendamment choisi dans le groupe constitué par un atome d'hydrogène, un groupe -alkyle en C_{1 à 4}, -alcoxy en C_{1 à 4}, -C (=O) H, -C (=O) -alkyle en C_{1 à 4}, -CO₂H, -CO₂-alkyle en C_{1 à 4}, -NH₂, -NH-alkyle en C_{1 à 4}, -N (alkyle en C_{1 à 4}) ₂, halogéno, hydroxy, nitro et oxo lorsqu'il est attaché à un atome de carbone ;
q vaut 1, 2 ou 3 ;
Z est choisi dans le groupe constitué par un groupe hydroxy, -NH₂, -NH-alkyle en C_{1 à 8}, -N(alkyle en C_{1 à 8})₂, O-alkyl en C_{1 à 8}-OH, -O-alkyl en C_{1 à 8}-alcoxy en C_{1 à 8}, -O-alkylcarbonyl en C_{1 à 8}-alkyle en C_{1 à 8}, -O-alkyl en C_{1 à 8}-CO₂H, -O-alkyl en C_{1 à 8}-C(O)O-alkyle en C_{1 à 8}, -O-alkyl en C_{1 à 8}-O-C(O)alkyle en C_{1 à 8}, -O-alkyl en C_{1 à 8}-NH₂, -O-alkyl en C_{1 à 8}-NH-alkyle en C_{1 à 8}, -O-alkyl en C_{1 à 8}-N(alkyle en C_{1 à 8})_{2,} -O-alkylamide en C_{1 à 8}, -O-alkyl en C_{1 à 8}-C(O)-NH-alkyle en C_{1 à 8}, -O-alkyl en C_{1 à 8}-C(O)-N (alkyle en C_{1 à 8})₂ et -NHC (O) alkyle en C_{1 à 8};
et sels, mélanges racémiques et énantiomères pharmaceutiquement acceptables de celui-ci.

20. Composé selon la revendication 1 de formule (I.2) : 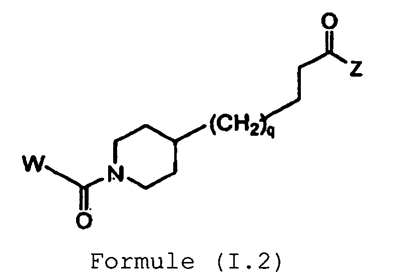 dans laquelle
W est choisi dans le groupe constitué par un groupe -alkyl en C_{0 à 4} (R₁) et -alkyl en C_{0 à 4}-phényl (R₁, R₈ ;
R₁ est choisi dans le groupe constitué par un groupe -NH(R₆), -dihydro-1H-pyrrolo[2,3-b]pyridinyl(R₈), -tétrahydropyrimidinyl(R₈), -tétrahydro-1,8-naphtyridinyl(R₈), -tétrahydro-1H-azépino[2,3-b]pyridinyl(R₈) et -pyridinyl(R₈) ;
R⁶ est choisi dans le groupe constitué par un groupe -dihydroimidazolyl(R₈), -tétrahydropyridinyl(R₈), -tétrahydropyrimidinyl(R₈) et -pyridinyl(R₈) ;
R⁸ est un à quatre substituants indépendamment choisis dans le groupe constitué par un atome d'hydrogène et un groupe -alkyl en C_{1 à 4} (R₉) lorsqu'il est attaché à un atome d'azote ; et où R₈ est un à quatre substituants indépendamment choisis dans le groupe constitué par un atome d'hydrogène, un groupe -alkyl en C_{1à 4} (R₉) , -alcoxy en C_{1 à 4} (R₉), -O-aryl(R₁₀), et hydroxy lorsqu'il est attaché à un atome de carbone ;
R₉ est choisi dans le groupe constitué par un atome d'hydrogène, un groupe -alcoxy en C_{1 à 4}, -NH₂, -NH-alkyle en C_{1 à 4}, -N (alkyle en C_{1 à 4})₂, (halogéno)_{1 à 3} et hydroxy ;
R₁₀ est un à quatre substituants indépendamment choisis dans le groupe constitué par un atome d'hydrogène, un groupe -alkyle en C_{1 à 4}, -alcoxy en C_{1 à 4}, -C(=O)H, -C(=O)-alkyle en C_{1 à 4}, -CO₂H, -CO₂-alkyle en C_{1 à 4}, -NH₂, -NH-alkyle en C_{1 à 4}, -N (alkyle en C_{1 à 4}) ₂, halogéno, hydroxy, nitro et oxo lorsqu'il est attaché à un atome de carbone ;
q vaut 1, 2 ou 3 ;
Z est choisi dans le groupe constitué par un groupe hydroxy, -NH₂, -NH-alkyle en C_{1 à 8}, -N(alkyle en C_{1 à 8})₂, O-alkyl en C_{1 à 8}-OH, -O-alkyl en C_{1 à 8}-alcoxy en C_{1 à 8}, -O-alkylcarbonyl en C_{1 à 8}-alkyle en C_{1 à 8}, -O-alkyl en C_{1 à 8}-CO₂H, -O-alkyl en C_{1 à 8}-C(O)O-alkyle en C_{1 à 8}, -O-alkyl en C_{1 à 8}-O-C(O) alkyle en C_{1 à 8}, -O-alkyl en C_{1 à 8}-NH₂, -O-alkyl en C_{1 à 8}-NH-alkyle en C_{1 à 8}, -O-alkyl en C_{1 à 8}-N (alkyle en C_{1 à 8}) ₂, -O-alkylamide en C_{1 à 8}, -O-alkyl en C_{1 à 8}-C (O) -NH-alkyle en C_{1 à 8}, -O-alkyl en C_{1 à 8}-C(O) N(alkyle en C_{1 à 8})₂ et -NHC(O)alkyle en C_{1 à 8} ;
et sels, mélanges racémiques et énantiomères pharmaceutiquement acceptables de celui-ci.

21. Composé selon la revendication 1 de formule (I.3) : 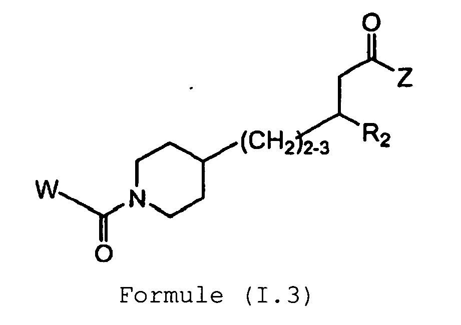 dans laquelle
W est choisi dans le groupe constitué par un groupe -alkyl en C_{0 à 4} (R₁) et -alkyl en C_{0 à 4}-phényl (R₁, R₈) ;
R₁ est choisi dans le groupe constitué par un groupe -NH(R₆), -dihydro-1H-pyrrolo[2,3-b]pyridinyl(R₈), -tétrahydropyrimidinyl(R₈), -tétrahydro-1,8-naphtyridinyl(R₈), -tétrahydro-1H-azépino[2,3-b]pyridinyl(R₈) et -pyridinyl(R₈) ;
R₂ est choisi dans le groupe constitué par un atome d'hydrogène, un groupe -tétrahydropyrimidinyl(R₈), -1,3-benzodioxolyl(R₈), -dihydrobenzofuranyl(R₈), -tétrahydroquinoléinyl(R₈), -phényl(R₈), -naphtalényl(R₈), -pyridinyl(R₈), -pyrimidinyl(R⁸) et -quinoléinyl(R₈) ;
R⁶ est un groupe -dihydroimidazolyl(R₈), -tétrahydropyridinyl(R₈), -tétrahydropyrimidinyl(R₈) ou -pyridinyl (R₈) ;
R⁸ est un à quatre substituants indépendamment choisis dans le groupe constitué par un atome d'hydrogène et un groupe -alkyl en C_{1 à 4} (R₉) lorsqu'il est attaché à un atome d'azote ; et où R₈ est un à quatre substituants indépendamment choisis dans le groupe constitué par un atome d'hydrogène, un groupe -alkyl en C_{1 à 4} (R₉) , -alcoxy en C_{1 à 4}(R₉), -O-aryl(R₁₀), et hydroxy lorsqu'il est attaché à un atome de carbone ; et
R₉ est choisi dans le groupe constitué par un atome d'hydrogène, un groupe -alcoxy en C_{1 à 4}. -NH₂, -NH-alkyle en C_{1 à 4}, -N(alkyle en C_{1 à 4}) ₂, (halogéno) _{1 à 3} et hydroxy ;
R₁₀ est un à quatre substituants indépendamment choisis dans le groupe constitué par un atome d'hydrogène, un groupe -alkyle en C_{1 à 4}, -alcoxy en C_{1 à 4}, -C (=O) H, -C(=O)-alkyle en C_{1 à 4}, -CO₂H, -CO₂-alkyle en C_{1 à 4}, -NH₂, -NH-alkyle en C_{1 à 4}, -N (alkyle en C_{1 à 4})₂, halogéno, hydroxy, nitro et oxo lorsqu'il est attaché à un atome de carbone ;
Z est choisi dans le groupe constitué par un groupe hydroxy, -NH₂, -NH-alkyle en C_{1 à 8}, -N (alkyle en C_{1 à 8})₂, O-alkyl en C_{1 à 8}-OH, -O-alkyl en C_{1 à 8}-alcoxy en C_{1 à 8}, -O-alkylcarbonyl en C_{1 à 8}-alkyle en C_{1 à 8}, -O-alkyl en C_{1 à 8}-CO₂H, -O-alkyl en C_{1 à 8}-C(O)O-alkyle en C_{1 à 8}, -O-alkyl en C_{1 à 8}-O-C(O)alkyle en C_{1 à 8}, -O-alkyl en C_{1 à 8}-NH₂, -O-alkyl en C_{1 à 8}-NH-alkyle en C_{1 à 8}, -O-alkyl en C_{1 à 8}-N (alkyle en C_{1 à 8})₂, -O-alkylamide en C_{1 à 8}, -O-alkyl en C_{1 à 8}-CO)-NH-alkyle en C_{1à 8}, -O-alkyl en C_{1 à 8}-C(O)-N (alkyle en C_{1 à 8})₂ et -NHC(O)alkyle en C_{1 à 8} **;**
et sels, mélanges racémiques et énantiomères pharmaceutiquement acceptables de celui-ci.

22. Composé selon la revendication 20 ou 21, dans lequel R₁ est choisi dans le groupe constitué par -NH(R₆), -tétrahydropyrimidinyl (R₈) et -tétrahydro-1,8-naphtyridinyl(R₈) ;
et toutes les autres variables sont telles que précédemment définies.

23. Composé de formule (I.4) : 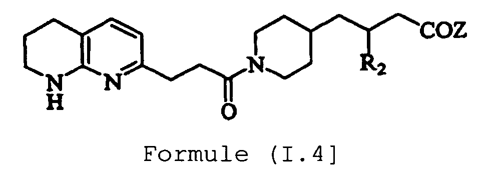 dans laquelle R₂ est choisi dans le groupe constitué par -2-benzofuranyle, -3-benzofuranyle, -4-benzofuranyle, -5-benzofuranyle, -6-benzofuranyle, -7-benzofuranyle, -benzo[b]thién-2-yle, -benzo[b]thién-3-yle, -benzo[b]thién-4-yle, -benzo[b]thién-5-yle, -benzo[b]thién-6-yle, -benzo[b]thién-7-yle, -1H-indol-2-yle, -1H-indol-3-yle, -1H-indol-4-yle, -1H-indol-5-yle, -1H-indol-6-yle, -1H-indol-7-yle, -2-benzoxazolyle, -4-benzoxazolyle, -5-benzoxazolyle, -6-benzoxazolyle, -7-benzoxazolyle, -2-benzothiazolyle, -3-benzothiazolyle, -4-benzothiazolyle, -5-benzothiazolyle, -6-benzothiazolyle, -7-benzothiazolyle, -1H-benzimidazolyl-2-yle, -1H-benzimidazolyl-4-yle, -1H-benzimidazolyl-5-yle, -1H-benzimidazolyl-6-yle, -1H-benzimidazolyl-7-yle, -2-quinoléinyle, -3-quinoléinyle, -4-quinoléinyle, -5-quinoléinyle, -6-quinoléinyle, -7-quinoléinyle, -8-quinoléinyle, -2H-1-benzopyran-2-yle, -2H-1-benzopyran-3-yle, -2H-1-benzopyran-4-yle, -2H-1-benzopyran-5-yle, -2H-1-benzopyran-6-yle, -2H-1-benzopyran-7-yle, -2H-1-benzopyran-8-yle, -4H-1-benzopyran-2-yle, -4H-1-benzopyran-3-yle, -4H-1-benzopyran-4-yle, -4H-1-benzopyran-5-yle, -4H-1-benzopyran-6-yle, -4H-1-benzopyran-7-yle, -4H-1-benzopyran-8-yle, -1H-2-benzopyran-1-yle, -1H-2-benzopyran-3-yle, -1H-2-benzopyran-3-yle, -1H-2-benzopyran-5-yle, -1H-2-benzopyran-6-yle, -1H-2-benzopyran-7-yle, -1H-2-benzopyran-8-yle, -1,2,3,4-tétrahydro-1-naphtalényle, -1,2,3,4-tétrahydro-2-naphtalényle, -1,2,3,4-tétrahydro-5-naphtalényle, 1,2,3,4-tétrahydro-6-naphtalényle, -2,3-dihydro-2-benzofuranyle, -2,3-dihydro-3-benzofuranyle, -2,3-dihydro-4-benzofuranyle, -2,3-dihydro-5-benzofuranyle, -2,3-dihydro-6-benzofuranyle, -2,3-dihydro-7-benzofuranyle, -2,3-dihydrobenzo[b]thién-2-yle, -2,3-dihydrobenzo[b]thién-3-yle, -2,3-dihydrobenzo[b]thién-4-yle, -2,3-dihydrobenzo[b]thién-5-yle, -2,3-dihydrobenzo[b]thién-6-yle, -2,3-dihydrobenzo[b]thién-7-yle, -2,3-dihydro-1H-indol-2-yle, 2,3-dihydro-1H-indol-3-yle, -2,3-dihydro-1H-indol-4-yle, -2,3-dihydro-1H-indol-5-yle, -2,3-dihydro-1H-indol-6-yle, -2,3-dihydro-1H-indol-7-yle, -2,3-dihydro-2-benzoxazolyle, -2,3-dihydro-4-benzoxazolyle, -2,3-dihydro-5-benzoxazolyle, -2,3-dihydro-6-benzoxazolyle, -2,3-dihydro-7-benzoxazolyle, -2,3-dihydro-1H-benzimidazol-2-yle, -2,3-dihydro-1H-benzimidazol-4-yle, -2,3-dihydro-1H-benzimidazol-5-yle, -2,3-dihydro-1H-benzimidazol-6-yle, -2,3-dihydro-1H-benzimidazol-7-yle, -3,4-dihydro-1(2H)-quinoléinyle, -1,2,3,4-tétrahydro-2-quinoléinyle, -1,2,3,4-tétrahydro-3-quinoléinyle, 1,2,3,4-tétrahydro-4-quinoléinyle, -1,2,3,4-tétrahydro-5-quinoléinyle, -1,2,3,4-tétrahydro-6-quinoléinyle, -1,2,3,4-tétrahydro-7-quinoléinyle, -1,2,3,4-tétrahydro-8-quinoléinyle, -3,4-dihydro-2H-1-benzopyran-2-yle, -3,4-dihydro-2H-1-benzopyran-3-yle, -3,4-dihydro-2H-1-benzopyran-4-yle, -3,4-dihydro-2H-1-benzopyran-5-yle, -3,4-dihydro-2H-1-benzopyran-6-yle, -3,4-dihydro-2H-1-benzopyran-7-yle, -3,4-dihydro-2H-1-benzopyran-8-yle, -3,4-dihydro-4H-1-benzopyran-2-yle, -3,4-dihydro-4H-1-benzopyran-3-yle, -3,4-dihydro-4H-1-benzopyran-4-yle, -3,4-dihydro-4H-1-benzopyran-5-yle, -3,4-dihydro-4H-1-benzopyran-6-yle, -3,4-dihydro-4H-1-benzopyran-7-yle, -3,4-dihydro-4H-1-benzopyran-8-yle, -3,4-dihydro-1H-2-benzopyran-2-yle, -3,4-dihydro-1H-2-benzopyran-3-yle, -3,4-dihydro-1H-2-benzopyran-4-yle, -3,4-dihydro-1H-2-benzopyran-5-yle, -3,4-dihydro-1H-2-benzopyran-6-yle, -3,4-dihydro-1H-2-benzopyran-7-yle et -3,4-dihydro-1H-2-benzopyran-8-yle
facultativement substitué si possible par des valences disponibles avec jusqu'à 7 substituants indépendamment choisis parmi un groupe méthyle lorsqu'ils sont attachés à un atome d'azote ; et indépendamment choisis parmi un groupe méthyle, méthoxy ou fluoro lorsqu'ils sont attachés à un atome de carbone ;
Z est choisi dans le groupe constitué par un groupe hydroxy, -NH₂, -NH-alkyle en C_{1 à 8}, -N(alkyle en C_{1 à 8}) ₂, O-alkyl en C_{1 à 8}-OH, -O-alkyl en C_{1 à 8}-alcoxy en C_{1 à 8}, -O-alkylcarbonyl en C_{1 à 8}-alkyle en C_{1 à 8}, -O-alkyl en C_{1 à 8}-CO₂H, -O-alkyl en C_{1 à 8}-C(O)O-alkyle en C_{1 à 8}, -O-alkyl en C_{1 à 8}-O-C(O) alkyle en C_{1 à 8}, -O-alkyl en C_{1 à 8}-NH₂, -O-alkyl en C_{1 à 8}-NH-alkyle en C_{1 à 8}, -O-alkyl en C_{1 à 8}-N(alkyle en C_{1 à 8})₂, -O-alkylamide en C_{1 à 8}, -O-alkyl en C_{1 à 8}-C(O)-NH-alkyle en C_{1 à 8}, -O-alkyl en C_{1 à 8}-C(O) - N (alkyle en C_{1 à 8})₂ et -NHC(O)alkyle en C_{1 à 8} ;
et sels, mélanges racémiques et énantiomères pharmaceutiquement acceptables de celui-ci.

24. Composition comprenant le composé selon l'une quelconque des revendications 1 à 3 ou 6 à 23 ou composé décrit dans la revendication 4 ou 5, et support pharmaceutiquement acceptable.

25. Composé selon l'une quelconque des revendications 1 à 3 ou 6 à 23 ou composé décrit dans la revendication 4 ou 5, à utiliser en médecine.

26. Composé selon l'une quelconque des revendications 1 à 3 ou 6 à 23 ou composé décrit dans la revendication 4 ou 5, à utiliser dans la prévention, le traitement ou l'amélioration d'un trouble véhiculé par l'αv intégrine, où le trouble véhiculé par l'αv intégrine est choisi dans le groupe constitué par les cancers, les pathologies associées au cancer, l'athérosclérose, les vasculopathies induites par une transplantation, la formation de la néointima, un papillome, la fibrose pulmonaire, la glomérulonéphrite, la glomérulosclérose, la dysplasie rénale multikystique congénitale, la fibrose rénale, la rétinopathie diabétique, la dégénérescence maculaire, le psoriasis, l'ostéoporose, la résorption osseuse, la polyarthrite inflammatoire, la polyarthrite rhumatoïde, la resténose et les adhérences et où le composé est administré dans une quantité thérapeutiquement efficace à un sujet qui le nécessite.

27. Composé selon la revendication 26, dans lequel la quantité thérapeutiquement efficace va d'environ 0,001 mg/kg/jour à environ 1000 mg/kg/jour.

28. Composé selon la revendication 26 ou la revendication 27, dans lequel la maladie véhiculée par des cellules exprimant pathologiquement une αv intégrine est choisie dans le groupe constitué par les cancers, les pathologies associées au cancer, la rétinopathie diabétique, la dégénérescence maculaire, l'ostéoporose, la résorption osseuse, la polyarthrite inflammatoire, la polyarthrite rhumatoïde et la resténose.
